(19) Europäisches Patentamt
European Patent Office
Office européen des brevets

(11) EP 2 119 455 A1

(12) EUROPEAN PATENT APPLICATION

(43) Date of publication:
18.11.2009 Bulletin 2009/47

(51) Int Cl.:
*A61K 47/48* (2006.01)

(21) Application number: 09169971.0

(22) Date of filing: 08.04.2005

(84) Designated Contracting States:
AT BE BG CH CY CZ DE DK EE ES FI FR GB GR HU IE IS IT LI LT LU MC NL PL PT RO SE SI SK TR

(30) Priority: 27.05.2004 US 575068 P
11.03.2005 US 661976 P
08.04.2004 EP 04008607

(62) Document number(s) of the earlier application(s) in accordance with Art. 76 EPC:
05732179.6 / 1 732 612

(71) Applicants:
- Max-Planck-Gesellschaft zur Förderung der Wissenschaften e.V. Berlin (DE)
- Technische Universität Dresden 01069 Dresden (DE)
- Jado Technologies GmbH 01307 Dresden (DE)

(72) Inventors:
- Braxmeier, Tobias 01069 Dresden (DE)
- Friedrichson, Tim 01277 Dresden (DE)
- Fröhner, Wolfgang 69190 Walldorf (DE)
- Jennings, Gary 01069 Dresden (DE)
- Munick, Michael 01277 Dresden (DE)
- Schlechtingen, Georg 01069 Dresden (DE)
- Schroeder, Cornelia 13088 Berlin (DE)
- Knölker, Hans-Joachim 01187 Dresden (DE)
- Simons, Kai 01309 Dresden (DE)
- Zerial, Marino 01309 Dresden (DE)
- Kurzchalia, Teymuras 01309 Dresden (DE)

(74) Representative: Heunemann, Dieter
Vossius & Partner
Siebertstrasse 4
81675 München (DE)

Remarks:
This application was filed on 10-09-2009 as a divisional application to the application mentioned under INID code 62.

# (54) Tripartite raftophilic structures and their use

(57) The present invention relates to a compound comprising a tripartite structure in the format C-B-A or C'-B'-A' wherein moiety A and moiety A' is a raftophile, moiety B and moiety B' is a linker, moiety C and moiety C' is a pharmacophore; and wherein moiety B and B' is a linker which has a backbone of at least 8 carbon atoms and wherein one or more of said carbon atoms may be replaced by nitrogen, oxygen or sulfur. Furthermore, specific medical and pharmaceutical uses of the compounds of the invention are disclosed.

EP 2 119 455 A1

SEQUENCE LISTING

<110> JadoLabs GmbH

Max-Planck-Gesellschaft zur Förderung der Wissenschaften e.V.

Technische Universität Dresden

<120> Tripartite raftophilic structures and their use

<130> G1331 EP/1 S3

<160> 8

<170> PatentIn version 3.1

<210> 1

<211> 3

<212> PRT

<213> artificial sequence

<220>

<221> source
<223> /note="Description of artificial sequence: part of inhibitor"

<400> 1

Glu Val Asn
1

<210> 2

<211> 4

<212> PRT

<213> artificial sequence

<220>
<221> source
<223> /note="Description of artificial sequence: part of inhibitor"

<400> 2

Val Ala Glu Phe
1

<210> 3

<211> 24

<212> PRT

<213> artificial sequence

<220>
<221> source
<223> /note="Description of artificial sequence: linker"

<400> 3

```
Arg His Asp Ser Gly Tyr Glu Val His His Gln Lys Leu Val Phe Phe
1               5                   10                  15

Ala Glu Asp Val Gly Ser Asn Lys
            20
```

<210> 4

<211> 3

<212> PRT

<213> artificial sequence

<220>

<221> source
<223> /note="Description of artificial sequence: part of inhibitor"

<400> 4

Glu Val Asn
1

<210> 5

<211> 28

<212> PRT

<213> ARTIFICIAL SEQUENCE

<220>
<221> source
<223> /note="Description of artificial sequence: part of inhibitor plus linker"

<400> 5

```
Val Ala Glu Phe Arg His Asp Ser Gly Tyr Glu Val His His Gln Lys
1               5                   10                  15

Leu Val Phe Phe Ala Glu Asp Val Gly Ser Asn Lys
            20                  25
```

<210> 6

<211> 4

<212> PRT

<213> artificial sequence

<220>
<221> source
<223> /note="Description of artificial sequence: part of inhibitor"

<400> 6

```
Gly Glu Val Asn
1
```

<210> 7

<211> 28

<212> PRT

<213> artificial sequence

<220>
<221> source
<223> /note="Description of artificial sequence: part of inhibitor plus Linker"

<400> 7

```
Val Ala Glu Phe Arg His Asp Ser Gly Tyr Glu Val His His Gln Lys
1               5                   10                  15
```

```
Leu Val Phe Phe Ala Glu Asp Val Gly Ser Asn Lys
            20                  25

<210>  8

<211>  36

<212>  PRT

<213>  artificial sequence


<220>
<221>  source
<223>  /note="Description of artificial sequence: inhibitor"

<400>  8

Tyr Thr Ser Leu Ile His Ser Leu Ile Glu Glu Ser Gln Asn Gln Gln
1               5                   10                  15

Glu Lys Asn Glu Gln Glu Leu Leu Glu Leu Asp Lys Trp Ala Ser Leu
            20                  25                  30

Trp Asn Trp Phe
        35
```

## Description

The present invention relates to a compound comprising a tripartite structure in the format C-B-A or C'-B'-A' wherein moiety A and moiety A' is a raftophile, moiety B and moiety B' is a linker, moiety C and moiety C' is a pharmacophore; and wherein moiety B and B' is a linker which has a backbone of at least 8 carbon atoms and one or more of said carbon atoms may be replaced by nitrogen, oxygen or sulfur. Furthermore, specific medical and pharmaceutical uses of the compounds of the invention are disclosed.

The lipid bilayer that forms cell membranes is a two dimensional liquid the organization of which has been the object of intensive investigations for decades by biochemists and biophysicists. Although the bulk of the bilayer has been considered to be a homogeneous fluid, there have been repeated attempts to introduce lateral heterogeneities, lipid microdomains, into our model for the structure and dynamics of the bilayer liquid (Glaser, Curr. Opin. Struct. Biol. 3 (1993), 475-481; Jacobson, Comments Mol. Cell Biophys. 8 (1992), 1-144; Jain, Adv. Lipid Res. 15 (1977), 1-60; Vaz, Curr. Opin. Struct. Biol. 3 (1993)).

The realization that epithelial cells polarize their cell surfaces into apical and basolateral domains with different protein and lipid compositions in each of these domains, initiated a new development that led to the "lipid raft" concept (Simons, Biochemistry 27 (1988), 6197-6202; Simons, Nature 387 (1997), 569-572). The concept of assemblies of sphingolipids and cholesterol functioning as platforms for membrane proteins was promoted by the observation that these assemblies survived detergent extraction, and are referred to as detergent resistant membranes, DRM (Brown, Cell 68 (1992), 533-544). This was an operational break-through where raft-association was equated with resistance to Triton-X100 extraction at 4˚C. The addition of a second criterion, depletion of cholesterol using methyl-β-cyclodextrin (Ilangumaran, Biochem. J. 335 (1998), 433-440; Scheiffele, Embo J. 16 (1997), 5501-5508), leading to loss of detergent resistance, prompted several groups in the field to explore the role of lipid microdomains in a wide spectrum of biological reactions. There is now increasing support for a role of lipid assemblies in regulating numerous cellular processes including cell polarity, protein trafficking and signal transduction.

Cell membranes are two-dimensional liquids. Thus, lateral heterogeneity implies liquid-liquid immiscibility in the membrane plane. It has been well known that hydrated lipid bilayers undergo phase transitions as a function of temperature. These transitions, which occur at defined temperatures for each lipid species, always involve some change in the order of the system. The most important of these transitions is the so-called "main" or "chain-melting" transition in which the bilayer is transformed from a highly ordered quasi-two dimensional crystalline solid to a quasi-two dimensional liquid. It involves a drastic change in the order of the systems, in particular of the translational (positional) order in the bilayer plane and of the conformational order of the lipid chains in a direction perpendicular to this plane. Translational order is related to the lateral diffusion coefficient in the plane of the membrane and conformational order is related to the *trans*/*gauche* ratio in the acyl chains. The main transition has been described as an ordered-to-disordered phase transition, so that the two phases may be labeled as solid-ordered ($s_o$) below the transition temperature and liquid-disordered ($l_d$) above that temperature. Cholesterol and phospholipids are capable of forming a liquid-ordered ($l_o$)) phase that can coexist with a cholesterol-poor liquid-disordered *($l_d$)* phase thereby permitting phase coexistence in wholly liquid phase membranes (Ipsen, Biochem. Biophys. Acta 905 (1987) 162-172; Ipsen, Biophys. J. 56 (1989), 661-667). Sterols do so as a result of their flat and rigid molecular structure, which is able to impose a conformational ordering upon a neighboring aliphatic chain (Sankaram, Biochemistry 29 (1990), 10676-10684), when the sterol is the nearest neighbor of the chain, without imposing a corresponding drastic reduction of the translational mobility of the lipid (Nielsen, Phys. Rev. E. Stat. Phys. Plasmas Fluids Relat. Interdiscip. Topics 59 (1999), 5790-5803). Due to the fact that the sterol does not fit exactly in the crystalline lattice of an $s_o$ (gel) lipid bilayer phase it will, if it dissolves within this phase, disrupt the crystalline translational order without significantly perturbing the conformational order. Thus, cholesterol at adequate molar fractions can convert $l_d$ or $s_o$ lipid bilayer phases to liquid-ordered ($l_o$) phases.

Rafts are lipid platforms of a special chemical composition (rich in sphingomyelin and cholesterol in the outer leaflet of the cell membrane) that function to segregate membrane components within the cell membrane. Rafts are understood to be relatively small (30-50 mn in diameter, estimates of size varying considerably depending on the probes used and cell types analysed) but they can be coalesced under certain conditions. Their specificity with regard to lipid composition is reminiscent of phase separation behavior in heterogeneous model membrane systems. In fact, many of their properties with regard to chemical composition and detergent solubility are similar to what is observed in model systems composed of ternary mixtures of an unsaturated phosphatidylcholine, sphingomyelin (or a long-chain saturated phosphatidylcholine), and cholesterol (de Almeida, Biophys. J. 85 (2003), 2406-2416). Rafts could be considered domains of a $l_o$ phase in a heterogeneous l phase lipid bilayer composing the plasma membrane. What the other coexisting phase (or phases) is (or are) is not clear at present. There is consensus that the biological membrane is a liquid, so $s_o$ phase coexistence may be ignored for most cases. Whether the other phase (phases) is (are) $l_d$ or $l_o$ phases will depend upon the chemical identity of the phospholipids that constitute this phase (these phases) and the molar fraction of cholesterol in them. Rafts may be equated with a liquid-ordered phase and refer to the rest of the membrane as the non-raft liquid phase. Within the framework of thermodynamics, a phase is always a macroscopic system consisting of large number

of molecules. However, in lipid bilayers the phases often tend to be fragmented into small domains (often only a few thousand molecules) each of which, per se, may not have a sufficient number of molecules to strictly satisfy the thermodynamic definition of a phase. In the absence of a better description for this sort of mesoscopic states and assuming that there are a large number of domains in a given system, the domains may be treated as if they were a part of a macroscopic phase so that the same properties are attributed to the domains that would describe the phase. This definition is probably adequate as long as the domains do not get too small. The liquid-ordered raft phase thus comprises all the domains (small or clustered) of the raft phase in the membranes. The rest of the membrane surrounding the rafts, the liquid phase, may be a homogeneous percolating liquid phase or may be further subdivided into liquid domains not yet characterized.

The prior art has speculated that some pharmaceuticals may be active on biological membranes like cell membranes or viral envelopes. For example, it was postulated that the anti-HIV agent cosalane acts by inhibition of binding of gp120 to CD4 as well as by inhibition of post-attachment event prior to reverse transcription; Cushman, J. Chem. 37 (1994), 3040. The cholestane moiety of cosalane is speculated to imbed into the lipid bilayer and Golebiewski, Bioorg. & Med. Chemistry 4 (1996), 1637 has speculated that the incorporation of a phosphate group into the linker chain of cosalane makes the resulting phosphodiester resemble the structure of a polylipid.

In Ruell, J. Org. Chem. 64 (1999), 5858 the work on cosalane compounds was extended. In particular, a cosalane pharmacophore analog is presented having short amide and methylene linkers attached to its terminal substituted benzoic acid rings instead of the originally proposed benzylic ether linkages. This work demonstrates that the proposed cosalane-type compound is accessible by routes that do not utilize cosalane itself as an intermediate or starting material and an in vitro effect on inhibition of the cytopathic effect of HIV-1 was shown. In Casimiro-Garcia, J. Bioorg. Med. Biochem. 8 (2000), 191 cosalane analogs are proposed where an amido group or an amino moiety was introduced into the alkenyl-linker chain of cosalane. Again, the cosalane analogs inhibited in vitro the cytopathic effect of HIV-1 and HIV-2. Further cosalane analogs are known from US 5,439,899, US 6,562,805 and US 2003/0212045. All these cosalane compounds/analogs comprise modifications in their linker structure. Yet, in particular the pharmacological part of cosalane was modified in this work. These modifications were made in an attempt to increase effectiveness of membrane integration, yet potency was reduced in every case. Again, all the known cosalane and cosalane analogs comprise a structure, which is incapable of discriminating between different biological membranes and/or partitioning in(to) different membranes. In 2001, Hussey Organic Letters 4, 415-418 the synthesis of a chimeric estradiol derivative linked to cholesterol and cholesterylamine, designed for the delivery of estradiol into cells by internalization was described. Similarly, Hussey, J. Am. Chem. Soc. 123 (2001), 1271-1273 has proposed a synthetic streptavidin protein conjugate for the intracellular delivery of macromolecules into mammalian cells. In Hussey, J. Am. Chem. Soc. 124 (2002), 6265-6273 a further synthetic molecule is described that enables cell uptake of streptavidin by non-covalent interactions with cholesterol and sphingolipid and lipid rafts are discussed. The corresponding compound comprises a derivative of cholesterylamine linked to D-biotin through an 11-atom tether. In Martin, Bioconjugate Chem. 14 (2003), 67-74, non-natural cell surface receptors are proposed which comprise peptides capped with cholesterylglycine. The ligand for these "non-natural receptors" is supposed to bind non-covalently to the peptide moiety and the proposed ligand comprising anti-HA, anti-Flag or streptavidin. Again, the non-natural cell surface receptors are proposed as a delivery strategy for macromolecular uptakes into cells.

A problem underlying the present invention was the provision of compounds and methods for medical/pharmaceutical intervention in disorders which are due to or linked to biochemical interactions or processes that take place on sphingolipid/cholesterol microdomains of and in mammalian cells.

The solution of this technical problem is achieved by providing the embodiments characterized in the claims.

Therefore, the present invention provides for a compound comprising a tripartite structure

C-B-A or C'-B'-A'

wherein moiety A and A' is a raftophile;
wherein moiety B and B' is a linker;
wherein moiety C and C' is a pharmacophore;
wherein the raftophilicity of moiety A and moiety A' comprises a partitioning into lipid membranes which are characterized by insolubility in non-ionic detergent at 4°C, and
wherein moiety B and B' is a linker which has a backbone of at least 8 carbon atoms and wherein one or more of said carbon atoms may be replaced by nitrogen, oxygen or sulfur.

The term "a tripartite structure" relates to compounds which comprise, covalently linked, a raftophile, a linker and a pharmacophore, whereby the individual moieties of said tripartite structure are denoted herein as "moiety A and A' " for a raftophile, "moiety B and B' " for a linker and "moiety C and C' " for a given pharmacophore. Yet, it is of note that the "tripartite structure" of the inventive compound may also comprise further structural or functional moieties. These

comprise, but are not limited to labels (like, e.g. radioactive labels, fluorescence labels, purification tags, etc.) which are attached to the N- or C-terminal end of the inventive construct or which may be linked, for example via side-chains, to the linker "C" and "C' ". Further functional or structural domains of the inventive construct comprise non-covalent cross-linking functions, such as charged groups, polar groups able to accept or donate hydrogen-bonding, amphiphilic groups able to mediate between lipophilic and hydrophilic compartments, groups able to interact with each other in order to thermodynamically support the enrichment of the inventive construct in lipid rafts. Additional functional or structural domains are preferably not directly attached to the pharmacophore part. Most preferably, said additional domains or moieties are in contact either direct or indirectly with the linker B/B'.

The term "raftophile" relates to a compound capable of interacting with membrane rafts. Rafts are known in the art, see, inter alia, Simons, (1988), loc. cit. or Danielson, Biochem. Biophys. 1617 (2003), 1-9. A "raftophile" comprises not only natural compounds but also synthetic compounds, as detailed herein below. The "raftophiles" comprised in the inventive tripartite structure have high affinity to the liquid ordered ($l_o$) (herein equated to rafts) phase of the membrane bilayer and spend more time in this phase compared to the liquid disordered ($l_d$) phases (herein equated to non-rafts). The partition into rafts may occur directly from the extracellular or vesicular luminal space or laterally from the bilayer. Accordingly, one of the features of "moiety A and A' " of the inventive construct relates to its capacity to be capable of partitioning into lipid membranes, preferably cellular lipid membranes, whereby said lipid membranes are characterized by insolubility in non-ionic detergents (like, e.g. 1.0% Triton X-100, 0.5% Lubrol WX or 0.5% Brij 96) at 4°C. This feature of "moiety A and A' " corresponds to the fact that a "raftophile" is capable of insertion into or interaction with sphingolipid- and cholesterol-rich microdomains on mammalian cells. Accordingly, the raft can be defined as a (non-ionic) detergent resistant membrane (DRM) structure, as defined above and taught in Simons (1988, 1997), loc. cit. and Brown (1992), loc. cit. Therefore, one possibility to verify whether a given compound (having a tripartite structure as defined herein) comprises a "moiety A" or "moiety A' " as defined herein or whether a given molecule may function as a "moiety A/A' " as defined herein is a detergent resistant membrane (DRM) test as disclosed in the prior art and as described in detail in the experimental part. In summary, the accumulation of the compound to be tested in cellular membrane fractions derived from non-raft and raft membrane is determined in said DRM assay. The test system involves treatment of cultured cells with test compound. Following incubation, cells are lysed in detergent solution and the DRM fraction (rafts) are isolated on a sucrose gradient. The DRM fraction is recovered and test compounds are measured by fluorimetry or quantitative mass spectrometry. Raftophilicity is determined as the proportion of test compound recovered in the DRM fraction compared to the amount of total membrane. An even better comparison of results of different experiments is achieved by comparing the raftophilicity of a test compound to that of a known, raftophilic standard. Corresponding examples are cholesteryl 4,4-difluoro-5,7-dimethyl-4-bora-3a,4a-diaza-*s*-indacene-3-dodecanoate (cholesteryl BODIPY® FL $C_{12}$ as provided by Molecular Probes, Eugene, US) or [$^3$H]cholesterol. More particularly, the DRM-test is carried out as follows. Cultured cells are incubated with the test compound for a period of time, e.g. 1 hour at 37°C, and then the cells are washed and extracted with cold detergent, usually 1% Triton X-100 in the cold (4°C). The lysate is centrifuged through a sucrose density gradient to produce a floating layer containing detergent resistant membranes. These can be equated to rafts for the purpose of the raftophilicity determination. The rafts and other materials are taken and analysed e.g. by mass-spectroscopy or fluorimetry (if the test compound is fluorescent) to determine the amount of test compound in each raft. The relative enrichment in the raft (raftophilicity) is then calculated. A corresponding example is provided in the experimental part.

As a natural raftophile, a derivative of raft-substituent lipids, e.g. cholesterol (sterol), sphingolipid (ceramide), GPI-anchor or saturated fatty acid may be considered. Derivatization of these classes of compounds is not supposed to interfere with their association with rafts and be at least as strong as the parent compound, as determined by raft-assays, as, inter alia, provided herein.

As discussed above, examples of such natural raftophiles are derivatives of cholesterol bearing a functional group attached to the hydroxyl group, sterol ring or the side chain. Further, corresponding examples are given below.

The linker (B/B') connects the raftophile (A/A') and the pharmacophore (C/C'). The precursors of the raftophile and the linker will contain functional groups which allow for covalent bonding there between. The nature of the functional groups is not particularly limited and corresponding examples are given herein below. Functional groups of the the raftophile (A/A'), which are used to covalently bind the raftophile (A/A') to the linker (B/B'), will herein also be referred to as "hooks". The chemical structure of these hooks is not particularly restricted and the only prerequisite is that the hooks do not interfere with the association of the tripartite structure to rafts. In a preferred embodiment, the raftophilicity of the raftophile (A/A'), and thus the raftophilicity of the present tripartite structure, is increased by an appropriate choice of the hook. The influence of the hook on raftophilicity of the raftophile (A/A') is demonstrated in the example section below.

In the case of the tripartite structure C'-B'-A', the raftophile A' is attached to a nucleophilic group on the linker B', i.e. to its N-terminus. As is evident from the specific structures shown below, the N-terminus of the linker does not necessarily comprise a nitrogen atom, but may also, for example, comprise an oxygen atom, as, e.g., in linker 22, where $X^{221}$ is oxygen. Examples of hooks that can be used to attach the raftophile A' to the N-terminus of a linker B' are succinyl and acetyl groups, wherein the N-terminus of the linker B' is attached to a carbonyl group of the succinyl or acetyl group.

Hooks that comprise an ether linkage, such as an acetyl group which is attached to an oxygen atom of the raftophile A' via the alpha-carbon atom of the acetyl group, are particularly preferred. Suitable amino acid hooks on raftophile A' are aspartic acid and glutamic acid, wherein the amino acid residue is attached to the raftophile via the side chain carboxylic acid group of the amino acid residue and the linker is attached to the alpha-carboxylic acid group of the same amino acid residue. The alpha-amino group of the amino acid residue can be protected, for example as its acetate.

In the case of the tripartite structure C-B-A. the raftophile A is attached to an electrophilic group on the linker B, i.e. to its C-terminus. As is evident from the specific structures shown below, the C-terminus of the linker is not necessarily a C=O group (as, e.g. in linkers **20, 21** and **22**), but may also be, for example, a sulfonyl ($SO_2$) group (cf., e.g., linker **24).** The raftophile A may be coupled directly to the C-terminus of a linker B by use of a terminal heteroatom of the raftophile A. Alternatively, an amino acid, for example, may be employed as hook to attach the raftophile A to the linker B , if a direct coupling is not appropriate or feasible: For example, raftophile A can be coupled to the epsilon-amino group of a lysine residue and the C-terminus of the linker B can be coupled to the alpha-amino group of the same lysine residue. Other suitable amino acid hooks on raftophile A are aspartic acid and glutamic acid, wherein the amion acid residue is attached to the raftophile via the side chain carboxylic acid group of the amino acid residue and the linker is attached to the alpha-amino group of the same amino acid residue. In the amino acid hooks the alpha-carboxylic acid group can be protected, for example as a primary amide.

A synthetic raftophile is a moiety or a precursor thereof that has high affinity to rafts but is not an analogue or a derivative of a natural raft lipid substituent. Again, examples of such synthetic raftophiles are provided herein.

As pointed out above, the propensity of a compound to partition into the raft domain from the aqueous phase or to laterally segregate into the raft domain from the surrounding non-raft bulk lipid (raftophilicity) lies in certain features of its structure which allow efficient integration or packing of the compound with the raft lipids. Specifically, the raftophilicity is determined by the compound's interaction with the lipid component of the raft or with a transmembrane part of a raft-associated membrane protein and may, inter alia, be determined by an assay provided herein, like the above outlined DRM assay or the LRA discussed below and documented in the examples.

Features relevant for raftophilicity may be, singularly or in combination, hydrophobicity and degree of branching of hydrocarbon chains or chains containing *trans*-unsaturations, hydrogen bonding capacity within the upper part of the raft such as demonstrated by sphingolipid and cholesterol, nearly flat carbocyclic ring structures, multiple hydrocarbon chains, structures which pack efficiently with sphingolipids and cholesterol, and structures whose integration is thermodynamically favourable.

In preferred raftophilic moieties "A/A' ", hydrocarbon chains are employed the overall length of which corresponds to hydrocarbon chains found in natural constituents of rafts, such as sphingolipids and cholesterol. For example, in moieties represented by formulae **2** and **3**, shown below, hydrocarbon chains having a length of approximately 8 to 12 carbon atoms are preferred. In moieties represented by formulae **4a** and **5a,** shown below, hydrocarbon chains having a length of approximately 18 to 24 carbon atoms are preferred. Furthermore, efficient packing with sphingolipids and cholesterol in the rafts is facilitated by choosing saturated, linear hydrocarbon chains. In raftophilic moieties "A/A' " having more than one long chain substituent, it is preferred that the difference in the number of carbon atoms between the long chain substituents is 4 or less, more preferably 2 or less. For example, if a raftophilic moiety "A/A' " bears a first long chain substitutent which is a linear $C_{18}$ alkyl group, it is preferred that a second long chain substituent is a linear $C_{14-22}$ alkyl group, more preferably a $C_{16-20}$ alkyl group. By minimizing the difference in the number of carbon atoms between two or more long chain substituents on a raftophilic moiety "A/A' " an overall cone shape of this moiety can be avoided and a disrafting effect of the raftophilic moiety "A/A' " upon incorporation into the raft can be minimized.

Certain structural features are excluded from the raft and therefore cannot be contained within raftophiles. Such features include hydrocarbon chains with multiple *cis*-unsaturations (e.g. dioleylphosphatidylcholine), orthogonal heterocyclic ring structures and nucleosides.

The propensity of a compound to partition into the raft domain may be determined in an assay measuring the concentration of the compound in the raft domain and that in the non-raft domain after a given incubation time with the lipid membrane system under study. Apart from the DRM test discussed above, a liposome raftophilicity assay (LRA) may be employed. Briefly, unilamellar liposomes composed of non-raft lipids (e.g. phosphatidylcholine and phosphatidylethanolamine) or liposomes composed of raft lipids (e.g. sphingolipid, phosphatidylcholine and cholesterol) are incubated in an aqueous suspension with the test compound, for example a tripartite structure compound of the invention or a precursor of a moiety suspected to be capable of functioning as "moiety A/A' " of the tripartite structure compound of the invention for a period of time e.g. 1 hour at 37°C. The fractions are separated and the amount of test compound in each is determined. For each liposome type a lipophilicity value is determined from the amount of compound taken up by the liposome. Raftophilicity is defined as the ratio of the lipophilicities of a given compound for raft versus non-raft liposomes. Again, a corresponding example is given in the experimental part. Yet, the person skilled in the art is readily in a position to carry out a LRA by carrying out the following, summarized protocol. Lipophilicity of a compound is, inter alia, measured by said LRA. For each liposome type (raft or non-raft) the lipophilicity is defined as the partitioning partitioning between an aqueous phase (i.e. concentration in the supernatant) versus a lipid phase (raft or non-raft), i.e.

the concentration in the lipids which constitute the liposome.

The test system comprises three components in which test compounds may be found, the lipid membrane, the aqueous supernatant and in the test tube wall. Following incubation, the liposomes are removed from the system and test compounds are measured in the aqueous and tube wall fraction by fluorimetry or quantitative mass spectrometry. Data may be computed to yield partition coefficients and raftophilicity.

Accordingly, the LRA described herein and also known in the art provides a further test system to elucidate the raftophilicity of a compound comprising the tripartite structure described herein or of a precursor of "moiety A" as well as "moiety A' " as defined herein and to be employed in a compound of the invention.

In context of this invention, it is of note that the person skilled in the art is readily in a position to generate liposomes. These liposomes comprise the above described "raft" liposomes, as well as "mixed" liposomes and "non-raft" liposomes. The corresponding lipids are known in the art. "Liposome-forming lipids" refers to amphipathic lipids which have hydrophobic and polar head group moieties, and which (a) can form spontaneously into bilayer vesicles in water, as exemplified by phospholipids, or (b) are stably incorporated into lipid bilayers, with the hydrophobic moiety in contact with the interior, hydrophobic region of the bilayer membrane, and the polar head group moiety oriented toward the exterior, polar surface of the membrane. The liposome-forming lipids of this type typically include one or two hydrophobic acyl hydrocarbon chains or a steroid group and may contain a chemically reactive group, such as an amine, acid, ester, aldehyde or alcohol, at the polar head group. Included in this class are the phospholipids, such as phosphatidyl choline (PC), phosphatidyl ethanolamine (PE), phosphatidic acid (PA), phosphatidyl inositol (PI), and sphingomyelin (SM), where the two hydrocarbon chains are typically between about 14 and 22 carbon atoms in length, and have varying degrees of unsaturation. Raft-lipids are defined herein.

The term "linker (linker structure)" as used in the context of the tripartite structure of the invention is employed to connect the raftophile A or A' and the pharmacophore C or C'. These subunits should neither compete in terms of raftophilicity with the raftophile A or A' nor compete in terms of pharmaceutical activity with the pharmacophore C or C'. In accordance with the present invention the linker rather provides covalent attachment of the raftophile to the pharmacophore and provides an ideal distance between the raftophile and the pharmacophore in order to enable the raftophile to pursue its function, e.g. enrichment and anchoring in lipid rafts, and in order to enable the pharmacophore to pursue its function, e.g. inhibition of enzymes. For this purpose the length of the linker is adapted to the situation in each case by modular assembly.

Subunits of said linker (moiety B and B') may be amino acids, derivatized or functionalized amino acids, polyethers, ureas, carbamates, sulfonamides or other subunits which fulfill the above mentioned requirement, i.e. providing for a distance between the raftophile ("moiety A and A' ") and the pharmacophore ("moiety C and C' ").

As discussed above, moiety B and B' is a linker which has a backbone of at least 8 carbon atoms (C) wherein one or more of said carbon atoms may be replaced by nitrogen (N), oxygen (O) or sulfur (S). Preferably said backbone has at least 8 atoms and at the most 390 atoms, more preferably said backbone has at least 9 atoms and at the most 385 atoms, even more preferably said backbone has at least 10 atoms and at the most 320 atoms.

If the linker B or B' comprises a sequence of covalently attached alpha- or beta-amino acids, the above recited atoms in the backbone are preferably at least 9 and 320 at the most, even more preferred is a linker consisting of amino acids which has a backbone of 10 to 80, more preferably of 20 to 70, even more preferably of 30 to 65, and most preferably of 34 to 60 C-atoms. In case said linker B or B' comprises a sequence of polyethers (amino acids with polyether backbones) said linker has preferably 9 to 285 atoms in the backbone. If the linker B or B' comprises urea, the preferred number of atoms in the backbone is from 10 to 381. A backbone structure made of carbamates has in moiety B or B' preferably from 10 to 381 atoms and a linker moiety B or B' consisting of sulfonamides comprises preferably at least 8 and and the most 339 atoms.

Accordingly, the overall length of moiety B or B' is 1 nm to 50 nm, more preferably from 5 to 40 nm, more preferably from 8 nm to 30 nm and most preferably from 10 nm to 25 nm.

The length of a structure/moiety as defined herein and particularly of a linker may be determined by methods known in the art, which comprise, but are not limited to molecular modelling (using preferably standard softwawre, like e.g. Hyperchem®, Furthermore, the corresponding length (or distance between moiety A/A' and moiety C/C') may also be deduced by crystallographic methods, in particular X-ray crystallography. Such X-ray crystallography methods are known in the art, see, inter alia, "X-ray chrystallography methods and interpretation" in McRee (1999), Practical Protein Crystallography, 2nd edition, Academic Press and corresponding information is also available from the internet, see, inter alia, X-ray crystallography structure and protein sequence/peptide sequence information available from the National Center for Biotechnology Information, U.S. National Library of Medicine, at http://www.ncbi.nlm.nih.gov/entrez/query.fcgi.

One function of the linker is to connect the raftophile to the pharmacophore (such as an inhibitor) in a way that the raftophile can be integrated into the lipid raft subcompartment of the bilayer (the raft) and the pharmacophore is able to bind to and/or interact with a specific site of action in the target molecule (e.g. inhibitor binding site and/or interaction site).

The linker is chosen to have a length which corresponds at least to the length of a backbone structure which has at least 8 carbon atoms and corresponds to the distance between the phosphoryl head groups or other equivalent

head groups of the raft lipids and the pharmacophore (preferably an inhibitor) binding and/or interaction site in the target molecule. Said binding and/or initiation site may be the active-site of an enzyme, a protein-protein docking site, a natural ligand binding site such as a ligand-receptor binding site or a site targeted by a virus to bind to a cellular membrane protein. Yet, the invention is not limited to the target molecules/sites listed herein above.

The length of the linker can be determined by information and methods known in the art, like X-ray crystallography, molecular modeling or screening with different linker lengths.

An example of how to determine the length of the linker is given by considering the BACE-1 beta-secretase protein. According examples are also provided in the experimental part. It is known that the distance between the transmembrane sequence and the cleavage site of the BACE-1 substrate amyloid-precursor protein (APP) is 29 amino acids (De Strooper, B., Annaert, W. (2000), Proteolytic processing and cell biological functions of the amyloid precursor protein, J. Cell Sci. 113, 1857-70.). Assuming a simple alpha-helix conformation this would mean that a linker of a length of 29 amino acids, or approximately 10 nm would be required to span the distance between the raftophile and the inhibitor binding site in this particular example.

Inhibitor III is a known inhibitor of BACE-1 beta-secretase protein. The inhibitor III sequence (Capell, J. Biol. Chem. 277 (2002), 5637; Tung, J. Med. Chem. 45 (2002), 259) replaces the primary beta-cleaved bond with a non-hydrolysable statine and also contains 4 more residues at the C-terminus. Hence 24 further amino acids are required from inhibitor III to the membrane. These amino acids correspond to the linker defined for delivery of inhibitor III to rafts in the example given herein for tripartite compound of the invention.

Accordingly, a suitable tripartite structure according to the present invention would be pharmacophore-Arg-His-Asp-Ser-Gly-Tyr-Glu-Val-His-His-Gln-Lys-Leu-Val-Phe-Phe-Ala-Glu-Asp-Val-Gly-Ser-Asn-Lys-raftophile, where the pharmacophore is e.g. Glu-Val-Asn-Sta-Val-Ala-Glu-Phe (where Sta is statine). For example, cholesteryl glycolic acid; can be employed as raftophile, see also compound having formula 24 described herein below.

Alternatively, the distance of 10 nm in the above example could also be spanned by a linker containing an appropriate number of polyethylene glycol units, see also compounds having formulae **25** and **25b,** in particular **25b,** described herein below. Linkers of this type are particularly preferred as they increase the solubility of the tripartite structure in aqueous media.

Since the targets of raftophile-linked inhibitors are likely to be enzymes or receptors with membrane proximal inhibitor-binding sites, the range of lengths expected to be spanned by the linker is from 1 nm to 50 nm, preferably 8 to 30 nm as discussed above. Said 1 nm to 50 nm corresponds to about 8 to 390 carbon atoms in a backbone. The person skilled in the art takes into account that the length of the linker defined herein is not only determined by its primary structure but also by its secondary structure (e.g. for peptide linkers alpha-helices and/or beta sheets). Furthermore, some naturally occurring amino acids, e.g. Pro, Met, Cys, may be comprised in the linker, but are considered as less suitable as building blocks for linkers in accordance with this invention, since these may induce turns in the geometry of the linker construct. This may lead to reduced flexibility or sensitivity to oxidation during their synthesis in vitro. Therefore, considering the above, a (peptide) linker of a length of 50 nm does not necessarily comprise only about 80 amino acids but may comprise more amino acids.

As an example and preferred embodiment of the invention, the range to be spanned would be equivalent to a polypeptide length of between 3 and 80 amino acids or a polyglycol length of 3 to 95 (ethylene)glycol units equivalent to 9 to 240 C-atoms. However, it is preferred that the linker comprises at least 3, more preferably at least 10, more preferably at least 15 amino acids or (ethylene)glycol units. Most preferred are linkers of 15 to 30 amino acids or (ethylene) glycol units. The invention is, however, not limited to linkers consisting of amino acids or (ethylene)glycol. It is of note that the upper limit of 80 units given above it is not limiting to the inventive construct. Even longer linkers comprising more than 80 units are envisaged. As pointed out above, the corresponding distance should be defined by the distance/ length between the phosphoryl head group or corresponding head group comprised in the raft lipids and the pharmacophore (preferably and inhibiting molecule) binding and/or interaction site as defined above and herein below.

Therefore, linkers in accordance with this invention preferably comprise 3 to 80 or more amino acids, wherein amino acids may be specified as $\alpha$- and $\beta$-amino acids (e.g. natural amino acids, such as His, Arg, Lys, Phe, Leu, Ala, Asn, Val, Gly, Ser, Gln, Tyr, Asp, Glu, Thr, and $\beta$-Ala) and wherein one amino acid side chain (e.g. of Glu or Lys) may comprise a (dye) label for detection in assays (e.g. rhodamine or synthetically modified derivatives thereof) or other labels known in the art. A possible compound of the invention, for example, comprises its tripartite structure but also an additional functional group, namely an additional label.

Another function of the linker is to keep the pharmacophore, e.g. inhibitor away from the hydrophobic lipid bilayer and to improve the solubility of the whole compound in aqueous media. The linker is, accordingly, most preferred polar. This may be achieved by the use of amphiphilic subunits or the introduction of polar functionalities into the linker. As an example the introduction of one or more arginine residues into a polypeptide linker increases polarity and solubility. In one preferred embodiment the linker contains polyethyleneglycol units which are known to enhance solubility in aqueous media.

Another linker function, which is envisaged, is to allow lateral movement of the raftophile in the lipid bilayer

and also rotational movement of the raftophile and pharmacophore such that the raftophile can position itself optimally for integration into the raft and the pharmacophore can position itself optimally for interaction with the inhibitor binding site.

The above described biological assays provided herein, like DRM and LRA, may benefit from the attachment of a fluorescent, radioactive or dye label (e.g. fluorescein, Mca, rhodamine B or synthetically modified derivatives thereof) to the compound of the invention. Preferably, said label is attached to the linker structure. In order to maintain undisturbed interaction of the raftophile and the pharmacophore with their surroundings, respectively, the label may be covalently attached to the linker (e.g. to the side chain of an amino acid, e.g. glutamic acid or lysine). Thus, if necessary, carrying a label for detection can be another function of the linker. Said (detectable) label may, however, also be part of "moiety A/A' " or moiety "C/C' " of the tripartite structured compound of the invention.

The linker may contain subunits, which can be referred to as linker building blocks (or units) of the linker. They are, inter alia, described below, and may comprise a carboxylic or sulfonic acid function (termed "acceptor-terminus") on one end and an amino or hydroxy function (termed "donor terminus") on the other. Depending on the chosen synthetic route and on the type of pharmacophore used, the pharmacophore may, e.g. be attached to the donor terminus of the linker via a carboxyl group (e.g. the C-terminus of an inhibitor peptide) and the raftophile can, e.g., be attached to the acceptor-terminus of the linker either via a heteroatom or via a lysine unit which is coupled by its ε-amino group to the carboxy end of a raftophile and by its α-amino function to the acceptor-terminus of the linker building block.

The term "pharmacophore" relates in context of the present invention to a covalently linked, active moiety comprised in the tripartite compound of the present invention, whereby the pharmacophore is preferably an inhibitory unit capable of interfering with molecular and/or biochemical processes taking place in the raft.

Apart from the active moiety the pharmacophore may also contain a hook portion (e.g. succinyl, acetyl) which binds to the linker. Furthermore, a dye label, preferably a fluorescent dye label, such as rhodamine, Mca, fluoresceine or synthetically modified derivatives thereof, may be attached to the pharmacophore.

The pharmacophore may be, inter alia, a small molecule drug with specificity for a binding site (for example an enzyme active site, protein-protein docking site, ligand-receptor binding site or viral protein attachment site). Yet, the pharmacophore may also be a peptidomimetic or peptide transtion-state inhibitor or polypeptide or (nucleic acid) aptamer. As detailed below, an example of the peptide transition-state inhibitor is the commercially available beta-secretase inhibitor III (Glu-Val-Asn-Sta-Val-Ala-Glu-Phe-$CONH_2$, where Sta is statine) (Calbiochem) which inhibits BACE-1 cleavage of APP at the beta-cleavage site. Other examples are the EGF receptor (Heregulin) inhibitor A30, a nucleic acid (RNA) aptamer (Chen, Proc. Natl. Acad. Sci. (USA) 100 (2003), 9226-31) or an anti-EGF receptor-blocking (monoclonal) antibody, e.g. trastuzumab (Herceptin). It is also envisaged that analogues of rifamycin (see US 6,143,740) are used in this context as small molecule EGF receptor antagonist. Furthermore, anti-HER2/neu peptidomimetic (AHNP) small-molecule inhibitors, (Park, Nat. Biotech. 18 (2000), 1948) may be a pharmacophore to be employed in the compounds of the invention. Furthermore, it is envisaged to employ influenza virus neuraminidase inhibitors, like Zanamivir (Relenza) and Oseltamivir (Tamiflu) which bind to the active site of neuraminidase. Other examples are provided below.

The main targets for the pharmacophores, preferably inhibitors, are those proteins whose (inhibitor) binding sites are accessible to the raftophile-linker-pharmacophore compounds of the invention. Hence, these will be, for example, proteins located in rafts or which move into rafts to execute a function.

The pharmacophore interaction sites on such target proteins will normally be from 1 nm to 50 nm from the phosphoryl head groups or other equivalent head groups of raft lipids in the extracellular space, in the case of the plasma membrane, or luminal space, in the case of vesicular membranes.

In accordance with the present invention it was surprisingly found that the novel compounds described herein and comprising the above defined tripartite structure are capable of linking specific pharmacophores (particularly inhibitors of biological/biochemical processes which take place in/on plasmamembrane- and/or vesicular rafts) to corresponding targets. Of particular importance to the invention is the linker structure which does not only provide the correct distance between the head groups of raft lipids and the binding and/or interaction site of the herein defined pharmacophores and their corresponding target molecules but also provides, together with the raftophile, "moiety A/A' ", for a distinct enrichment of the pharmacophore in the raft. In this context, it is of importance that the term "raft" as employed herein is not limited to rafts on the plasma membrane of a cell but also relates to intenal membranes and vesicular rafts. Enrichment of the pharmacophore in the raft leads to an unexpected increase in potency over and above the fold-enrichment based on its concentration. Thus, when the tripartite structured compound has a raftophilicity of e.g. 10, the increase in potency is of the order of 100. This is a result of the increase in the number of productive interactions between the pharmacophore and the active site of the target due to a longer residence time of the pharmacophore in the vicinity of the target.

In a more preferred embodiment of the invention, the tripartite structured compound comprises a "moiety A/A'" which is capable of partitioning into lipid membranes which comprise a lipid composition comprising cholesterol and/or functional analogues of cholesterol, sphingolipids and/or functional analogues of sphingolipid, glycolipid, and glycerophospholipids.

In the context of this invention, cholesterol-analogues are ergosterol, 7-dihydrocholesterol, or stigmasterol. Cholesterol analogues may be employed in the "rafts" for testing the compounds of the present invention. A preferred

sphingolipid is sphingomyelin, preferred sphingolipid analogues are ceramides, preferred glycolipids are gangliosides or cerebrosides or globosides or sulfatides, preferred glycerophospholipids are preferably saturated or mono-unsaturated (fatty-acylated) phosphatidylcholines, as well as phosphatidylethanolamines or phosphatidylserine.

The term "functional analogue" of cholesterol or of sphingolipids denotes, inter alia, corresponding steroid or lipid structures which contain structural features enabling raft formation (Xu, J. Biol. Chem. 276, (2001) 33540-33546, Wang, Biochemistry 43, (2004)1010-8).

In a more preferred embodiment of the tripartite structured compound of the invention, the lipid composition (into which moiety A/A' partitions) comprises glycolipids which are gangliosides or cerebrosides. It is also envisaged that globosides are comprised in said lipid composition. Said lipid composition is considered a "raft" lipid composition in contrast to a "non-raft" lipid composition. Accordingly said lipid composition is most preferably rich in cholesterol and sphingolipid. Yet, as mentioned above, also gangliosides may be comprised. These gangliosides may be, inter alia, GM1, GD1a, GD1b, GD3, GM2, GM3, GQ1a or GQ1b. These gangliosides are known in the art, see, inter alia, Svennerholm, Asbury, Reisfeld, "Biological Function of Gangliosides", Elsevier Science Ltd, 1994.

As discussed above, the raftophilicity as well as the biological, biopharmaceutical and/or pharmaceutical properties of a compound of the invention may be tested in vitro. The appended examples provide further guidance therefor. Yet, preferably, the corresponding tests are carried out on "rafts" comprising lipid compositions which comprise cholesterol and/or functional analogues of cholesterol in a range of 5 to 60%, sphingolipids and/or functional analogues of sphingolipid in a range of 5 to 40% and glycerophospholipids in a range of 20 to 80 %. Most preferably, said lipid membrane of the raft comprises cholesterol, sphingomyelin, phosphatidylcholine, phosphatidylethanolamine, and gangliosides (bovine brain, Type III, Sigma-Aldrich Co.). In a more preferred embodiment, said lipid membrane of the raft comprises cholesterol in the range of 40 to 60%, sphingomyelin in the range of 10 to 20%, phosphatidylcholine in the range of 10 to 20%, phosphatidyl ethanolamine in the range of 10 to 20%, and gangliosides in the range of 1 to 10%. A good example and a most preferred "artificial" raft comprises a lipid membrane that consists of 50% of cholesterol, 15% of sphingomyelin, 15% of phosphatidylcholine, 15% of phosphatidyl ethanolamine, and 5% of gangliosides.

Yet, it is also envisaged that the lipid membrane to be used for testing the compounds of the present invention and, in particular the precursor of "moiety A" of said tripartite structured compound comprises said cholesterol, said sphingolipid and/or functional analogues thereof and phospholipid in equal parts. For example said "artificial" raft may consist of a lipid membrane which comprises 33% cholesterol, 33% sphingomyelin/ceramide and 33% phophatidylcholine. Examples for "non-raft" lipid structures and liposomes are also given herein and in the appended examples.

In the following, examples of tripartite structured compounds of the invention are given. Particular embodiments of the inventive compounds are also given in the appended claims.

In the following formulae, --- is used to represent a single bond or a double bond, and ≡ is employed to denote a single bond, a double bond or a triple bond.

"Hydrocarbon" is used to denote a straight chain or branched, saturated or unsaturated, noncyclic or cyclic, but non-aromatic, group based on carbon and hydrogen atoms. The hydrocarbon group can also contain combinations of these groups. Optionally part of the hydrogen atoms can be replaced by fluorine atoms. For example, a hydrocarbon group can, among others, include an alkyl group, an alkenyl group, an alkynyl group, a cycloalkyl group, a cycloalkenyl group, an alkylene-cycloalkyl group, a cycloalkylene-alkyl group, an alkylene-cycloalkenyl group and a cycloalkenylene-alkyl group. Cycloalkyl and cycloalkylene groups preferably have 3 to 8 carbon atoms in their ring. Cycloalkenyl and cycloalkenylene groups preferably have 5 to 8 carbon atoms in their ring.

The present invention is intended to include pharmaceutically acceptable salts of the present compounds. Pharmaceutically acceptable salts of compounds of the present invention can be formed with various organic and inorganic acids and bases. Examplary acid addition salts comprise acetate, adipate, alginate, ascorbate, benzoate, benzenesulfonate, hydrogensulfate, borate, butyrate, citrate, caphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, nitrate, oxalate, pectinate, persulfate, 3-phenylsulfonate, phosphate, picate, pivalate, propionate, salicylate, sulfate, sulfonate, tartrate, thiocyanate, toluenesulfonate, such as tosylate, undecanoate and the like. Exemplary base addition salts comprise ammonium salts, alkali metall salts, such as sodium, lithium and potassium salts; earth alkali metall salts, such as calcium and magnesium salts; salts with organic bases (such as organic amines), such as benzazethine, dicyclohexylamine, hydrabine, N-methyl-D-glucamine, N-methyl-D-glucamide, t-butylamine, salts with amino acids, such as arginine, lysine and the like.

Furthermore, the general formulas given in the present invention are intented to cover all possible stereoisomers and diastereomers of the indicated compounds.

Moieties represented by the following formulae 2 and 3 are useful as the raftophile A or A' in the present invention:

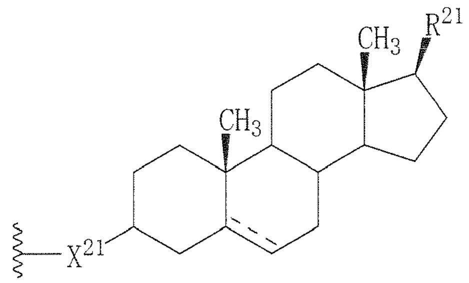

2

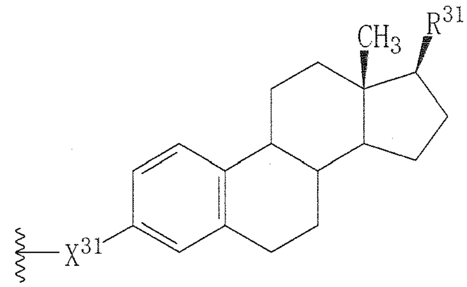

3

When the tripartite structure is C-B-A, $X^{21}$ and $X^{31}$ are directionally selected from NH, O, S, $NH(CH_2)_cOPO_3^-$, $NH(CH_2)_cSO_2CF_2$, $NH(CH_2)_cSO_2NH$, NHCONH, NHCOO, $NHCH(CONH_2)(CH_2)_dCOO$, $NHCH(COOH)(CH_2)_dCOO$, $NHCH(CONH_2)(CH_2)_dCONH$, $NHCH(COOH)(CH_2)_dCONH$, $NHCH(CONH_2)(CH_2)_4NH((CO)CH_2O)_f$ and $NHCH(COOH)(CH_2)_4NH((CO)CH_2O)_f$, preferably NH, $NH(CH_2)_cOPO_3^-$ and NHCONH, wherein the linker is bonded to $X^{21}$ or $X^{31}$. In another preferred embodiment, $X^{21}$ and $X^{31}$ are $NHCH(CONH_2)(CH_2)_dCOO$. In the context of the invention "directionally" means that the moieties given for $X^{21}$ and $X^{31}$ are bonded to the linker and the adjacent structure in the indicated direction. For example, in the case of $NH(CH_2)_cOPO_3^-$, NH is bonded to the linker and $OPO_3^-$ is bonded to the steroid structure. c is an integer from 2 to 3, preferably 2. d is an integer from 1 to 2, preferably 1. f is an integer from 0 to 1, Preferably 0. When the tripartite structure is C'-B'-A', $X^{21}$ and $X^{31}$ are $CO(CH_2)_b(CO)_aNH$, $CO(CH2)_b(CO)_aO$, $CO(CH_2)_bS$, $CO(CH_2)_bOPO_3^-$, $CO(CH_2)_bSO_2CF_2$, $CO(CH_2)_bSO_2NH$, $CO(CH_2)_bNHCONH$, $CO(CH_2)_bOCONH$, $CO(CH_2)_eCH(CONH_2)NHCO(CH_2)_b(CO)_aNH$, $CO(CH_2)_eCH(COOH)NHCO(CH_2)_b(CO)_aNH$, $CO(CH_2)_eCH(CONH_2)NHCO(CH_2)_b(CO)_aO$, $CO(CH_2)_eCH(COOH)NHCO(CH_2)_b(CO)_aO$, $COCH(NH_2)(CH_2)_eCOO$ or $COCH(NHCOCH_3)(CH_2)_eCOO$, preferably $CO(CH_2)_b(CO)_aNH$, $CO(CH_2)_b(CO)_aO$, $CO(CH_2)_bSO_2NH$, $CO(CH_2)_bNHCONH$ or $CO(CH_2)_bOCONH$, more preferably $CO(CH_2)_b(CO)_aNH$ or $CO(CH_2)_b(CO)_aO$, wherein the linker is bonded to the terminal carbonyl group of $X^{21}$ or $X^{31}$. In another preferred embodiment, $X^{21}$ and $X^{31}$ are $CO(CH_2)_eCH(CONH_2)NHCO(CH_2)_b(CO)_aNH$, $CO(CH_2)_eCH(COOH)NHCO(CH_2)_b(CO)_aNH$, $CO(CH_2)_eCH(CONH_2)NHCO(CH_2)_b(CO)_aO$, $CO(CH_2)_eCH(COOH)NHCO(CH_2)_b(CO)_aO$, $COCH(NH_2)(CH_2)_eCOO$ or $COCH(NHCOCH_3)(CH_2)_eCOO$. a is an integer from 0 to 1. b is an integer from 1 to 3. If a is 0, b is preferably 1. If a is 1, b is preferably 2. e is an integer from 1 to 2, preferably 1.

$R^{21}$ and $R^{31}$ are a $C_{4-20}$ hydrocarbon group, wherein one or more hydrogens are optionally replaced by fluorine. Preferably, $R^{21}$ and $R^{31}$ are a $C_{4-20}$ hydrocarbon group, optionally including one or more trans double bonds, more preferably a $C_{4-20}$ alkyl group. Even more preferably, $R^{21}$ and $R^{31}$ are a $C_{8-12}$ alkyl group. Most preferably, $R^{21}$ and $R^{31}$ are the branched $C_8H_{17}$ alkyl group present in naturally occurring cholesterol.

The stereocenter at C3 of moiety 2 is preferably as in naturally occurring cholesterol.

In a preferred embodiment, --- is a single bond. Incorporaton of a single bond between carbons 5 and 6 of the steroid scaffold allows for a more facile synthesis. Moreover, derivatives having a single bond between carbons 5 and 6 of the steroid scaffold often show an even higher raftophilicity than the corresponding unsaturated derivatives.

The following moieties **200a** to **200m** and **300a** to **300g** are preferred examples of moieties **2** and **3** for the raftophile A':

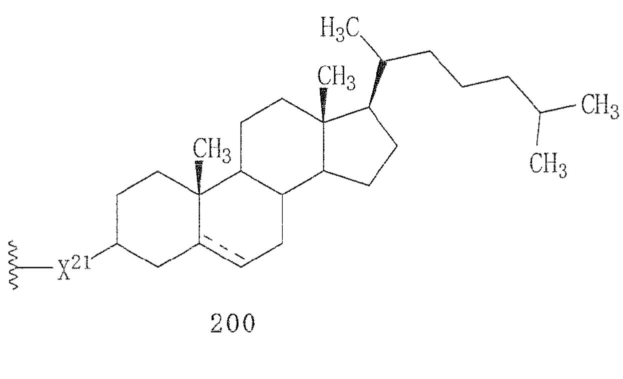

200

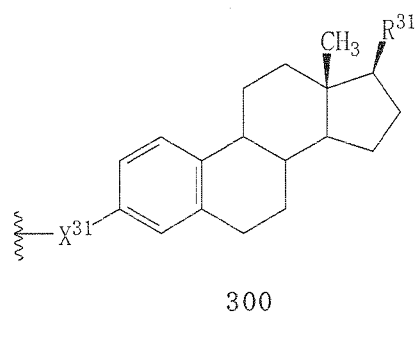

300

| 200 | $X^{21}$ | --- | 300 | $X^{31}$ | $R^{31}$ |
|---|---|---|---|---|---|
| **a** | $COCH_2O$ | double bond | **a** | $COCH_2O$ | $(CH_2)_{11}CH_3$ |
| **b** | $COCH_2O$ | single bond | **b** | $COCH_2O$ | $(CH_2)_{17}CH_3$ |
| **c** | $COCH_2NH$ | double bond | **c** | $COCH_2NH$ | $(CH_2)_{17}CH_3$ |
| **d** | $COCH_2NH$ | single bond | **d** | $COCH_2CH_2CONH$ | $(CH_2)_{17}CH_3$ |
| **e** | $COCH_2CH_2COO$ | single bond | **e** | $COCH_2SO_2NH$ | $(CH_2)_{17}CH_3$ |
| **f** | $COCH_2CH_2CONH$ | single bond | **f** | $COCH_2O$ | $(CH_2)_2$-cyclohexyl |
| **g** | $COCH_2SO_2NH$ | double bond | **g** | $COCH_2O$ | $-(CH=CH-)_2CH=CH_2$, all trans |
| **h** | $COCH_2NHCONH$ | double bond | | | |
| **i** | $COCH_2OCONH$ | double bond | | | |
| **j** | $COCH_2CH_2COO$ | double bond | | | |
| **k** | $COCH(NH_2)CHCOO$ | single bond | | | |
| **l** | $COCH(NHCOCH_3)CH_2COO$ | single bond | | | |
| **m** | $NHCH(CONH_2)CH_2COO$ | single bond | | | |

Moieties **200a, 200b, 200c, 200e, 200f, 200j, 200k** and **200l** are preferred examples of the raftophile A'. Moieties **200b** and **200f** are more preferred examples of the raftophile A'. Moiety **300a** is also a preferred example of the raftophile A'. Moiety **200m** is a particularly preferred example of the raftophile A.

Moieties represented by the following formulae **4a, 4b, 5a** and **5b** are useful as the raftophile A or A' in the present invention:

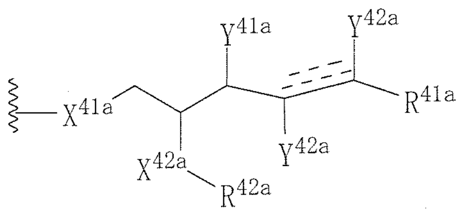

4a

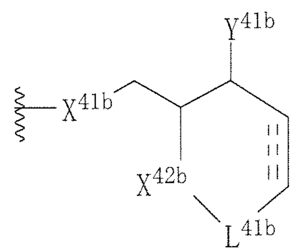

4b

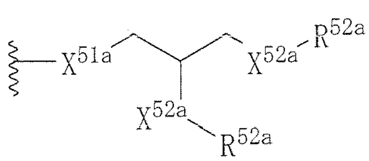

5a

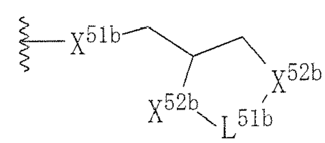

5b

When the tripartite structure is C-B-A, $X^{41a}$, $X^{41b}$, $X^{51a}$ and $X^{51b}$ are directionally selected from NH, O, $NH(CH_2)_cOPO_3^-$, $NH(CH_2)_cSO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, preferably O, $NH(CH_2)_cOPO_3^-$ and NHCOO, wherein the linker is bonded to $X^{41a}$, $X^{41b}$, $X^{51a}$ or $X^{51b}$. In another preferred embodiment, $X^{41a}$, $X^{41b}$, $X^{51a}$ and $X^{51b}$ are $NHCH(CONH_2)(CH_2)_dCOO$. c is an integer from 2 to 3, preferably 2. d is an integer from 1 to 2, preferably 1. When the tripartite structure is C'-B'-A', $X^{41a}$, $X^{41b}$, $X^{51a}$ and $X^{51b}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, $CO(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, preferably $CO(CH_2)_b(CO)_aNH$ or $CO(CH_2)_b(CO)_aO$, wherein the linker is bonded to the

terminal carbonyl group of $X^{41a}$, $X^{41b}$, $X^{51a}$ or $X^{51b}$. a is an integer from 0 to 1. b is an integer from 1 to 3. If a is 0, b is preferably 1. If a is 1, b is preferably 2. e is an integer from 1 to 2, preferably 1.

$X^{42a}$, $X^{42b}$, each $X^{52a}$ and each $X^{52b}$ are independently NH, O, S, OCO, NHCO, NHCONH, $NHCO_2$ or $NHSO_2$, preferably NH, O, NHCO, NHCONH, $NHSO_2$ or OCO, more preferably NHCO or $NHSO_2$, even more preferably NHCO.

$Y^{41a}$ and $Y^{41b}$ are $NH_2$, $NHCH_3$, OH, H, halogen or O, provided that when $Y^{41a}$ or $Y^{41b}$ is $NH_2$, $NHCH_3$, OH, H or halogen then --- is a single bond and when $Y^{41a}$ or $Y^{41b}$ is O then --- is a double bond. $Y^{41a}$ and $Y^{41b}$ are preferably OH or O, even more preferably OH.

Each $Y^{42a}$ is independently H or OH, provided that when --- is a triple bond, each $Y^{42a}$ is not present. Each $Y^{42a}$ is preferably H.

$R^{41a}$ is a $C_{10-30}$ hydrocarbon group, wherein one or more hydrogens are optionally replaced by fluorine. Preferably, $R^{41a}$ is a $C_{10-30}$ hydrocarbon group, optionally including one or more trans double bonds. More preferably, $R^{41a}$ is a $C_{13-19}$ alkyl group.

$R^{42a}$ and each $R^{52a}$ are independently a $C_{14-30}$ hydrocarbon group, wherein one or more hydrogens are optionally replaced by fluorine. Preferably, $R^{42a}$ and each $R^{52a}$ are independently a $C_{14-30}$ alkyl group, optionally including one or more trans double bonds. More preferably, $R^{42a}$ and each $R^{52a}$ are independently a $C_{14-30}$ alkyl group. Even more preferred groups for $R^{42a}$ and each $R^{52a}$ are $C_{16-26}$ alkyl groups, $C_{18-24}$ alkyl groups and $C_{18-20}$ alkyl groups.

$L^{41b}$ and $L^{51b}$ are a $C_{24-40}$ alkylene group, a $C_{24-40}$ alkenylene group or a $C_{24-40}$ alkynylene group, wherein one or more hydrogens are optionally replaced by fluorine.

With regard to the side chains of moieties **4a** and **5a,** i.e. $R^{41a}$, $R^{42a}$ and each $R^{52a}$, it is preferred that these groups do not contain any double or triple bonds. Furthermore, it is preferred that these groups are linear, i.e. do not contain any branching. In a particularly preferred embodiment, the difference in the number of carbon atoms between the groups $R^{41a}$ and $R^{42a}$ is two or less, even more preferred one or less, and the difference in the number of carbon atoms between the two groups $R^{52a}$ is four or less, even more preferred two or less. These preferences are chosen in view of optimizing the geometrical conformation of the raftophile to fit into the overall structure of the raftophile. Saturated, linear side chains are considered to provide the highest degree of conformational flexibility in the side chains to facilitate incorporation into lipid rafts. By choosing the difference in the number of carbon atoms in two side chains in one raftophilic moiety as small as possible, i.e. by avoiding an overall conical shape of the raftophile, a potential destabilizing effect of the raftophile on the raft assembly upon incorporation therein is believed to be minimized.

The stereocenters in moieties **4a, 4b, 5a** and **5b** are preferably as in naturally occurring sphingosine.

In moieties **4a** and **4b,** when --- is a double bond, it can be either in the cis configuration or in the trans configuration. In moieties **4a** and **4b,** when --- is a double bond, it is preferably in the trans configuration.

The following moieties **400aa** to **400ap, 400ba, 500aa** to **500ae** and **500ba** are examples of moieties **4a, 4b, 5a** and **5b** for the raftophile A':

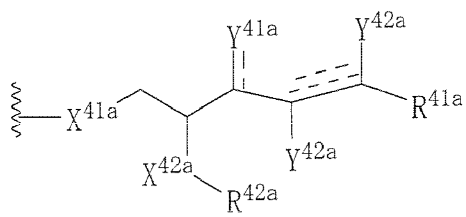

400a

| **400a** | $X^{41a}$ | $Y^{41a}$ | $Y^{42a}$ | --- (double bonds are trans) | $R^{41a}$ | $X^{42a}$ | $R^{42a}$ (double bonds are *trans*) |
|---|---|---|---|---|---|---|---|
| **a** | $COCH_2CH_2COO$ | OH | H | double bond | $(CH_2)_{12}CH_3$ | NHCO | $(CH_2)_{14}CH_3$ |
| **b** | $COCH_2O$ | OH | H | double bond | $(CH_2)_{12}CH_3$ | NHCO | $(CH_2)_{14}CH_3$ |
| c | $COCH_2CH_2COO$ | O | H | double bond | $(CH_2)_{12}CH_3$ | NHCO | $(CH_2)_{14}CH_3$ |
| **d** | $COCH_2CH_2COO$ | OH | H | triple bond | $(CH_2)_{12}CH_3$ | NHCO | $(CH_2)_{14}CH_3$ |

(continued)

| **400a** | $X^{41a}$ | $Y^{41a}$ | $Y^{42a}$ | - - - (double bonds are trans) | $R^{41a}$ | $X^{42a}$ | $R^{42a}$ (double bonds are *trans*) |
|---|---|---|---|---|---|---|---|
| **e** | $COCH_2CH_2COO$ | OH | H | single bond | $(CH_2)_{12}CH_3$ | NHCO | $(CH_2)_{14}CH_3$ |
| **f** | $COCH_2CH_2COO$ | OH | H | double bond | $(CH_2)_{12}CH_3$ | NHCO | $(CH_2)_{18}CH_3$ |
| **g** | $COCH_2CH_2COO$ | OH | H | double bond | $(CH_2)_{12}CH_3$ | NHCO | $(CH_2)_7CHCH(CH_2)_5CH_3$ |
| **h** | $COCH_2CH_2COO$ | OH | H | double bond | $(CH_2)_{17}CH_3$ | NHCO | $(CH_2)_{28}CH_3$ |
| **i** | $COCH_2CH_2CONH$ | OH | H | double bond | $(CH_2)_{12}CH_3$ | NHCO | $(CH_2)_{14}CH_3$ |
| **j** | $COCH_2CH_2COO$ | OH | H | double bond | $(CH_2)_{12}CH_3$ | NH | $(CH_2)_{15}CH_3$ |
| **k** | $COCH_2CH_2COO$ | OH | OH | single bond | $(CH_2)_{12}CH_3$ | NHCO | $(CH_2)_{14}CH_3$ |
| **l** | $COCH_2CH_2COO$ | OH | H | double bond | $(CH_2)_{12}CH_3$ | $NHSO_2$ | $(CH_2)_{14}CH_3$ |
| **m** | $COCH_2CH_2COO$ | OH | H | double bond | $(CH_2)_{12}CH_3$ | NHCONH | $(CH_2)_{17}CH_3$ |
| **o** | $COCH_2CH_2COO$ | OH | H | double bond | $(CH_2)_{17}CH_3$ | OCO | $(CH_2)_{28}CH_3$ |
| **p** | $COCH_2CH_2COO$ | OH | H | double bond | $(CH_2)_{12}CH_3$ | NHCONH | $(CH_2)_{15}CH_3$ |

In moieties **400aa, 400ab,** and **400ad** to **400ap** $Y^{41a}$ is bonded to the carbon backbone via a single bond. In moiety **400ac** $Y^{41a}$ is bonded to the carbon backbone via a double bond.

Moieties **400aa, 400ad, 400af, 400aj, 400ak, 400al** and **400ap** are preferred examples of the raftophile A'.

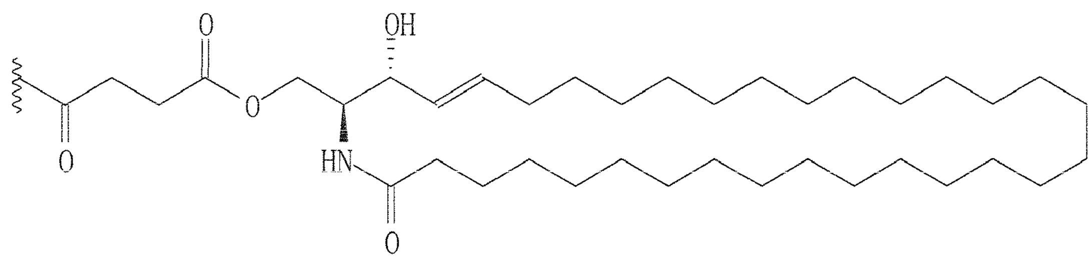

400ba

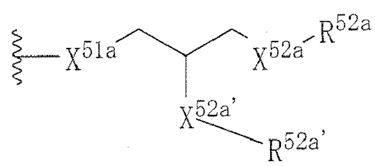

500a

| **500a** | $X^{51a}$ | $X^{52a}$ | $R^{52a}$ | $X^{52a'}$ | $R^{52a'}$ |
|---|---|---|---|---|---|
| **a** | $COCH_2CH_2COO$ | OCO | $(CH_2)_{18}CH_3$ | OCO | $(CH_2)_{18}CH_3$ |
| **b** | $COCH_2O$ | OCO | $(CH_2)_{18}CH_3$ | OCO | $(CH_2)_{18}CH_3$ |
| **c** | $COCH_2CH_2COO$ | OCO | $(CH_2)_{18}CH_3$ | OCO | $(CH_2)_{28}CH_3$ |
| **d** | $COCH_2CH_2COO$ | OCO | $(CH_2)_{28}CH_3$ | OCO | $(CH_2)_{28}CH_3$ |
| **e** | $COCH_2CH_2COO$ | O | $(CH_2)_{19}CH_3$ | O | $(CH_2)_{19}CH_3$ |

Moieties **500aa** and **500ae** are preferred examples of the raftophile A'. Particularly preferred is moiety **500ae.**

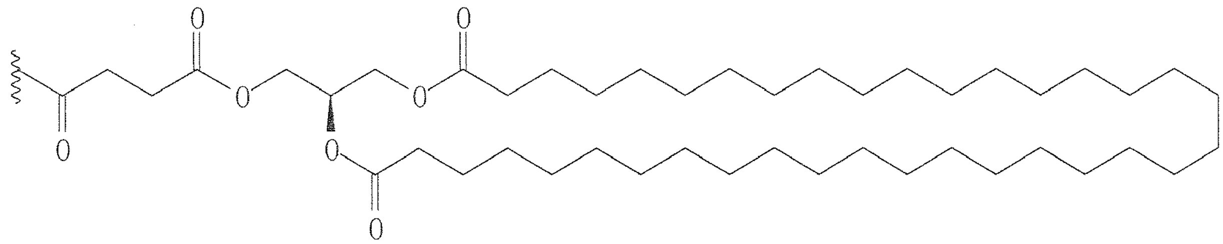

500ba

Moieties represented by the following formulae **6** and **7** are useful as the raftophile A or A' in the present invention:

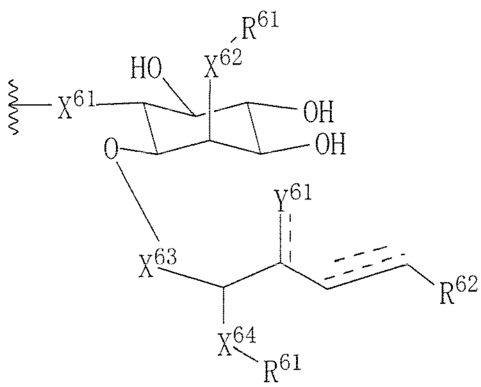

6

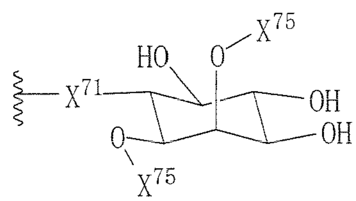

7

When the tripartite structure is C-B-A, $X^{61}$ and $X^{71}$ are O, wherein the linker is bonded to $X^{61}$ or $X^{71}$. When the tripartite structure is C'-B'-A', $X^{61}$ and $X^{71}$ are $CO(CH_2)_b(CO)_aO$, wherein the linker is bonded to the terminal carbonyl group of $X^{61}$ or $X^{71}$. a is an integer from 0 to 1. b is an integer from 1 to 3. If a is 0, b is preferably 1. If a is 1, b is preferably 2.

Each $X^{75}$ is independently a CO-$C_{13-25}$ hydrocarbon group, wherein one or more hydrogens are optionally replaced by fluorine, a group of the following formula:

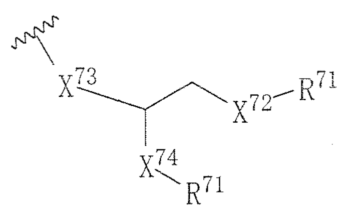

or a group of the following formula:

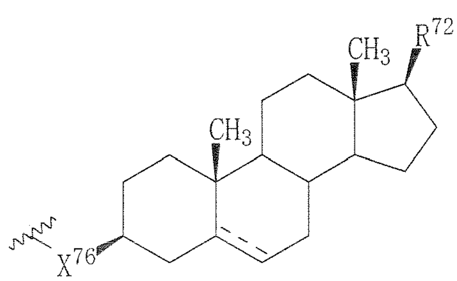

Preferably, $X^{75}$ is a CO-$C_{13-25}$ hydrocarbon group, wherein one or more hydrogens are optionally replaced by

fluorine, even more preferably a CO-$C_{18-20}$ alkyl group. In an even more preferred embodiment, $X^{75}$ is a group of the formula:

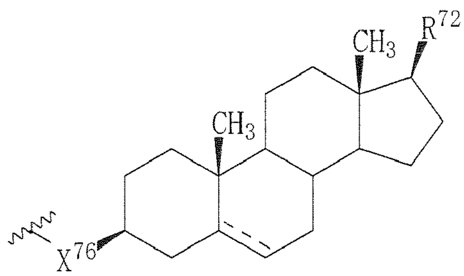

wherein --- is preferably a single bond.

$X^{62}$ and each $X^{72}$ are independently O or OCO, preferably OCO.

$X^{63}$ and $X^{73}$ are directionally selected from $PO_3^-CH_2$, $SO_3CH_2$, $CH_2$, $CO_2CH_2$ and a direct bond, preferably $PO_3^-CH_2$.

$X^{64}$ and $X^{74}$ are NH, O, S, OCO, NHCO, NHCONH, $NHCO_2$ or $NHSO_2$.

$X^{76}$ is directionally selected from $CO(CH_2)_b(CO)_aO$ and $CO(CH_2)_b(CO)_aNH$, preferably $CO(CH_2)_b(CO)_aO$. a is an integer from 0 to 1. b is an integer from 1 to 3. If a is 0, b is preferably 1. If a is 1, b is preferably 2. Most preferably, $X^{76}$ is $COCH_2O$.

$Y^{61}$ is $NH_2$, $NHCH_3$, OH, H, halogen or O, provided that when $Y^{61}$ is $NH_2$, $NHCH_3$, OH, H or halogen then --- is a single bond and when $Y^{61}$ is O then --- is a double bond. Preferably $Y^{61}$ is OH.

Each $R^{61}$ and each $R^{71}$ are independently a $C_{16-30}$ hydrocarbon group, wherein one or more hydrogens are optionally replaced by fluorine. Preferably, each $R^{61}$ and each $R^{71}$ are independently a $C_{16-24}$ hydrocarbon group, optionally including one or more trans double bonds. More preferably, each $R^{61}$ and each $R^{71}$ are independently a $C_{16-20}$ alkyl group.

$R^{62}$ is a $C_{13-25}$ hydrocarbon group, wherein one or more hydrogens are optionally replaced by fluorine. Preferably, $R^{62}$ is a $C_{13-25}$ hydrocarbon group, optionally including one or more trans double bonds. More preferably, $R^{62}$ is a $C_{13-19}$ alkyl group.

$R^{72}$ is a $C_{4-20}$ hydrocarbon group, wherein one or more hydrogens are optionally replaced by fluorine. Preferably, $R^{72}$ is a $C_{4-20}$ hydrocarbon group, optionally including one or more trans double bonds, more preferably a $C_{4-20}$ alkyl group. Even more preferably, $R^{72}$ is a $C_{8-12}$ alkyl group. Most preferably, $R^{72}$ is the branched $C_8H_{17}$ alkyl group present in naturally occurring cholesterol.

In moiety **6**, when --- is a double bond, it can be either in the cis configuration or in the trans configuration. In moiety **6,** when --- is a double bond, it is preferably in the trans configuration.

With regard to the side chains of moieties **6** and **7**, i.e. $R^{61}$, $R^{62}$ and $R^{71}$, it is preferred that these groups do not contain any double or triple bonds. Furthermore, it is preferred that these groups are linear, i.e. do not contain any branching. In a particularly preferred embodiment, the difference in the number of carbon atoms between the groups $R^{61}$ and $R^{62}$ or between the three groups $R^{71}$ is four or less, even more preferred two or less. If $X^{75}$ is a CO-$C_{13-25}$ hydrocarbon group, it is preferred that the difference in the number of carbon atoms between the groups $R^{71}$ and $X^{75}$ is four or less, even more preferred two or less These preferences are chosen in view of optimizing the geometrical conformation of the raftophile to fit into the overall structure of the raftophile. Saturated, linear side chains are considered to provide the highest degree of conformational flexibility in the side chains to facilitate incorporation into rafts. By choosing the difference in the number of carbon atoms in two side chains in one raftophilic moiety as small as possible, i.e. by avoiding an overall conical shape of the raftophile, a potential destabilizing effect of the raftophile on the raft assembly upon incorporation therein is believed to be minimized.

The following moieties **600** and **700** are preferred examples of moieties **6** and **7** for the raftophile A':

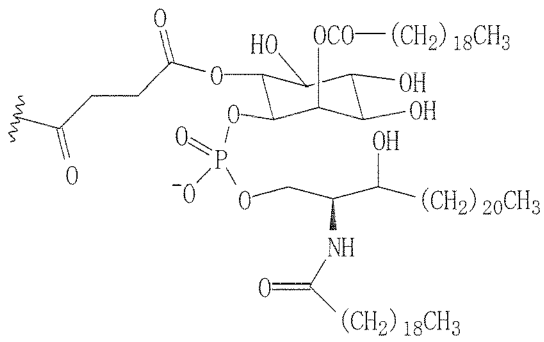

600

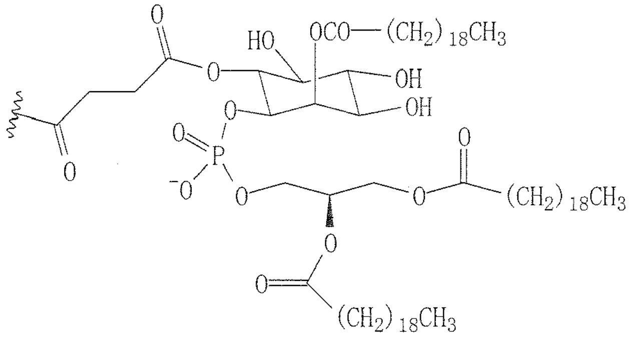

700

The following moieties **700a, 700b** and **700c** are particularly preferred examples of moiety **7** for the raftophile A':

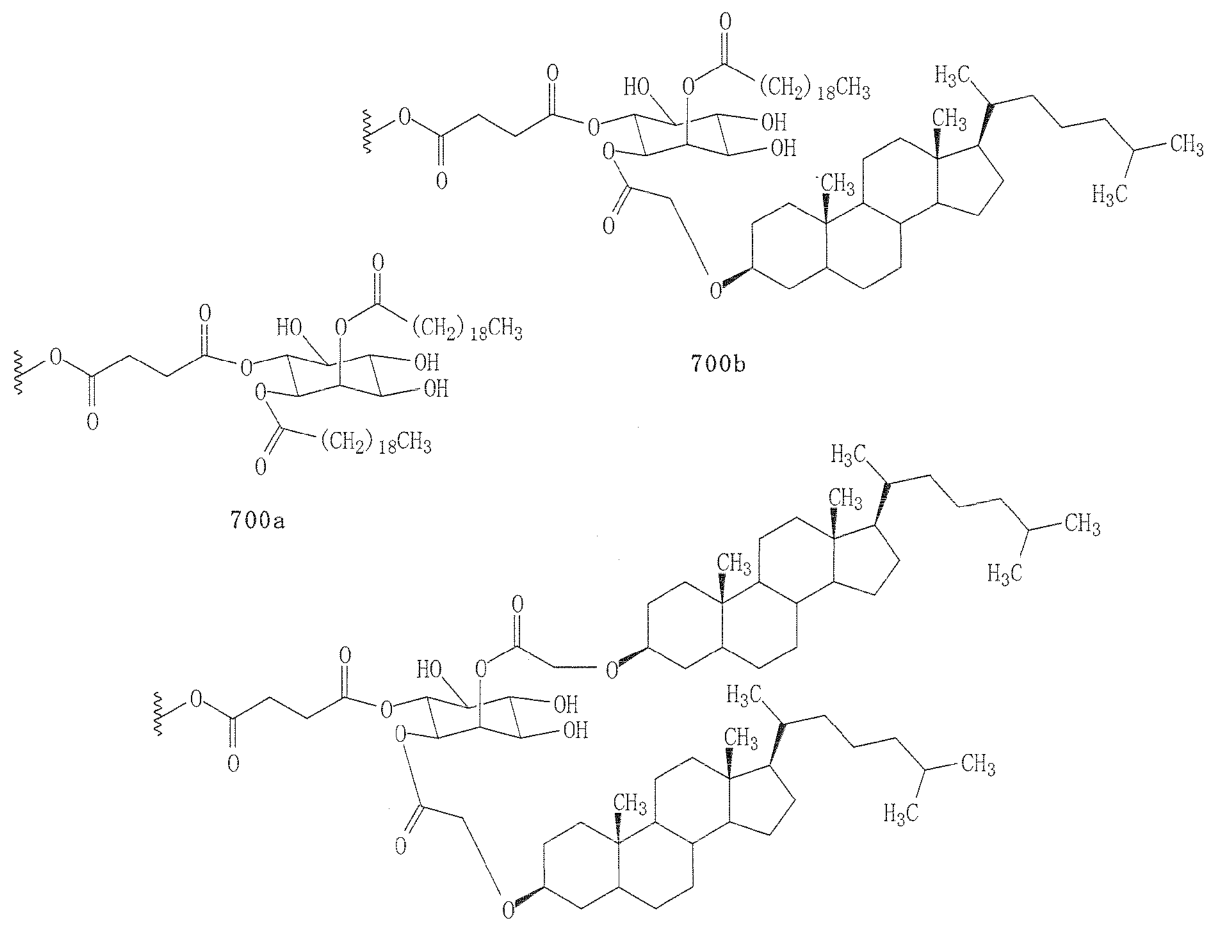

700b

700a

700c

Moieties represented by the following formulae **8a, 8b**, **9** and **10** are useful as the raftophile A or A' in the present invention:

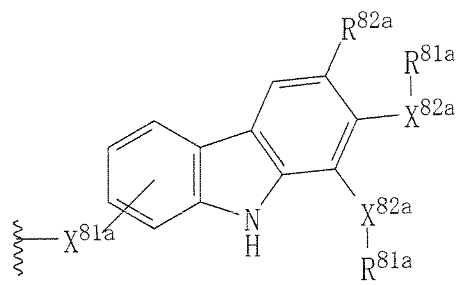

8a

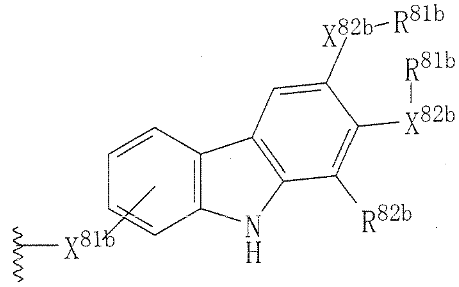

8b

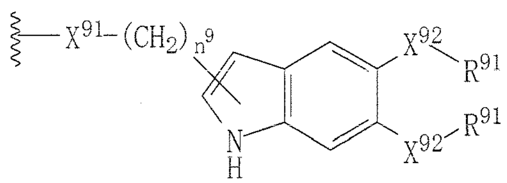

9

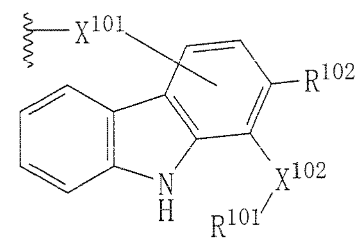

10

When the tripartite structure is C-B-A, $X^{81a}$, $X^{81b}$, $X^{91}$ and $X^{101}$ are directionally selected from NH, O, NH $(CH_2)_cOPO_3^-$, $NH(CH_2)_cSO_2NH$, NHCONH and NHCOO, preferably NH and NHCONH, wherein the linker is bonded to $X^{81a}$, $X^{81b}$, $X^{91}$ or $X^{101}$. c is an integer from 2 to 3, preferably 2. When the tripartite structure is C'-B'-A', $X^{81a}$, $X^{81b}$, $X^{91}$ and $X^{101}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNH$-CONH, $CO(CH_2)OCONH$, $CO(CH_2)_bOSO_3$, or $CC(CH_2)_bNHCO_2$, preferably $CO(CH_2)_b(CO)_aNH$ or $CO(CH_2)_b(CO)_aO$, wherein the linker is bonded to the terminal carbonyl group of $X^{81a}$, $X^{81b}$, $X^{91}$ or $X^{101}$. a is an integer from 0 to 1.b is an integer from 1 to 3. If a is 0, b is preferably 1. If a is 1, b is preferably 2.

Each $X^{82a}$, each $X^{82b}$, each $X^{92}$ and $X^{102}$ are independently $CH_2$ or O, preferably $CH_2$.

$n^9$ is an integer from 1 to 2.

Each $R^{81a}$, each $R^{81b}$ and each $R^{91}$ are independently H or a $C_{16-30}$ hydrocarbon group, wherein one or more hydrogens are optionally replaced by fluorine, provided that at least one $R^{81a}$, at least one $R^{81b}$ and at least one $R^{91}$ are a $C_{16-30}$ hydrocarbon group, wherein one or more hydrogens are optionally replaced by fluorine. Preferably, each $R^{81a}$, each $R^{81b}$ and each $R^{91}$ are independently H or a $C_{16-30}$ hydrocarbon group, optionally including one or more trans double bonds or one or more triple bonds, provided that at least one $R^{81a}$, at least one $R^{81b}$ and at least one $R^{91}$ are a $C_{16-30}$ hydrocarbon group. More preferably, each $R^{81a}$, each $R^{81b}$ and each $R^{91}$ are independently H or a $C_{16-30}$ alkyl group, provided that at least one $R^{81a}$, at least one $R^{81b}$ and at least one $R^{91}$ are a $C_{16-30}$ alkyl group.

$R^{101}$ is a $C_{16-30}$ hydrocarbon group, wherein one or more hydrogens are optionally replaced by fluorine. Preferably, $R^{101}$ is a $C_{16-30}$ hydrocarbon group, optionally including one or more trans double bonds or one or more triple bonds. More preferably, $R^{101}$ is a $C_{16-30}$ alkyl group.

$R^{82a}$, $R^{82b}$ and $R^{102}$ are H, a $C_{1-15}$ hydrocarbon group, wherein one or more hydrogens are optionally replaced by fluorine, or a $C_{1-15}$ hydrocarbonoxy group, wherein one or more hydrogens are optionally replaced by fluorine. Preferably, $R^{82a}$, $R^{82b}$ and $R^{102}$ are H, a $C_{1-15}$ alkyl group or a $C_{1-15}$ alkoxy group.

Preferably, $X^{81a}$ is bonded to the benzo ring in the 6 position. Preferably, $X^{81b}$ is bonded to the benzo ring in the 7 position. Preferably, $X^{91}$-$(CH_2)_{n9}$- is bonded to the pyrrole ring in the 3 position. Preferably, $X^{101}$ is bonded to the benzo ring in the 3 position.

The following moieties **800a, 900** and **1000** are preferred examples of moieties **8a**, **9** and **10** for the raftophile A':

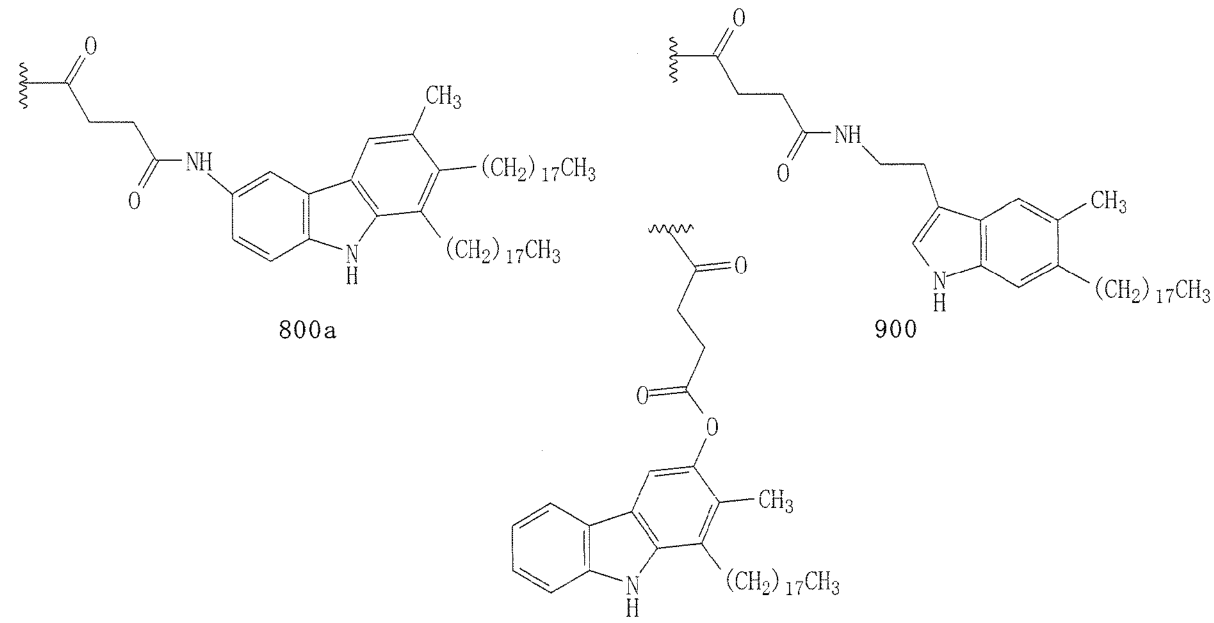

800a 900

1000

Moieties represented by the following formulae **11** and **12** are useful as the raftophile A or A' in the present invention:

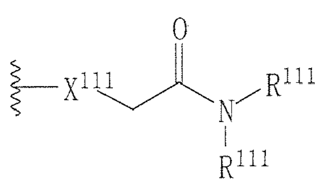

11

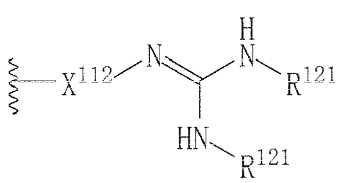

12

When the tripartite structure is C-B-A, $X^{111}$ is directionally selected from O, NH, $O(CH_2)_cO$ and $NH(CH_2)_cSO_2NH$, wherein the linker is bonded to $X^{111}$. c is an integer from 2 to 3. When the tripartite structure is C'-B'-A', $X^{111}$ is $CO(CH_2)_b(CO)_aO$ or $CO(CH_2)_b(CO)_aNH$, wherein the linker is bonded to the terminal carbonyl group of $X^{111}$. a is an integer from 0 to 1. b is an integer from 1 to 3. If a is 0, b is preferably 1. If a is 1, b is preferably 2.

When the tripartite structure is C-B-A, $X^{112}$ is directionally selected from $(CH_2)_cNH$ or a direct bond, wherein the linker is bonded to $X^{112}$. c is an integer from 2 to 3, preferably 2. When the tripartite structure is C'-B'-A', $X^{112}$ is $CO(CH_2)_bO(CO)$ or $CO(CH_2)_b$, wherein the linker is bonded to the carbonyl group of the $CO(CH_2)_b$ moiety of $X^{112}$. b is an integer from 1 to 3, preferably 2.

Each $R^{111}$ and each $R^{121}$ are independently a $C_{16-30}$ hydrocarbon group, wherein one or more hydrogens are optionally replaced by fluorine. Preferably, each $R^{111}$ and each $R^{121}$ are independently a $C_{16-30}$ hydrocarbon group, optionally including one or more trans double bonds or one or more triple bonds. More preferably, each $R^{111}$ and each $R^{121}$ are independently a $C_{16-30}$ alkyl group.

The following moieties **1100a, 1100b, 1200a** and **1200b** are preferred examples of moieties **11** and **12** for the raftophile A':

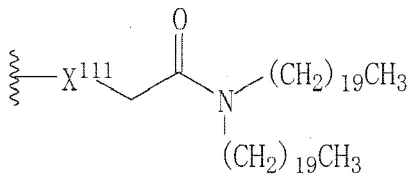

1100

| 1100 | $X^{111}$ |
|---|---|
| a | $COCH_2O$ |
| b | $COCH_2NH$ |

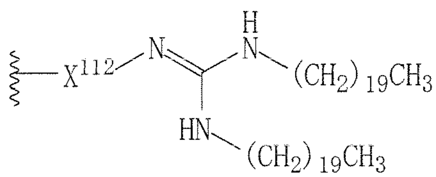

1200

| 1200 | $X^{112}$ |
|---|---|
| a | $COCH_2CH_2OCO$ |
| b | $COCH_2$ |

A moiety represented by the following formula 13 is useful as the raftophile A or A' in the present invention:

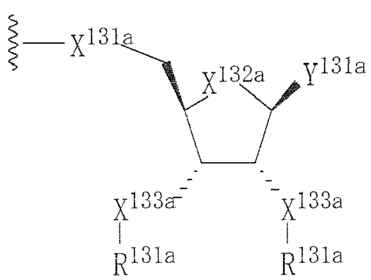

13a

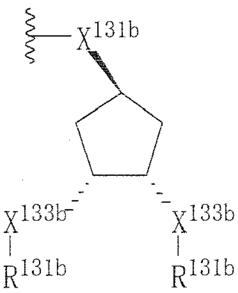

13b

When the tripartite structure is C-B-A, $X^{131a}$ and $X^{131b}$ are directionally selected from NH, O, $NH(CH_2)_cOPO_3^-$, $NH(CH_2)_cSO_2NH$, NHCONH and NHCOO, wherein the linker is bonded to $X^{131a}$ or $X^{131b}$. c is an integer from 2 to 3, preferably 2. When the tripartite structure is C'-B'-A', $X^{131a}$ and $X^{131b}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, or $CO(CH_2)_bNHCO_2$, preferably $CO(CH_2)_b(CO)_aO$, wherein the linker is bonded to the terminal carbonyl group of $X^{131a}$ or $X^{131b}$. a is an integer from 0 to 1. b is an integer from 1 to 3. If a is 0, b is preferably 1. If a is 1, b is preferably 2. $X^{132a}$ is NH, O or $SO_2$ preferably NH or O, more preferably O.

Each $X^{133a}$ and each $X^{133b}$ are independently O, NH, $CH_2$, OCO or NHCO, preferably OCO or NHCO.

$Y^{131a}$ is $NH_2$, $NHCH_3$, CH, H or halogen, preferably H or OH.

Each $R^{131a}$ and each $R^{131b}$ are independently a $C_{16-30}$ hydrocarbon group, wherein one or more hydrogens are optionally replaced by fluorine. Preferably, each $R^{131a}$ and each $R^{131b}$ are independently a $C_{16-30}$ hydrocarbon group, optionally including one or more trans double bonds or one or more triple bonds. More preferably, each $R^{131a}$ and each $R^{131b}$ are independently a $C_{16-30}$ alkyl group.

The following moieties **1300aa** to **1300ac** are preferred examples of moiety **13a** for the raftophile A':

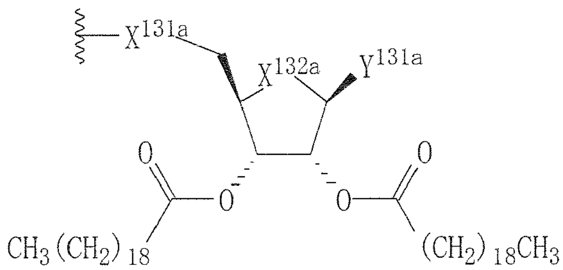

1300a

| 1300a | $X^{131a}$ | $X^{132a}$ | $Y^{131a}$ |
|---|---|---|---|
| **a** | $COCH_2CH_2COO$ | O | OH |
| **b** | $COCH_2CH_2COO$ | NH | H |

The following moiety **1300b** is a preferred example of moiety **13b** for the raftophile A':

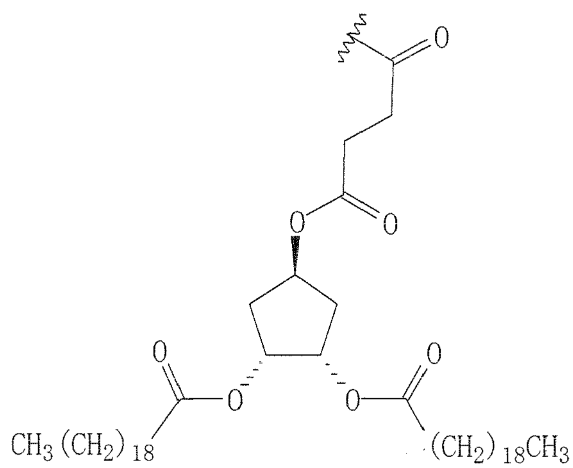

1300b

Naphthalene moieties **14a,** phenanthrene moieties **14b** and chrysene moieties **14c,** each substituted by one 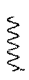-$X^{141}$- and one -$X^{142}$-$R^{141}$ in any available position, provided that the carbon atoms to which 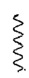-$X^{141}$- and -$X^{142}$-$R^{141}$ are bonded are separated by at least 2 carbon atoms, are useful as the raftophile A or A' in the present invention, wherein unsubstituted naphthalene, unsubstituted phenanthrene and unsubstituted chrysene are represented by the following formulae **14a-1, 14b-1** and **14c-1,** respectively:

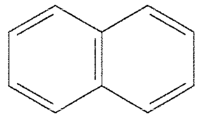

14a-1

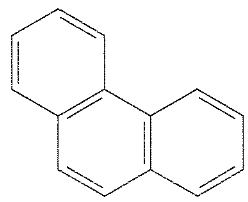

14b-1

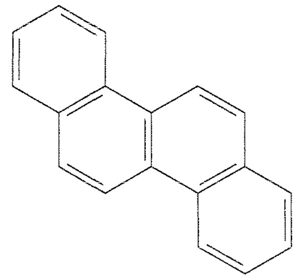

14c-1

The term "separated by at least 2 carbon atoms" means that the shortest route between the carbon atoms to which 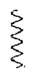 $-X^{141}-$ and $-X^{142}-R^{141}$ are bonded contains at least 2 carbon atoms, not counting the carbon atoms to which 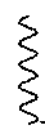 $-X^{141}-$ and $-X^{142}-R^{141}$ are bonded.

When the tripartite structure is C-B-A, $X^{141}$ is directionally selected from NH, O, $NH(CH_2)_cOPO_3^-$, $NH(CH_2)_cSO_2NH$, NHCONH and NHCOO, preferably NH and NHCONH, wherein the linker is bonded to $X^{141}$. c is an integer from 2 to 3. When the tripartite structure is C'-B'-A', $X^{141}$ is $CO(CH_2)_b(CO)_aNH$, $CO(CH_2)_b(CO)_aO$, $CO(CH_2)_bS$, $CO(CH_2)_bOPO3^-$, $CO(CH_2)_bSO_2NH$, $CO(CH_2)_bNHCONH$, $CO(CH_2)_bOCONH$, $CO(CH_2)_bOSO_3$, or $CO(CH_2)_bNHCO_2$, preferably $CO(CH_2)_b(CO)_aNH$, $CO(CH_2)_b(CO)_aO$ or $CO(CH_2)_bSO_2NH$, wherein the linker is bonded to the terminal carbonyl group of $X^{141}$. a is an integer from 0 to 1. b is an integer from 1 to 3. If a is 0, b is preferably 1. If a is 1, b is preferably 2.

$X^{142}$ is O or $CH_2$.

$R^{141}$ is a $C_{12-30}$ hydrocarbon group, wherein one or more hydrogens are optionally replaced by fluorine. Preferably, $R^{141}$ is a $C_{12}$-$_{30}$ hydrocarbon group, optionally including one or more trans double bonds or one or more triple bonds. More preferably, $R^{141}$ is a $C_{12}$-$_{30}$ alkyl group.

The following moieties **1400aa** to **1400ae** are preferred examples of the naphthalene moieties for the raftophile A':

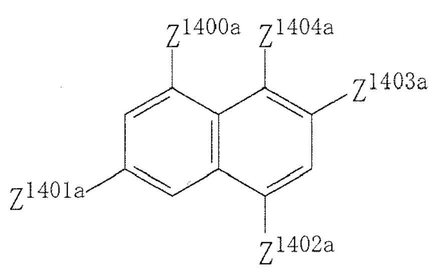

1400a

| **1400a** | $Z^{1400a}$ | $Z^{1401a}$ | $Z^{1402a}$ | $Z^{1403a}$ | $Z^{1404a}$ |
|---|---|---|---|---|---|
| **a** | $-COCH_2CH_2CONH$ | H | $(CH_2)_{15}CH_3$ | H | H |
| **b** | $-COCH_2SO_2NH$ | H | $O(CH_2)_{17}CH_3$, | H | H |
| **c** | H | $-COCH_2NH$ | H | $(CH_2)_{15}CH_3$ | H |
| **d** | H | $-COCH_2CH_2CONH$ | H | $(CH_2)_{15}CH_3$ | H |
| **e** | H | H | $(CH_2)_{15}CH_3$ | H | $-COCH_2CH_2CONH$ |

The following compound **1400b** is a preferred example of the phenanthrene moiety for the raftophile A':

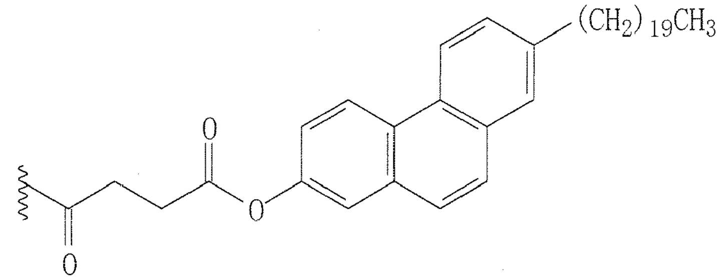

1400b

Moieties represented by the following formulae **15** and **16** are useful as the raftophile A or A' in the present invention:

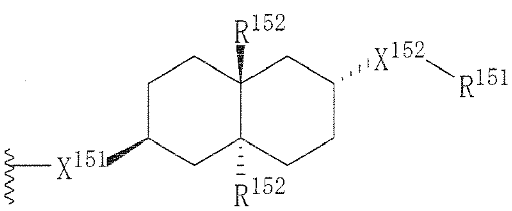

15

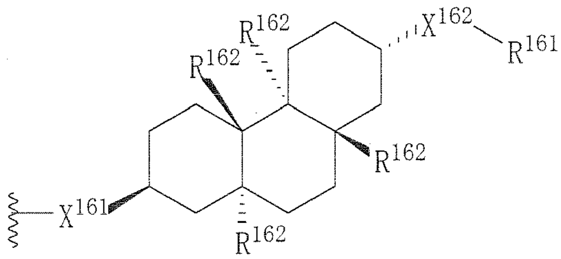

16

When the tripartite structure is C-B-A, $X^{151}$ and $X^{161}$ are directionally selected from NH, O, $NH(CH_2)_cOPO_3^-$, $NH(CH_2)_cSO_2NH$, NHCONH and NHCOO, preferably NH and NHCONH, wherein the linker is bonded to $X^{151}$ or $X^{161}$. c is an integer from 2 to 3, preferably 2. When the tripartite structure is C'-B'-A', $X^{151}$ and $X^{161}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, or $CO(CH_2)_bNHCO_2$, preferably $CO(CH_2)_b(CO)_aNH$ or $CO(CH_2)_b(CO)_aO$, wherein the linker is bonded to the terminal carbonyl group of $X^{151}$ or $X^{161}$. a is an integers from 0 to 1. b is an integer from 1 to 3. If a is 0, b is preferably 1. If a is 1, b is preferably 2.

$X^{152}$ and $X^{162}$ are $CH_2$ or O.

$R^{151}$ and $R^{161}$ are a $C_{14-30}$ hydrocarbon group, wherein one or more hydrogens are optionally replaced by fluorine. Preferably, $R^{151}$ and $R^{161}$ are a $C_{14-30}$ hydrocarbon group, optionally including one or more trans double bonds or one or more triple bonds. More preferably, $R^{151}$ and $R^{161}$ are a $C_{14-30}$ alkyl group.

Each $R^{152}$ and each $R^{162}$ are independently hydrogen, $CH_3$ or $CH_2CH_3$, preferably hydrogen.

The following moieties 1500a and 1600a are preferred examples of moieties **15** and **16** for the raftophile A':

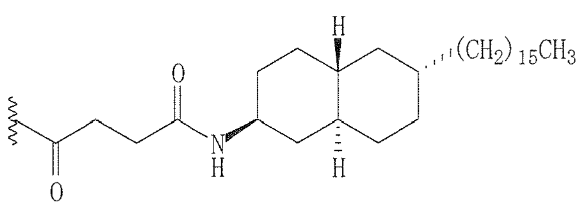

1500a

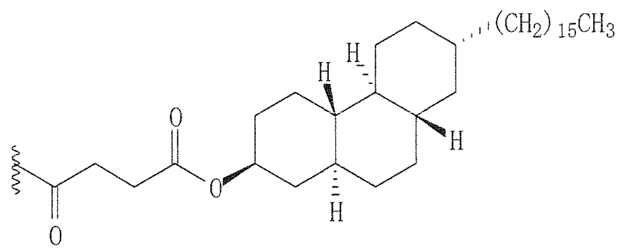

1600a

The moieties represented by the following formulae **18a** and **18b** are useful as the raftophile A or A' in the

present invention:

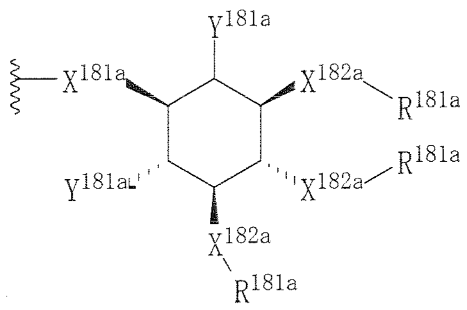

18a

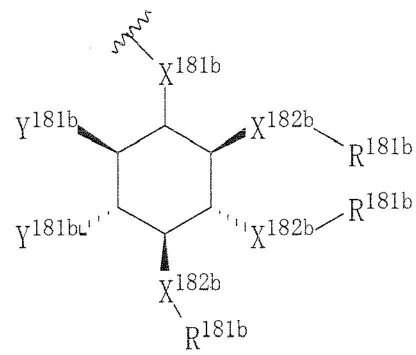

18b

When the tripartite structure is C-B-A, $X^{181a}$ and $X^{181b}$ are directionally selected from NH, O, $NH(CH_2)_cOP0_3^-$, $NH(CH_2)_cSO_2NH$, NHCONH and NHCOO, preferably O and $NH(CH_2)_eOPO_3^-$, wherein the linker is bonded to $X^{181a}$ or $X^{181b}$. c is an integer from 2 to 3, preferably 2. When the tripartite structure is C'-B'-A', $X^{181a}$ and $X^{181b}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$ $CO(CH_2)_bNHCONH$, $CO(CH_2)_bOCONH$, $CO(CH_2)_bOSO_3$ or $CO(CH_2)_bNHCO_2$, preferably $CO(CH_2)_b(CO)_aO$, wherein the linker is bonded to the terminal carbonyl group of $X^{181a}$ or $X^{181b}$. a is an integer from 0 to 1. b is an integer from 1 to 3. If a is 0, b is preferably 1. If a is 1, b is preferably 2.

Each $Y^{181a}$ and each $Y^{181b}$ is independently $NH_2$, $NHCH_3$, OH, H or halogen, preferably OH.

Each $X^{182a}$ and each $X^{182b}$ is independently O, NH, OCO or NHCO, preferably OCO.

Each $R^{181a}$ and each $R^{181b}$ is independently a $C_{15-30}$ hydrocarbon group, wherein one or more hydrogens are optionally replaced by fluorine. Preferably, each $R^{181a}$ and each $R^{181b}$ is independently a $C_{15-30}$ hydrocarbon group, optionally including one or more trans double bonds. More preferably, each $R^{181a}$ and each $R^{181b}$ is independently a $C_{15-24}$ alkyl group.

With regard to the side chains of moieties **18a** and **18b**, i.e. $R^{181a}$ and $R^{181b}$, it is preferred that these groups do not contain any double or triple bonds. Furthermore, it is preferred that these groups are linear, i.e. do not contain any branching. In a particularly preferred embodiment, the difference in the number of carbon atoms between each of the groups $R^{181a}$ or between each of the groups $R^{181b}$ is four or less, even more preferred two or less. These preferences are chosen in view of optimizing the geometrical conformation of the raftophile to fit into the overall structure of the raftophile. Saturated, linear side chains are considered to provide the highest degree of conformational flexibility in the side chains to facilitate incorporation into rafts. By choosing the difference in the number of carbon atoms in two side chains in one raftophilic moiety as small as possible, i.e. by avoiding an overall conical shape of the raftophile, a potential destabilizing effect of the raftophile on the raft assembly upon incorporation therein is believed to be minimized.

The following moieties **1800a** to **1800c** are preferred examples of moiety **18a** for the raftophile A':

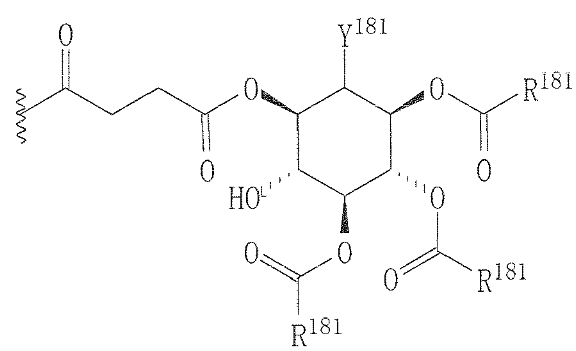

1800

| 1800 | $Y^{181}$ | $R^{181}$ |
|---|---|---|
| **a** | (S)-OH | $(CH_2)_{19}CH_3$ |
| **b** | (S)-OH | $(CH_2)_{23}CH_3$ |
| **c** | (R)-OH | $(CH_2)_{23}CH_3$ |

The following moiety **1800d** is a preferred example of moiety **18d** for the raftophile A':

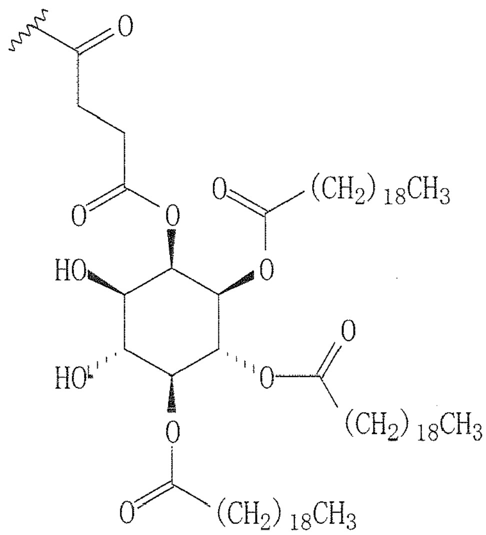

1800d

Moieties represented by the following formulae **19a** and **19b** are useful as the raftophile A or A' in the present invention:

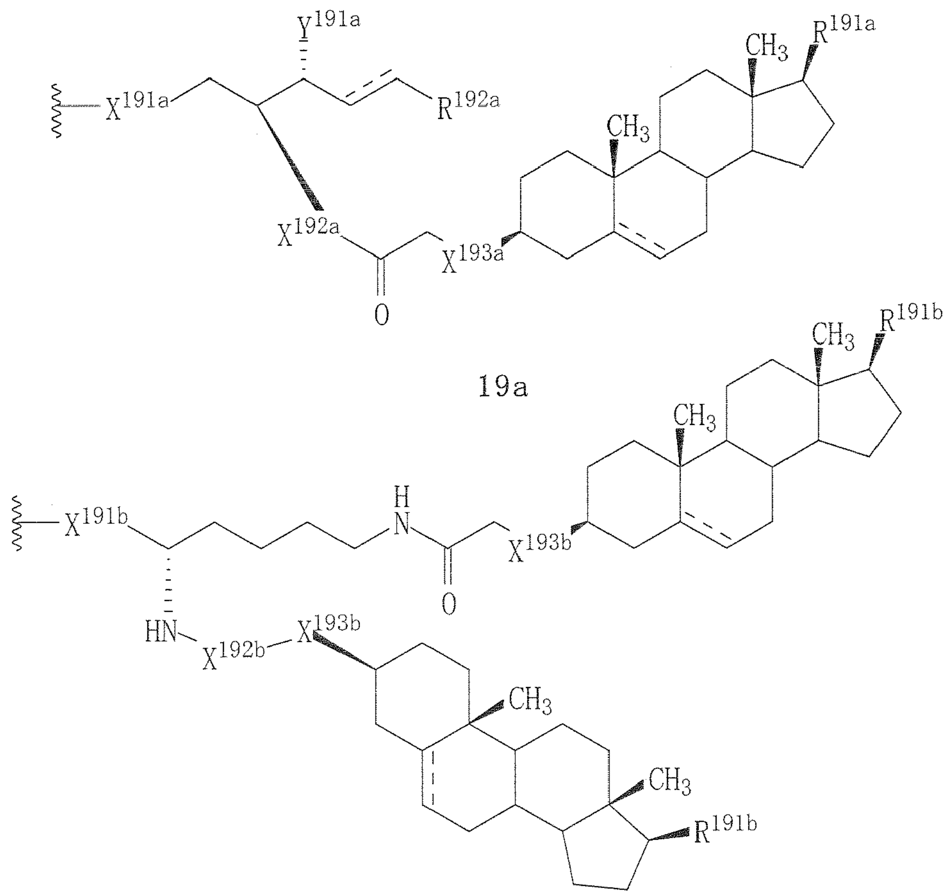

19a

19b

When the tripartite structure is C-B-A, $X^{191a}$ is directionally selected from NH, O, $NH(CH_2)_cOPO_3^-$, $NH(CH_2)$, $SO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, preferably O and NHCOO. wherein the linker is bonded to $X^{191a}$. In another preferred embodiment, $X^{191a}$ is $NHCH(CONH_2)(CH_2)_dCOO$. c is an integer from 2 to 3, preferably 2. d is an integer from 1 to 2, preferably 1. When the tripartite structure is C'-B'-A', $X^{191a}$ is $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bO$-CONH, $CO(CH_2)_bOSO_3$, $CO(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, preferably $CO(CH_2)_b(CO)_aO$, wherein the linker is bonded to the terminal carbonyl group of $X^{191a}$. a is an integer from 0 to 1. b is an integer from 1 to 3. If a is 0, b is preferably 1. If a is 1, b is preferably 2. e is an integer from 1 to 2, preferably 1.

When the tripartite structure is C-B-A, $X^{191b}$ is $NH(CH_2)$,NHCO, wherein the linker is bonded to the terminal amino group of $X^{191b}$. c is an integer from 2 to 3, preferably 2. When the tripartite structure is C'-B'-A', $X^{191b}$ is CO, wherein the linker is bonded to $X^{191b}$.

$X^{192a}$ is directionally selected from $NHCOCH_2NH$ or $NHCOCH_2OCH_2CH_2OCH_2CH_2NH$.

$X^{192b}$ is directionally selected from $COCH_2CH_2NHCOCH_2$ or $COCH_2$.

$X^{193a}$ and each $X^{193b}$ are independently directionally selected from O, NH, $C_{1-8}$ alkylene-O and $C_{1-8}$ alkylene-NH.

$Y^{191a}$ is $NH_2$, OH or H, preferably OH.

$R^{191a}$ and each $R^{191b}$ are independently a $C_{4-18}$ hydrocarbon group. Preferably, $R^{191a}$ and each $R^{191b}$ are independently a $C_{4-18}$ hydrocarbon group, optionally including one or more trans double bonds. More preferably, $R^{191a}$ and each $R^{191b}$ are independently a $C_{4-18}$ alkyl group. Most preferably, $R^{191a}$ and each $R^{191b}$ are the branched $C_8H_{17}$ alkyl group present in naturally occurring cholesterol.

$R^{192a}$ is a $C_{13-25}$ hydrocarbon group, wherein one or more hydrogens are optionally replaced by fluorine. Preferably, $R^{192a}$ is a $C_{13-25}$ hydrocarbon group, optionally including one or more trans double bonds. More preferably, $R^{192a}$ is a $C_{13-19}$ alkyl group.

In moiety **19a**, when ---, which is not part of the cyclic system, is a double bond, it can be either in the cis

configuration or in the trans configuration. In moiety **19a,** when ---. which is not part of the cyclic system is a double bond, it is preferably in the trans configuration.

In moieties **19a** and **19b**, ---, which is part of the cyclic system, is preferably a single bond. In this context, the same remarks that were made above with respect to moiety **2** apply.

The following moieties **1900a** and **1900b** are preferred examples of the moieties **19a** and **19b** for the raftophile A'.

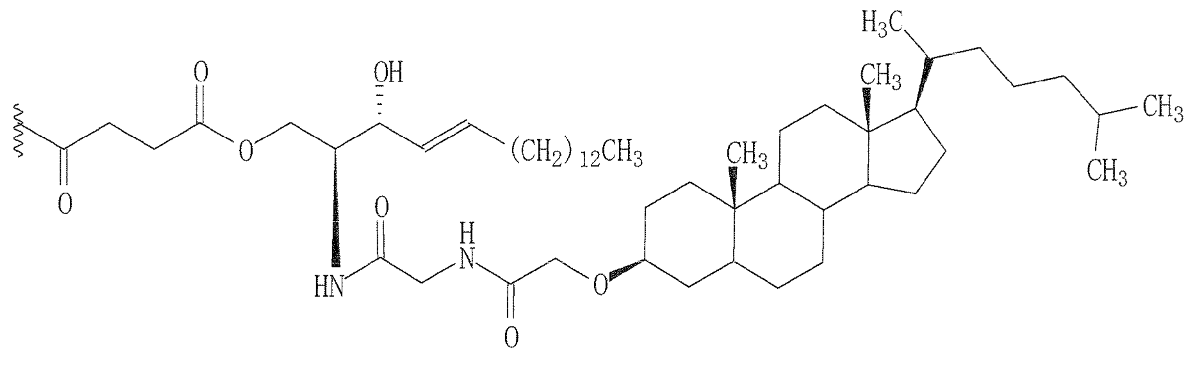

1900a

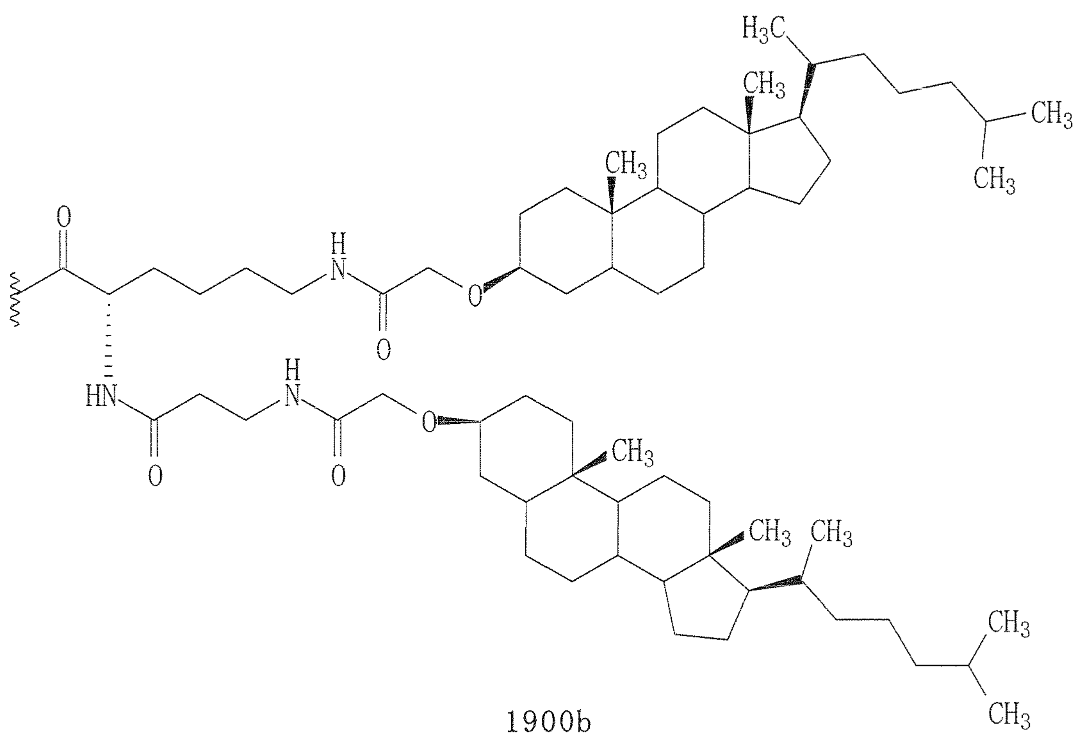

1900b

In the following the syntheses of precursors that when coupled to a linker yield the moieties that were described above as being useful as the raftophile A or A' in the compounds of the present invention will be described.

Syntheses of cholesteryl glycolic acid, 3-cholesterylamine, and cholesteryl glycine are described in the literature (S. L. Hussey, E. He, B. R. Peterson, J. Am. Chem. Soc. 2001, 123, 12712-12713; S. L. Hussey, E. He, B. R. Peterson, Org. Lett. 2002, 4, 415-418; S. E. Martin, B. R. Peterson, Bioconjugate Chem. 2003, 14, 67-74). Precursors of moiety **2** having an amide, sulfonamide, urea or carbamate function at position 3 of the steroid structure can be prepared from 3-cholesterylamine. For example, 3-cholesterylamine can be reacted with succinic anhydride in the presence of DMAP to afford the corresponding succinyl substituted compound. The corresponding sulfonamide can be obtained by reaction of 3-cholesterylamine with chlorosulfonylacetic acid, which can be prepared as described in the literature (R. L. Hinman, L. Locatell, J. Am. Chem. Soc. 1959, 81, 5655-5658). The corresponding urea or carbamate can be prepared according

to literature procedures via the corresponding isocyanate (H.-J. Knölker, T. Braxmeier, G. Schlechtingen, Angew. Chem. Int. Ed. 1995, 34, 2497; H.-J. Knölker, T. Braxmeier, G. Schlechtingen, Synlett 1996, 502; H.-J. Knölker, T. Braxmeier, Tetrahedron Lett. 1996, 37, 5861). Precursors of moiety **2** having a phosphate or carboxymethylated phosphate at position 3 of the steroid structure can be prepared as described in the literature (Golebriewski, Keyes, Cushman, Bioorg. Med. Chem. 1996, 4, 1637-1648; Cusinato, Habeler, et al., J. Lipid Res. 1998, 39, 1844-1851; Himber, Missano, et al., J. Lipid Res. 1995, 36, 1567-1585). Precursors of moiety **2** having a thiol at position 3 of the steroid structure can be prepared as described in the literature (J. G. Parkes, H. R. Watson, A. Joyce, R. Phadke, I.C. P. Smith, Biochim. Biophys. Acta 1982, 691, 24-29), the corresponding carboxymethylated thiols are obtainable by simple alkylation as described for the corresponding amines and alcohols. Precursors of moiety **2** having a difluoromethylenesulfone derivative at position 3 of the steroid structure can be prepared as described in the literature (J. Lapierre, V. Ahmed, M.-J. Chen, M. Ispahany, J. G. Guillemette, S. D. Taylor, Bioorg. Med. Chem. Lett. 2004, 14, 151-155). Introduction of various side chains at position 17 of precursors of moiety **2** can be achieved by use of literature protocols starting from dehydroisoandrosterone or pregnenolone (E. D. Bergmann, M. Rabinovitz, Z. H. Levinson, J. Am. Chem. Soc. 1959, 81, 1239-1243 and references therein). Precursors of moiety **2** which are derived from cholestane are obtainable from the corresponding precursors of moiety **2** which are derived from cholesterol by reduction of the 5,6-double bond using literature protocols, e.g. hydrogenation in the presence of various transition metal catalysts.

Precursors of moiety **3** having an oxygen derived substituent at position 3 are prepared in a similar manner as described for the precursors of moiety **2** starting from estrone. Precursors of moiety **3** having nitrogen derived substitution at position 3 can be prepared in a similar manner as described for precursors of moiety **2** starting from 3-amino estrone, which can be prepared as described in the literature (X. Zhang, Z. Sui, Tetrahedron Lett. 2003, 44, 3071-3073; L. W. L. Woo, M. Lightowler, A. Purohit, M. J. Reed, B. V. L. Potter, J. Steroid Biochem. Molec. Biol. 1996, 57, 79-88). Precursors of moiety **3** having a sulfur derived substituent at position 3 can be prepared in a similar manner as described for precursors of moiety **2** starting from 3-thioestrone, which can be prepared as described in the literature (L. W. L. Woo, M. Lightowler, A. Purohit, M. J. Reed, B. V. L. Potter, J. Steroid Biochem. Molec. Biol. 1996, 57, 79-88). Introduction of various side chains at position 17 of the estrone structure can be achieved by a Wittig approach, followed by hydrogenation of the resulting double bond as described in the literature (R. H. Peters, D. F. Crowe, M. A. Avery, W. K. M. Chong, M. Tanabe, J. Med. Chem. 1989, 32, 1642-1652; A. M. Krubiner, E. P. Oliveto, J. Org. Chem. 1966, 31, 24-26). Further manipulations within the side chain (e.g. double bond constructions, cycloalkyl decorations) can be achieved by standard protocols (Suzuki- couplings, etc.).

Precursors of moiety **4a** belonging to the class of ceramides, dehydroceramides and dihydroceramides with different hydrocarbon groups are obtainable as outlined in the literature (A. H. Merrill, Jr., Y. A. Hannun (Eds.), Methods in Enzymology, Vol. 311, Academic Press, 1999; P. M. Koskinen, A. M. P. Koskinen, Synthesis 1998, 1075). In particular, sphingosine base can be used as key intermediate for all precursors of moiety **4a** having oxygen derived substitution at position 1 of the sphingosine backbone. The corresponding amino derivatives are obtainable by substitution of the sulfonates, which can be prepared from the alcohols according to known protocols. Alkylation and acylation of 1-amino or 1-hydroxy derivatives can be achieved by reaction with bromo acetic acid and succinic anhydride, respectively. The thioacetylated derivative can be prepared by substitution of a sulfonate with mercapto acetic acid. Phosphate and sulfate derivatives are obtainable as described in the literature (A. H. Merrill, Jr., Y. A. Hannun (Eds.), Methods in Enzymology, Vol. 311, Academic Press, 1999; P. M. Koskinen, A. M. P. Koskinen, Synthesis 1998, 1075). Acylation, sulfonylation, urea and carbamate formation can be achieved by standard procedures. Precursors of moiety **4a** wherein $X^{42a}$ is an amino or amino derived function can be prepared starting from sphingosine base, which is available as published by Koskinen (P. M. Koskinen, A. M. P. Koskinen, Synthesis 1998, 1075), using standard protocols. The corresponding 2-oxygen substituted sphingolipids can be obtained by a strategy published by Yamanoi (T. Yamanoi, et al., Chem. Lett. 1989, 335). Precursors of moiety **4a**, wherein both $Y^{42a}$ represent a hydroxy group, are obtainable by bishydroxylation of the corresponding alkene using known protocols. The corresponding monohydroxy derivatives can be prepared as described in the literature (A. R. Howell, A. J. Ndakala, Curr. Org. Chem. 2002, 6, 365-391). Precursors of moiety **4a** having a triple bond incorporated at position 4 of the sphingosine backbone are obtainable as described in the literature (P. Garner, et al., J. Org. Chem. 1988, 53, 4395; P. Herold, Helv. Chim. Acta 1988, 74, 354; H.-E. Radunz, et al., Liebigs Ann. Chem. 1988, 1103). Modification of substituents $R^{41a}$ and $R^{42a}$ in precursors of moiety **4a** can be achieved by protocols and strategies outlined in various review articles (H. J. Harwood, Chem. Rev. 1962, 62, 99-154; W. J. Gensler, Chem. Rev. 1957, 57, 191-280).

Precursors of moiety **4b** are obtainable by protocols described in the literature (S. Müller, et al., J. Prakt. Chem. 2000, 342, 779) and by combinations thereof with protocols described for the preparation of precursors of moiety **4a**.

Precursors of moiety **5a**, wherein $X^{51a}$ and $X^{52a}$ are oxygen derived substituents, can be prepared starting from commercially available (*R*)-(-)-2,2-dimethyl-1,3-dioxolane-4-methanol as outlined by Fraser-Reid (U. Schlueter, J. Lu, B. Fraser-Reid, Org. Lett. 2003, 5, 255-257). Variation of substituents $R^{52a}$ in compounds **5a** can be achieved by protocols and strategies outlined in various review articles (H. J. Harwood, Chem. Rev. 1962, 62, 99-154; W. J. Gensler, Chem. Rev. 1957, 57, 191-280). Precursors of moiety **5a,** wherein $X^{51a}$ and $X^{52a}$ are nitrogen derived substituents, are

obtainable either starting from the corresponding oxygen substituted systems by nucleophilic replacement of the corresponding sulfonates and further modifications as outlined above, or starting from 1,2,3-triaminopropane which is obtainable as described in the literature (K. Henrick, M. McPartlin, S. Munjoma, P. G. Owston, R. Peters, S. A. Sangokoya, P. A. Tasker, J. Chem. Soc. Dalton Trans. 1982, 225-227).

Precursors of moiety **5b** are obtainable in a similar fashion as precursors of moiety **4b** or alternatively by ring closing metathesis of ω-ethenylated precursors of moiety **5a**.

Precursors of moieties **6** and **7** are obtainable by synthetic strategies described in the literature (J. Xue, Z. Guo, Bioorg. Med. Chem. Lett. 2002, 12, 2015-2018; J. Xue, Z. Guo, J. Am. Chem. Soc. 2003, 16334-16339; J. Xue, N. Shao, Z. Guo, J. Org. Chem. 2003, 68, 4020-4029; N. Shao, J. Xue, Z. Guo, Angew. Chem. Int. Ed. 2004, 43, 1569-1573) and by combinations thereof with methods described above for the preparation of precursors of moieties **4a** and **5a**.

Precursors of moieties **8a**, **8b** and **10** are obtainable by total synthesis following synthetic strategies described in the literature (H.-J. Knölker, Chem. Soc. Rev. 1999, 28, 151-157; H.-J. Knölker, K. R. Reddy, Chem. Rev. 2002, 102, 4303-4427; H.-J. Knölker, J. Knöll, Chem. Commun. 2003, 1170-1171; H.-J. Knölker, Curr. Org. Synthesis 2004, 1, in preparation).

Precursors of moiety **9** can be prepared by Nenitzescu-type indole synthesis starting from 4-methoxy-3-methylbenzaldehyde to afford 6-methoxy-5-methylindole. Ether cleavage, triflate formation and Sonogashira coupling leads to the corresponding 6-alkynyl substituted 5-methylindole. Vilsmeier formylation and subsequent nitromethane addition yields the 3-nitrovinyl substituted indole derivative which is subjected to a global hydrogenation resulting in the formation of the 6-alkyl substituted 5-methyltryptamine. Acylation of the amino group using succinyl anhydride completes the preparation.

Precursors of moiety **11** can be prepared in analogy to reported structures in the literature (N. K. Djedovic, R. Ferdani, P. H. Schlesinger, G. W. Gokel, Tetrahedron 2002, 58, 10263-10268).

Precursors of moiety **12** can be prepared by known guanidine formation via the corresponding thiourea followed by simple alkylation or acylation.

Precursors of moiety **13a** can be prepared in a similar manner as published by Grinstaff (G. S. Hird, T. J. McIntosh, M. W. Grinstaff, J. Am. Chem. Soc. 2000, 122, 8097-8098) starting from the corresponding ribose, or azaribose derivative, respectively.

Precursors of moiety **13b** can be prepared starting from cyclopentadiene. Monoepoxidation followed by treatment with lithium aluminium hydride yields 3-cyclopentene-1-ol which is silyl protected. Bishydroxylation gives the corresponding diol which is then acylated using fatty acids. After desilylation the hydroxy function is either alkylated or acylated.

Precursors of moiety **14a** can be prepared from the corresponding commercially available bromo- and nitro-substituted naphthalenes by palladium mediated couplings to introduce alkyl substituted alkynes. Subsequent reduction of both nitro to amino functions and alkyne to alkyl groups followed by either acylation of the amino group with succinyl anhydride or alkylation with bromoacetic acid results in the desired compound.

Precursors of moiety **15** can be prepared in a similar way as described in the literature (J. G. Witteveen, A. J. A. Van der Weerdt, Rec. Trav. Chim. Pays-Bas 1987, 106, 29-34).

Precursors of moiety **14b** can be prepared starting from 2,7-phenanthrenediol which is synthesized as described in literature (M. S. Newman, R. L. Childers, J. Org. Chem 1967, 32, 62-66), by monoprotection and subsequent triflate formation followed by Sonogashira coupling, reduction of the alkyne to alkyl, deprotection and acylation or alkylation, respectively.

Precursors of moiety **16** can be prepared in a similar manner as described in the literature (W. Sucrow, H. Minas, H. Stegemeyer, P. Geschwinder, H. R. Murawski, C. Krueger, Chem. Ber. 1985, 118, 3332-3349; H. Minas, H. R. Murawski, H. Stegemeyer, W. Sucrow, J. Chem. Soc. Chem. Commun. 1982, 308-309).

Precursors of moiety **18** can be prepared starting from *myo*-or *scyllo*-inositol by combination of protocols outlined in the literature (N. Shao, J. Xue, Z. Guo, Angew. Chem. Int. Ed. 2004, 43, 1569-1573, and references cited therein; D.-S. Wang, C.-S. Chen, J. Org. Chem. 1996, 61, 5905-5910, and references cited therein).

Precursors of moiety **19a** can be prepared in a similar fashion as described for precursors of moiety **4a,** with the free amino function of sphingosine base being acylated either with glycine or 2-(2-aminoethoxy)ethoxy acetic acid followed by acylation of the free *N*-terminus with a corresponding cholesteryl or cholestanyl derivative, which can be prepared as described above.

Precursors of moiety **19b** can be prepared by acylation of the ε-amino function with cholesteryl or cholestanyl derivatives, the preparation of which is described above, and acylation of the α-amino function with either cholesteryl- or cholestanyl derivatives or with β-alanine followed by acylation of the *N*-terminus with cholesteryl or cholestanyl derivatives.

A moiety represented by the following formula **20** is useful as the linker B or B' in the present invention:

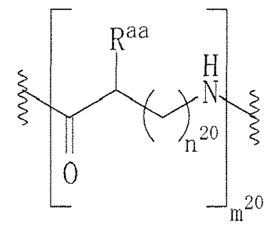

20

$m^{20}$ is an integer from 3 to 80, preferably from 5 to 80, more preferably from 5 to 40, most preferably from 5 to 20. Each $n^{20}$ is independently an integer from 0 to 1, more preferably 0. Each $R^{aa}$ is independently any of the side chains of naturally occurring amino acids, optionally substituted with a dye label which is preferably a fluorescent dye label. The dye label may be rhodamine, Mca, fluoresceine or synthetically modified derivatives thereof. The C-terminus is bonded to the raftophile A and the N-terminus is bonded to the pharmacophore C in the tripartite structure C-B-A. The N-terminus is bonded to the raftophile A' and the C-terminus is bonded to the pharmacophore C' in the tripartite structure C'-B'-A'.

The following moiety **2000** is an example of moiety **20** for the linkers B and B':

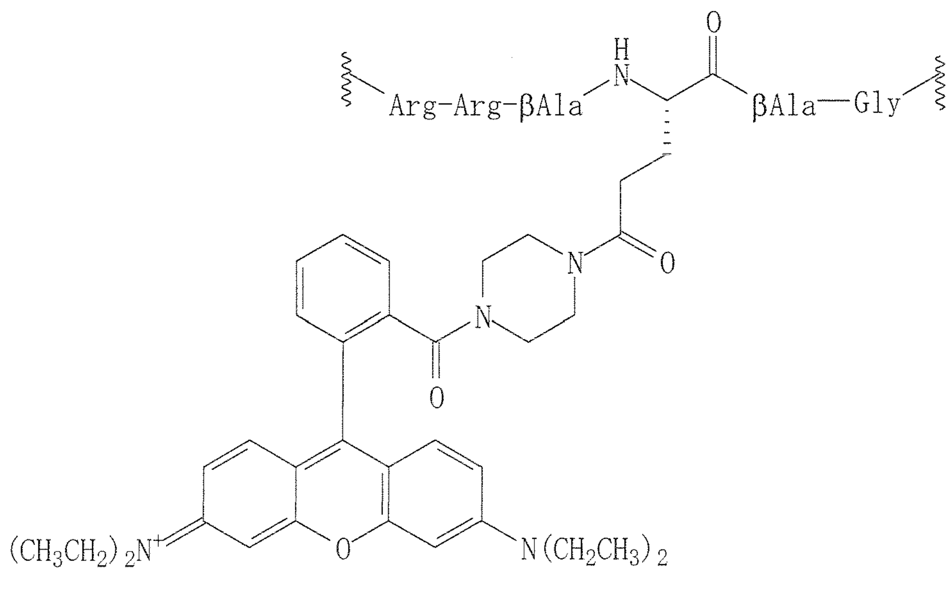

2000

The following moiety **2001** is a preferred example of moiety **20** for the linkers B and B'. Linker **2001** is particularly suitable for a compound comprising a tripartite structure for the inhibition of the BACE-1 beta-secretase protein.

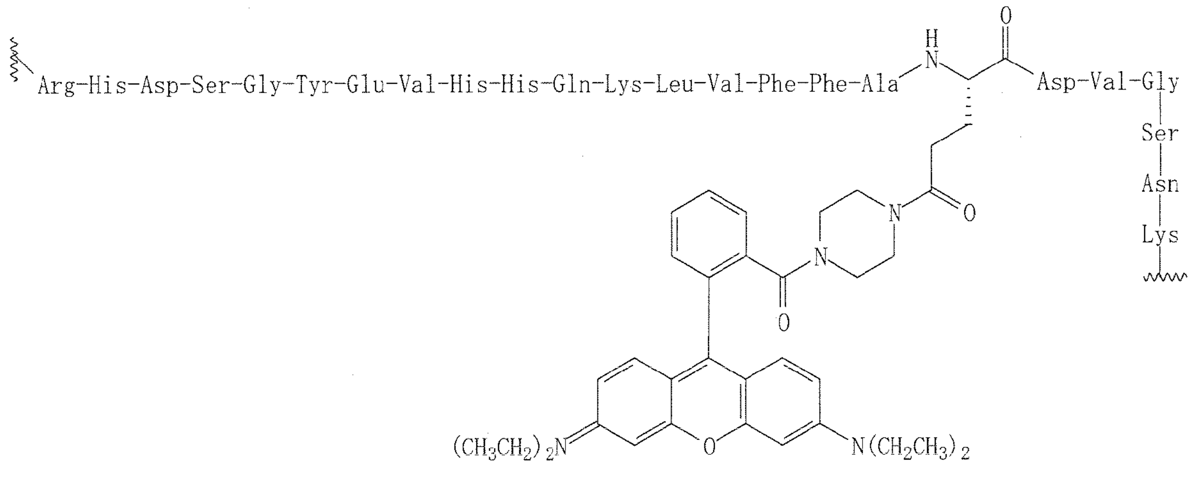

2001

Moieties represented by the following formula **21** are useful as the linker B or B' in the present invention:

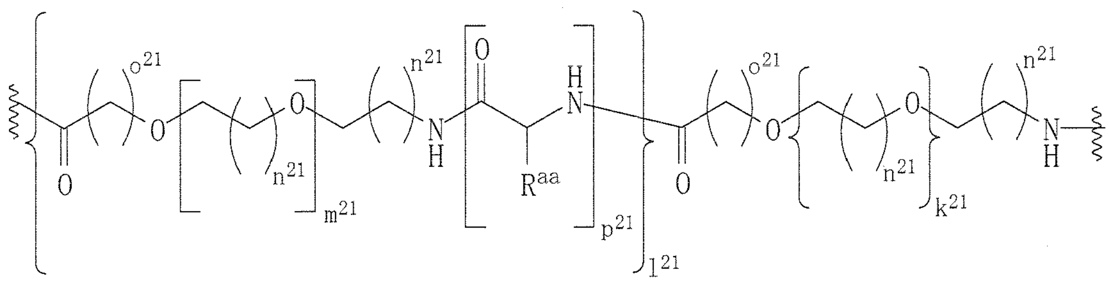

21

Each $n^{21}$ is independently an integer from 1 to 2, preferably I. Each $o^{21}$ is independently an integer from 1 to 3, preferably 1 to 2, more preferably 1. Each $p^{21}$ is independently an integer from 0 to 1. $k^{21}$ and each $m^{21}$ are independently integers from 0 to 5, preferably 1 to 4, more preferably 1 to 3. $1^{21}$ is an integer from 0 to 10, preferably 1 to 5, more preferably 2 to 3, provided that the sum of $k^{21}$ and $l^{21}$ is at least 1. Each $R^{aa}$ is independently any of the side chains of naturally occurring amino acids, optionally substituted with a dye label which is preferably a fluorescent dye label. The dye label may be rhodamine, Mca, fluoresceine or synthetically modified derivatives thereof. The C-terminus is bonded to the raftophile A and the N-terminus is bonded to the pharmacophore C in the tripartite structure C-B-A. The N-terminus is bonded to the raftophile A' and the C-terminus is bonded to the pharmacophore C' in the tripartite structure C'-B'-A'.

Preferred examples of moiety **21** for the linkers B and B' contain polyglycols units i.e. each $n^{21}$ is 1.

The following moiety **2100** is a preferred example of moiety **21** for the linkers B and B':

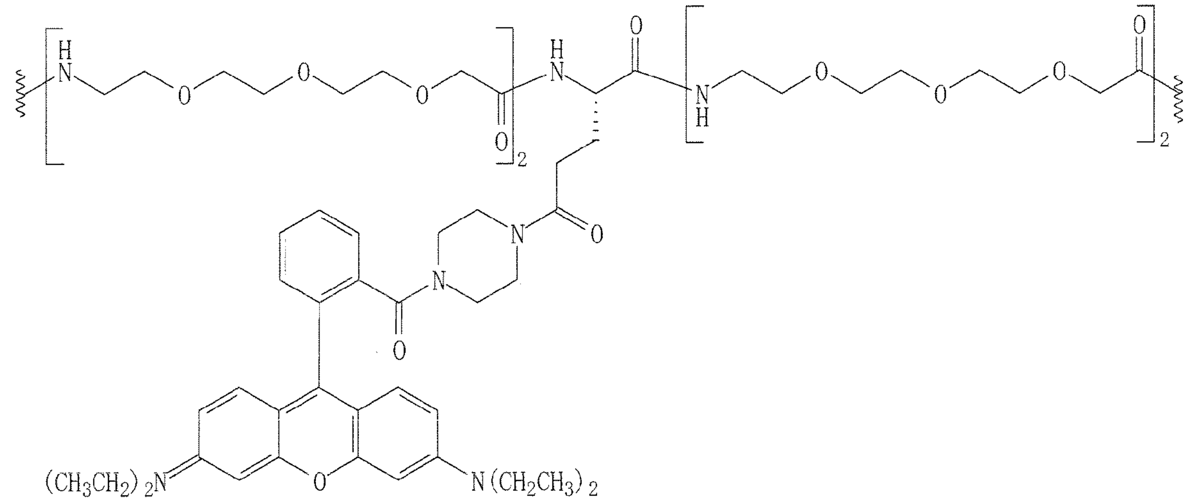

2100

In particularly preferred examples of moiety **21** for the linkers B and B' each or any, preferably each, $n^{21}$ is 1, each or any, preferably each, $o^{21}$ is 2 and each or any, preferably each, $p^{21}$ is 0. One example of a moiety of this type is the following moiety **2001**:

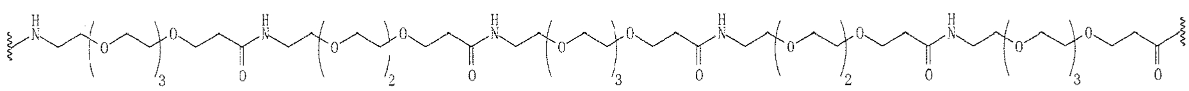

2101

The usefulness of a moiety having formula **2101** as linker B is demonstrated in the appended examples.

Moieties represented by the following formula **22** are useful as the linker B or B' in the present invention:

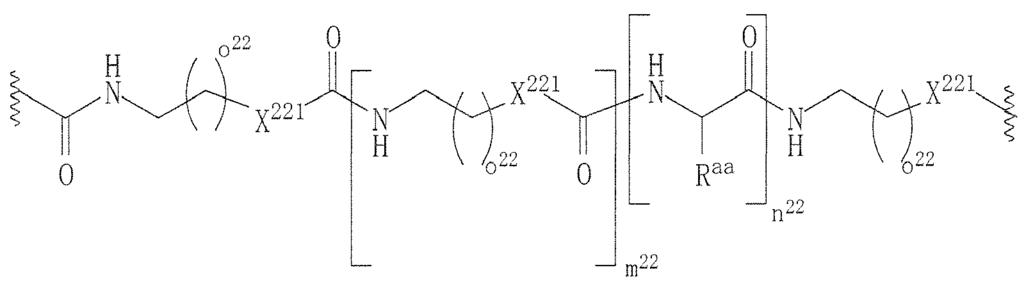

22

$m^{22}$ is an integer from 0 to 40, preferably 2 to 30, more preferably 4 to 20. $n^{22}$ is an integer from 0 to 1. Each $o^{22}$ is independently an integer from 1 to 5, preferably 1 to 3. Each $X^{221}$ is independently NH or O. Each $R^{aa}$ is independently any of the side chains or naturally occurring amino acids, optionally substituted with a dye label which is preferably a fluorescent dye label. The dye label may be rhodamine, Mca, fluoresceine or synthetically modified derivatives thereof. The C-terminus is bonded to the raftophile A and the $X^{221}$- terminus is bonded to the pharmacophore C in the tripartite structure C-B-A. The $X^{221}$- terminus is bonded to the raftophile A' and the C-terminus is bonded to the pharmacophore C' in the tripartite structure C'-B'-A'.

Moieties represented by the following formula **23** are useful as the linker B or B' in the present invention:

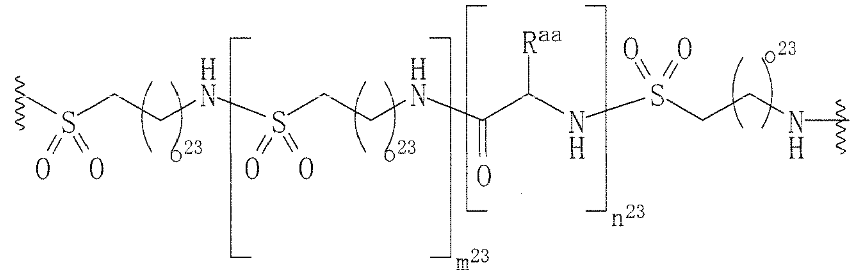

23

$m^{23}$ is an integer from 0 to 40, preferably 2 to 30, more preferably 4 to 20. $n^{23}$ is an integer from 0 to 1. Each $o^{23}$ is independently an integer from 1 to 5, preferably 1 to 3. Each $R^{aa}$ is independently any of the side chains of naturally occurring amino acids, optionally substituted with a dye label which is preferably a fluorescent dye label. The dye label may be rhodamine, Mca, fluoresceine or synthetically modified derivatives thereof. The $SO_2$-terminus is bonded to the raftophile A and the N-terminus is bonded to the pharmacophore C in the tripartite structure C-B-A. The N-terminus is bonded to the raftophile A' and the $SO_2$-terminus is bonded to the pharmacophore C' in the tripartite structure C'-B'-A'.

Of the various moieties that can be employed as linker B and B', moieties represented by the general formula **21** are preferred. Moieties containing polyglycol units, for example moieties represented by general formula **21**, wherein each $n^{21}$ is 1, each $o^{21}$ is 2 and each $p^{21}$ is 0, are particularly preferred.

As pointed out above, the pharmacophore comprised in the tripartite structured compound of the invention is a molecule, preferably a small molecule which comprises a specificity to a binding or interaction site (like an enzyme, active site, a protein-protein interaction site, a receptor-ligand interaction site or, inter alia, a viral bacterial or parasitic attachment site). Accordingly, most preferably, said pharmacophore is a molecule capable of interacting with the before mentioned biological systems and is capable of interfering with said systems, e.g. with the interaction of signalling molecules or receptor-ligand-interactions (like, e.g. EGF-receptors and their corresponding ligands).

Therefore, the pharmacophore "C" or "C'" comprised in the tripartite structured compound of the present invention may be selected from the group consisting of an enzyme, an enzyme inhibitor, a receptor inhibitor, an antibody or a fragment or a derivative thereof, an aptamer, a peptide, a fusion protein, a small molecule inhibitor, a heterocyclic or carbocyclic compound, and a nucleoside derivative.

As discussed above, "moiety C" and "moiety C' " of the tripartite structured compound of the invention may also be an antibody or a fragment or derivative thereof. For example, the well-known anti-HER2 (Herceptin) (or a functional fragment or derivatives thereof) antibody employed in the management of breast cancer may be employed. The term "antibody" also comprises derivatives or fragments thereof which still retain the binding specificity. These are considered as "functional fragments or derivatives". Techniques for the production of antibodies are well known in the art and described, e.g. in Harlow and Lane "Antibodies, A Laboratory Manual", CSH Press, Cold Spring Harbor, 1988.

The present invention, accordingly, includes compounds comprising, as "moiety C/C' " chimeric, single chain and humanized antibodies, as well as antibody fragments, like, inter alia, Fab fragments. Antibody fragments or derivatives further comprise F(ab')2, Fv or scFv fragments; see, for example, Harlow and Lane, loc. cit.. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies to polypeptide(s) of this invention. Also, transgenic animals may be used to express humanized antibodies to polypeptides of this invention. Most preferably, the antibody useful in context of this invention is a monoclonal antibody. For the preparation of monoclonal antibodies, any technique which provides antibodies produced by continuous cell line cultures can be used. Examples for such techniques include the hybridoma technique, the trioma technique, the human B-cell hybridoma technique and the EBV-hybridoma technique to produce human monoclonal antibodies. Techniques describing the production of single chain antibodies (e.g., US Patent 4,946,778) can be adapted to produce single chain antibodies to immunogenic polypeptides as described above. It is in particular preferred that the antibodies/antibody constructs as well as antibody fragments or derivatives to be employed in accordance with this invention are capable to be expressed in a cell. This may, inter alia, be achieved by direct injection of the corresponding proteineous molecules or by injection of nucleic acid molecules encoding the same. In context of the present invention, the term "antibody molecule" comprised as "Moiety C/C' " in the tripartite construct also relates to full immunoglobulin molecules as well as to parts of such immunoglobulin molecules. Furthermore, the term relates, as discussed above, to modified and/or altered antibody molecules, like chimeric and humanized antibodies. The term also relates to monoclonal or polyclonal antibodies as well as to recombinantly or

synthetically generated/synthesized antibodies. The term also relates to intact antibodies as well as to antibody fragments thereof, like, separated light and heavy chains, Fab, Fab/c, Fv, Fab', F(ab')2. The term "antibody molecule" also comprises bifunctional antibodies and antibody constructs, like single chain Fvs (scFv) or antibody-fusion proteins.

Also aptamers or aptamer-parts are considered as pharmacophores to be comprised in the inventive compounds. In accordance with the present invention, the term "aptamer" means nucleic acid molecules that can bind to target molecules. Aptamers commonly comprise RNA, single stranded DNA, modified RNA or modified DNA molecules. The preparation of aptamers is well known in the art and may involve, inter alia, the use of combinatorial RNA libraries to identify binding sides (Gold, Ann. Rev. Biochem. 64 (1995), 763-797). An example of an aptamer to be used in the tripartite structural compound of the invention is given herein and comprises the aptamer A30 as discussed below.

Said pharmacophore "C" and "C'" may also be an enzyme inhibitor. Most preferably, and as documented herein, said enzyme inhibitor is beta-secretase inhibitor III.

As pointed out above, the pharmacophore C/C' may be a receptor inhibitor, for example an receptor inhibitor which interferes with the interaction of the receptor with its corresponding ligand. Such a receptor inhibitor may be EGF receptor inhibitor Herstatin (Azios, Oncogene, 20, (2001) 5199-5209) or aptamer A30 (Chen, Proc. Natl. Acad. Sci. USA, 100 (2003) 9226-9231).

In a preferred embodiment, the pharmacophore C/C' comprised in the inventive compound is an antiviral agent. Preferred the antiviral agents are known in the art and comprise, but are not limited to, Zanamivir (2,4-dideoxy-2,3-didehydro-4-guanidinosialic acid; von Itzstein M. Nature. (1993) 363, 418-23; Woods JM. Antimicrob Agents Chemother. (1993) 37, 1473-9.) or Oseltamivir (ethyl(3R,4R,5S)-4-acetoamido-5-amino-3-(1-ethylpropoxy)-1-cyclohexene-1-carboxylate; Eisenberg EJ. Antimicrob Agents Chemother. (1997) 41, 1949-52; Kati WM. Biochem Biophys Res Commun. (1998) 244, 408-13.). These compounds are particularly useful in the treatment or alleviation of an influenza infection. Also preferred are the influenza virus binding agents, RWJ-270201 (Peramivir), BCX-1812, BCX-1827, BCX-1898, and BCX-1923 (Babu YS, J Med Chem. (2000) 43, 3482-6; Smee DF. Antimicrob Agents Chemother. (2001) 45, 743-8.), Norakin (1-tricyclo-(2,2,1,0)-heptyl-(2)-1-phenyl-3-piperidine-propanol; triperiden), Akineton (alpha-5-norbornen-2-yl-alpha-phenyl-1-piperidine propanol; biperiden), Antiparkin (ethylbenzhydramin) or Parkopan (trihexyphenidyl). The antiviral agent may also be selected from the group consisting of Fuzeon (Hartt JK. Biochem Biophys Res Commun. (2000) 272, 699-704; Tremblay CL. J Acquir Immune Defic Syndr. (2000) 25, 99-102.), T1249 (a 39-mer peptide; Trimeris Inc.), coselane, AMD3100, AMD070, SCH351125, AD101 (all bicyclams; De Clercq. E Antimicrob Agents Chemother. (1994) 38, 668-74 ; Palani A. J Med Chem. (2001) 44, 3339-42.). Also envisaged in this context are cyclopentane neuraminidase-inhibitors as pharmacophores; see, inter alia, Smee, Antimicrob. Agents Chemother. 45 (2001), 743-748. These corresponding tripartite compounds may be employed in the management of HIV-infections and AIDS.

Also to be employed as a pharmacophore C/C' are anilino-naphtalene compounds, like ANS, AmNS, or bis-ANS. The corresponding inventive tripartite compounds are particularly useful in the treatment or prevention of PvP-related diseases, like transmissible spongiform encephalopathies. ANS, AmNS and bis-ANS are defined herein below in context of their medical use in prion-related disorders.

Accordingly, the compounds of the present invention, i.e. the tripartite structure compound described herein, are particularly useful in medical settings which comprise not only their use as pharmaceuticals but also their use as comparative test substances. For example, as pointed out herein, tripartite structured compounds, like the compound shown in formula **24** may comprise additional functional parts or structures, like labeled structures. The corresponding compound may be employed in the raftophilicity assay as described herein and may be used in comparative as well as non-comparative test settings. However, the most important use of the compounds provided herein is their use as pharmaceuticals. Accordingly, the present invention also relates to a pharmaceutical composition comprising any of the tripartite structured compounds described herein.

The compounds of the present invention may be administered as compounds per se or may be formulated as pharmaceutical compositions, optionally comprising pharmaceutically acceptable excipients, such as carriers, diluents, fillers, desintegrants, lubricating agents, binders, colorants, pigments, stabilizers, preservatives or antioxidants.

The pharmaceutical compositions can be formulated by techniques known to the person skilled in the art, such as the techniques published in Remington's Pharmaceutical Sciences, 20th Edition. The pharmaceutical compositions can be formulated as dosage forms for oral, parenteral, such as intramuscular, intravenous, subcutaneous, intraarterial, rectal, nasal, topical or vaginal administration. Dosage forms for oral administration include coated and uncoated tablets, soft gelatine capsules, hard gelatine capsules, lozenges, troches, solutions, emulsions, suspensions, syrups, elixiers, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insuflation, for example by a metered inhaler. Dosage forms for topical administration include cremes, gels, ointments, salves, patches and transdermal delivery systems.

The present invention also provides for a method of treatment, amelioration or prevention of disorders or

diseases which are due to (or which are linked to) biochemical and/or biophysiological processes which take place on or within raft structures of a mammalian cell. In a most preferred setting, the compounds of the present invention are used in these treatment methods by administration of said compounds to a subject in need of such treatment, in particular a human subject.

The tripartite structured compounds of the present invention are particularly useful in medical settings since besides lipids clustering, several specific cellular proteins partition into the liquid-ordered raft phase (Simons, Annu. Rev. Biophys. Biomol. Struct. 33 (2004), 269-295). For example, GPI-anchored proteins are commonly used as markers of lipid rafts whereas Transferrin Receptor is typically excluded from rafts and marks the liquid disordered phase (Harder, J. Cell Biol. 141 (1998), 929-942). Such partitioning can also be modulated, thereby regulating the activity and complex formation of raft proteins (Harder, Curr. Opin. Cell Biol. 9 (1997), 534-542). For example, H-Ras resides in the inactive state in rafts and functions in signaling upon exit from these microdomains. By contrast, APP processing by β-secretase requires partitioning into rafts (see below). The importance of lipid rafts in membrane compartmentalization and cell physiology is underscored by their involvement in pathological processes. Some examples of the role of lipid rafts and their modulation in key human diseases are given below.

Alzheimer disease (AD) depends on the formation of senile plaques containing the amyloid-β-peptide (Aβ), a fragment derived from the large type I transmembrane protein APP, the amyloid precursor protein (London, Curr. Opin. Struct. Biol. 12 (2002), 480-486). The Aβ fragment is cleaved sequentially by enzymes termed β-secretase (BACE) and beta-secretase. BACE is an aspartyl-protease that cleaves APP in its luminal domain, generating a secreted ectodomain. The resulting 10-kDa C-terminal fragment is subsequently cleaved by beta-secretase, which acts at the transmembrane domain of APP, thus releasing Aβ. A third enzymatic activity, the beta-secretase, counteracts the activity of BACE by cleaving APP in the middle of the Aβ region, yielding products that are non-amyloidogenic: The beta fragment (a secreted ectodomain) and the short C-terminal stub that is also cleaved by beta-secretase. Therefore, beta-cleavage directly competes with beta-cleavage for their common substrate APP.

Lipid rafts play a role in regulating the access of beta- and beta-secretase to the substrate APP. Cholesterol depletion inhibits beta-cleavage and Aβ formation in neurons and other cells, resulting in a higher proportion of beta-cleavage (London, Biochim. Biophys. Acta 1508 (2000), 182-195). APP and BACE co-patch with one another following antibody cross-linking within lipid rafts (Ehehalt, J. Cell Biol. 160 (2003), 113-123). A fraction of APP and BACE is found in DRMs, a biochemical hallmark of localization to lipid rafts (Simons, Proc. Natl. Acad. Sci. USA 95 (1998), 6460-6464; Riddell, Curr. Biol. 11 (2001) 1288-1293). Aβ production is strongly stimulated upon rafts clustering that brings together surface rafts containing APP and BACE (Ehehalt, (2003), loc. cit.). In demonstrating a causal relationship between raft partitioning and Aβ production, these data provide the means of 1) interfering with the partitioning of APP and BACE in rafts, 2) their intracellular trafficking to meet within the same rafts and 3) the activity of BACE in rafts, to inhibit Aβ fragment production and generation of Alzheimer disease. A corresponding preferred construct for the intervention in Alzheimer's disease is provided herein; see, for example, formulae **24** and **25,** as well as **25b,** a particularly preferred embodiment of the invention. It is also envisaged that corresponding compounds may be employed in the treatment of Down's syndrome.

Also infectious diseases may be treated or even prevented by the use of the tripartite structured compounds provided herein. These comprise but are not limited to infection by measles virus, respiratory syncytial cell virus, Ebolavirus, Marburgvirus, Ebstein-Barr virus, echovirus 1, papillomaviruses (e.g. simian virus 40), polyomaviruses or bacterial infections, like mycobacterial infection, inter alia infections with M. tuberculosis, M. kansaii or M. bovis. Also infection by Escherichia coli, Campylobacter jejuni, Vibrio cholerae, Clostridium difficile, Clostridium tetani, Salmonella or Shigella is envisaged to be treated or prevented by compounds as provided herein. Several viruses and bacteria employ lipid rafts to infect host cells. The above mentioned pathogens and specific examples given below are linked by their requirement of rafts during their infection cycle.

A first example of a virus to be characterized with respect to rafts requirement was influenza virus (Ipsen, (1987), loc. cit.). Rafts play a role in the virus assembly process. The virus contains two integral glycoproteins, hemagglutinin and neuraminidase. Both glycoproteins are raft-associated as judged by cholesterol-dependent detergent resistance (Ipsen, (1987), loc. cit.). Influenza virus buds out from the apical membrane of epithelial cells, which is enriched in raft lipids. Influenza virus preferentially includes raft lipids in its envelope during budding through polymerization of M proteins that drives raft clustering (Ipsen, (1987), loc. cit.).

The herein described tripartite compounds provide a medical tool for the intervention in influenza infections. Specific corresponding pharmacophores were given herein above.

Rafts are also implicated in four key events the HIV life cycle. 1) Passage across the host's mucosa. HIV binds to the glycosphingolipid galactosylceramide at the apical surface of mucosal epithelial cells and then transcytoses across the epithelium to be released on the basolateral side. Disrupting raft association blocks viral transcytosis (Israelachvili, Biochim. Biophys. Acta 469 (1977), 221-225). 2) Viral entry into immune cells. During infection of target cells, the viral envelope components, as well as the internal Gag protein (which is essential for assembly of the viral envelope; (Jacobson (1992), loc. cit.) are all initially associated with rafts, as evidenced by partitioning into DRMs. Indeed, viral glycoproteins

can co-patch with known raft-associated proteins on the surface of living cells after cross-linking with specific antibodies (Jain (1977), loc. cit.). Interestingly, the virus receptors on the host cell surface are also raft-associated. The HIV glycoprotein gp120 co-patches with the cell surface receptor CD4 and with the co-receptors, the chemokine receptors CCR5 and CXLR4. CD4, CCR5, and CXLR4 are found in DRMs. Binding of the virus to its surface receptors, first to CD4 and then to the chemokine receptor, seems to lead to raft clustering and lateral assembly of a protein complex in the membrane to initiate fusion of the virus envelope with the cell membrane. Both cholesterol and specific glycosphingolipid species serve as crucial elements in organizing the fusion complex (Jorgensen, J. Phys. Chem. 104 (2000), 11763-11773; Keller, Phys. Rev. Lett. 81 (1998), 5019-5022). 3) Alteration of signaling in host cells. HIV prepares the host cell by changing the cellular state of signaling. Nef, an early HIV gene product, promotes infectivity of the virus via lipid rafts (Kenworthy, Mol. Biol. Cell 11 (2600), 1645-1644); infection with HIV-1 virions lacking Nef does not progress to AIDS (Kholodenko, Trends Cell Biol. 10 (2000), 173-178). The Nef protein is a peripheral, myristoylated membrane protein with a proline-rich repeat that can bind to raft-associated nonreceptor tyrosine kinases of the Src family. It associates with DRMs and seems to prime the host cells for HIV infection by lowering the threshold necessary for T cell activation (Kenworthy (2000), loc. cit.). Resting T cells do not support a productive HIV infection, but Nef activates T cells by increasing IL-2 secretion and obviates the need for costimulatory signals. By clustering lipid rafts carrying relevant host cell surface proteins, Nef oligomerization may aid in organizing the T cell signaling complex and the HIV budding site (Kenworthy (2000), loc. cit.; Kurzchalia, Curr. Opin. Cell Biol. 11 (1999), 424-431;). 4) Viral exit from cells and dispersion through the host's vascular system. HIV exit from the cell, another raft-dependent step, depends critically on the viral Gag protein (Jorgensen (2000), loc. cit.; Lipowsky, J. Biol. Phys. 28 (2002), 195-210). Viruses contain 1,200-1,500 Gag molecules, which multimerize on the cytosolic leaflet of the membrane, driving viral assembly and budding. In this process the Gag-Gag interactions collect the virus spike proteins to the bud site. This process requires palmitoylation of gp120 and myristoylation of Gag, and it can be blocked by cholesterol depletion (Jorgensen (2000), loc. cit.). Thus, one can envisage that Gag proteins specifically bind to rafts containing HIV spike proteins, which cluster rafts together to promote virus assembly. The interaction between HIV-1 protein and lipid rafts may cause a conformational change in Gag required for envelope assembly (Jacobson (1992), loc. cit.).

Recent studies have demonstrated that budding of HIV virions as well as fusion with the target cells occurs through lipid rafts. Budding occurs presumably through preferential sorting of HIV Gag to lipid rafts (Nguyen, J. Virol. 74 (2000) 3264-72). HIV-1 particles produced by infected T-cell lines acquire raft components such as the GPI-linked proteins Thy-1 and CD59,and the ganglioside GM1, which is known to partition preferentially into lipid rafts. Assembly of infectious human immunodeficiency virus type 1 (HIV-1) virions requires incorporation of the viral envelope glycoproteins gp41 and gp120. The HIV envelope glycoprotein gp41 also plays an important role in the fusion of viral and target cell membranes. The extracellular domain of gp41 contains three important functional regions, i.e. fusion peptide (FP), N- terminal heptad repeats (NHR) and C-terminal heptad repeats (CHR). During the process of fusion of HIV with the membrane of the target cells, FP inserts into the target cell membrane and subsequently the NHR and CHR regions change conformations and associate with each other to form a fusion-active gp41 core. Peptides derived from NHR and CHR regions, designated N- and C-peptides, respectively, have potent inhibitory activity against HIV fusion by binding to the CHR and NHR regions, respectively, to prevent the formation of the fusion-active gp41 core. Small molecular non-peptide HIV fusion inhibitors having a mechanism of action similar to the C-peptides have been recently developed (Jiang, Curr. Pharm. Des. 8 (2002) 563-80. Wadia, Nat, Med. 10 (2004) 310-315). Accordingly, these peptide and non-peptide inhibitors can be used as pharmacophores C/C' in the compound of the invention.

Accordingly, the present invention provides also for tripartite structured compounds as described above which comprise as pharmacophore "C/C'" specific compounds which inhibit the life cycle of HIV. Examples of such pharmacophores are, but are not limited to, cosalane, AMD3100, AMD070, Fuzeon™, T20, T1249, DP178 and the like. As pointed out herein, particular preferred pharmacophores C/C' in this context are the peptide analogues T20/T1249/Fuzeon™ or "enfuvirtide.

As mentioned herein above and as documented below, the pharmacophore C/C' may also comprise or be a peptide or peptide derivative. A corresponding, non-limiting example is the inhibitory "HR2 peptide" known in the art as "T20". Said peptide is shown to be active in the medical management of HIV/AIDS. "T20"is also known as "DP178" and related peptides and/or derivatives thereof are well known in the art and are described for their anti-retroviral activity; see, inter alia, Wild (1992) PNAS 91, 9770; WO 94/282920, US 5,464,933. Also the peptide "T1249" is known in the art and may be employed in medical interventions. T1249 may be comprised as a pharmacophore C/C' in the tripartite structures of this invention. Again, such a tripartite raftophile in accordance with this invention is particularly useful in the treatment and/or medical intervention of retroviral infection and in particular in AIDS management and/or HIV infections. T20 and T1249 may also be comprised in the herein described inventive construct in form of the described pegylated form(s) which are known and, inter alia, described in WO2004013165. A preparation of T1249 is, inter alia, described in US 5,955,422 or US 6,348,568. Further details on a corresponding tripartite construct of the present invention are given in the appended examples and are illustrated in appended figure 3. A corresponding inventive construct is, inter alia, depicted in formula **25c**.

Tuberculosis is a further example of a bacterial-caused infectious disease involving rafts. First, Complement receptor type 3 (CR3) is a receptor able to internalize zymosan and C3bi-coated particles and is responsible for the nonopsonic phagocytosis of Mycobacterium kansasii in human neutrophils. In these cells CR3 has been found associated with several GPI-anchored proteins localized in lipid rafts of the plasma membrane. Cholesterol depletion markedly inhibits phagocytosis of M. kansasii, without affecting phagocytosis of zymosan or serum- opsonized M. kansasii. CR3, when associated with a GPI protein, relocates in cholesterol-rich domains where M. kansasii are internalized. When CR3 is not associated with a GPI protein, it remains outside of these domains and mediates phagocytosis of zymosan and opsonized particles, but not of M. kansasii isopentenyl pyrophosphate (IPP), a mycobacterial antigen that specifically stimulates Vgamma9Vdelta2 T cells, and compare This delay, which likely accounts for the delay observed in TNF-alpha production, is discussed in terms of the ability of the antigen to cross-link and recruit transducing molecules mostly anchored to lipid rafts (Peyron, J. Immunol. 165 (2000), 5186-5191). Accordingly, the tripartite structured compounds of the present invention are also useful in the prevention, amelioration and/or treatment of tuberculosis and/or other disorders caused by mycobacteria, like M. tuberculosis, M. bovis, etc..

Furthermore, malaria infections of human erythrocytes by malarial parasite is blocked following raft-cholesterol disruption. Erythrocyte rafts serve as an entry route to the parasite (Samuel, J. Biol. Chem. 276 (2001), 29319-29329). Therefore, the compounds of the present invention are useful in inhibiting the infectious route of Plasmodium falciparum. It is, e.g. envisaged that anti-CD36 antibodies or functional fragments thereof be used as pharmacophores in the compounds of the present invention. Such antibodies are known in the art, see, e.g. Alessio, Blood 82 (1993), 3637-3647.

Yet, in a further embodiment of the invention tripartite structured compounds of the invention may be employed as pharmaceuticals in the management of prion diseases.

A conformational change resulting in amyloid formation is also involved in the pathogenesis of prion disease. Prion diseases are thought be promoted by an abnormal form (PrPsc) of a host- encoded protein (PrPc). PrPsc can interact with its normal counterpart PrPc and change the conformation of PrPc so that the protein turns into PrPsc. PrPsc then self-aggregates in the brain, and these aggregates are thought to cause the disorders manifested in humans as Creutzfeldt-Jakob disease, Kuru, or Gerstmann-Sträussler-Scheinker syndrome (McConnell, Annu, Rev. Biophys. Biomol. Struct. 32 (2003), 469-492). The mechanism by which PrPc is converted to PrPsc is not known, but several lines of evidence suggest that lipid rafts are involved (McLaughlin, Annu. Rev. Biophys. Biomol. Struct. 31 (2002), 151-175; Milhiet, Single Mol. 2 (2001), 119-121).

PrP is a GPI-anchored protein. Both PrPc and PrPsc are associated with DRMs in a cholesterol-dependent manner. Cholesterol depletion of cells leads to decreased formation of PrPsc from PrPc. The GPI anchor is required for conversion. When the GPI anchor is exchanged with a transmembrane domain, conversion to abnormal proteins is blocked. In vitro, the conversion of PrPc to PrPsc, as monitored by PrP protease resistance, occurs when microsomes containing PrPsc are fused with DRMs containing PrP (McLaughlin (2002), loc. cit.). Extraction with detergent leads to raft clustering in DRMs. Fusion of microsomes with DRMs was necessary in this experiment because simply mixing the membranes did not lead to measurable generation of new PrPsc. On the other hand, releasing PrP ectodomains from PrPsc by phospholipase C treatment also stimulated conversion of PrP to PrPsc in this system. Baron et al. (McLaughlin (2002), loc. cit.) hypothesize that membrane components exchange between apposed cells; a possible mechanism for such an exchange is that the cells release membrane vesicles containing PrPsc that fuse with neighboring cells. Indeed, a similar process has been found to mediate transfer of the raft-associated chemokine receptor CCR5 (Murata, Proc. Nat. Acad. Sci. USA 92 (1995), 10339-10343). Alternatively, GPI-anchored PrPsc could be released as such from one cell and move across the extracellular aqueous phase to be inserted into another cell. Recently, it was shown that direct cell-cell contact is required for transfer of PrPsc infectivity in cell culture (Nielsen (1999), loc. cit.). Therefore, the inventive construct is useful in the management of PrPsc infections.

The prion protein (PrP) is the protein implicated in the pathognetic mechanisms underlying transmissible spongiform encephalopathies. A conformational change of the PrP(C) into the pathogenic PrP(Sc) form involves the conversion of alpha-helical structures into beta-sheet- enriched structures. Anilino-naphtalene compounds such as bis-ANS (4,4'-dianilino-1,1'-binaphthyl-5,5'-sulfonate), ANS (1-anilinonaphthalene-8-sulfonate), and AmNS (1-amino-5-naphtalenesulfonate) inhibit prion peptide aggregation, by directly interacting with PrP (Cordeiro, J. Biol. Chem. 279(7) (2004), 5346-5352). Since PrP is a GPI-anchored protein and both PrPc and PrPsc are associated with lipid rafts, the activity of Anilino-naphtalene compounds is enhanced through the preferential targeting of such pharmacophores to rafts.

Also asthma is a target disease for the use of the tripartite structured compounds of the invention.

The cells used most intensively to study the role of lipid rafts in FcεRI-mediated signaling are rat basophilic leukemia (RBL) cells. A role for rafts in the interactions that follow FcεRI aggregation, mainly in signaling complexes assembled around the linker for activation of T cells (LAT). The involvement of rafts in the immediate events following antigen-mediated FcεRI clustering has been described.

Rafts are important in controlling and integrating signal progression following FcεRI activation in the mast cell system. Accordingly, the tripartite structured compound of the invention may interfere with this signal progression.

Furthermore, the compounds of the present invention are useful in the management of proliferative disorders,

since a large number of signaling components are regulated through their partitioning to rafts. For example, the tyrosine kinase activity of EGF receptor is suppressed in rafts and cholesterol play a regulatory role in this process (Ringerike, J. Cell Sci. 115 (2002), 1331-1340). Similarly, H-Ras is inactive in rafts and its signaling activity occurs upon exiting rafts (Parton, Trends Cell Biol. 14 (2004), 141-147). The list of signaling factors whose activity depends on rafts is extended to various types of ligand-receptor complexes and downstream signaling components (Simons (2004), loc. cit.; Miaczynska, Curr. Opin. Cell Biol. 16 (2004), in progress). Accordingly, as documented above, specific pharmacophores capable of interfering with these signaling features may be introduced in the inventive tripartite structured compound. Preferably, the compound of the invention is used in the treatment of breast cancer, colon cancer, stomach cancer, mogenital cancers, lung cancer, or skin cancer, like melanomas. For the treatment/prevention of breast cancer it is also envisaged that antiestrogens, like tamoxifen, fulvestrant or anastrole are employed as pharmcophores C/C' in the compound of the present invention.

For example, the peptide hormone endothelin transmits proliferative signals through G protein-coupled receptors, the endothelin type A (ETAR) and B (ETBR) receptors. These molecules are therefore important therapeutic targets in the development of anti-tumor therapy. ETAR and ETBR are important in the development of melanoma. ETBR forms a complex with caveolin-1 and thus localizes in the specialized form of lipid rafts called caveolae (Yamaguchi, Eur. J. Biochem. 270 (2003) 1816-1827). The small molecule A-192621, is an nonpeptide ETBR antagonist that significantly inhibits melanoma growth in nude mice by blocking signaling pathways downstream ETBR which are important in host-tumor interactions and cancer progression (Bagnato, Cancer Res. 64, (2004) 1436-1443). Accordingly, A-192621 and similar derivatives can be used as pharmacophore in the compound of the invention.

Recent studies have shown that insulin signaling leading to GLUT-4 translocation depends on insulin receptor signalling emanating from caveolae or lipid rafts at the plasma membrane (Khan, Diabetologia 45 (2002), 1475-1483). Accordingly, the described tripartite structured compound is also useful in the medical management of diabetes.

In a further embodiment, the tripartite structured compound may be employed in the medical/pharmaceutical intervention of a parsite infection, as pointed out above for malaria. Yet, also other parasite infections, like Trypanosoma-, Leishmania-, or Toxoplasma gondii-infections are envisaged to be treated by administration of the inventive tripartite compound.

It is also envisaged that compounds of the present invention be employed in the medical management of hypertension and/or congestive heart failure. As corresponding pharmacophores C/C' receptor inhibitors like Losartan, Valsartan, Candesartan Cilexetil, or Irbesartan or TCV-116 (2-Ethoxy-1-[2'-(1H-tetrazol-5-yl) biphenyl-4-yl]-1-benzimidazole-7-carboxylate. Following the teachings of the present invention, the compounds as disclosed herein are also useful in the treatment, amelioration and/or prevention of disorders and diseases, like hyperallergenic response and asthma, T-cell and B-cell response, autoimmune disease, chronic inflammation, atherosclerosis, lysosomal storage disease, Niemann-Pick disease, Tay-Sachs disease, Fabry's disease, metachromatic leukodystrophy, hypertension, Parkinson's disease, polyneuropathies, demyelenating diseases, as well as muscular dystrophy.

As disclosed above, the present invention also provides for a method for the preparation of a compound as described herein, wherein said method comprises preferably the steps of a) defining the distance between (a) phosphoryl head group(s) or (an) equivalent head group(s) of (a) raft lipid(s) and a binding and/or interaction site of a pharmacophore C/C' on a raft-associated target molecule; b) selecting a linker B/B' which is capable of spanning the distance as defined in a); and c) bonding a raftophile A/A' and the pharmacophore C/C' by the linker as selected in b).

Corresponding working examples for such a method are given herein and are also illustrated in the appended examples. The person skilled in the art is in a position to deduce relevant binding sites or interactions sites of a given or potential pharmacophore and, accordingly, to determine the distance between (a) phosphoryl head group(s) or (an) equivalent head group(s) of (a) raft lipid(s) and a binding and/or interaction site of a pharmacophore C/C' on said target molecule. Such methods comprise, but are not limited to molecular modelling, in vitro and/or molecular-interaction or binding assays (like, e.g. yeast two or three hybrid systems, peptide spotting, overlay assays, phage display, bacterial displays, ribosome displays), atomic force microscopy as well as spectroscopic methods and X-ray crystallography. Furthermore, methods such as site-directed mutagenesis may be employed to verify deduced interaction sites of a given pharmacophore or of a candidate pharmacophore and its corresponding target.

As illustrated above, the target molecule is most preferably a molecule which is involved in biological processes which take place on or in lipid rafts (i.e. cholesterol-sphingolipid microdomains). Non-limiting examples for target molecules are beta-secretase (BACE-1), but also amyloid-precursor-protein (APP), raft-associated viral receptors or bacterial receptors (as illustrated above), Prp/PrP(SC), hormone receptors (such as, e.g., insulin receptors, endothelin receptors or angiotensin II receptors), receptors for growth factors (such as, e.g., EGF-receptors), Ig-receptors (such as, e.g., IgE receptor FcεRI), cell surface proteins (such as, e.g., surface glycoprotein CD36 (GPIV)). Preferably, said target molecules are enzymes, receptor molecules and/or signal transduction molecules. Further examples of target molecules are given herein above. The term "raft-associated target molecule" means in the context of this invention that the molecule may either be comprised in rafts or may be translocated into rafts upon corresponding stimulation and/or modification (e.g. secondary modification by phosphorylation etc.)

The selection of a linker was illustrated herein above and is also shown in the experimental part. Such a selection comprises the selection of linkers known in the art as well as the generation and use of novel linkers, e.g. by molecular modelling and corresponding synthesis or further methods provided herein above and known in the art.

The term "spanning" as employed herein above in step b) means that the length of the linker B/B' is selected so that it places the specific pharmacophore (preferably an inhibitor) at the correct locus on the target molecule, e.g. an enzyme, a signal transduction molecule or a receptor, and that the raftophile A/A' is part of the lipid layer of the raft.

Due to the medical importance of the tripartite structured compounds of the present invention, the invention also provides for a method for the preparation of a pharmaceutical composition which comprises the admixture of the herein defined compound with one or more pharmaceutically acceptable excipients. Corresponding excipients are mentioned herein above and comprise, but are not limited to cyclodextrins. As pointed out above, should the pharmaceutical composition of the invention be administered by injection or infusion it is preferred that the pharmaceutical composition is an emulsion.

It is to be stressed that the person skilled in the art is readily in the position to deduce, verify and/or evaluate the raftophilicity of a given tripartite structure as well as of the individual moiety A/A' as described herein. Corresponding test assays are provided herein and are also illustrated in the appended examples.

For example, for evaluation of the various raftophilic moieties described herein, rhodamine-labeled conjugates were prepared comprising the raftophile to be evaluated and a literature-known modified rhodamine dye as described in example 32. For ease of preparation and modularity, the modified rhodamine dye was attached to the side chain of glutamic acid and the resulting labeled amino acid was used as dye marker The raftophile and the rhodamine-labeled glutamic acid were coupled via a linker building block, for example Arg-Arg-βAla or 3Gl (12-amino-4,7,10-trioxadodecanoic acid).

In the case of steroid-derived raftophile moieties, compounds comprising a single bond between positions 5 and 6 of the steroid-derived scaffold are preferred over compounds with a double bond at that position. For example, evaluation of raftophile moiety **200a** in the LRA assay resulted in a raftophilicity Rf of 16.5 (and relative raftophilicity $r_{rel}$ 0.493 in the DRM assay), while raftophile moiety **200b** comprising identical linker and dye label substructure provided a raftophilicity of Rf 42.7 in the LRA assay (and relative raftophilicity $r_{rel}$ 0.536 in the DRM assay).

Raftophile moieties having structure **19b** are preferred, as demonstrated by the comparison of moiety **19b** and moiety **200b** coupled to identical linker and dye label substructures. In the LRA assay, the raftophilicity Rf of moiety **19b** was calculated as 76.3, while moiety **200b** provided a Rf value of 58.6. Evaluation of the same structures in the DRM assay resulted in a relative raftophilicity $r_{rel}$ 0.503 for moiety **19b** and relative raftophilicity $r_{rel}$ 0.336 for moiety **200b**.

In the case of ceramide-derived raftophile moieties of the general structure 400a, when considering the chain length of substituents $R^{41a}$ and $R^{42a}$, an overall symmetrical shape is preferred in order to obtain high raftophilicity values. For example, when comparing raftophile moieties **400aa** and **400af** comprising identical linker and dye label substructure, in the DRM assay a higher relative raftophilicity $r_{rel}$ of 0.772 was obtained for the more symmetrical moiety **400aa** as compared to a relative raftophilicity $r_{rel}$ of 0.560 for moiety **400af.** The higher symmetry results from the incorporation of a palmitoyl (C16) side chain in moiety **400aa** compared to the eicosanoyl (C20) side chain of moiety **400af**.

In the case of raftophile moieties **7**, compounds comprising steroid-derived substructures as side chains are preferred over compounds displaying simple acyl side chains. For example, raftophile moiety **700c** is preferred over raftophile moiety **700b**, which itself is preferred over raftophile moiety **700a,** as demonstrated in both LRA and DRM evaluation. The raftophilicity of **700c** was calculated as Rf 37.3 in the LRA assay and the relative raftophilicity as $r_{rel}$ 0.414 in the DRM assay, while measurement of **700b** provided Rf 28.8 in the LRA and $r_{rel}$ 0.403 in the DRM assay. Evaluation of simple fatty acid decorated moiety **700a** resulted in a raftophilicity of Rf 18.6 in the LRA and a relative raftophilicity $r_{rel}$ 0.266 in the DRM assay.

In general, for all raftophilic moieties A/A' described in the present invention an acetic hook is preferred over a succinic hook. For example, the raftophilicity of raftophile moiety **200e** was determined in the LRA assays as Rf 8.1, while raftophile moiety **200b** resulted in an Rf of 42.7 in the LRA assay, when comparing compounds comprising identical linker and dye label substructures. In the same comparison using the DRM assay relative raftophilicities ($r_{rel}$) of 0.468 and 0.536 were obtained, respectively. Thus, in particular, raftophile moieties comprising an ether or amine function at position 3 of a steroid-derived scaffold or at position 1 of a sphingosine-derived structure are preferred over similar moieties displaying an amide or ester function at these positions. This holds true also from the viewpoint of chemical stability, as ether and amine functions are more stable against solvolysis and enzyme- mediated cleavage than amide and ester functions, and amide functions are more stable than ester functions in that very respect.

In order to evaluate the influence of the linker moiety on the raftophilicity of a given raftophile, raftophile moiety **200b** was coupled to the modified rhodamine-dye via a 12-amino-4,7,10-trioxadodecanoic acid linker in a manner that **200b** was attached to the 12-amino function and the N-terminus of the modified dye building block was attached to the C-terminus. Using the LRA assay, a raftophilicity (Rf) of 58.6 was calculated. When testing an analogous conjugate prepared with a peptidic linker of the sequence Arg-Arg-betaAla, a Rf of 42.6 was obtained, while testing an analogue conjugate prepared with a peptidic linker of the sequence Lys-Lys-betaAla resulted in a Rf of 24.1. Thus, in order to

obtain high raftophilicities, linkers made from polyethers are preferred over linkers made from peptides, and linkers comprising arginine units in proximity to the raftophile moiety are preferred over linkers having lysine units at that position. Moreover, a qualitative solubility assessment of compounds **24b** and **25b** demonstrated unambiguously the higher solubility of compound **24b** comprising a polyether linker in an aqueous medium. The invention will now be described by reference to the following chemical, biological and biochemical examples which are merely illustrative and are not to be construed as a limitation of the scope of the present invention.

The invention is also illustrated by the following illustrative figures. The appended figures show:

Figure 1
*Top*, proposed mechanism of action of the tripartite structure 25b and BACE inhibitor III; *Bottom*, inhibition of BACE activity by compound 25b and BACE inhibitor III. Control (no inhibitors) activity was set to 100%.

Figure 2
*Top*, proposed mechanism of action of inhibition of beta-secretase (BACE) activity in whole neuronal cells employing compound 25b and BACE inhibitor III; *Bottom*, inhibition of beta-secretase (BACE) activity by compound 25b and BACE inhibitor III (see also Example 36).

Figure 3
*Top*, proposed mechanism of action of a tripartite structure incorporating the HIV membrane fusion inhibitor, enfuvirtide. HIV spike proteins dock onto cell membrane receptors in rafts and facilitate membrane fusion. Enfuvirtide prevents conformational changes in the docked spike protein to prevent membrane fusion. Potency of the tripartite inhibitor is proposed to be 100 - 1000 fold higher due to enrichment in the raft.

**Examples**

Abbreviations

| | |
|---|---|
| DCC | N,N'-dicyclohexyl carbodiimide |
| Dde | 1-(4,4-dimethyl-2,6-dioxocyclohexyliden)ethyl |
| DIPEA | diisopropylethylamine |
| DMAP | dimethylamino pyridine |
| DMF | dimethylformamide |
| Fmoc | N-alpha-(9-fluorenylmethyloxycarbonyl) |
| HATU | 2-(7-aza-1H-benzotriazole-1-yl)-1,1,3,3-tetramethyluronium hexafluorophosphate |
| HBTU | 2-(1H-benzotriazole-1-yl)-1,1,3,3-tetramethyluronium hexafluorophosphate |
| NMO | N-methyl morpholine N-oxide |
| Pbf | 2,2,4,6,7-pentamethyldihydrobenzofuran-5yl-sulfonyl |
| PE | petroleum ether |
| RT | retention time |
| TBDMS | tert-butyldimethyl silyl |
| TBDPS | tert-butyldiphenyl silyl |
| THF | tetrahydrofurane |
| Trt | trityl |
| βAla: | β-alanine |
| Sta: | statine |
| Chol: | cholesteryl |
| Dhc: | dihydrocholesteryl |
| Glc: | $-O-CH_2-CO-$ |
| Succ: | $-CO-CH_2-CH_2-CO-$ |
| 4GI: | |

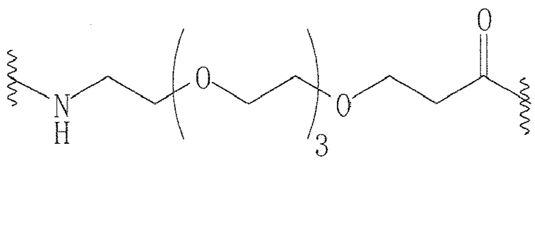

3GI:

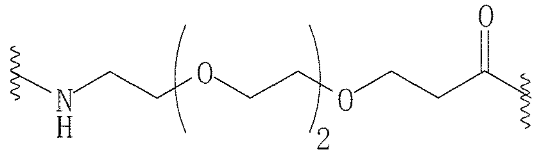

Glu(Rho):

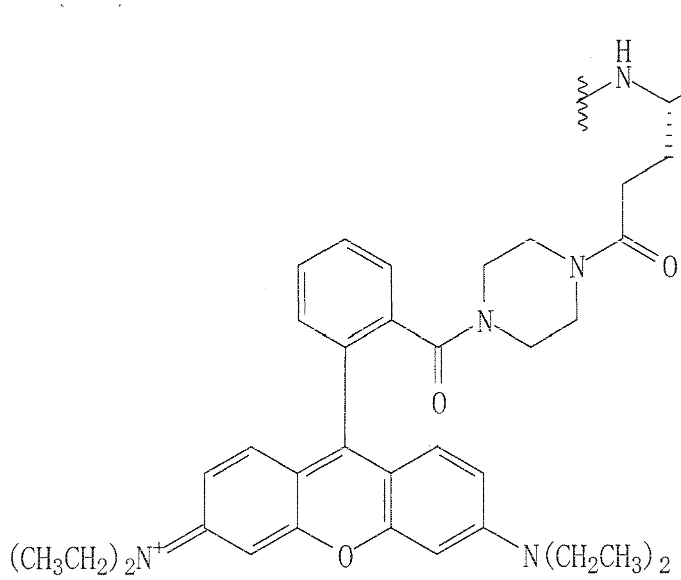

## General Procedures

### Acylation to introduce side chain (ceramides)

DIPEA (2.55 eq) was added to the solution of the corresponding acid (1.2 eq) and HATU (1.2 eq) in DMF/$CH_2Cl_2$ (1:1) with stirring at room temperature for 5 min. The solution was than added to the solution of 3 (1.0 eq) in $CH_2Cl_2$ and stirred at room temperature for 2 h. Reaction mixture was diluted with $CH_2Cl_2$ (100 mL) and washed with 1 N HCl solution and extracted with $CH_2Cl_2$ (3 x 100 mL). The combined organic layers were dried over sodium sulfate and concentrated in vacuo. Purification of the residue by flash chromatography (silica, PE/EtOAc) yielded product.

### Esterification (inositols and glycerols)

To a solution of alcohol (1.0 eq) in $CH_2Cl_2$ (5 mL) was added DCC (1.4 eq), DMAP (0.66 eq) and the corresponding acid (1.4 eq) and stirred under argon atmosphere for 24 h at room temperature. The solvent was removed under reduced pressure and the residue was subjected to flash chromatography (silica, petroleum ether/EtOAc) to afford the product.

### Attachment of succinic head group to ceramides

Succinic anhydride (1.1 eq) was added to the stirred solution of ceramide (1.0 eq) in $CH_2Cl_2$ (10 mL). After adding DMAP (1.2 eq), the reaction mixture was stirred at room temperature for 16 h. The mixture was diluted with 50 mL $CH_2Cl_2$ and washed with 1 N HCl solution and extracted with $CH_2Cl_2$ (3 x 100 mL). The combined organic layers were dried over sodium sulfate and concentrated in vacuo. Purification of the residue by flash chromatography (silica, hexane/EtOAc) yielded product.

### Attachment of succinic head group to inositols and glycerols

Succinic anhydride (2.0 eq) was added to the stirred solution of alcohol (1.0 eq) in $CH_2Cl_2$ (10 mL). After adding DMAP (2.0 eq), the reaction mixture was stirred at room temperature for 48 h. The mixture was diluted with $CH_2Cl_2$,

washed with sat. NaCl solution and extracted with $CH_2Cl_2$. The combined organic layers were dried over sodium sulfate and concentrated in vacuo. Purification of the residue by flash chromatography (silica, PE/EtOAc) yielded product.

**Removal of TBDPS group**

A solution of tetrabutylammonium fluoride (1 M solution in THF) (4.25 eq) was added to the solutions of TBDPS-protected ceramide (1.0 eq) in THF (15 mL) and heated at 60˚C for 3 h. Reaction mixture was cooled and diluted with $CH_2Cl_2$ (100 mL) and washed with 1 N HCl solution and extracted with $CH_2Cl_2$ (3 x 100 mL). The combined organic layers were dried over sodium sulfate and concentrated in vacuo. Purification of the residue by flash chromatography (silica, hexane/EtOAc/MeOH) yielded product.

**Removal of Benzyl group**

10% Palladium on carbon was added to a solution of the benzyl-protected inositol (1.0 eq) in a mixture of methanol (5 mL) and $CH_2Cl_2$ (5 mL) and vigorously stirred under $H_2$ atmosphere (800-900 torr) for 24 h. The reaction mixture was filtered over a short path of celite (which was subsequently washed with methanol/$CH_2Cl_2$) and the solvent was evaporated. The residue was subjected to flash chromatography (silica, methanol/$CH_2Cl_2$) on silica gel column to afford the product.

**Deallylation**

To a solution of *O*-allyl-inositol (1.0 eq) in a mixture of $CH_2Cl_2$ (10 mL), acetic acid (19 mL) and $H_2O$ (1 mL) was added palladium-(II)-chloride (1.6 eq), sodium acetate (4.0 eq) and stirred at room temperature for 24 h. The solvent was removed under reduced pressure and the residue was dissolved in EtOAc and washed with saturated $NaHCO_3$. The combined organic layers were washed with brine and dried over sodium sulfate and concentrated in vacuo. Purification of the residue by flash chromatography (silica, petroleum ether/EtOAc) yielded product.

**Example 1: Preparation of succinic mono (D-erythro-$C_{16}$-ceramidyl) ester, a precursor** of moiety 400aa

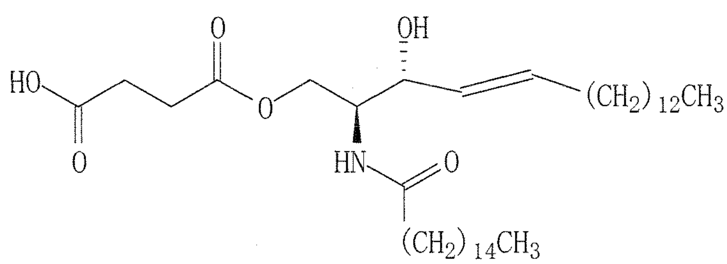

Dimethylaminopyridine (0.25g, 2.1mmol) and succinic anhydride (0.21g, 2.1mmol) were added to a solution of 3-O-$^t$butyldiphenylsilyl-D-erythro-$C_{16}$-ceramide (0.85g, 1.09mmol) in dichloromethane (5ml). The resulting slurry was stirred at room temperature for 2 days to give a light yellow solution. After dilution with dichloromethane (50ml), the reaction mixture was washed with 1M HCl and $H_2O$, and dried over $Na_2SO_4$. Crude yield: 0.86g colorless oil. The light yellow solution of the above described crude material (0.82g, 0.94mmol) and tetrabutylammonium fluoride (75% in $H_2O$, 1.05g, 3mmol) in tetrahydrofurane (4ml) was stirred at 60˚C for 3h. After cooling to room temperature, the reaction mixture was quenched by addition of 1M HCl (50ml) and extracted with ethyl acetate (50ml). The organic phase was separated, washed with 1M HCl and $H_2O$, and dried ($Na_2SO_4$). The crude material (0.69g, white solid) was purified by column chromatography on silica gel (petroleum ether/ethyl acetate/methanol 10:10:1) to give 0.3g of succinic mono (D-erythro-$C_{16}$-ceramidyl) ester as a white solid.
$^1$H-NMR (300 MHz, $CDCl_3$): d = 0.88 (t, 6H), 1.26 (s, 46H), 1.60 (m, 2H), 2.03 (q, 2H), 2.20 (dt, 2H), 2.64 (m, 4H), 4.19 (m, 3H), 4.33 (m, 1H), 5.46 (dd, 1H), 5.75 (dt, 1H), 6.10 (d, 1H). MS (ESI): m/z = 660 (M+Na).

**Example 2: Preparation of succinic mono (D-erythro-$C_{20}$-ceramidyl) ester, a precursor of moiety 400af**

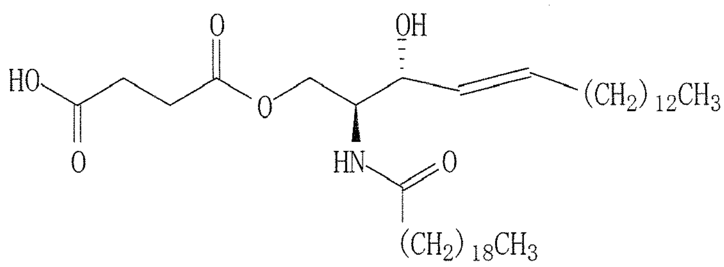

Dimethylaminopyridine (0.32g, 2.6mmol) and succinic anhydride (0.22g, 2.2mmol) were added to a solution of 3-O-$^t$butyldiphenylsilyl-D-erythro-$C_{20}$-ceramide (1g, 1.2mmol) in dichloromethane (5ml). The resulting slurry was stirred at room temperature for 3h. After dilution with dichloromethane (50ml), the reaction mixture was washed with 1M HCl and $H_2O$, and dried over $Na_2SO_4$. Crude yield: 1.02g colorless oil.

The crude material (1.02g, 1.09mmol) and TBAF (75% in $H_2O$, 1.05g, 3mmol) were dissolved in THF (3ml) to give a light yellow solution which was stirred at 50°C for 3h. After cooling to room temperature, the reaction mixture was quenched by addition of 1M HCl (50ml) and extracted with ethyl acetate (50ml). The organic phase was separated, washed with aqueous saturated sodium chloride solution, and dried ($Na_2SO_4$). The crude material (0.89g white solid) was purified by column chromatography on silica gel (petroleum ether/ethyl acetate/methanol 10:10:1) to give 0.14g of succinic acid mono (D-erythro-$C_{20}$-ceramidyl) ester as a white solid.
$^1$H-NMR (300 MHz, $CDCl_3$): d = 0.88 (t, 6H), 1.25 (s, 54H), 1.60 (m, 2H), 2.03 (q, 2H), 2.20 (dt, 2H), 2.64 (m, 4H), 4.18-4.33 (m, 4H), 5.46 (dd,1H), 5.75 (dt, 1H), 6.05 (d, 1H).
MS (ESI): m/z = 716 (M+Na).

**Example 3: Preparation of succinic mono (D-erythro-$C_{16}$-ceramidyl) ester, a precursor** for moiety 400ad

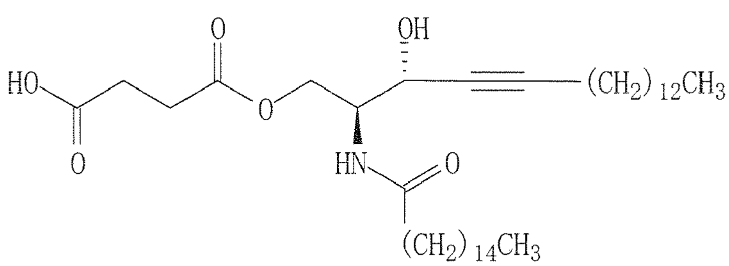

Dimethylaminopyridine (0.33g, 2.7mmol) and succinic anhydride (0.2g, 2mmol) were added to a solution of 3-O-$^t$butyldiphenylsilyl-4,5-dehydro-D-erythro-$C_{16}$-ceramide (0.84g, 1.08 mmol) in dichloromethane (5ml). The resulting slurry was stirred at room temperature for 2h. After dilution with dichloromethane (50ml), the reaction mixture was washed with 1M HCl and $H_2O$, and dried over $Na_2SO_4$. Crude yield: 0.83g colorless oil. The crude material and tetrabutylammonium fluoride (75% in $H_2O$, 1.1g, 3.2mmol) were dissolved in tetrahydrofurane (4ml) to give a light yellow solution which was stirred at 60°C for 3.5h. After cooling to room temperature, the reaction mixture was quenched by addition of $H_2O$ (50ml) and extracted with ethyl acetate (50ml). The organic phase was separated, washed with aqueous saturated sodium chloride solution, and dried ($Na_2SO_4$). The crude material which was a waxy, light yellow solid (0.82g) was purified by column chromatography on silica gel (petroleum ether/ethyl acetate/methanol 10:10:1) to give 0.28g of succinic mono (D-erythro-$C_{16}$-ceramidyl) ester as a white solid.
$^1$H-NMR (300 MHz, $CDCl_3$): d = 0.88 (t, 6H), 1.26 (s, 44H), 1.49 (q, 2H), 1.62 (m, 2H), 2.22 (q, 4H), 2.65 (br s, 4H), 4.27 (t, 1H), 4.36 (m, 2H), 4.53 (br s, 1H), 6.13 (d, 1H).
MS (ESI): m/z = 658 (M+Na).

**Example 4: Preparation of a precursor for moiety 400al**

The precursor to compound **400al** was obtained by the following reaction sequence:

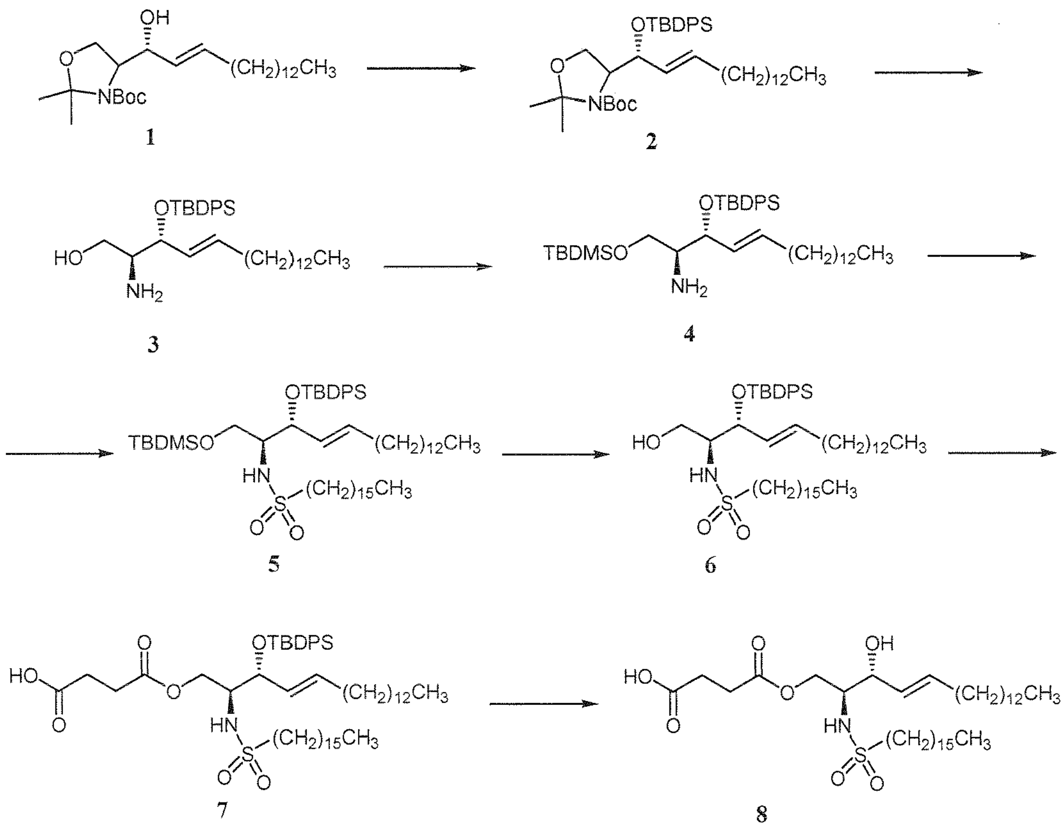

1 2 3 4 5 6 7 8

Compound **1** was obtained as per literature procedure (*Synthesis*, 1998, 1075).

The solution of **1** (10.9 g, 24.8 mmol), imidazole (3.4 g, 50 mmol) and TBDPSCl (10.4 mL, 40 mmol) in DMF (25 mL) was stirred at 80˚C for 3 h and further at 100˚C for 2 h. Reaction mixture was cooled to room temperature and quenched with $H_2O$ (300 mL) and extracted with $Et_2O$ (2 x 150 mL). The combined organic layers were washed with 1 N HCl (100 mL) solution, saturated $NaHCO_3$ solution (100 mL) and $H_2O$ (200 mL); dried over sodium sulfate and concentrated in vacuo. Purification of the residue by flash chromatography (silica, PE/EtOAc 30:1) yielded compound **2** as colorless oil (13.7 g, 81%).
$^1$H-NMR (300 MHz, $CDCl_3$): δ = 0.86 (m, 3H), 1.03 (s, 12H), 1.16 (m, 18H), 1.39 (m, 15H), 1.63 (br s, 2H), 3.85 (m, 2H), 4.12 (m, 2H), 4.90 (m, 1H), 5.18 (m, 1H), 7.34 (m, 6H), 7.61 (m, 4H).

1M HCl (25 mL) was added to the solution of **2** (13.7 g, 20.2 mmol) in 1,4-dioxane (250 mL) and heated at 100˚C for 1 h. The reaction was cooled to room temperature and quenched with sat. $NaHCO_3$ (100 mL) solution and extracted with $Et_2O$ (2 x 150 mL). The combined organic layers were washed with brine (100 mL) and dried over sodium sulfate and concentrated in vacuo. Purification of the residue by flash chromatography (silica, $CH_2Cl_2$/MeOH 20:1) yielded **3** as a light yellow oil (7.97 g, 73%).
$^1$H-NMR (300 MHz, $CDCl_3$): δ = 0.81 (m, 3H), 1.05 (s, 9H), 1.14 (m, 22H), 1.81 (m, 2H), 2.02 (br s, 3H), 2.80 (m, 1H), 3.42 (m, 1H), 3.59 (m, 1H), 4.01 (m, 1H), 5.21 (m, 2H), 7.31 (m, 6H), 7.62 (m, 4H).

To a solution of **3** (1.076 g, 2.0 mmol) in $CH_2Cl_2$ (20 mL) were added DMAP (488.7 mg, 4.0 mmol) and TBDMSCl (0.603 g, 4.0 mmol). The mixture was stirred for 16 h at room temperature. Reaction mixture was diluted with $CH_2Cl_2$ (100 mL) and washed with 1 N HCl solution and extracted with $CH_2Cl_2$ (3 x 100 mL). The combined organic layers were dried over sodium sulfate and concentrated in vacuo. Purification of the residue by flash chromatography (silica, $CH_2Cl_2$/MeOH 20:1) yielded **4** as a light yellow oil (1.31 g, 100%).
$^1$H-NMR (300 MHz, $CDCl_3$): δ = - 0.10 (m, 6H), 0.72 - 0.82 (m, 21H), 0.92 - 1.16 (s, 22H), 1.73 (m, 2H), 3.08 (m, 1H), 3.67 (d, J = 5.6 Hz, 2H), 4.23 (m, 1H), 5.16 (m, 2H), 7.22 (m, 6H), 7.51 (m, 4H).

To a solution of **4** (1.311 g, 2.01 mmol) in $CH_2Cl_2$ (20 mL) were added DMAP (492 mg, 4.03 mmol) and 1-hexadecanesulfonyl chloride (1.334 g, 4.10 mmol). The mixture was heated at reflux for 20 h. Reaction mixture was diluted with $CH_2Cl_2$ (100 mL) and quenched with $H_2O$ (1000 mL), washed with NaCl solution and extracted with $CH_2Cl_2$ (3 x 100 mL). The combined organic layers were dried over sodium sulfate and concentrated in vacuo. Purification of the residue by flash chromatography (silica, $CH_2Cl_2$/MeOH 20:1) yielded **5** as a light yellow oil (1.486 g, 79%). Crude product was subjected to the next step.

1M HCl (10 mL) was added to the solution of **5** (1.486 g, 1.58 mmol) in dioxane (10 mL) and heated at 80°C for 2 h and further at 100°C for 2 h. The reaction was quenched with sat. $NaHCO_3$ (50 mL) solution and extracted with $CH_2Cl_2$ (3 x 100 mL). The combined organic layers were dried over sodium sulfate and concentrated in vacuo. Purification of the residue by flash chromatography (silica, Hexane/EtOAc 4:1) yielded **6** as a waxy solid (642 mg, 49%).
$^1$H-NMR (300 MHz, $CDCl_3$): δ = 0.86 (m, 6H), 1.07 (s, 9H), 1.26 (m, 46H), 1.73 (m, 5H), 2.22 (m, 1H), 2.90 (m, 1H), 3.34 (m, 1H), 3.62 (m, 1H), 3.70 (s, 2H), 3.83 (m, 1H), 4.32 (m, 1H), 4.59 (d, J = 8.06 Hz, 1H), 5.32 (m, 2H), 7.33 (m, 6H), 7.60 (m, 4H).
MS (ESI): m/z = 843.6 ($M+NH_4$)

Succinic head group was attached as described in the general procedure to obtain compound **7** (598 mg; 89%).
$^1$H-NMR (300 MHz, $CDCl_3$): δ = 0.86 (m, 6H), 1.05 (s, 9H), 1.26 (m, 46H), 1.70 (m, 5H), 2.09 (m, 1H), 2.22 (m, 1H), 2.58 (m, 5H), 2.89 (m, 2H), 3.65 (m, 1H), 4.22 (m, 2H), 4.41 (d, J = 8.7 Hz, 1H), 5.30 (m, 2H), 7.33 (m, 6H), 7.60 (m, 4H).
MS (ESI): m/z = 943.6 ($M+NH_4$)

Protecting group was removed as per the general procedure to obtain compound **400al** (300 mg; 72%).
$^1$H-NMR (300 MHz, $CDCl_3$): δ = 0.86 (m, 6H), 1.26 (s, 46H), 1.52(m, 1H), 1.70 (m, 7H), 2.09 (m, 1H), 2.24 (m, 2H), 2.47 (m, 2H), 2.52 (br s, 2H), 3.03 (m, 1H), 3.39 (m, 4H), 4.21 (m, 2H), 4.85 (d, J = 8.7 Hz, 1H), 5.30 (m, 1H), 5.78 (m, 1H).
MS (ESI): m/z = 705.5 ($M+NH_4$)

**Example 5: Preparation of a precursor for moiety 400ak**

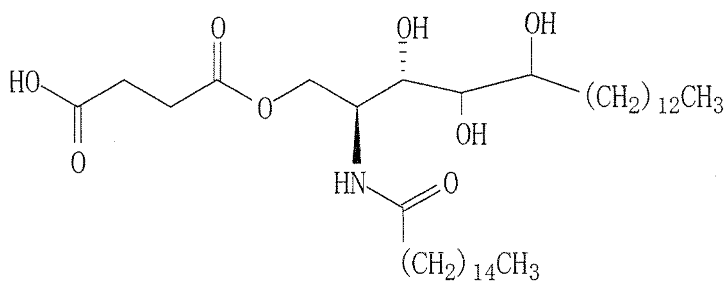

To a solution of the compound obtained in example 1 (98 mg, 0.154 mmol) in $CH_2Cl_2$ (10 mL) was added NMO (19 mg, 0.162 mmol) and $OsO_4$ (39 mg, 0.154 mmol). The mixture was stirred at room temperature for 3 h and subsequently diluted with 50 mL $CH_2Cl_2$ and washed with $H_2O$ (250 mL) and subsequently with IN HCl and extracted with $CH_2Cl_2$. The combined organic layers were dried over sodium sulfate and concentrated in vacuo. Purification of the residue by flash chromatography (silica, $CH_2Cl_2$/MeOH 1:1) yielded the precursor of moiety **400ak** as a waxy solid (106 mg, 100%).
MS (ESI): m/z = 672.5 (M+1)

**Example 6: Preparation of a precursor for moiety 400ap**

The precursor of moiety **400ap** was obtained by the following reaction sequence.

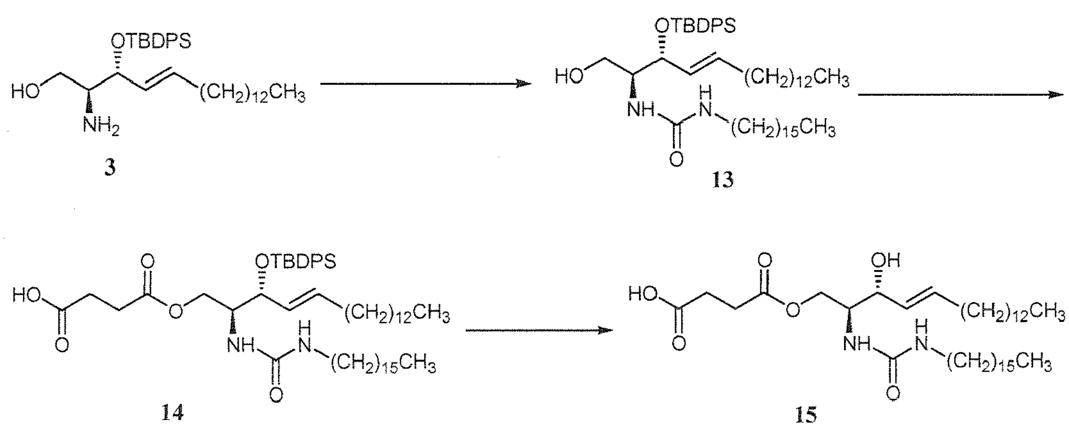

3 13 14 15

Compound **3** was prepared as described in example 4 above.

Hexadecyl isocyanate (0.81 mL, 2.6 mmol) was added to the solution of **3** in $CH_2Cl_2$ (5 mL) and stirred at room temperature for 2 h. Reaction mixture was diluted with $CH_2Cl_2$ (100 mL) and washed with 1 N HCl solution and extracted with $CH_2Cl_2$ (3 x 100 mL). The combined organic layers were dried over sodium sulfate and concentrated in vacuo. Purification of the residue by flash chromatography (silica, PE/EtOAc 3:1) yielded product **13** as colourless oil (0.72 g, 35%).

Succinic head group was attached as described in the general procedure to afford the product **14** (780 mg; 97%). Crude product was subjected to the next step.

Protecting group was removed as per the general procedure to afford the precursor of moiety **400ap** (440 mg; 76%).

$^1$H-NMR (300 MHz, $CDCl_3$): δ = 0.76 (m, 6H), 1.14 (s, 49H), 1.35 (m, 1H), 1.90 (m, 2H), 2.50 (m, 4H), 2.96 (m, 2H), 3.81 (m, 1H), 3.97 (m, 1H), 4.04 (m, 1H), 4.11 (m, 1H), 5.33 (m, 1H), 5.58 (m, 1H).

MS (ESI): m/z = 667.5 (M+1)

**Example 7: Preparation of a precursor for moiety 400aj**

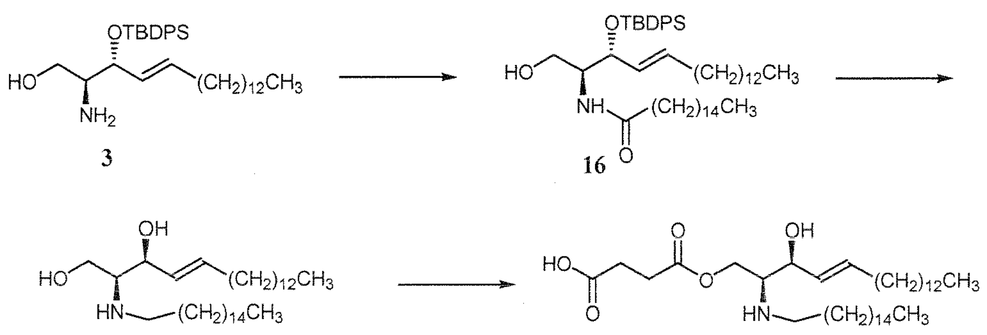

3 16 17 18

Compound **3** was prepared as described in example 4 above.

A solution of palmitic acid (0.77 g, 3.0 mmol) and HATU (1.142 g, 3.0 mmol) in a mixture of DMF/$CH_2Cl_2$ (1:1) (10 mL) was stirred for 5 minutes. DIPEA (0.825 g, 6.38 mmol) and a solution of 3 (1.345 g, 2.5 mmol) in $CH_2Cl_2$ (10 mL) were added to the reaction mixture and stirred at room temperature for 2 h and subsequently diluted with 100 mL $CH_2Cl_2$ and washed with 1 N HCl solution and extracted with $CH_2Cl_2$ (3 x 100 mL). The combined organic layers were dried over sodium sulfate and concentrated in vacuo. Purification of the residue by flash chromatography (silica, Hexane/EtOAc 4:1) yielded **16** as a waxy solid (1.685 g, 87%).

$^1$H-NMR (300 MHz, $CDCl_3$): δ = 0.84 (m, 6H), 1.04 (s, 6H), 1.24 (m, 44H), 1.49 (m, 4H), 1.86 - 2.33 (m, 6H), 2.79 - 2.95 (m, 2H), 3.58 (m, 1H), 3.85 (m, 2H), 4.32 (m, 1H), 5.38 (m, 2H), 5.92 (m, 1H), 7.35 (m, 6H), 7.59 (m, 4H).

MS (ESI): m/z = 776 (M+1)

A solution of **13** (217 mg, 0.28 mmol) in dry THF (15 mL) was cooled to 0˚C and a solution of $LiAlH_4$ (1M solution in THF) (0.842 mL, 0.84 mmol) was added dropwise. The mixture was stirred at 0˚C for 2 h and at room temperature for 16 h. The reaction was quenched with water (100 mL) and extracted with $CH_2Cl_2$ (3 x 100 mL). The combined organic layers were dried over sodium sulfate and concentrated in vacuo. Purification of the residue by flash chromatography (silica, EtOAc) yielded **17** as a white solid (83 mg, 57%).

$^1$H-NMR (300 MHz, $CDCl_3$): δ = 0.86 (m, 6H), 1.26 (s, 46H), 1.52(m, 5H), 2.05 (m, 2H), 2.43 (m, 2H), 2.70 (m, 2H), 3.42 (m, 1H), 3.73 (br s, 2H), 4.21 (m, 1H), 5.42 (m, 1H), 5.75 (m, 1H).

MS (ESI): m/z = 524.6 (M+1)

Succinic head group was attached as described in the general procedure to afford the product **18** (98 mg; 41%).

$^1$H-NMR (300 MHz, $CDCl_3$): δ = 0.86 (m, 6H), 1.26 (s, 46H), 1.52(m, 5H), 2.05 (m, 2H), 2.54 (m, 6H), 2.95 (m, 2H), 3.25 (m, 1H), 3.97 (m, 2H), 4.72 (m, 1H), 5.42 (m, 1H), 5.75 (m, 1H).

MS (ESI): m/z = 624.5 (M+1)

**Example 8: Preparation of succinic acid mono (2,3-di-eicosyloxycarbonyl-propyl) ester, a precursor for moiety 500aa**

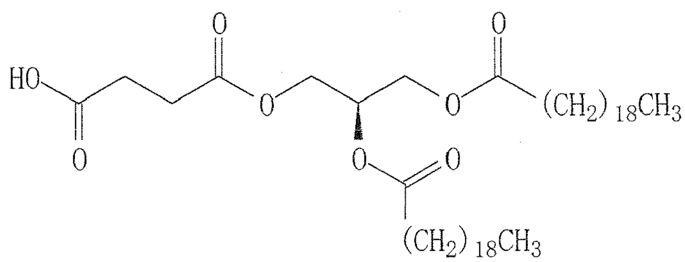

**3-O-p-methoxybenzyl-*sn*-glycerol**

A solution of 3.0 g (22.70 mmol) (*R*)-2,2-dimethyl-1,3-dioxalane-4-methanol in dry dimethyl formamide (60 ml) under argon atmosphere was cooled to 0°C and p-methoxybenzyl chloride (3.70 ml, 4.27 g, 27.24 mmol) was added. After 15 minutes NaH (0.625 g, 26.05 mmol) was added slowly. The reaction mixture was allowed to warm up to room temperature and was stirred for 20 h.

The reaction was quenched by adding 5 ml ethanol. The mixture was poured into aqueous saturated sodium chloride solution, and the aqueous layer was extracted twice with ethyl acetate. The combined organic layer was washed with water, dried with $Na_2SO_4$, filtered and evaporated to the p-methoxybenzyl derivative, which was used in the next step without further purification.

The material was dissolved in a mixture of methanol (60 ml) and acidic acid (50 ml) and stirred for 4 days at room temperature. The solvents were removed by continuous coevaporation with dioxane. The residue was purified by flash chromatography on silica gel (ethyl acetate) to give 3-O-p-methoxybenzyl-*sn*-glycerol (2.57 g, 12.10 mmol) as a colorless oil.

$^1$H-NMR (300 MHz, $CDCl_3$): δ = 7.24 (d, J = 8.6 Hz, 2 H), 6.88 (d, J = 8.6 Hz, 2 H), 4.42 (s, 3 H), 3.85 - 3.94 (m, 1 H), 3.81 (s, 3 H), 3.64 - 3.70 (m, 2 H), 3.52 - 3.56 (m, 2 H). $^{13}$C-NMR (125 MHz, $CDCl_3$): δ = 159.40 (C), 129.72 (C), 129.45 (CH), 113.90 (CH), 73.27 ($CH_2$), 71.53 ($CH_2$), 70.53 (CH), 64.12 ($CH_2$), 55.30 ($CH_3$).

**(*R*)-1,2-Di-eicosyloxycarbonyl-3-(p-methoxybenzyl)-*sn*-glycerol**

p-Methoxybenzyl glycerol (212 mg, 1 mmol), eicosanoic acid (781 mg, 2.5 mmol) and dimethylaminopyridine (24 mg, 0.2 mmol) were dissolved in dry dichloromethane (50 ml) and cooled to 0°C. Dicyclohexylcarbodiimide (516 mg, 2.5 mmol) was dissolved in 10 ml dry dichloromethane and was added slowly to the stirred reaction mixture. The reaction mixture was allowed to warm up to room temperature and stirred for 22 h. The solvent was removed under reduced pressure und the residue was purified by flash chromatography on silica gel (petroleum ether/ethyl acetate: 6: 1) to give (R)-1,2-di-eicosyloxycarbonyl-3-(p-methoxybenzyl)-sn-glycerol (724 mg, 90%).

$^1$H-NMR (500 MHz, $CDCl_3$): δ = 7.22 (d, J = 8.6 Hz, 2 H), 6.86 (d, J = 8.6 Hz, 2 H), 5.21 (m, 1 H), 4.45 (m, 2 H), 4.14 - 4.33 (m, 2 H), 3.79 (s, 3 H), 3.53 - 3.56 (m, 2 H), 2.30 (t, J = 7.5 Hz, 2 H), 2.28 (t, J = 7.5 Hz, 2 H), 1.54 - 1.62 (m, 4 H), 1.24 (s, br, 64 H), 0.87 (t, J = 6.8 Hz, 6 H).

$^{13}$C-NMR (125 MHz, $CDCl_3$): δ = 173.42 (C=O), 173.12 (C=O), 159.30 (C), 129.76(C), 129.29 (2 × CH), 113.80 (2 × CH), 72.95 ($CH_2$), 70.00 (CH), 67.89 ($CH_2$), 62.69 ($CH_2$), 55.25($CH_3$), 34.33 ($CH_2$), 34.12 ($CH_2$), 31.92 ($CH_2$), 29.70 ($CH_2$), 29.66 ($CH_2$), 29.50 ($CH_2$), 29.36 ($CH_2$), 29.30 ($CH_2$), 29.13 ($CH_2$), 29.09 ($CH_2$), 24.96 ($CH_2$), 24.88 ($CH_2$), 22.69 ($CH_2$), 14.12 (2 × $CH_3$).

MS (ESI): 823.9 (M + $Na^+$).

**(*R*)-1,2-Di-eicosyloxycarbonyl-*sn*-glycerol**

(R)-1,2-Di-eicosyloxycarbonyl-3-(p-methoxybenzyl)-sn-glycerol (434 mg, 0.55 mmol) and DDQ 2,3-dichloro-5,6-dicyano-1,4-benzoquinone (160 mg, 0.70 mmol) were dissolved in 25 ml dichloromethane. Water (1,5 ml) was added and the mixture was stirred under air for 24 hours.

The solution was filtered and an aqueous saturated sodium bicarbonate solution was added. The aqueous layer was extracted twice with dichloromethane (2 x 50 ml) and the combined organic layer was washed with an aqueous

saturated sodium bicarbonate solution and an aqueous saturated sodium chloride solution, dried with $Na_2SO_4$, filtered and the solvent was removed under reduced pressure. The residue could be used without further purification.
$^1$H-NMR (500 MHz, $CDCl_3$): δ = 5.07 (m, 1 H), 4.21 - 4.33 (m, 2 H), 3.72 (d, J = 4.4 Hz, 2 H), 2.33 (t, J = 7.5 Hz, 2 H), 2.31 (t, J = 7.5 Hz, 2 H), 1.57 - 1.65 (m, 4 H), 1.26 (s, br, 64 H), 0.87 (t, J = 6.8 Hz, 6 H).
$^{13}$C-NMR (125 MHz, $CDCl_3$): δ =173.80 (C=O), 173.43 (C=O), 72.09 (CH), 61.96 ($CH_2$), 61.57 ($CH_2$), 34.29 ($CH_2$), 34.10 ($CH_2$), 31.92 ($CH_2$), 29.70 ($CH_2$), 29.66 ($CH_2$), 29.62 ($CH_2$), 29.48 ($CH_2$), 29.36 ($CH_2$), 29.27 ($CH_2$), 29.12 ($CH_2$), 29.09 ($CH_2$), 24.94 ($CH_2$), 24.89 ($CH_2$), 22.69 ($CH_2$), 14.12 (2 × $CH_3$).
MS (ESI): 703.6 (M + $Na^+$).

**Succinic acid mono (2,3-di-eicosyloxycarbonyl-propyl) ester**

(R)-1,2-Di-eicosyloxycarbonyl-sn-glycerol (272 mg, 0.4 mmol), succinic anhydride (50 mg, 0.5 mmol) and dimethylaminopyridine (61 mg, 0.5 mmol) were dissolved in dry dichloromethane (25 ml). The reaction mixture was stirred for 20 hours.

Aqueous saturated sodium chloride solution (5 ml) was added and the mixture was stirred for 5 minutes. The mixture was diluted with dichloromethane (50 ml) and water (20 ml). The aqueous layer was extracted twice with dichloromethane (25 ml). The combined organic layer was dried ($Na_2SO_4$), filtered and the solvent was removed. The residue was purified by flash chromatography on silica gel (petroleum ether/ethyl acetate: 1:1) to give succinic acid mono (2,3-di-eicosyloxycarbonyl-propyl) ester (204 mg, 0.26 mmol) as a colorless powder.
**$^1$H-NMR (500 MHz, $CDCl_3$):** δ = 5.26 (m, 1 H), 4.26 - 4.33 (m, 2 H), 4.11-4.19 (m, 2 H),2.62 - 2.69 (m, 4 H), 2.31 (t, J = 7.5 Hz, 2 H), 2.30 (t, J = 7.5 Hz, 2 H), 1.56 - 1.63 (m, 4 H), 1.24 (s, br, 64 H), 0.87 (t, J = 6.8 Hz, 6 H).
**$^{13}$C-NMR (125 MHz, $CDCl_3$):** δ = 175.68 (C=O), 173.37 (C=O), 172.95 (C=O), 171.58 (C=O), 68.71 (CH), 62.63 ($CH_2$), 62.01 ($CH_2$), 34.17 ($CH_2$), 34.04 ($CH_2$), 31.92 ($CH_2$), 29.70 ($CH_2$), 29.66 ($CH_2$), 29.49 ($CH_2$), 29.36 ($CH_2$), 29.28 ($CH_2$), 29.12 ($CH_2$), 29.07 ($CH_2$), 28.71 ($CH_2$), 28.46($CH_2$), 24.89 ($CH_2$), 24.88 ($CH_2$), 22.69 ($CH_2$), 14.12 (2 × $CH_3$).
**MS (ESI):** 803.9 (M + $Na^+$).

**Example 9: Preparation of a precursor for moiety 500ae**

The precursor for compound **500ae** was obtained by the following reaction sequence.

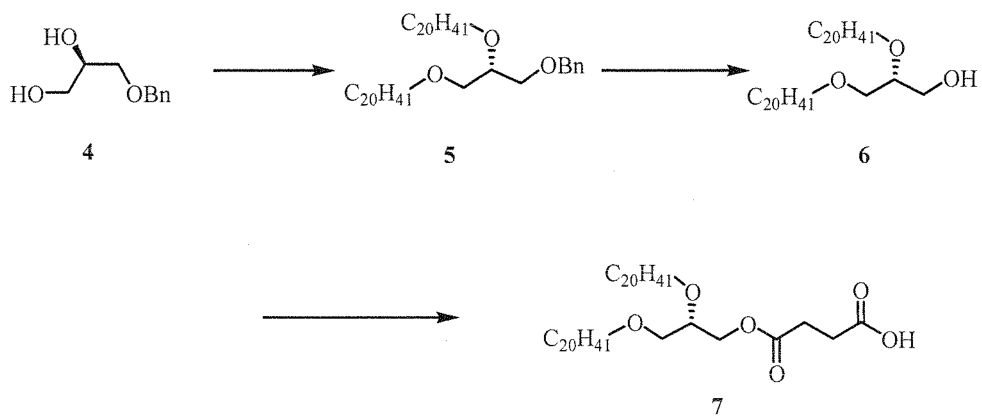

4 5 6

7

**(*R*)-1,2-*O*-Di-eicosyl-3-*O*-benzyl-*sn*-glycerol (5)**

To a solution of 3-*O*-benzyl-*sn*-glycerol **4** (182 mg, 1 mmol) in dry DMF (20 mL) was added eicosylbromid (904 mg, 2.5 mmol) and NaH (60%, 100 mg, 2.5 mmol) under argon atmosphere and heated at 100°C for 22 h and further at 130°C for 24 h. Reaction mixture was cooled to room temperature and diluted with $CH_2Cl_2$ (50 mL) and washed with $H_2O$. The combined organic layers were dried over sodium sulfate and concentrated in vacuo. Purification of the residue by flash chromatography (silica, PE/EtOAc 20:1) yielded compound **5** (493 mg, 66 %) as a colourless waxy solid.
$^1$H-NMR (300 MHz, $CDCl_3$): δ = 7.28 - 7.34 (m, 5 H), 4.55 (s, 2 H), 3.48 - 3.59 (m, 5 H), 3.42 (t, J = 6.6 Hz, 2 H), 1.52 - 1.57 (m, 4 H) ,1.20 - 1.35 (m, 68 H), 0.88 (t, *J* = 6.7 Hz, 6 H). MS (ESI): 743.7 (M + $H^+$), 760.7 (M + $NH_4^+$)

**(*R*)-1,2-*O*-Di-eicosyl-3-*O*-benzyl-*sn*-glycerol (6)**

The benzyl group was removed as described in the general procedure to obtain compound **6** (349 mg, 81 %)) as a colourless waxy solid.
$^{1}$H-NMR (300 MHz, $CDCl_3$): δ = 3.41 - 3.71 (m, 9 H), 1.52 - 1.57 (m, 4 H) ,1.20 - 1.39 (m, 68 H), 0.88 (t, *J* = 6.7 Hz, 6 H).
$^{13}$C-NMR (125 MHz, $CDCl_3$): δ = 78.24 (CH), 71.86 ($CH_2$), 70.93 ($CH_2$), 70.39 ($CH_2$), 63.13 ($CH_2$), 31.92 ($CH_2$), 30.08 ($CH_2$), 29.70 ($CH_2$), 29.62 ($CH_2$), 29.47 ($CH_2$), 29.36 ($CH_2$), 26.10 ($CH_2$), 22.68 ($CH_2$), 14.10 (2 x $CH_3$).
MS (ESI): 670.7 (M + $NH_4^+$), 675.7 (M + $Na^+$)

**(*R*)-1,2-*O*-Di-eicosyl-*sn*-glycerol-3-succinate (7)**

Succinic head group was attached as described in the general procedure to obtain compound 7 (345.7 mg, 90%) as a colourless waxy solid.
$^{1}$H-NMR (300 MHz, $CDCl_3$): δ = 4.25 (dd, *J* = 11.5, 4.3 Hz, 2 H), 3.60 - 3.63 (m, 1 H), 3.54 (t, *J* = 6.7 Hz, 2 H), 3.39 - 3.47 (m, 4 H), 2.66 (t, *J* = 3.2 Hz, 4 H) 1.47 - 1.55 (m, 4 H) ,1.20 - 1.39 (m, 68 H), 0.87 (t, *J* = 6.7 Hz, 6 H).
$^{13}$C-NMR (125 MHz, $CDCl_3$): δ = 171.99 (C=O), 171.16 (C=O), 77.43 (CH), 71.80 ($CH_2$), 70.68 ($CH_2$), 70.23 ($CH_2$), 64.41 ($CH_2$), 60.39 (2 x $CH_2$), 31.92 ($CH_2$), 29.99 ($CH_2$), 29.70 ($CH_2$), 29.64 ($CH_2$), 29.48 ($CH_2$), 29.35 ($CH_2$), 28.87 ($CH_2$), 26.08 ($CH_2$), 26.04 ($CH_2$), 22.68 ($CH_2$), 14.18 ($CH_3$), 14.10 ($CH_3$).
MS (ESI): 770.8 (M + $NH_4^+$), 775.7 (M + $Na^+$)

**Example 10: Preparation of a precursor for moiety 700a**

The precursor for compound **700a** was obtained by the following reaction sequence.

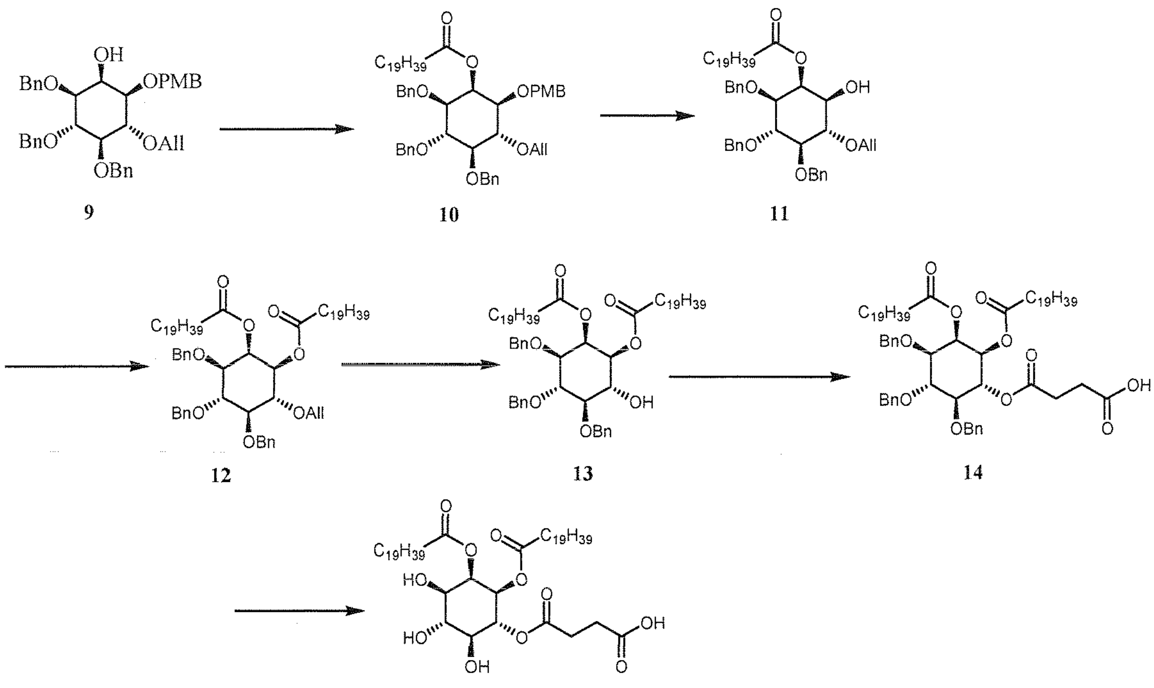

9 10 11

12 13 14

15

Compound **9** was obtained as per literature (J. Org. Chem. 2003, 68, 4020).
Esterification was performed as described in the general procedure to obtain compound **10** (583 mg, 98 %) as a waxy solid.
$^{1}$H-NMR (500 MHz, $CDCl_3$): δ = 7.25 - 7.34 (m, 17 H), 6.85 (d, *J* = 8.6 Hz, 2 H), 5.94 - 6.00 (m, 1 H), 5.82 (t, *J* = 2.7 Hz, 1 H), 5.26 (dd, *J* = 17.2, 1.6 Hz, 1 H), 5.15 (dd, *J* = 10.4, 1.3 Hz, 1 H), 4.80 - 4.87 (m, 3 H), 4.76 (d, *J* = 10.6 Hz, 1 H), 4.71 (d, *J* = 11.2 Hz, 1 H), 4.63 (d, *J* = 10.8 Hz, 1 H), 4.50 (d, *J* = 11.2 Hz, 1 H), 4.44 (d, *J* = 10.8 Hz, 1 H), 4.36 (dd, *J* = 12.1, 5.7 Hz, 1 H), 4.25 (dd, *J* = 12.1, 5.7 Hz, 1H), 3.81 (t, *J* = 9.6 Hz, 1 H), 3.80 (s, 3 H), 3.69 (t, *J* = 9.5 Hz, 1 H), 3 . 3 6 - 3.45 (m, 3 H), 2.36 (t, *J* = 7.3 Hz, 2 H), 1.58 - 1.63 (m, 2 H), 1.16 - 1.29 (m, 32 H), 0.87 (t, *J* = 6.9 Hz, 3 H).

$^{13}$C-NMR (125 MHz, $CDCl_3$): δ = 173.28 (C=O), 159.24 (CH), 138.71 (C), 138.52 (C), 137.72 (C), 135.30 (CH), 129.93 (C), 129.62 (CH), 128.41 (CH), 128.32 (CH), 128.21 (CH), 128.11 (CH), 127.98 (CH), 127.72 (CH), 127.57 (CH), 116.63 ($CH_2$), 113.71 (CH), 82.91 (CH), 81.36 (CH), 81.12 (CH), 78.34 (CH), 77.90 (CH), 76.29 ($CH_2$), 75.91 ($CH_2$), 74.61 ($CH_2$), 72.11 ($CH_2$), 66.37 (CH), 55.23 ($CH_3$), 34.51 ($CH_2$), 32.79($CH_2$), 31.91 ($CH_2$), 30.90 ($CH_2$), 29.70 ($CH_2$), 29.65 ($CH_2$), 29.61 ($CH_2$), 29.50 ($CH_2$), 29.47 ($CH_2$), 29.40 ($CH_2$), 29.35 ($CH_2$), 29.27 ($CH_2$), 28.97 ($CH_2$), 26.41 ($CH_2$), 25.51 ($CH_2$), 25.45 ($CH_2$), 25.32 ($CH_2$), 24.70 ($CH_2$), 22.68 ($CH_2$), 14.12 ($CH_3$).

To a solution of **10** (213 mg, 0.24 mmol) in a mixture of acetonitril (23 mL), toluene (2 mL) and water (1 mL) at 0°C was added cerium ammonium nitrate (645 mg, 1.18 mmol) and stirred for 30 minutes at 0°C and than, for 2 h on warming to room temperature. Reaction mixture was diluted with EtOAc and washed with sat. $NaHCO_3$ solution. The combined organic layers were dried over sodium sulfate and concentrated in vacuo. Purification of the residue by flash chromatograph (silica, PE/EtOAc 5:1) yielded compound **11** (162 mg, 88 %) as a colourless oil.

$^{1}$H-NMR (500 MHz, $CDCl_3$): δ = 7.25 - 7.34 (m, 15 H), 5.90 - 5.98 (m, 1 H), 5.74 (t, *J* = 2.6 Hz, 1 H), 5.27 (dd, *J* = 17.2, 1.5 Hz, 1 H), 5.18 (dd, *J* = 10.4, 1.3 Hz, 1 H), 4.89 (d, *J* = 10.6 Hz, 1 H), 4.88 (d, *J* = 10.5 Hz, 1 H), 4.72 - 4.79 (m, 3 H), 4.49 (d, *J* = 11.2 Hz, 1 H), 4.42 (dd, *J* = 12.5, 5.5 Hz, 1 H), 4.23 (dd, *J* = 12.5, 6.0 Hz, 1H), 3.82 (t, *J* = 9.5 Hz, 1 H), 3.58 - 3.64 (m, 2 H), 3.51 (dd, *J* = 9.6, 2.8 Hz, 1H), 3.46 (t, *J* = 9.2 Hz, 1 H), 2.39 (t, *J* = 7.4 Hz, 2 H), 2.29 (s, br 1 H) 1.60 -1.67 (m, 2 H), 1.16 - 1.29 (m, 32 H), 0.87 (t, *J* = 6.9 Hz, 3 H).

$^{13}$C-NMR (125 MHz, $CDCl_3$): δ = 173.28 (C=O), 138.57 (C), 138.28 (C), 137.61 (C), 134.78 (CH), 129.45 (CH), 128.34 (CH), 128.21 (CH), 128.08 (CH), 127.93 (CH), 127.79 (CH), 127.76 (CH), 127.61 (CH), 117.39 ($CH_2$), 83.12 (CH), 81.89 (CH), 81.17 (CH), 78.50 (CH), 75.96 ($CH_2$), 75.93 ($CH_2$), 74.34 ($CH_2$), 72.08 ($CH_2$), 70.17 (CH), 68.85 (CH), 34.50 ($CH_2$), 29.69 ($CH_2$), 29.66 ($CH_2$), 29.52 ($CH_2$), 29.37 ($CH_2$), 29.35 ($CH_2$), 29.02 ($CH_2$), 25.24 ($CH_2$), 22.68 ($CH_2$), 14.12 ($CH_3$).

Esterification was performed as described in the general procedure to obtain compound **12** (271 mg, 91 %) as a waxy solid.

$^{1}$H-NMR (500 MHz, $CDCl_3$): δ = 7.26 - 7.32 (m, 15 H), 5.84 - 5.90 (m, 1 H), 5.73 (t, *J* = 2.7 Hz, 1 H), 5.22 (dd, *J* = 17.2, 1.5 Hz, 1 H), 5.12 (dd, *J* = 10.5, 1.3 Hz, 1 H), 4.80 - 4.88 (m, 4 H), 4.76 (d, *J* = 10.6 Hz, 1 H), 4.67 (d, *J* = 11.1 Hz, 1 H), 4.46 (d, *J* = 11.1 Hz, 1 H), 4.27 (dd, *J* = 12.5, 5.5 Hz, 1 H), 4.17 (dd, *J* = 12.5, 6.0 Hz, 1 H), 3.81 (t, *J* = 9.6 Hz, 1 H), 3.77 (t, *J* = 9.8 Hz, 1 H), 3.58 (dd, *J* = 9.7, 2.7 Hz, 1 H), 3.49 (t, *J* = 9.4 Hz, 1 H) 2.35 (t, *J* = 7.3 Hz, 2 H), 2.27 (t, *J* = 7.4 Hz ,2 H) 1.60 - 1.67 (m, 4 H), 1.16 -1.29 (m, 64 H), 0.87 (t, *J* =6.9 Hz, 6 H).

$^{13}$C-NMR (125 MHz, $CDCl_3$): δ = 172.85 (C=O), 172.82 (C=O), 138.55 (C), 138.29 (C), 137.50 (C), 134.82 (CH), 128.44 (CH), 128.33 (CH), 128.19 (CH), 128.11 (CH), 127.93 (CH), 127.80 (CH), 127.76 (CH), 127.61 (CH), 116.68 ($CH_2$), 82.84 (CH), 81.30 (CH), 79.07 (CH), 78.10 (CH), 76.31 ($CH_2$), 75.95 ($CH_2$), 74.38 ($CH_2$), 72.14 ($CH_2$), 71.30 (CH), 67.37 (CH), 34.40 ($CH_2$), 34.26 ($CH_2$), 31.91 ($CH_2$), 29.71 ($CH_2$), 29.66 ($CH_2$), 29.58 ($CH_2$), 29.48 ($CH_2$), 29.41 ($CH_2$), 29.36 ($CH_2$), 29.32 ($CH_2$), 29.13 ($CH_2$), 29.04 ($CH_2$), 25.34 ($CH_2$), 24.77 ($CH_2$), 14.12 (2 × $CH_3$).

Allyl group was removed as per the general procedure to obtain compound **13** (199 mg, 79 %) as a colourless oil.

$^{1}$H-NMR (500 MHz, $CDCl_3$): δ = 7.26 - 7.35 (m, 15 H), 5.75 (t, *J* = 2.7 Hz, 1 H), 4.95 (d, J = 11.1 Hz, 1 H), 4.91 (d, J = 10.7 Hz, 1 H), 4.73 - 4.79 (m, 4 H), 4.68 (d, J = 11.1 Hz, 1 H), 4.47 (d, *J* = 11.1 Hz, 1 H), 3.98 (t, *J* = 10.0 Hz, 1 H), 3.84 (t, *J* = 9.5 Hz, 1 H), 3.60 (dd, *J* = 9.7, 2.8 Hz, 1 H), 3.40 (t, *J* = 9.3 Hz, 1 H) 2.35 (t, *J* = 7.3 Hz, 2 H), 2.28 (t, *J* = 7.4 Hz , 2 H) 1.55 - 1.64 (m, 4 H), 1.16 - 1.31 (m, 64 H), 0.87 (t, *J* = 6.9 Hz, 6 H).

$^{13}$C-NMR (125 MHz, $CDCl_3$): δ = 173.18 (C=O), 172.83 (C=O), 138.42 (C), 138.23 (C), 137.42 (C), 128.64 (CH), 128.38 (CH), 128.36 (CH), 128.14 (CH), 128.06 (CH), 128.02 (CH), 127.98 (CH), 127.94 (CH), 127.81 (CH), 127.69 (CH), 82.72 (CH), 81.08 (CH), 78.71 (CH), 75.90 ($CH_2$), 75.80 ($CH_2$), 72.12 ($CH_2$), 71.05 (CH), 70.95 (CH), 67.18 (CH), 34.34 ($CH_2$), 34.13 ($CH_2$), 31.91 ($CH_2$), 29.71 ($CH_2$), 29.65 ($CH_2$), 29.55 ($CH_2$), 29.46 ($CH_2$), 29.36 ($CH_2$), 29.29 ($CH_2$), 29.06 ($CH_2$), 29.05 ($CH_2$), 25.26 ($CH_2$), 24.75 ($CH_2$), 22.68 ($CH_2$), 14.12 (2 × $CH_3$).

Succinic head group was attached as described in the general procedure to obtain compound 14 (126 mg, 62 %) as a colourless oil.

$^{1}$H-NMR (500 MHz, $CDCl_3$): δ = 7.22 - 7.32 (m, 15 H), 5.73 (t, *J* = 2.7 Hz, 1 H), 5.48 (t, J = 10.2 Hz), 4.83 - 4.89 (m, 3 H), 4.73 (d, J = 10.6 Hz, 1 H), 4.69 (d, J = 11.1 Hz, 1 H), 4.62 (d, J = 11.3 Hz, 1 H), 4.47 (d, *J* = 10.1 Hz, 1 H), 3.91 (t, *J* = 9.5 Hz, 1 H), 3.60 (dd, *J* = 9.7, 2.8 Hz, 1 H), 3.53 (t, *J* = 9.5 Hz, 1 H) 2.32 - 2.53 (m, 6 H), 2.21 (t, *J* = 7.4 Hz ,2 H) 1.50 - 1.70 (m, 4 H), 1.16 -1.31 (m, 64 H), 0.87 (t, *J* = 6.9 Hz, 6 H).

$^{13}$C-NMR (125 MHz, $CDCl_3$): δ = 172.81 (C=O), 173.06 (C=O), 172.91 (C=O), 170.86 (C=O), 138.32 (C), 138.13 (C), 137.36 (C), 128.42 (CH), 128.36 (CH), 128.11 (CH), 128.03 (CH), 127.91 (CH), 127.85 (CH), 127.78 (CH), 127.71 (CH), 81.30 (CH), 80.56 (CH), 77.91 (CH), 76.00 ($CH_2$), 75.74 ($CH_2$), 72.19 ($CH_2$), 71.63 (CH), 69.20 (CH), 66.96 (CH), 34.27 ($CH_2$), 33.98 ($CH_2$), 31.93 ($CH_2$), 29.72 ($CH_2$), 29.66 ($CH_2$), 29.55 ($CH_2$), 29.49 ($CH_2$), 29.36 ($CH_2$), 29.30 ($CH_2$), 29.07 ($CH_2$), 29.05 ($CH_2$), 28.75 ($CH_2$), 28.21 ($CH_2$), 25.20 ($CH_2$), 24.70 ($CH_2$), 22.67 ($CH_2$), 14.11 (2 × $CH_3$).

Benzyl groups were removed as per the general procedure to obtain compound **15.**

MS (ESI): 886.7 (M + $NH_4^+$), 867.4 (M - $H^+$)

**Example 11: Preparation of a precursor for moiety 700b**

The precursor for compound **700b** was obtained by the following reaction sequence.

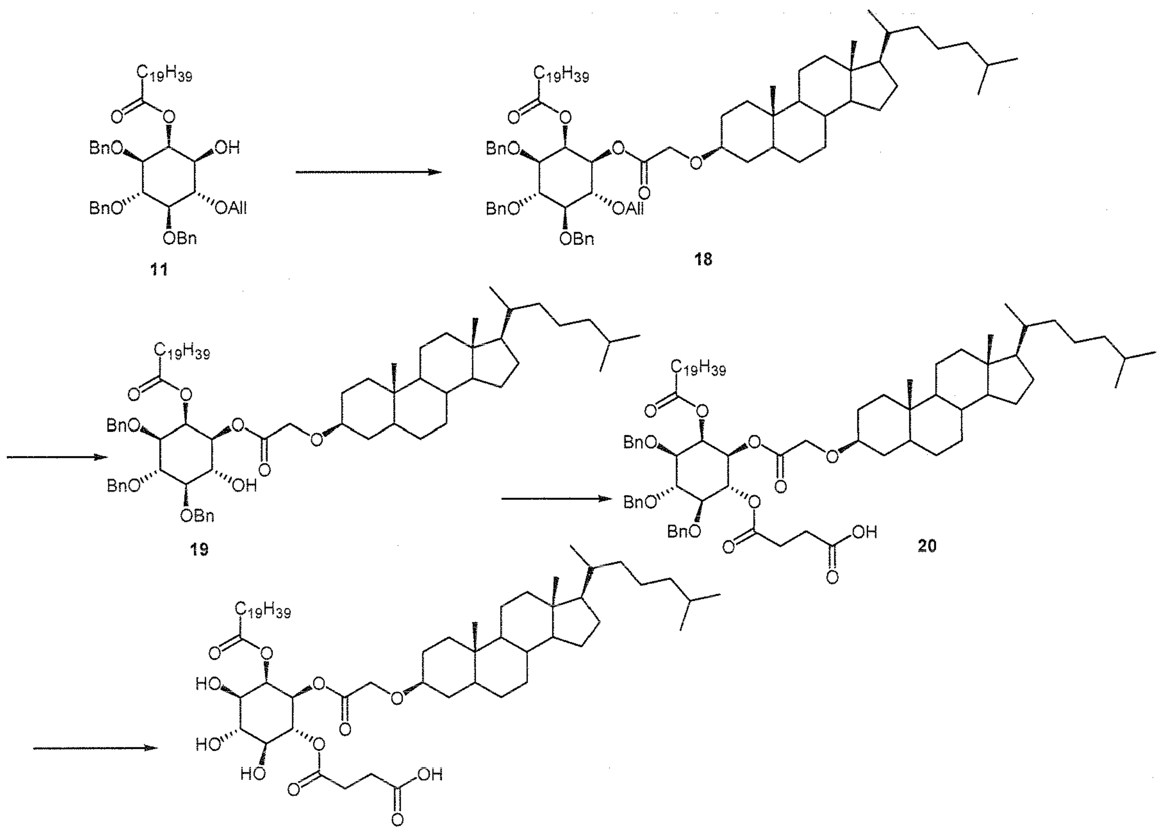

**11** **18** **19** **20** **21**

Esterification was performed as described in the general procedure to obtain compound **18** from compound **11** (250 mg, 99 %) as a waxy solid.
$^1$H-NMR (500 MHz, $CDCl_3$): δ = 7.26 - 7.32 (m, 15 H), 5.83 - 5.89 (m, 1H), 5.72 (t, *J* = 2.7 Hz, 1 H), 5.21 (dd, *J* = 17.2, 1.6 Hz, 1 H), 5.18 (dd, *J* = 10.4, 1.3 Hz, 1 H), 4.90 (dd, *J* = 10.3, 2.8 Hz, 1 H), 4.87 (d, J = 10.7 Hz, 1 H), 4.85 (d, J = 10.5 Hz, 1 H), 4.81 (d, J = 10.4 Hz, 1 H), 4.77 (d, J = 10.6 Hz, 1 H), 4.67 (d, J = 11.1 Hz, 1 H), 4.47 (d, *J* = 11.1 Hz, 1 H), 4.27 (dd, *J* = 12.5, 5.6 Hz, 1 H), 4.15 (dd, *J* = 12.3, 5.3 Hz, 1H), 4.07 (d, J = 2.1 Hz, 2 H), 3.82 (t, *J* = 9.5 Hz, 1 H), 3.77 (t, *J* = 9.8 Hz, 1 H), 3.58 (dd, *J* = 9.7, 2.7 Hz, 1 H), 3.50 (t, *J* = 9.4 Hz, 1 H), 3.30 - 3.40 (m, 1 H), 2.35 (dt, *J* = 7.4, 2.1 Hz, 2 H), 2.30 (m, 1 H), 1.93 - 1.97 (m, 1 H), 1.78 -1.86 (m, 2 H), 1.71 (dt, *J* = 6.8, 3.5 Hz, 1 H), 1.58 - 1.66 (m, 4 H), 1.56 (s, 3 H), 1.45 - 1.54 (m, 4 H), 1.35 - 1.41 (m, 1 H), 1.19 -1.33 (m, 32 H), 1.16 (s, 1 H), 0.93 -1.15 (m, 11 H), 0.84 - 0.92 (m, 15 H), 0.79 (s, 3 H), 0.64 (s, 3 H),0.60 - 0.62 (m, 1 H). $^{13}$C-NMR (125 MHz, $CDCl_3$): δ = 172.85 (C=O), 170.32 (C=O), 138.52 (C), 138.24 (C), 137.44 (C), 134.81 (CH), 128.44 (CH), 128.35 (CH), 128.19 (CH), 128.09 (CH), 127.92 (CH), 127.82 (CH), 127.80 (CH), 127.63 (CH), 116.66 ($CH_2$), 82.74 (CH), 81.97 (CH), 79.40 (CH), 78.97 (CH), 76.30 ($CH_2$), 75.95 ($CH_2$), 74.34 ($CH_2$), 72.19 ($CH_2$), 71.83 (CH), 67.34 (CH), 65.19 ($CH_2$), 60.39 ($CH_2$), 56.46 (CH), 56.27 (CH), 54.33 (CH), 44.75 (CH), 42.57 (C), 40.01 ($CH_2$), 39.49 ($CH_2$), 36.85 ($CH_2$), 36.15 ($CH_2$), 35.78 (CH), 35.69 (C), 35.46 (CH), 34.41 ($CH_2$), 34.35 ($CH_2$), 32.07 ($CH_2$), 31.92 ($CH_2$), 29.75 ($CH_2$), 29.72 ($CH_2$), 29.66 ($CH_2$), 29.62 ($CH_2$), 29.45 ($CH_2$), 29.36 ($CH_2$), 29.08 ($CH_2$), 28.77 ($CH_2$), 28.00 ($CH_3$), 27.78 ($CH_2$), 25.29 ($CH_2$), 24.20 ($CH_2$), 23.82 ($CH_2$), 22.81 ($CH_3$), 22.69 ($CH_2$), 22.55 ($CH_3$), 21.23 ($CH_2$), 21.05 (CH), 18.65 ($CH_3$), 14.13 ($CH_3$), 12.26 ($CH_3$), 12.05 ($CH_3$).
MS (ESI): 1230.9 (M + $NH_4^+$), 1235.9 (M + $Na^+$).

Allyl group was removed as per the general procedure to obtain compound **19** (190 mg, 86%) as a waxy solid.
$^1$H-NMR (500 MHz, $CDCl_3$): δ = 7.26 - 7.35 (m, 15 H), 5.74 (t, *J* = 2.7 Hz, 1 H), 4.95 (d, J = 11.1 Hz, 1 H), 4.91 (d, J = 10.7 Hz, 1 H), 4.84 (dd, *J* = 10.4 2.8 Hz, 1 H), 4.76 (d, J = 10.7 Hz, 1 H), 4.73 (d, J = 11.1 Hz, 1 H), 4.68 (d, J = 11.1 Hz, 1 H), 4.48 (d, *J* = 11.1 Hz, 1 H), 4.03 - 4.14 (m, 2 H), 3.98 (t, *J* = 9.8 Hz, 1 H), 3.84 (t, *J* = 9.5 Hz, 1 H), 3.61 (dd, *J* = 9.7, 2.8 Hz, 1H), 3.40 (t, *J* = 9.3 Hz, 1 H), 3.28 - 3.37 (m, 1 H), 2.35 (dt, *J* = 7.5, 3.6 Hz, 2 H), 2.25 (br, 1 H), 1.93 -1.97 (m, 1 H), 1.74 - 1.86 (m, 2 H), 1.71 (m, 1 H), 1.43 - 1.68 (m, 11 H), 1.35 -1.41 (m, 1 H), 1.19 - 1.33 (m, 34 H), 0.93 -

1.17 (m, 11 H), 0.84 - 0.92 (m, 15 H), 0.78 (s, 3 H), 0.63 (s, 3 H),0.60 - 0.62 (m, 1 H).
$^{13}$C-NMR (125 MHz, $CDCl_3$): $\delta$ = 172.82 (C=O), 170.62 (C=O), 138.39 (C), 138.19 (C), 137.36 (C), 128.66 (CH), 128.39 (CH), 128.12 (CH), 128.08 (CH), 127.98 (CH), 127.85 (CH), 127.71 (CH), 82.60 (CH), 81.05 (CH), 79.48 (CH), 79.18 (CH), 75.93 ($CH_2$), 75.82 ($CH_2$), 72.17 ($CH_2$), 71.60 (CH), 70.84 (CH), 67.15 (CH), 65.16 ($CH_2$), 60.40 (C), 56.48 (CH), 56.28 (CH), 54.31 (CH), 44.75 (CH), 42.58 (C), 40.02 ($CH_2$), 39.50 ($CH_2$), 36.85 ($CH_2$), 36.16 ($CH_2$), 35.79 (CH), 35.69 (C), 35.46 (CH), 34.43 ($CH_2$), 34.30 ($CH_2$), 32.07 ($CH_2$), 31.93 ($CH_2$), 29.75 ($CH_2$), 29.67 ($CH_2$), 29.60 ($CH_2$), 29.42 ($CH_2$), 29.37 ($CH_2$), 29.09 ($CH_2$), 28.77 ($CH_2$), 28.25 (CH), 28.00 ($CH_2$), 27.76 ($CH_2$), 25.22 ($CH_2$), 24.21 ($CH_2$), 22.81 ($CH_3$), 22.70 ($CH_2$), 22.55 (CH), 21.23 ($CH_2$), 18.65 ($CH_3$), 14.13 ($CH_3$), 12.26 ($CH_3$), 12.06 ($CH_3$).
MS (ESI): 1190.9 (M + $NH_4$+), 1195.9 (M + $Na^+$).

Succinic head group was attached as described in the general procedure to obtain compound **20** (150 mg, 90%) as a waxy solid.
$^1$H-NMR (500 MHz, $CDCl_3$): $\delta$ = 7.20 - 7.32 (m, 15 H), 5.70 (t, *J* = 2.7 Hz, 1 H), 5.48 (t, *J* = 10.1 Hz, 1 H), 4.97 (dd, *J* = 10.6, 2.8 Hz, 1 H), 4.87 (d, J = 10.6 Hz, 1 H), 4.83 (d, J = 11.4 Hz, 1 H), 4.74 (d, J = 10.6 Hz, 1 H), 4.67 (d, J = 11.2 Hz, 1 H), 4.60 (d, J = 11.4 Hz, 1 H), 4.49 (d, *J* = 11.2 Hz, 1 H), 4.00 - 4.14 (m, 2 H), 3.93 (t, *J* = 9.5 Hz, 1 H), 3.60 (dd, *J* = 9.5, 2.8 Hz, 1H), 3.52 (t, *J* = 9.5 Hz, 1 H), 3.33 - 3.38 (m, 1 H), 2.29 - 2.54 (m, 6 H), 2.25 (br, 1 H), 1.93 - 1.97 (m, 1 H), 1.75 - 1.84 (m, 2 H), 1.69 - 1.73 (m, 1 H), 1.58 - 1.65 (m, 5 H), 1.42 - 1.57 (m, 5 H), 1.19 - 1.40 (m, 35 H), 0.93 - 1.17 (m, 11 H), 0.84 - 0.92 (m, 15 H), 0.77 (s, 3 H), 0.63 (s, 3 H), 0.56 - 0.61 (m, 1 H).
$^{13}$C-NMR (125 MHz, $CDCl_3$): $\delta$ = 173.28 (C=O), 173.03 (C=O), 170.75 (C=O), 170.11 (C=O), 138.24 (C), 138.02 (C), 137.24 (C), 128.45 (CH), 128.40 (CH), 128.37 (CH), 128.08 (CH), 128.03 (CH), 127.98 (CH), 127.90 (CH), 127.81 (CH), 127.74 (CH), 81.25 (CH), 80.47 (CH), 80.14 (CH), 77.69 (CH), 76.02 ($CH_2$), 75.78 ($CH_2$), 72.24 ($CH_2$), 71.09 (CH), 69.60 (CH), 67.02 (CH), 65.02 ($CH_2$), 60.40 (C), 56.44 (CH), 56.28 (CH), 54.29 (CH), 44.76 (CH), 42.56 (C), 39.99 ($CH_2$), 39.49 ($CH_2$), 36.79 ($CH_2$), 36.15 ($CH_2$), 35.78 (CH), 35.64 (C), 35.44 (CH), 34.34 ($CH_2$), 34.25 ($CH_2$), 32.04 ($CH_2$), 31.92 ($CH_2$), 29.74 ($CH_2$), 29.72 ($CH_2$), 29.66 ($CH_2$), 29.61 ($CH_2$), 29.42 ($CH_2$), 29.36 ($CH_2$), 29.08 ($CH_2$), 28.70 ($CH_2$), 28.23 (CH), 28.00 ($CH_2$), 27.49 ($CH_2$), 25.16 ($CH_2$), 24.19 ($CH_2$), 23.83 ($CH_2$), 22.81 ($CH_3$), 22.69 ($CH_2$), 22.55 (CH), 21.22 ($CH_2$), 18.64 ($CH_3$), 14.12 ($CH_3$), 12.23 ($CH_3$), 12.05 ($CH_3$).
MS (ESI): 1290.8 (M + $NH_4^+$), 1295.9 (M + $Na^+$), 1271.7 (M - $H^+$).

Benzyl groups were removed as per the general procedure to obtain compound **21** (81.9 mg, 80%) as a colourless solid.
MS (ESI): 1020.8 (M + $NH_4^+$), 1025.8 (M + $Na^+$), 1001.5 (M - $H^+$).

**Example 12: Preparation of a precursor for moiety 700c**

The precursor for compound **700c** was obtained by the following reaction sequence.

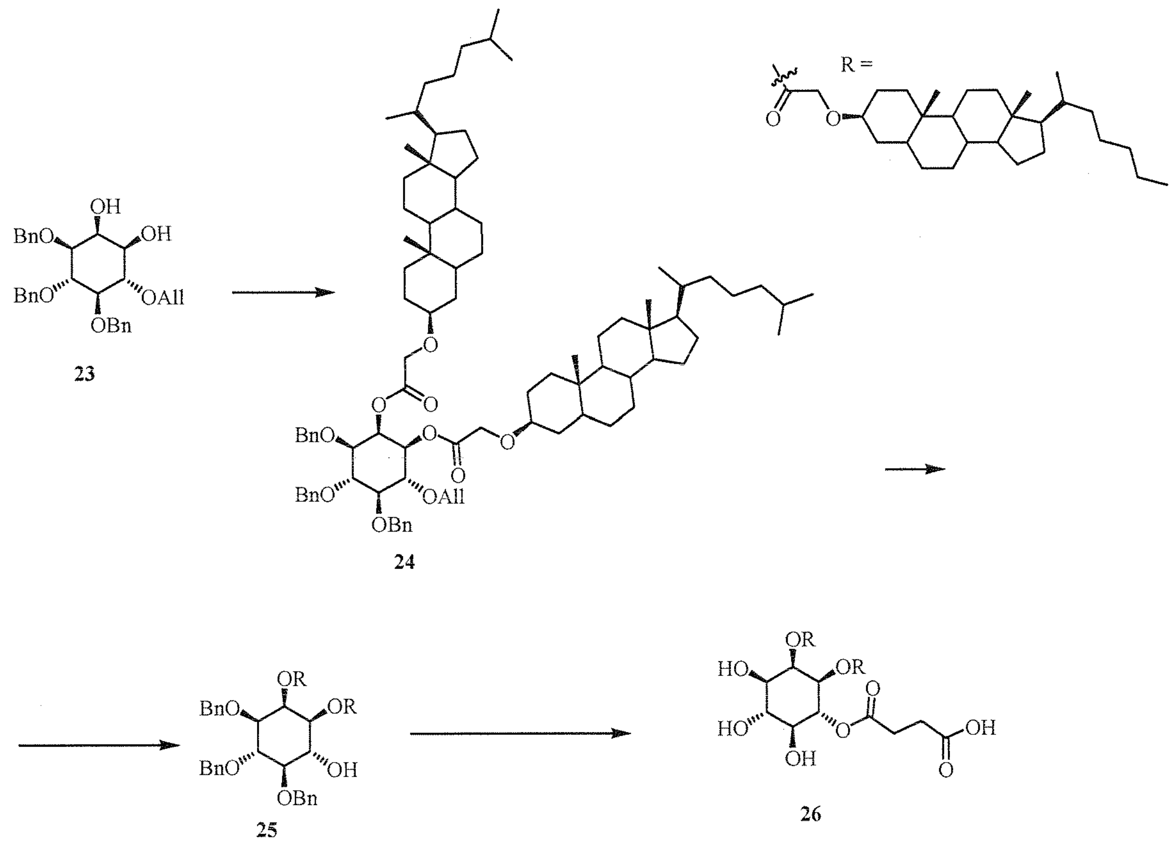

23

24

25

26

Esterification was performed as described in the general procedure to obtain compound **24** from compound **23** (171.5 mg, 80%) as a waxy solid.

$^1$H-NMR (300 MHz, $CDCl_3$): δ = 7.25 - 7.32 (m, 15 H), 5.79 - 5.92 (m, 1 H), 5.77 (t, *J* = 2.6 Hz, I H), 5.21 (dd, *J* = 17.2, 1.7 Hz, 1 H), 5.12 (d, *J* = 11.7 Hz, 1 H), 4.93 (dd, *J* = 9.9, 2.6 Hz, 1 H), 4.83 - 4.89 (m, 3 H), 4.78 (d, *J* = 10.8 Hz, 1 H), 4.67 (d, *J* = 11.1 Hz, 1 H), 4.50 (d, J = 11.4 Hz, 1 H), 4.24- 4.34 (m, 2 H), 4.08 - 4.17 (m, 4 H), 3.79 - 3.86 (m, 2 H), 3.60 (dd, *J* = 10.1, 3.0 Hz, 1H), 3.52 (t, *J* = 9.3 Hz, 1 H), 3.28-3.37 (m, 2H), 1.92-1.98 (m, 2 H), 1.76 - 1.85 (m, 4 H), 1.62 - 1.72 (m, 8 H), 1.36 - 1.56 (m, 10 H) 1.20-1.31 (m, 18 H), 1.00 - 1.15 (m, 16 H), 0.87 - 0.97 (m, 20 H), 0.78 (d, *J* = 5.7 Hz, 6 H), 0.63 (s, 6 H), 0.54 - 0.61 (m, 2 H).

MS (ESI): 1365.0 (M + $NH_4^+$), 1370.0 (M + $Na^+$).

Allyl group was removed as per the general procedure to obtain compound **25** (123.0 mg, 74%) as a waxy solid.

$^1$H-NMR (300 MHz, $CDCl_3$): δ = 7.26 - 7.34 (m, 15 H), 5.78 (t, *J* = 2.5 Hz, 1 H), 4.94 (d, *J* = 11.1 Hz, 1 H), 4.90 (d, *J* = 10.7 Hz, 1H), 4.86 (dd, *J* = 10.4; 2.6 Hz, 1 H), 4.77 (d, *J* = 10.9 Hz, 1 H), 4.74 (d, *J* = 11.2 Hz, 1 H), 4.67 (d, *J* = 11. Hz, 1 H), 4.50 (d, *J* = 11.1 Hz, 1 H), 4.04 - 4.21 (m, 4 H), 3.96 (t, *J* = 9.8 Hz, 1 H), 3.83 (t, *J* = 9.5 Hz, 1 H), 3.63 (dd, *J* = 9.7, 2.6 Hz, 1H), 3.40 (t, *J* = 9.6 Hz, 1 H), 3.27 - 3.33 (m, 2 H), 1.92 -1.98 (m, 2 H), 1.76 -1.85 (m, 4 H), 1.62 -1.72 (m, 8 H), 1.36 - 1.56 (m, 10 H) 1.20 - 1.31 (m, 18 H), 1.00 - 1.15 (m, 16 H), 0.87 - 0.97 (m, 20 H), 0.78 (d, *J* = 5.7 Hz, 6 H), 0.63 (s, 6 H), 0.54 - 0.61 (m, 2 H).

MS (ESI): 1365.0 (M + $NH_4^+$), 1370.0 (M + $Na^+$).

Attachment of succinic head group followed by removal of the benzyl group as described in general procedures afforded compound **26** (80 mg, 93% over two steps) as a waxy solid.

MS (ESI): 1154.6 (M + $NH_4^+$), 1159.7 (M + $Na^+$), 1135.6 (M - $H^+$)

### Example 13: Preparation of a precursor for moiety 1800d

The precursor for compound **1800d** was obtained by the following reaction sequence.

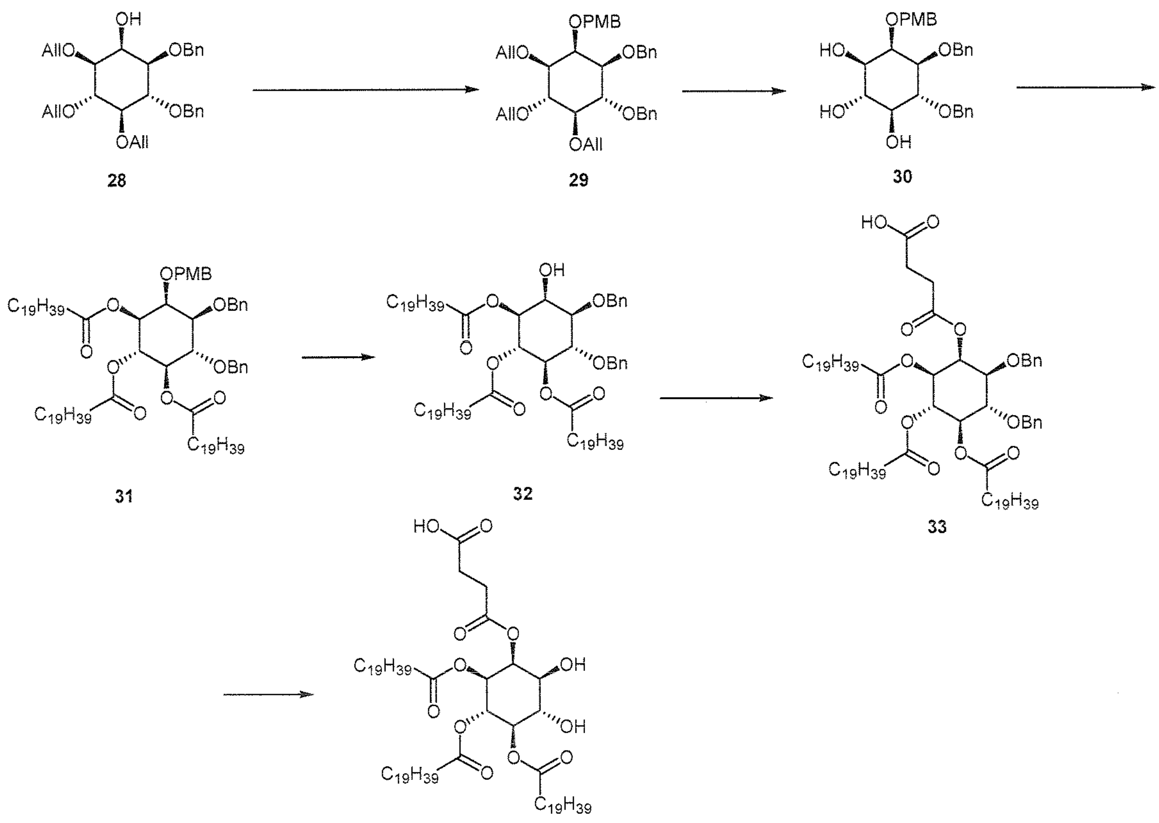

28 29 30 31 32 33

34

Compound **28** was obtained as per the literature procedure (Biochemistry, 1994, 33, 11586).

To a solution of **28** (889.4 mg, 1.85 mmol) in $CH_2Cl_2$ (20 mL) was added 4-methoxybenzylchloride (434.7 mg, 2.78 mmol) and sodium hydride (60%, 111 mg, 2.78 mmol) at -15°C and stirred for 1 h and for 18 h at room temperature. Reaction mixture was diluted with EtOAc (50 mL) and washed with sat. NaCl solution and water and extracted with EtOAc. The combined organic layers were dried over sodium sulfate and concentrated in vacuo. Purification of the residue by flash chromatography (silica, PE/EtOAc 6:1) yielded compound **29** (1.023 g, 92%) as a colourless oil.

$^1$H-NMR (500 MHz, $CDCl_3$): δ = 7.28 - 7.36 (m, 12 H), 6.84 (d, *J* = 8.7 Hz, 2 H), 5.87 - 6.03 (m, 3 H), 5.27 - 5.33 (m, 3 H), 5.14 - 5.18 (m, 3 H), 4.87 (d, *J* = 10.5 Hz, 1 H), 4.80 - 4.83 (m, 3 H), 4.65 (d, *J* = 11.8 Hz, 1 H), 4.58 (d, *J* = 11.7 Hz, 1 H), 4.27 - 4.37 (m, 4 H), 4.06 - 4.12 (m, 2 H), 3.95 - 4.05 (m, 2 H), 3.81 - 3.84 (m, 1 H), 3.80 (s, 3 H), 3.30 (dd, *J* = 9.9, 2.3 Hz, 1 H), 3.26 (t, *J* = 9.3 Hz, 1 H), 3.14 (dd, *J* = 9.9, 2.3 Hz, 1 H).

$^{13}$C-NMR (125 MHz, $CDCl_3$): δ = 158.91 (C), 138.86 (C), 138.48 (C), 135.51 (CH), 135.40 (CH), 134.98 (CH), 131.03 (C) 129.38 (CH), 128.28 (CH), 128.25 (CH), 128.13 (CH), 127.47 (CH), 127.44 (CH), 116.46 ($CH_2$), 116.40 ($CH_2$), 116.34 ($CH_2$), 113.43 (CH), 83.28 (CH), 81.52 (CH), 81.33 (CH), 80.69 (CH), 80.44 (CH), 75.83 ($CH_2$), 74.55 ($CH_2$), 74.48 ($CH_2$), 73.79 (CH), 73.52 ($CH_2$), 72.65 ($CH_2$), 71.61 ($CH_2$), 55.21 ($CH_3$).

MS (ESI): 618.2 (M + $NH_4^+$), 623.2 (M + $Na^+$)

To a solution of **29** (216.5 mg, 0.36 mmol) in ethanol (20 mL) was added 10% Pd/C (100 mg) and p-toluenesulfonic acid (180 mg, 0.95 mmol) and heated at reflux for 2 h. The solvent was removed under reduced pressure and the residue was subjected to the flash chromatography (silica, EtOAc) to afford the product (97 mg, 56%) as a colourless oil.

$^1$H-NMR (500 MHz, $CDCl_3$): δ = 7.25 - 7.34 (m, 12 H), 6.86 (d, *J* = 8.6 Hz, 2 H), 4.97 (m, 2 H), 4.75 (d, *J* = 10.1 Hz, 1 H), 4.68 (s, 2 H), 4.60 (d, *J* = 10.2 Hz, 1 H), 3.99 - 4.01 (m, 1 H), 3.83 (t, *J* = 9.5 Hz, 1 H), 3.79 (s, 3 H), 3.70 (t, *J* = 9.5 Hz, 1 H), 3.43 - 3.45 (m, 1 H), 3.32 - 3.38 (m, 2 H), 3.03 (s, br, 1 H), 2.77 (s, br, 1 H), 2.45 (s, br, 1 H).

$^{13}$C-NMR (125 MHz, $CDCl_3$): δ = 159.26 (C), 138.57 (C), 138.00 (C), 130.60 (C) 129.48 (CH), 128.48 (CH), 128.47 (CH), 128.39 (CH), 128.00 (CH), 127.78 (CH), 127.70 (CH), 127.61 (CH), 113.62 (CH), 81.13 (CH), 80.80 (CH), 76.83 (CH), 75.43 ($CH_2$), 74.53 ($CH_2$), 74.38 (CH), 73.59 (CH), 72.85 ($CH_2$), 72.06 (CH), 55.25 ($CH_3$).

Esterification was performed as described in the general procedure to obtain compound **31** from compound **30** (97 mg, 56%) as a waxy solid.

$^1$H-NMR (500 MHz, $CDCl_3$): δ = 7.20 - 7.33 (m, 12 H), 6.83 (d, *J* = 8.7 Hz, 2 H), 5.56 (t, *J* = 10.1 Hz, 1 H), 5.10 (t, *J* = 9.6 Hz, 1 H), 4.84 (d, *J* = 11.4 Hz, 1 H), 4.78 (dd, *J* = 10.5, 2.4 Hz, 1 H), 4.73 (d, *J* = 11.4 Hz, 1 H), 4.59 - 4.64 (m, 4 H), 4.10 (t, *J* = 2.2 Hz, 1 H), 4.06 (t, *J* = 9.7 Hz, 1 H), 3.79 (s, 3 H), 3.57 (dd, *J* = 9.7, 2.1 Hz, 1 H), 2.10 - 2.30 (m, 6 H), 1.40

- 1.52 (m, 6 H), 1.15 - 1.30 (m, 96 H), 0.87 (t, *J* = 6.9 Hz, 9 H).

$^{13}C$-NMR (125 MHz, $CDCl_3$): δ = 172.95 (C=O), 172.68 (C=O), 172.63 (C=O), 159.20 (C), 138.37 (C), 137.89 (C), 130.24 (C) 129.51 (CH), 128.42 (CH), 128.26 (CH), 127.74 (CH), 127.60 (CH), 127.53 (CH), 127.50 (CH), 113.64 (CH), 80.46 (CH), 78.96 (CH), 75.45 ($CH_2$), 74.37 ($CH_2$), 74.09 (CH), 72.94 ($CH_2$), 72.47 (CH), 71.52 (CH), 69.96 (CH), 55.23 ($CH_3$), 34.19 ($CH_2$), 34.17 ($CH_2$), 31.92 ($CH_2$), 29.71 ($CH_2$), 29.66 ($CH_2$), 29.52 ($CH_2$), 29.46 ($CH_2$), 29.36 ($CH_2$), 29.34 ($CH_2$), 29.21 ($CH_2$), 29.19 ($CH_2$), 24.93 ($CH_2$), 24.84 ($CH_2$), 24.80 ($CH_2$), 22.69 ($CH_2$), 14.12 ($CH_3$).

To a solution of **31** (100 mg, 0.073 mmol) in a mixture of $CH_2Cl_2$ (10 mL) and water (0.2 mL) was added dichlorodicyanobenzoquinone (25 mg, 0.101 mmol) and stirred for 5 h at room temperature. Reaction mixture was diluted with $CH_2Cl_2$ and washed with sat. $NaHCO_3$ solution and extracted with $CH_2Cl_2$. The combined organic layers were dried over sodium sulfate and concentrated in vacuo. Purification of the residue by flash chromatography (silica, PE/EtOAc 8:1) yielded product **32** (88.6 mg, 97%) as a waxy solid.

$^{1}H$-NMR (500 MHz, $CDCl_3$): δ = 7.22 - 7.32 (m, 10 H), 5.55 (t, *J* = 10.2 Hz, 1 H), 5.12 (t, *J* = 9.9 Hz, 1 H), 4.88 (dd, *J* = 10.3, 2.8 Hz, 1 H), 4.83 (d, *J* = 11.4 Hz, 1 H), 4.67 (s, 2 H), 4.62 (d, *J* = 11.4 Hz, 1H), 4.28 (t, *J* = 2.5 Hz, 1 H), 3.98 (t, *J* = 9.7 Hz, 1 H), 3.61 (dd, *J* = 9.5, 2.5 Hz, 1 H), 2.25 - 2.35 (m, 2 H), 2.09 - 2.23 (m, 4 H), 1.45 - 1.58 (m, 6 H), 1.09 - 1.39 (m, 96 H), 0.87 (t, *J* = 6.9 Hz, 9 H).

$^{13}C$-NMR (125 MHz, $CDCl_3$): δ = 172.92 (C=O), 172.63 (C=O), 172.53 (C=O), 138.17 (C), 137.23 (C), 128.58 (CH), 128.34 (CH), 128.14 (CH), 127.94 (CH), 127.63 (CH), 127.58 (CH), 79.54 (CH), 78.64 (CH), 75.59 ($CH_2$), 73.02 ($CH_2$), 72.06 (CH), 70.84 (CH), 69.21 (CH), 67.83 (CH), 34.17 ($CH_2$), 33.79 ($CH_2$), 31.92 ($CH_2$), 29.72 ($CH_2$), 29.52 ($CH_2$), 29.49 ($CH_2$), 29.38 ($CH_2$), 29.33 ($CH_2$), 29.28 ($CH_2$), 29.21 ($CH_2$), 29.19 ($CH_2$), 29.12 ($CH_2$), 28.40 ($CH_2$), 24.94 ($CH_2$), 24.92 ($CH_2$), 24.79 ($CH_2$), 22.69 ($CH_2$), 14.12 ($CH_3$).

Succinic head group was attached as described in the general procedure to afford the product **33** (213 mg, 78%) as a colourless solid.

$^{1}H$-NMR (300 MHz, $CDCl_3$): δ = 7.19 - 7.32 (m, 10 H), 5.77 (t, *J* = 2.6 Hz, 1 H), 5.41 (t, *J* = 10.3 Hz, 1 H), 5.14 (t, *J* = 9.9 Hz, 1 H), 4.91 (dd, *J* = 10.4, 2.7 Hz, 1H), 4.84 (d, *J* = 11.4 Hz, 1 H), 4.66 (d, *J* = 11.0 Hz, 1 H), 4.59 (d, *J* = 11.4 Hz, 1 H), 4.49 (d, *J* = 11.4 Hz, 1 H), 3.87 (t, *J* = 9.5 Hz, 1 H), 3.67 (dd, *J* = 10.1, 3.1 Hz, 1 H), 2.77 - 2.81 (m, 2 H), 2.65 - 2.70 (m, 2 H), 2.12 - 2.28 (m, 6 H), 1.41 - 1.59 (m, 6 H), 1.16 -1.38 (m, 96 H), 0.87 (t, *J* = 6.8 Hz, 9 H).

MS (ESI): 1361.1 (M + $NH_4^+$), 1341.9 (M - $H^+$)

Benzyl groups were removed as per the general procedure to obtain compound **34** (160 mg, 97%) as a colourless solid.

$^{1}H$-NMR (500 MHz, $CDCl_3$): δ = 5.45 (t, *J* = 2.7 Hz, 1 H), 5.26 (t, *J* = 10.2 Hz, 1 H), 4.85 - 4.94 (m, 2 H), 3.69 (t, *J* = 9.8 Hz, 1 H), 3.56 (dd, *J* = 9.9, 2.8 Hz, 1 H), 2.59 - 2.62 (m, 2 H), 2.53 - 2.56 (m, 2 H), 2.16 - 2.25 (m, 2 H), 2.06 - 2.12 (m, 4 H), 1.41 - 1.59 (m, 6 H), 1.16 - 1.38 (m, 96 H), 0.75 (t, *J* = 6.7 Hz, 9 H).

$^{13}C$-NMR (125 MHz, $CDCl_3$): 174.45 (C=O), 173.27 (C=O), 172.77 (C=O), 172.50 (C=O), 171.71 (C=O), 72.55 (CH), 70.94 (CH), 70.81 (CH), 69.64 (CH), 69.41 (CH), 69.26 (CH), 44.34 ($CH_2$), 33.95 ($CH_2$), 33.91 ($CH_2$), 33.66 ($CH_2$), 31.69 ($CH_2$), 29.47 ($CH_2$), 29.12 ($CH_2$), 28.95 ($CH_2$), 28.82 ($CH_2$), 24.69 ($CH_2$), 24.40 ($CH_2$), 22.44 ($CH_2$), 22.23 ($CH_2$), 21.89 ($CH_2$), 13.78 ($CH_3$).

MS (ESI): 1186.0 (M + $Na^+$), 1161.7 (M - $H^+$)

### Example 14: Preparation of a precursor for moiety 200a

The free acid derivative of moiety **200a** was prepared as follows.

Ethyl diazoacetate (1.93 g, 16.8 mmol) and a catalytic amount of borontrifluoride ether complex (670 μL) was added subsequently to a solution of commercially available cholesterol (5 g, 12.9 mmol) in dichloromethane (100 mL) under an atmosphere of argon. The resulting reaction mixture was stirred at room temperature for about 3 h. After slow addition of saturated aqueous sodium hydrogencarbonate solution (60 mL), the organic layer was separated and washed again with saturated sodium hydrogencarbonate solution. The solvent was removed under reduced pressure, and the crude material was purified by column chromatography on silica gel (hexane/diethyl ether 5:1). The corresponding alkylated cholesteryl derivative was obtained as colourless solid (3.67 g, 60% yield).

The so obtained material was dissolved in ethanol (200 mL) and the solution heated to 50°C.

After addition of solid potassium hydroxide (1.2 g, 22.3 mmol) the resulting reaction mixture was stirred at 50°C for about 1 h. The mixture was acidified by addition of hydrochloric acid and partitioned between dichloromethane (800 mL) and water (800 mL). The organic layer was separated, dried over sodium sulfate and the solvent was removed under reduced pressure. The crude product was purified by column chromatography on silica gel (dichloromethane/methanol 95:5). The free acid derivative of moiety **200a** was obtained as colourless solid (3.04 g, 92% yield).

$^{1}H$-NMR (300 MHz, $CDCl_3$): δ = 5.36 (d, *J* = 5.1 Hz, 1 H), 4.14 (s, 2 H), 3.29 (m, 1 H), 2.22 - 2.38 (m, 3 H), 1.76 - 2.1 (m, 6 H), 0.84 - 1.67 (m, 28 H), 1.0 (s, 3 H), 0.67 (s, 3 H).

MS (ESI): *m*/*z* = 443.3 $(M-H)^-$

**Example 15: Preparation of a precursor for moiety 200b**

The free acid derivative of moiety **200b** was prepared as follows.

Ethyl diazoacetate (3.73 g, 32.8 mmol) was added to a solution of commercially available dihydrocholesterol (9.8 g, 25.2 mmol) in anhydrous dichloromethane (50 mL) under an atmosphere of argon. After portionwise addition of a catalytic amount of boron trifluoride etherate (1 mL of a 1M solution in diethyl ether), the resulting reaction mixture was stirred for 36 hours at room temperature. The reaction mixture was poured onto a saturated aqueous solution of sodium hydrogencarbonate (1 L) and extracted with ethyl acetate (1 L). After washing with water (1 L), the organic layer was dried over magnesium sulfate and the solvent removed under reduced pressure. The crude product was purified by column chromatography on silica gel (pure dichloromethane as eluent).

The obtained product was dissolved in dichloromethane (15 mL) and a 1M solution of potassium hydroxide in water (20 mL) was added. The resulting reaction mixture was stirred vigorously at room temperature for about 48 hours. A 1 M aqueous solution of hydrochloric acid was added, until the pH of the aqueous layer was adjusted at about pH 1-2. The mixture was partitioned between water (1 L) and ethyl acetate (900 mL). After separation the organic layer was washed with water (1 L), dried over magnesium sulfate, and the solvent was removed under reduced pressure to afford the analytically pure product as colourless solid (5.39 g, 48% overall yield).
$^1$H-NMR (300 MHz, $CDCl_3$): $\delta$ = 3.75 (s, 2 H), 3.32 (m, 1 H), 0.85 - 2.1 (m, 40 H), 0.80 (s, 3 H), 0.64 (s, 3 H).
MS (ESI): *m*/*z* = 445.2 $(M-H)^-$

**Example 16: Preparation of a precursor for moiety 200c**

The free acid derivative of moiety **200c** was prepared as follows.

The free acid derivative of moiety **200c** was prepared according to a synthetic strategy described in detail by B. R. Peterson et al. in the literature (S. L. Hussey, E. He, B. R. Peterson, J. Am. Chem. Soc. 2001, 123, 12712-12713; S. E. Martin, B. R. Peterson, Bioconjugate Chem. 2003, 14, 67-74). Actually, instead of the free acid derivative of moiety **200c** itself, the corresponding *N*-nosyl protected derivative was incorporated by solid phase synthesis, and the nosyl protecting group was removed after conjugate assembly by an experimental protocol described in the above cited publications of B. R. Peterson. However, the final raftophile building block was represented by the free acid derivative of moiety **200c.**

Example 17: Preparation of a precursor for moiety 200e

The free acid derivative of moiety 200e was prepared as follows.

Triethylamine (284 mg, 2.81 mmol) was added to a solution of commercially available dihydrocholesterol (840 mg, 2.16 mmol), succinic anhydride (281 mg, 2.81 mmol) and DMAP (342 mg, 2.81 mmol) in dichloromethane (10 mL) and the resulting reaction mixture was stirred at room temperature overnight. After dilution with ethyl acetate (900 mL) the reaction mixture was washed subsequently with 0.1M aqueous hydrochloric acid (1 L) and water (2 $\times$ 1 L). The organic layer was dried over sodium sulfate and the solvent removed under reduced pressure to afford the analytically pure product as colourless solid (926 mg, 87% yield).
$^1$H-NMR (300 MHz, $CDCl_3$): $\delta$ = 4.71 (m, 1 H), 2.67 (m, 2 H), 2.59 (m, 2 H), 1.96 (m, 1 H), 0.85 - 1.81 (m, 39 H), 0.81 (s, 3 H), 0.64 (s, 3 H).

**Example 18: Preparation of a precursor for moiety 200f**

The free acid derivative of moiety **200f** was prepared as follows.

A solution of commercially available dihydrocholesterol (10 g, 25.7 mmol), triphenylphosphine (20.3 g, 77.2 mmol) and methanesulfonic acid (5.2 g, 53.9 mmol) in anhydrous THF (250 mL) was heated to 42°C under an atmosphere of argon. After addition of diisopropylazodicarboxylate (15.6 g, 77.2 mmol) the resulting reaction mixture was stirred for about 18 h at 40°C. Water (20 mL) was added and the reaction mixture was stirred for 10 min at room temperature. After further addition of water (400 mL) and dichloromethane (200 mL), the organic layer was separated. The aqueous layer was extracted again with dichloromethane (200 mL), and the combined organic layers were washed with brine (800 mL). After drying of the organic layer over sodium sulfate, the solvent was removed under reduced pressure and the crude material was subjected to purification by column chromatography on silica gel using a gradient elution (petrol ether/ethyl acetate 10:1 to 6:1). The expected mesylate was obtained as white solid (4.1 g, 34% yield). The so obtained material (4.1 g, 8.8 mmol) was dissolved in DMSO (80 mL) and sodium azide (5.7 g, 88 mmol) was added. The resulting reaction mixture was stirred for about 18 h at 90°C. After addition of water (500 mL), the mixture was extracted with dichloromethane (400 mL). The organic layer was separated and washed thoroughly with water (4 $\times$ 700 mL). After drying over sodium sulfate, the solvent was removed under reduced pressure and the obtained analytically pure azide

was dried at high vacuum (2.7 g, 75% yield).

The so obtained azide (2.5 g, 6 mmol) was dissolved in anhydrous diethyl ether (25 mL) and the resulting solution was added dropwise to a suspension of lithiumaluminium hydride (690 mg, 18.3 mmol) in anhydrous diethyl ether (50 mL) at 36°C under an atmosphere of argon. The resulting reaction mixture was stirred for about 18 h at reflux temperature, then cooled down in an ice-water bath, and water was added dropwise until the gas evolution ceased. A aqueous solution of 2M sodium hydroxide (1 L) was added and the mixture was extracted with diethyl ether (500 mL). The aqueous layer was extracted again with dichloromethane (2 × 500 mL), and the combined organic layers were dried over sodium sulfate. The solvent was removed under reduced pressure and the expected amine was obtained analytically pure after drying under high vacuum (1.3 g, 55% yield).

A solution of the above described amine (160 mg, 0.41 mmol), DMAP (125 mg, 1 mmol) and succinic anhydride (103 mg, 1 mmol) in dichloromethane (10 mL) was stirred at room temperature for about 48 h. The solvent was removed under reduced pressure and the obtained solid residue was dissolved in ethyl acetate (20 mL). After subsequent addition of saturated aqueous sodium hydrogencarbonate solution (20 mL) and a catalytic amount of DMAP, the resulting mixture was stirred for 1 h at room temperature. An aqueous solution of 0.1M hydrochloric acid (500 mL) was added and the aqueous layer was extracted with ethyl acetate (2 × 400 mL). After drying of the organic layer over sodium sulfate, the solvent was removed under reduced pressure to afford the free acid derivative of moiety **200f** analytically pure (78 mg, 40% yield).

$^1$H-NMR (300 MHz, $CDCl_3$): $\delta$ = 3.78 (m, 1 H), 2.66 (m, 4 H), 1.97 (m, 1 H), 0.85 - 1.81 (m, 40 H), 0.78 (s, 3 H), 0.64 (s, 3 H).
MS (ESI): *m*/*z* = 488.4 $(M+H)^+$

**Example 19: Preparation of a precursor for moiety 200j**

The free acid derivative of moiety **200j** was prepared from commercially available cholesterol using the same protocol as described above for the free acid derivative of moiety **200e.**

The free acid derivative of moiety **200j** was obtained as colourless solid (1.2 g, 95% yield). $^1$H-NMR (300 MHz, $CDCl_3$): $\delta$ = 5.37 (d, *J* = 4.1 Hz, 1 H), 4.63 (m, 1 H), 2.66 - 2.70 (m, 2 H), 2.58 - 2.62 (m, 2 H), 2.32 (d, *J* = 7.8 Hz, 2 H), 1.77 - 2.05 (m, 5 H), 0.85 - 1.65 (m, 30 H), 1.02 (s, 3 H), 0.68 (s, 3 H).
MS (ESI): *m*/*z* = 485.1 $(M-H)^-$

**Example 20: Preparation of a compound comprising moiety 200k**

The free carboxylic acid function of the side chain of commercially available Fmoc-Asp-OtBu was coupled with dihydrocholesterol using standard esterification protocols known to the person skilled in the art. The resulting dihydrocholesteryl ester of Fmoc-Asp-OtBu was then subjected to cleavage of the OtBu ester using the standard trifluoroacetic acid protocol to provide the corresponding dihydrocholesteryl ester of Fmoc-Asp.

This building block was then attached to the N-terminus of a given rhodamine-labeled linker substructure followed by standard Fmoc deprotection to provide a compound comprising moiety **200k.**

**Example 21: Preparation of a compound comprising moiety 2001**

A compound comprising moiety **200l** was obtained from the compound comprising moiety **200k** obtained in example 20 by simple acetyl capping using standard protocols known in the literature.

**Example 22: Preparation of a precursor for moiety 200m**

A compound comprising moiety **200m** was prepared by attachment of the dihydrocholesteryl ester of Fmoc-Asp obtained in example 20 onto solid support followed by Fmoc deprotection of the N-terminus and solid phase peptide chemistry to assemble the linker and pharmacophore substructures onto the free N-terminus, as described for the preparation of compound **25b.**

**Example 23: Preparation of a precursor for moiety 300a**

The free acid derivative of moiety **300a** was prepared as follows.

A suspension of sodium hydride (500 mg suspension in mineral oil, 12.25 mmol sodium hydride) in anhydrous DMSO (15 mL) was heated to 70°C for about 45 min under an atmosphere of argon. After addition of a solution of commercially available dodecylphosphonium bromide in anhydrous DMSO (20 mL) the resulting red solution was kept at about 60 - 65°C for about 10 min. Then, a solution of commercially available estrone (668 mg, 2.47 mmol) in anhydrous DMSO (20 mL) was added to the hot solution, and the reaction mixture was stirred at 60°C for 18 hours. The mixture

was poured into water (1 L) and extracted with diethyl ether (2 × 500 mL). The combined organic layers were washed repeatedly with water (4 × 1L) and dried over sodium sulfate. After removal of the solvent under reduced pressure, the crude material was purified by column chromatography on silica gel (petrol ether/ethyl acetate 4:1). The expected 17-dodecylidenylated estrone was obtained as white solid (531 mg, 51% yield).
$^1$H-NMR (300 MHz, $CDCl_3$): δ = 7.08 (d, *J* = 8.5 Hz, 1 H), 6.56 (dd, *J* = 2.7, 8.5 Hz, 1 H), 6.49 (d, *J* = 2.7 Hz, 1 H), 4.98 (t, *J* = 7.4 Hz, 1 H), 4.58 (s, 1 H), 2.75 (m, 2 H), 2.04 - 2.41 (m, 9 H), 1.15 - 1.87 (m, 24 H), 0.84 (s, 3 H), 0.82 (t, *J* = 6.9 Hz, 3 H).
MS (ESI): *m*/*z* = 422.6 ($M^+$)
Hydrogenation of the 17,20 double bond in above described material was achieved by treatment of a solution of the 17-dodecylidenylated estrone (475 mg, 1.12 mmol) in dichloromethane (10 mL) and palladium (120 mg 10% on charcoal, 0.11 mmol) under an atmosphere of hydrogen for 36 hours at room temperature. The reaction mixture was filtered through a pad of celite and the solvent removed under reduced pressure to afford the analytically pure 17β-dodecyl substituted estrone as colourless solid (452 mg, 95% yield).
$^1$H-NMR (300 MHz, $CDCl_3$): δ = 7.09 (d, *J* = 8.4 Hz, 1 H), 6.55 (dd, *J* = 2.6, 8.4 Hz, 1 H), 6.49 (d, *J* = 2.6 Hz, I H), 4.51 (s, 1 H), 2.73 - 2.77 (m, 2 H), 2.15 - 2.19 (m, 2 H), 1.77 - 1.82 (m, 2 H), 1.03 - 1.65 (m, 16 H), 0.81 (t, *J* = 6.9 Hz, 3 H), 0.53 (s, 3 H).
MS (ESI): *m*/*z* = 424.7 ($M^+$)

A solution of the above described 17β-dodecyl substituted estrone (440 mg, 1.04 mmol) in DMF (6 mL) and dichloromethane (6 mL) was added to sodium hydride (50 mg suspension in mineral oil, 1.14 mmol sodium hydride) and the resulting suspension was heated to reflux for about 20 min. Tert-butyl bromoacetate (242 mg, 1.24 mmol) was added and the reaction mixture was stirred at reflux for about 28 hours. After pouring into water (1 L) and extraction with dichloromethane (600 mL), the organic layer was washed with water (3 × 800 mL) and dried over magnesium sulfate. The solvent was removed under reduced pressure to afford an analytically pure product as colourless solid. The obtained material was dissolved in dichloromethane (10 mL), and after addition of trifluoroacetic acid (2.5 mL) the resulting reaction mixture was stirred at room temperature for 3 h. The solvents were removed under reduced pressure and the obtained material was dried for 18 h at high vacuum. The free acid derivative of moiety **300a** was obtained as pale yellow solid (394 mg, 79% overall yield).
MS (ESI): *m*/*z* = 4881.3 $(M-H)^-$

**Example 24: Preparation of a precursor for moiety 1900a**

The free acid derivative of moiety 1900a was prepared as follows.

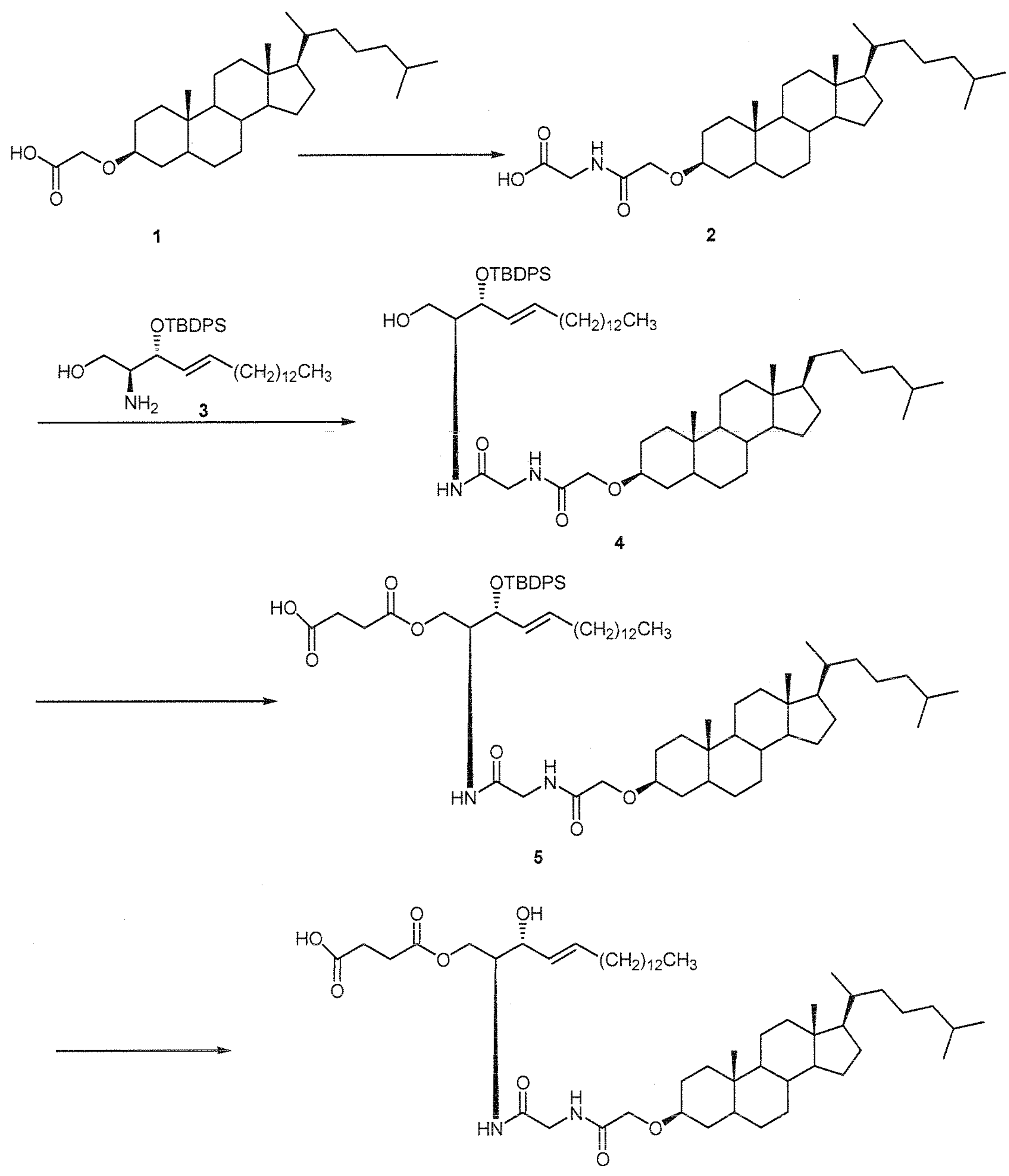

1 2 3 4 5 6

A solution of 1 (447 mg, 1 mmol), HATU (380 mg, 1 mmol), H-Gly-2Cl-Trt resin (0.54 mmol) (available from Novabiochem, catalog no. 04-12-2800) and DIPEA (259 mg, 2 mmol) in N-methyl-2-pyrrolidone (4 mL) was shaken for 1 h in peptide synthesizer. N-Methyl-2-pyrrolidone washing was carried out followed by $CH_2Cl_2$ washings. The resin thus obtained was cleaved by treatment with 1% trifluoroacetic acid solution in $CH_2Cl_2$. The product was washed with water (100 mL) and extracted with $CH_2Cl_2$ (3 x 100 mL). The combined organic layers were dried over sodium sulfate and concentrated in vacuo yielding 2 as a white solid (250 mg, 100%).
$^1$H-NMR (300 MHz, $CDCl_3$): δ = 0.58 (s, 6H), 0.70 - 1.77 (series of m, 36H), 1.87 (d, J = 12.3 Hz, 2H), 3.09 (m, 2H), 3.26 (m, 1H), 3.98 (m, 2H), 4.04 (m, 2H), 7.23 (m, 1H), 9.7 (br s, 1H).
MS (ESI): m/z = 502 (M - 1)

Steroid containing side chain was introduced as described in the general procedure to obtain compound 4 (281 mg, 62%) as a white solid.
$^1$H-NMR (300 MHz, $CDCl_3$): δ = 0.65 (s, 3H), 0.79 (s, 3H), 0.85 - 0.91 (m, 18H), 1.06 - 1.45 (m, 50H), 1.46 - 2.17 (m, 9H), 3.27 (m, 1H), 3.56 - 4.13 (m, 8H), 4.35 (m, 1H), 5.41 (m, 2H), 6.12 (m, 1H), 7.36 (m, 6H), 7.59 (m, 4H).

MS (ESI): m/z =1023 (M+1)

Succinic head group was attached as described in the general procedure to obtain compound 5 (224 mg, 73%) $^1$H-NMR (300 MHz, $CDCl_3$): δ = 0.63 (s, 3H), 0.78 (s, 3H), 0.84 - 0.95 (m, 18H), 0.98 - 1.31 (m, 50H), 1.41 - 1.82 (m, 10H), 1.94 (br d, J = 12.13 Hz, 2H), 2.47 (m, 4H), 3.27 (m, 1H), 3.48 (m, 1H), 3.95 (br s, 2H), 4.19 (m, 4H), 5.28 (m, 2H), 7.29 (m, 6H), 7.59 (m, 4H).
MS (ESI): m/z = 1123.7 (M+1)

Protecting group was removed as per the general procedure to obtain compound 6.
MS (ESI): m/z = 885.6 (M+1)

**Example 25: Preparation of a precursor for moiety 1900b**

The free acid derivative of moiety **1900b** was prepared starting from commercially available Fmoc-Lys(Dde) using solid phase peptide chemistry known to the person skilled in the art. After initial attachment of the orthogonally protected amino acid described in example 24 to solid support, the Dde protecting group was removed by literature-known protocols followed by capping with the free acid of moiety **200b** using standard peptide couplings. The preparation of the free acid of moiety **200b** is described herein above. Then, the Fmoc protecting group was removed followed by successive couplings of commercially available Fmoc-β-Ala and the free acid of raftophile moiety **200b.** Final cleavage from the solid support under standard conditions provided the free acid of moiety **1900b.**

**General procedure for the synthesis of compounds of the present invention**

Tripartite compounds as described herein may be synthesized on solid support using an Applied Biosystems 433A peptide synthesizer equipped with a series 200 UV/VIS detector (also referred to as ABI 433A and ABI 433 herein below). All peptide syntheses are, for example, carried out using the Fmoc method with piperidine as the deprotecting reagent and 2-(1H-benzotriazole-1-yl)-1,1,3,3-tetramethyluronium hexafluorophosphate (HBTU) or O-(7-azabenzotriazol-1-yl)-1,1,3,3-tetra-methyluronium hexafluorophosphate (HATU) as the coupling reagent. The principles of this synthetic method are described in common textbooks (e.g. G. A. Grant (Ed.), "Synthetic Peptides: A User's Cuide", W. H. Freeman & Co., New York 1992). Detailed descriptions of the synthetic procedures used by the ABI 433A are documented in the ABI 433A user's manuals, part numbers 904855 Rev. C and 904856C (©2001 by Applied Biosystems) and in the manual "Running the 433A with the Series 200 UV Detector" (©2002 by Applied Biosystems). The amide resin Fmoc-PAL-PEG-PS (Applied Biosystems, Part No. GEN913398) may be used as the solid support. Amino acid building blocks, coupling reagents and solvents were purchased ready-for-use from either Applied Biosystems or Novabiochem. Amino acids with polyglycol backbone were prepared according protocols known in the literature (D. Boumrah, M. M. Campbell, S. Fenner, R. G. Kinsman, Tetrahedron 1997, 53, 6977-6992) or purchased from Novabiochem.

Lipid building blocks, which can not be processed by the ABI 433A (e.g. because of low solubility), were (for example) coupled manually to the N-terminus of peptides on solid support generated as described above. After completion of synthesis the final product was cleaved off from solid support. A typical procedure is as follows: A cleavage cocktail containing trifluoroacetic acid (87%), water (4%), anisole (3%), thioanisole (3%), and triisopropylsilane (3%) is freshly prepared. 4 ml of this mixture are cooled in an ice-bath and added to 70 mg of resin-bound peptide or lipopeptide. The mixture is stirred at 5±2°C for 90 to 120 min. The mixture is filtered into 100 ml of an ice-cold mixture of diethyl ether and hexane (2:1) and the resin is washed with several portions of cleavage cocktail, which are filtered off in the same way. The diethyl ether/hexane mixture containing the combined filtrates is cooled in a freezer (-18°C) and the crude peptide or lipopeptide is isolated by membrane filtration. The crude product is washed with diethyl ether/hexane (2:1), dried under high vacuum and purified by preparative reversed phase HPLC.

**Example 26**

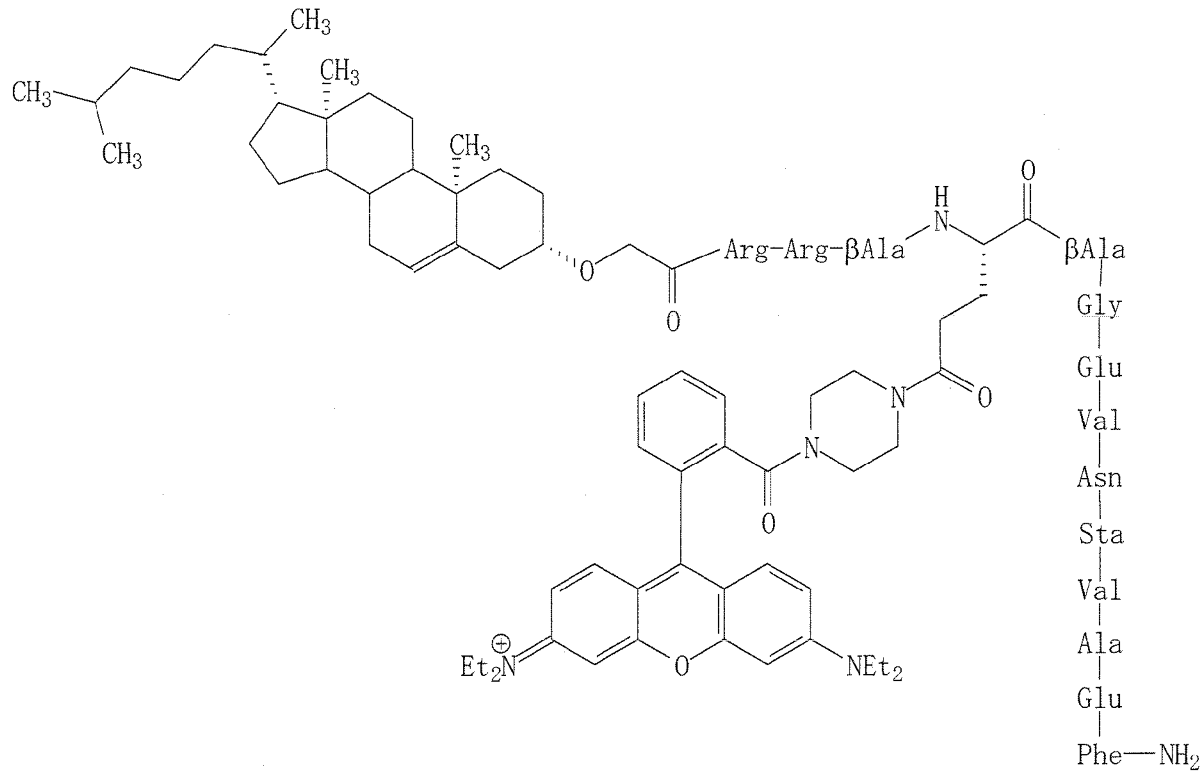

26

Fmoc-PAL-PEG-PS resin (610 mg, 0.25 mmol, loading: 0.41 mmol/g) was subjected to the following operations inside a reactor vessel using an automated peptide synthesizer: washing with dichloromethane, washing with N-methyl-2-pyrrolidone, cleavage of terminal Fmoc group using 20% piperidine in N-methyl-2-pyrrolidone (controlled by UV monitoring), washing with N-methyl-2-pyrrolidone.

Activation and coupling of the amino acid was achieved as follows: Fmoc-Phe (1 mmol) was transformed into the corresponding N-hydroxy-1H-benzotriazole ester (activation) in a gastight cartush by addition of HBTU (1 mmol, 2.2 mL of a 0.45 M solution in N-methyl-2-pyrrolidone) and diisopropylethylamine (2 mmol, 0.5 mL of a 2 M solution in N-methyl-2-pyrrolidone) followed by passing nitrogen gas through the reaction mixture until a clear solution resulted. The mixture was transferred into the reactor vessel and shaken with the resin for 30 min (coupling). The resin was drained and washed with N-methyl-2-pyrrolidone. The above mentioned sequence of operations was repeated for each of the following amino acids, i.e. Fmoc-Glu(*t*Bu), Fmoc-Ala, Fmoc-Val, Fmoc-Sta, Fmoc-Asn(Trt), Fmoc-Val, Fmoc-Glu(*t*Bu), Fmoc-Gly, Fmoc-βAla, resulting in a protected derivative of inhibitor III as obtainable from Calbiochem, Catalog No. 565780.

After a final washing with dichloromethane, the resin was dried under high vacuum and stored at -18˚C. Preparation of **26** was continued with a portion of the resin.

Coupling of rhodamine-labelled glutamic acid was done manually in a round bottom flask. Neat diisopropylethylamine (78 mg, 0.6 mmol) was added to a solution of Fmoc-Glu(Rho) (295 mg, 0.3 mmol) and HATU (115 mg, 0.3 mmol) in N-methyl-2-pyrrolidone (6 mL) and the resulting reaction mixture was stirred for 10 min at room temperature (activation). After addition of this mixture to the resin (341 mg, 0.1 mmol) described above, the resulting heterogenous reaction mixture was stirred carefully for 1 h at room temperature. The resin was drained, transferred to the reactor vessel and washed subsequently with N-methyl-2-pyrrolidone and dichloromethane using the automated peptide synthesizer.

Completion of the peptide strand was achieved by subsequent activation and coupling of Fmoc-βAla, Fmoc-Arg(Pbf), and Fmoc-Arg(Pbf) in the same manner as described above using the automated peptide synthesizer.

Coupling of cholesteryl glycolic acid (i.e. a precursor of unit **200a**) was done again manually in a round bottom flask. Neat diisopropylethylamine (42 mg, 0.16 mmol) was added to a solution of cholesteryl glycolic acid (73 mg, 0.16 mmol), HBTU (62 mg, 0.16 mmol) and N-Hydroxybenzotriazole (25 mg, 0.16 mmol) in dimethylformamide (2 mL) and the resulting reaction mixture was stirred for 10 min at room temperature (activation). After addition of this mixture to the resin (186 mg, 0.054 mmol) described above, the resulting heterogenous reaction mixture was stirred carefully for 2 h at room temperature. The resin was drained, transferred to the reactor vessel and washed subsequently with N-methyl-2-pyrrolidone and dichloromethane using the automated peptide synthesizer.

Cleavage from the resin was done as described in the general part: The resin (134 mg) was suspended in a mixture of trifluoroacetic acid (87%), water (4%), triisopropylsilane (3%), thioanisole (3%) and anisole (3%) and stirred for 90 min at 5°C (±2°C). The resin was drained and washed repeatedly with the above described cleavage cocktail (4 × 1 mL). The filtrate was poured into ice-cold diethyl ether (40 mL) and precipitation was completed by dilution to a volume of 100 mL with hexane/diethyl ether (1:2). The product was separated by membrane filtration (PP membrane, 0.45 μm), washed with hexane/diethyl ether (1:2) and dried under high vacuum (crude yield: 41 mg).

Preparative HPLC purification (Vydac-C8-column, 40 mL/min, A: water + 0.1% trifluoroacetic acid, B: acetonitrile + 0.1% trifluoroacetic acid, gradient elution from 51% to 63% B over a period of 15 min, retention time observed: 9.8 min) of the crude product yielded 26 as reddish pink foam (5.6 mg) after removal of the solvents under high vacuum and freeze drying from acetic acid.

HPLC analysis: Agilent Zorbax-$C_8$ Column 4.6 x 125 mm, flow rate 1 mL/min, A: water + 0.1% trifluoroacetic acid, B: acetonitril + 0.1% trifluoroacetic acid, gradient elution from 10% to 100% B in 45 min, retention time: 30.8 min, detection at 215 nm, 91% purity.

ESI-MS: 1262.4 $[M+H]^{2+}$, 842.3 $[M+2H]^{3+}$.

**Example 27**

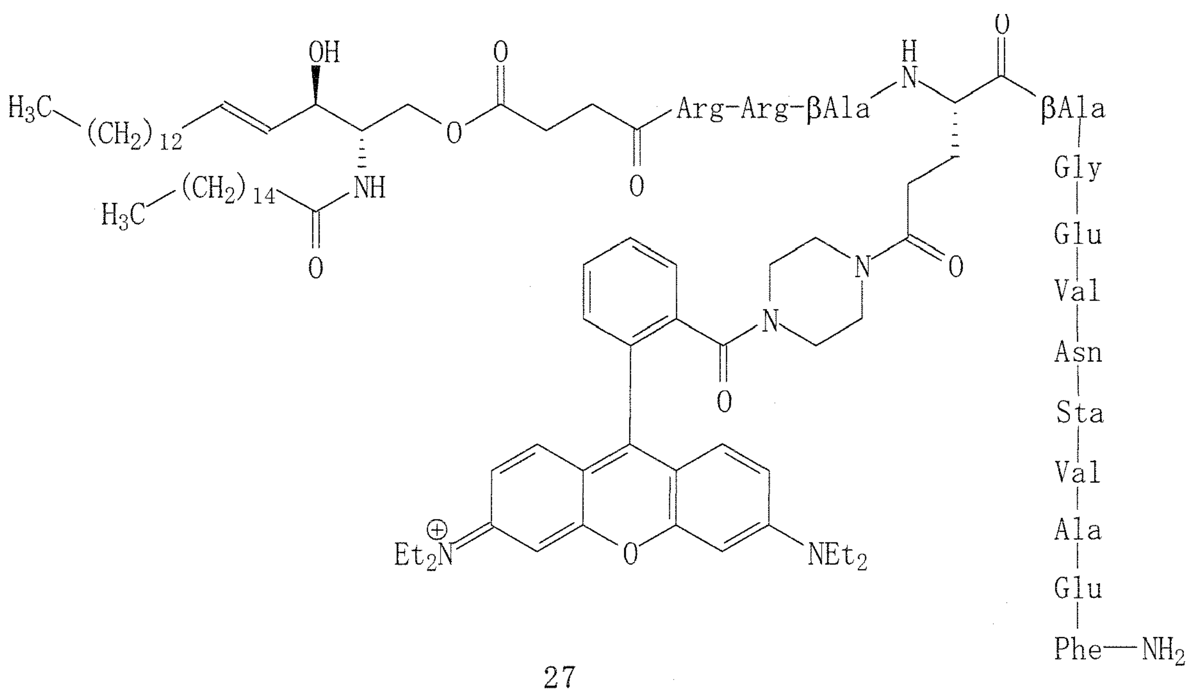

27

Preparation of compound **27** was accomplished as described for compound **26** by coupling of succinic mono (D-erythro-$C_{16}$-ceramidyl) ester (i.e. a precursor of moiety **400aa**) instead of cholesteryl glycolic acid (precursor of moiety **200a**) to the N-terminal arginine. Cleavage and purification were achieved as described for compound **26.** Compound **27** was obtained as a red powder (4.1 mg).

HPLC analysis: same protocol as described for compound **26,** but using an isocratic elution with 66% acetonitrile + 0.1% trifluoroacetic acid in 45 min; retention time: 13.5 min; detection at 215 nm; 90% purity.

ESI-MS: 1358 $[M+2H]^{2+}$, 906 $[M+2H]^{3+}$.

**Example 28: Preparation of a compound of the invention having formula 24**

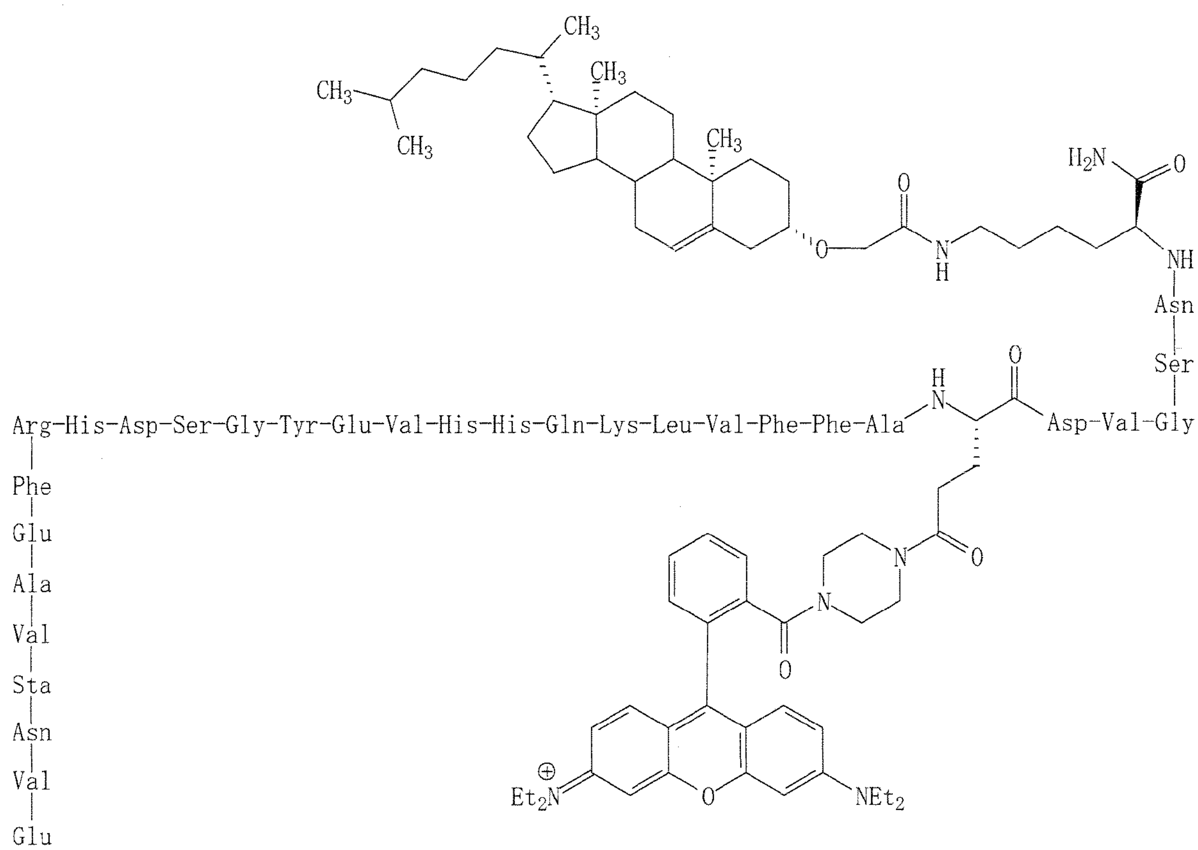

24

The preparation of **24** is achieved as outlined above in the general description. Using Fmoc-PAL-PEG-PS amide resin and automated solid phase peptide synthesis protocols, successive coupling of Fmoc-Lys(CholGlc), Fmoc-Asn, Fmoc-Ser(tBu), Fmoc-Gly, Fmoc-Val, Fmoc-Asp(OtBu), Fmoc-Glu(Rho), Fmoc-Ala, Fmoc-Phe, Fmoc-Phe, Fmoc-Val, Fmoc-Leu, Fmoc-Lys(Trt), Fmoc-Gln, Fmoc-His, Fmoc-His, Fmoc-Val, Fmoc-Glu(OtBu), Fmoc-Tyr, Fmoc-Gly, Fmoc-Ser, Fmoc-Asp(OtBu), Fmoc-His, Fmoc-Arg(Pbf), Fmoc-Phe, Fmoc-Glu(OtBu), Fmoc-Ala, Fmoc-Val, Fmoc-Sta, Fmoc-Asn, Fmoc-Val, Fmoc-Glu(OtBu) yields the pharmacophore-peptide linker sequence on solid phase. Subsequent manual coupling of cholesteryl glycolic acid (precursor of moiety **200a**) using standard peptide coupling techniques provides **24** fixed to a solid support *via* its C-terminus. Finally, cleavage from the resin following the general cleavage procedure described above results in amide **24** after purification by preparative HPLC.

The linker length was calculated by a MM+ forcefield optimization using Hyperchem® software to be 8.87 nm.

**Example 29: Preparation of a compound of the invention having formula 24b**

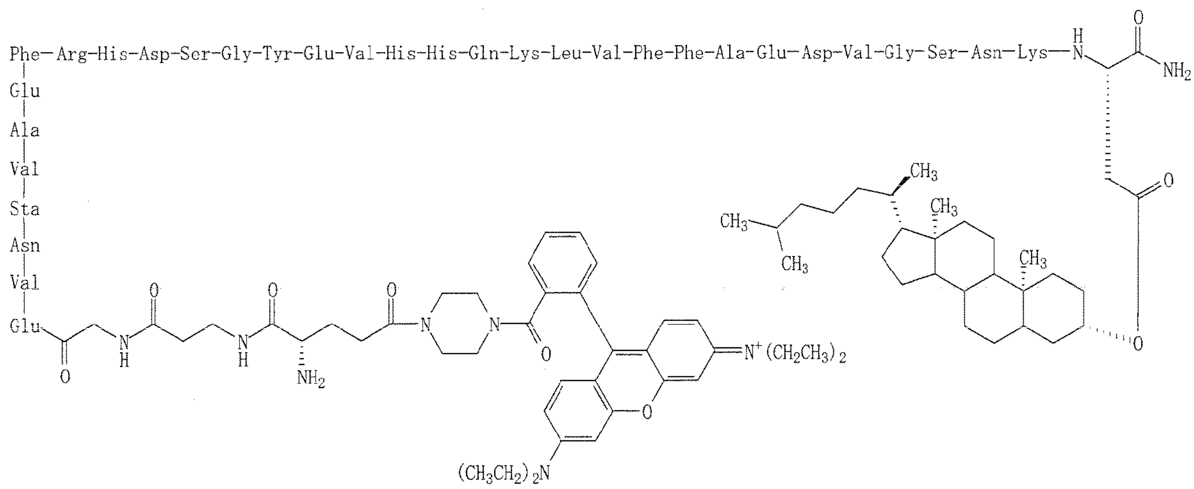

24b

Glu(Rho)-βAla-Gly-Glu-Val-Asn-Sta-Val-Ala-Glu-Phe-Arg-His-Asp-Ser-Gly-Tyr-Glu-Val-His-His-Gln-Lys-Leu-Val-Phe-Phe-Ala-Glu-Asp-Val-Gly-Ser-Asn-Lys-Asp(dihydrocholesteryl)-$NH_2$

Fmoc-Asp(dihydrocholesteryl) (prepared as described for moiety **200k** above) was loaded onto 0,1 mmol of PAL-PEG-PS resin as described for compound **25b** below. After automated washing, capping and deprotection, the following amino acid, Fmoc-Lys(Boc) was loaded manually using 234 mg (0,5 mmol) of Fmoc-Lys, 190 mg (0,5 mmol) of HATU, 190 μl (1,0 mmol) of DIPEA, procedure as before. The remaining sequence until βAla was built using the ABI 433 peptide synthesizer as described for compound **25b** below. Glu(Rho) was attached manually using 244 mg (0,25 mmol) of Fmoc-Glu(Rho), 95 mg (0,25 mmol) of HATU, 84 μl of DIPEA and 3 ml DMF in a similar manner as for compound **25b** below. The resin was deprotected and washed using the ABI 433 and dried in vacuo. Cleavage was carried out using trifluoroacetic acid/$H_2O$/triisopropylsilane/anisol/thioanisol (87:4:3:3:3) as described for compound **25b** below. HPLC-purification was carried out using a gradient of 42 to 46% B over 30 min, other conditions as described below in the preparation of compound **25b** (RT ≃ 24 min). Drying yielded 12,7 mg of red solid.

Analytical HPLC-MS was carried out using a C8 column type Vydac 208TP104, the same eluents as for the preparative separation, 1 ml/min flow rate and a gradient of 42 to 56% B over 35 min. The total purity was found to be 80,9% by MS-trace. Two impurities co-eluting inside the product peak amounted to 12,3%. ESI-MS: 1246,1 $[M]^{4+}$. MALDI: 4977,7 $[M]^+$.

**Example 30: Preparation of a compound of the invention having formula 25**

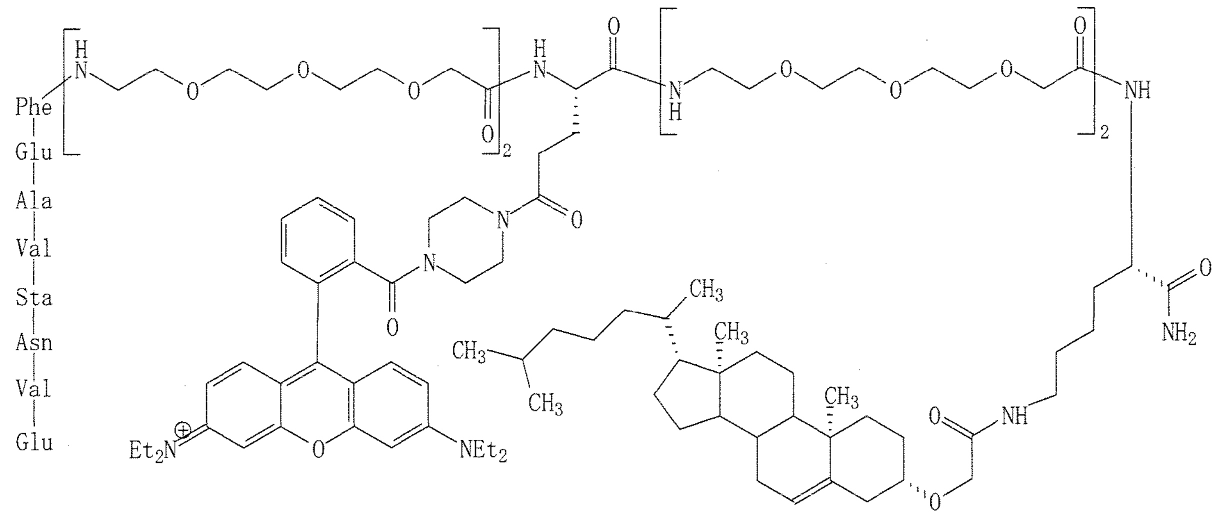

25

The preparation of **25** is achieved as outlined above in the general description. After manual coupling of cholesteryl glycolic acid (precursor of moiety **200a**) to the ε-amino group of lysine the resulting lysine derivative is coupled *via* its C-terminus to Fmoc-PAL-PEG-PS amide resin followed by automated solid phase peptide synthesis coupling successively twice 2-[2-(2-aminoethoxy)ethoxy]ethoxy acetic acid, rhodamine labelled glutamic acid, twice 2-[2-(2-aminoethoxy)ethoxy] ethoxy acetic acid, phenylalanine, glutamic acid, alanine, valine, statine, asparagine, valine, and glutamic acid to obtain the pharmacophore-polyglycol linkerraftophile conjugate on a solid support. Subsequent cleavage from the resin following the general cleavage procedure described above results in **25** after purification by preparative HPLC.

**Example 31: Preparation of a compound of the invention having formula 25b**

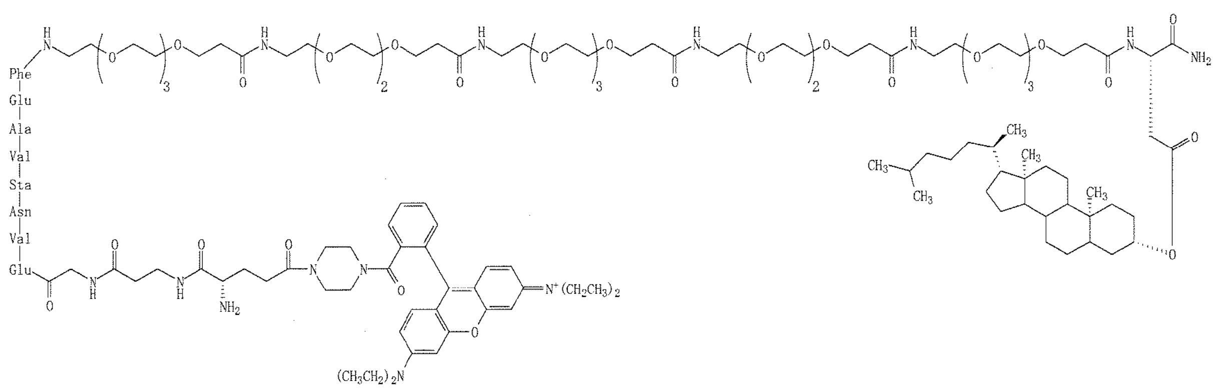

25b

Glu(Rho)-βAla-Gly-Glu-Val-Asn-Sta-Val-Ala-Glu-Phe-4Gl-3Gl-4Gl-3Gl-4Gl-Asp(dihydrocholesteryl)-$NH_2$

An active ester solution was prepared from 363 mg (0,5 mmol) of Fmoc-Asp(dihydrocholesteryl) (prepared as described for moiety **200k** above), 190 mg (0,5 mmol) of HATU, 190 μl (1,0 mmol) of DIPEA, 2 ml of $CH_2Cl_2$ and 1 ml of DMF. This solution was added to 100 μmol of deprotected, $CH_2Cl_2$-wet PAL-PEG-PS-resin (loading: 0,21 mmol/g). The amino acid was allowed to couple for 1 h, during which time 1 ml of DMF was added to remove a precipitate. Washing and deprotection were carried out on the ABI-433 synthesizer. Except for Glu(Rho), the remaining sequence was built on the ABI-433. Since low-load resin and a long sequence were processed, a 0,25 mmol chemistry program was used which allows for greater reaction volume and uses more solvent for washing. Furthermore, the coupling time was extended to 50 min and the second "residue" (4Gl) was attached via double coupling. After the final deprotection and washing, the N-terminal Glu(Rho) was attached in a similar way as described above for the coupling of Fmoc-Asp(DHC) using 293 mg (0,3 mmol) of Glu(Rho), 114 mg (0,3 mmol) of HATU, 102 µl (0,6 mmol) of DIPEA, 2 ml of DMF and 2 ml of $CH_2Cl_2$ and 1,5 h of coupling time. Final deprotection and washing were done using the ABI-433. Cleavage and deprotection were carried out using trifluoroacetic acid/$H_2O$/anisol/triisopropylsilane (90:4:3:3) and 90 min of reaction time. The product was precipitated with ether/petroleum ether (30:70), taken up in MeCN/MeOH (1:1), rotavapped to dryness and dried in high vacuum. Preparative HPLC-purification was carried out using a gradient of 40 to 57% B over 25 min (RT = 24,06 min). After drying, 34,1 mg of red solid were obtained. Analytical HPLC provided a retention time (RT) of 32,1 min and purity of 93% (215 nm). MALDI: 3346,9 $[M]^+$.

**Example 32: Preparation of rhodamine-labeled raftophiles for the evaluation of raftophilicity in LRA and DRM assays**

General remarks

Peptide couplings were performed on an ABI-433 synthesizer using the Fmoc-protocol and HBTU as a coupling reagent. Typically, 4 equivalents of active ester relative to resin and a coupling time of 1 h were used. Expensive amino acids or difficult couplings were carried out using HATU instead of HBTU, extended coupling time and sometimes reduced amounts (less than 2 equivalents of active ester) to maximise compound usage.

The use of very acid-labile Sieber resin is preferred to avoid side reactions / decomposition, e.g. of ceramides during cleavage from the solid support. Amino acids like Arg(Pbf) require more than 85% trifluoroacetic acid and more than 1 h of reaction time for complete deprotection. PAL-PEG-PS-Resin is preferred in these cases, since the Sieber linker gives rise to side reactions in concentrated trifluoroacetic acid.

Typical procedures

Preparation of rhodamine-labeled raftophile moiety 200b having a short peptide linker between raftophile and dye label and using a cleavage protocol employing concentrated trifluoroacetic acid

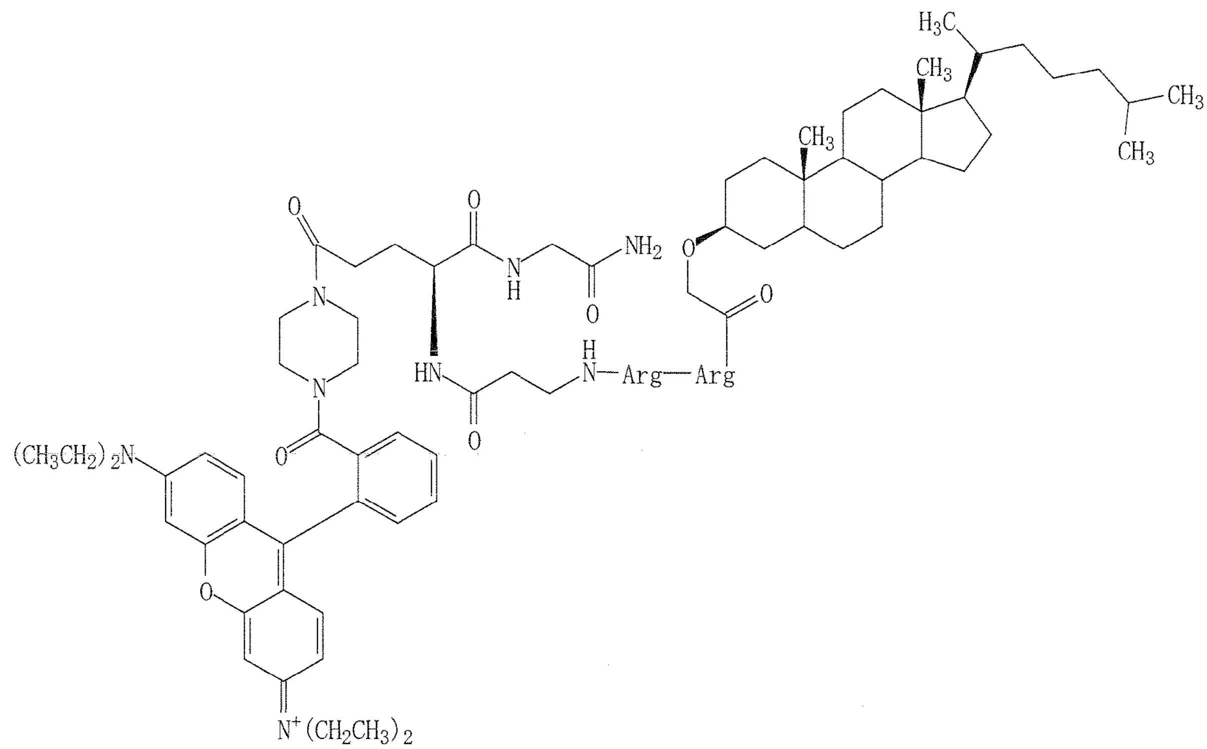

DHC-Glc-Arg-Arg-βAla-Glu(Rho)-Gly-$NH_2$

0,25 mmol of Sieber amide resin were loaded with Fmoc-Gly as outlined above and deprotected with piperidine. The resin was washed with N-methyl-2-pyrrolidone and $CH_2Cl_2$ and transferred to a flask equipped with argon inlet, septum and stirring bar. The flask was quickly evacuated and refilled with argon twice. In a separate flask, 590 mg (0,6

mmol) of Fmoc-Glu(Rho) and 229 mg (0,6 mmol) of HATU were suspended in 3 mL of DMF with stirring under argon. 208 μL (1,2 mmol) of DIPEA were added and the solids were dissolved by stirring and sonication for 5-10 min. The resulting deep-red solution was added to the resin via syringe. The resin was gently stirred for 1 h in this solution. The liquid was filtered off, the resin was briefly washed with N-methyl-2-pyrrolidone and $CH_2Cl_2$, transferred back to the synthesizer and washed with N-methyl-2-pyrrolidone until the washings come off colourless. Automated synthesis was continued by coupling of Fmoc-βAla and 2 x Fmoc-Arg. UV-monitoring was used to ensure completeness of coupling steps. After the final deprotection, the resin was washed with N-methyl-2-pyrrolidone and $CH_2Cl_2$, split in portions of ca. 50 μmol and dried in vacuo. Dihydrocholesteryl glycolic acid (142 mg, 318 μmol) and HATU (121 mg, 318 μmol) were placed under Ar. DMF (3 ml), $CH_2Cl_2$ (2 mL) and DIPEA (104 μL, 636 μmol) were added and the mixture was stirred and sonicated until a clear solution was obtained (ca. 5 min). This solution was transferred to a 50 μMol portion of the aforementioned resin and the resulting suspension was gently stirred under Ar for 1 h. The resin was briefly washed manually with DMF and $CH_2Cl_2$, transferred to the ABI synthesizer, washed with N-methyl-2-pyrrolidone and $CH_2Cl_2$ and dried in vacuo. To the dried resin was added 3-4 mL of a mixture of trifluoroacetic acid/$H_2O$/anisol/thioanisol/triisopropylsilane (87:4:3:3:3) and the resulting suspension was gently stirred under Ar for 2 h. The resin was filtered off and washed with ca. 2 ml of cleavage cocktail. The product was precipitated from the filtrate by addition of cold ether/petroleum ether (1:2, ca. 100 mL) and separated by centrifugation. The supernatant was discarded and the oily, red precipitate was taken up in MeCN/MeOH (2:1), rotavapped to dryness and dried in vacuo.

Preparative RP-HPLC purification was carried out using a Vydac C8 column (30x250 mm) type 208TP1030, $H_2O$/MeCN/MeOH (90:5:5) + 0,1% trifluoroacetic acid as eluent A, MeCN + 0,1% trifluoroacetic acid as eluent B, a flowrate of 40 mL/min and a gradient of 50 to 65% B over 30 min. (RT = 14 min, UV-detection at 215 nm.) The combined product frations were rotavapped to dryness and dried in HV to give 24,4 mg of dark purple solid.

Analytical HPLC was carried out using the same eluents as above, a Vydac 208TP104 column (4,6x250 mm) and a gradient of 45 to 70% B over 25 min at 1 ml/min. RT = 18,3 min, purity: 99% (215 nm). ESI-MS: 754,5 $[M+H]^{2+}$.

Preparation of rhodamine-labeled raftophile moiety **200j** having a short glycol linker between raftophile and dye label and using a mild cleavage protocol employing 1% trifluoroacetic acid in dichloromethane

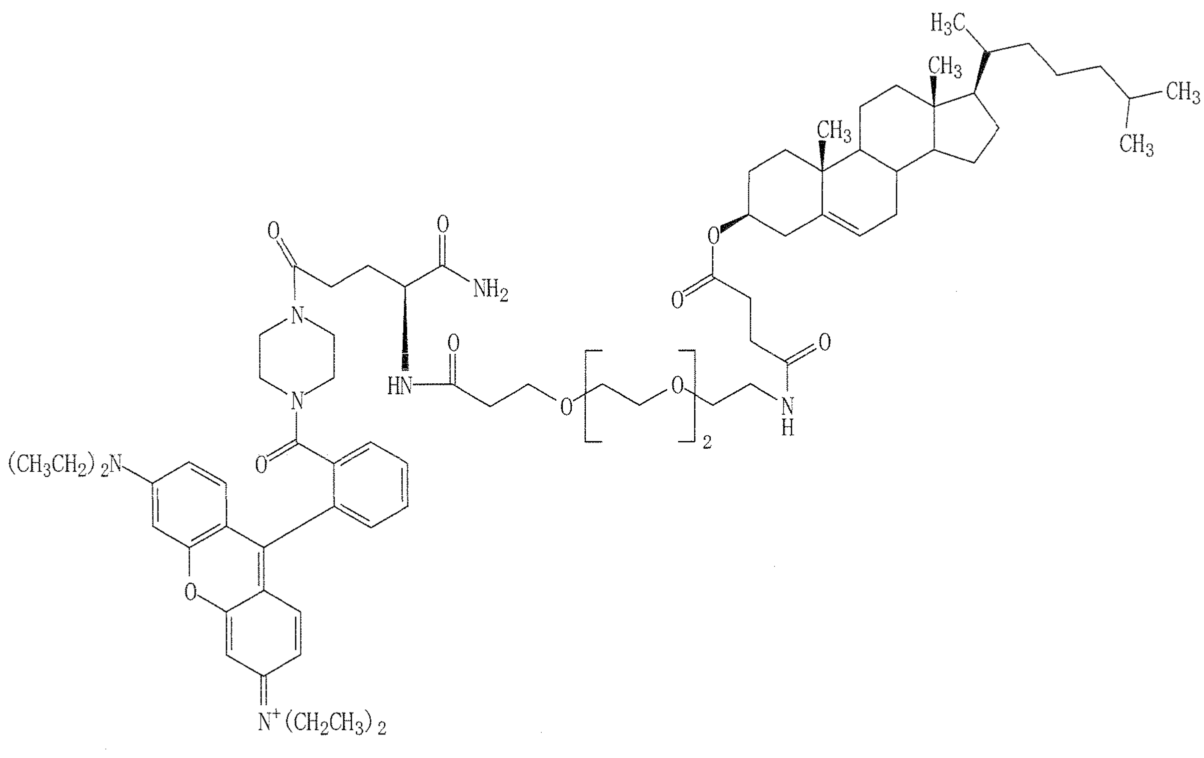

Chol-Suc-3Gl-Glu(Rho)-$NH_2$

250 μmol of 3Gl-Glu(Rho)-NH-[Sieber Resin] were prepared in a similar way as described above using commercially available Fmoc-12-amino-4,7,10-trioxadodecanoic acid as linker building block. To a 50 μmol portion of this resin was added an active ester solution prepared from cholesterylhemisuccinate (97,4 mg, 200 mmol), HATU (76 mg, 200 μmol), $CH_2Cl_2$ (1,5 mL), DMF (0,5 mL) and DIPEA (68 μL, 400 μmol) as described above. After 2,5 h of reaction

time, the resin was washed as before. The $CH_2Cl_2$-wet resin was repeatedly shaken with portions of 2-4 mL of 1% trifluoroacetic acid in $CH_2Cl_2$ for 2-3 min and filtered, until the acid solution comes off colourless (ca. 8-10 times). The combined filtrates were rotavapped to dryness in vacuo at 28°C bath temperature. The residue was taken up in acetonitrile, transferred to a smaller flask, rotavapped down again and dried in vacuo.

HPLC purification was carried out as above, but using $H_2O$/MeCN/MeOH (85:10:5) + 0,1% trifluoroacetic acid as eluent A and a gradient of 64 to 74% B over 20 min. (RT: 14,5 min.) Analytical HPLC was carried out using the same eluents and a gradient of 10 to 100% B over 45 min. (Other conditions as in the previous example.) RT: 38,4 min, purity: 96% (215 nm).

ESI-MS: 1310,8 $[M]^+$.

**Example 33: Liposome Raftophile Assay (LRA) and Detergent Resistant Membrane Assay (DRM)**

In accordance with the present invention, raftophilicity of a compound of the present invention may be determined by in vitro testing of the synthesized compounds. Said in vitro tests comprise the test provided herein. The assays provided herein and described in detail below may be employed as single assays or in combination.

**A. Principle of LRA**

The partition of test compounds into liposomes representing either non-raft or raft membrane is determined. The test system contains 3 components in which test compounds may be found, the lipid membrane (non-raft or raft), the aqueous supernatant and the test tube wall. Following incubation, the liposomes are removed from the system and the test compounds are measured in the aqueous and tube wall fraction by fluorimetry using a Tecan Safire multifunctional double-monochromateor fluorescence intensity reader or quantitative mass spectrometry. Mass spectrometrical analysis was performed by combination of HPLC and mass spectrometry (HPLC-MS) using a Hewlett-Packard 1100 (for HPLC) and an Esquire-LC (for mass spectrometry); the method used for mass spectrometry was electrospray ionisation (ESI) as also used for chemistry. Data are computed to yield partition coefficients and raftophilicity.

**Experimental protocol**

1. Add raft (R) or non-raft (N) liposomes (see Liposome Preparation; see below) to replica tubes and preincubate for 1 h at 37°C.

2. Add test compounds (usually in dimethylsulfoxide (DMSO)) and incubate for 1 h. Remove liposomes from one set of tubes, elute adherent compound from tube wall with 100 µl 40 mM octyl-β-D-glucopyranoside (OG) / phosphate-buffered solution (PBS) (A). Centrifuge a second set of tubes at 400,000 x g and collect supernatant (S) according to scheme 1.

Scheme 1

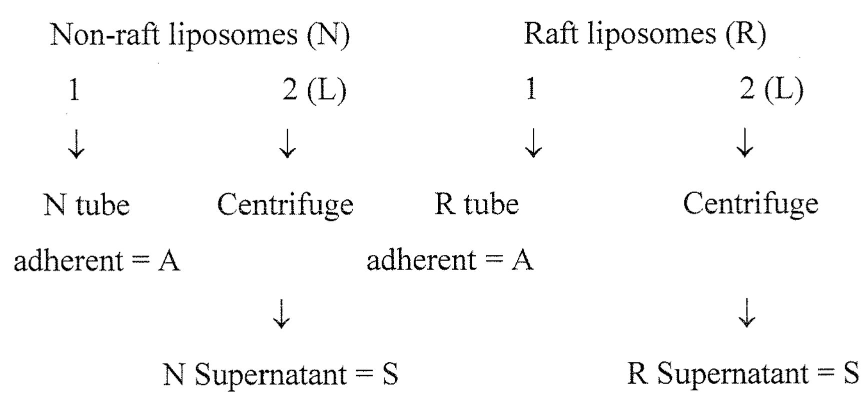


Compounds are detected in the aqueous supernatant and the adherent fraction by fluorimetry or quantitative mass spectrometry.

**Computation of partition coefficients and raftophilicity**

**Fractions**

| | |
|---|---|
| **L** | liposomes (before centrifugation) |
| **A** | tube-adherent |
| **S** | supernatant, free compound concentration |
| **I** | input concentration = L + A |

**Concentration in membrane fraction (M)**

$$M = L - S \qquad (1)$$

$$[M] = M * f\,(\text{volume ratio factor}) \qquad (2)$$

f = volume ratio of aqueous : membrane at 1 mg lipid/ml = 878.65

**Partition coefficients and raftophilicity**

Partition coefficient Cp is the ratio of compound concentrations in the membrane and the aqueous phase:

$$Cp = [M] / S \qquad (3)$$

**Raftophilicity (Rf):**

$$Rf = Cp(R) / Cp(N) \qquad (4)$$

**Liposome preparation**

Preparation and storage of lipid mixes

Lipid solutions and mixes are usually made up at 10 mg/ml.

Example of a composition of liposome mixes.

| Raft (R) | Mol % | mM lipid / mM phosphatidylcholine |
|---|---|---|
| Cholesterol | 50 | 2.03 |
| Sphingomyelin | 15 | 1.08 |
| Gangliosides type III | 5 | 0.43 |

(continued)

| Raft (R) | Mol % | mM lipid / mM phosphatidylcholine |
|---|---|---|
| Phosphatidylcholine (PC) | 15 | 1.00 |
| Phosphatidylethanolamine | 15 | 1.02 |
| | | |
| **Non-raft (N)** | | |
| Phosphatidylcholine | 50 | 1.00 |
| Phosphatidylethanolamine | 50 | 1.02 |

**Formation of liposomes by dialysis**

1. Take up lipids in 600μl 400 mM 1-octyl-β-D-glucoside (OG) in PBS or other buffer at room temperature, 37 ˚C (non-raft lipids) or 50 ˚C (raft lipids) in a rotary evaporator. When dissolved, vortex for 10 s. Vary detergent concentration proportionally with lipid concentration.

2. Dilute lipids to 1 mg/ml. Add 5.4 ml buffer (cell culture quality) at room temperature and vortex for 10 s. If a lipid residue remains rotate for another 5-10 min. at 37˚C or 50 ˚C. At the beginning of dialysis, raft lipid:detergent ratio should be 0.04.

3. In a 22 ˚C room prepare a 5 1 glass beaker with 5 1 PBS and 20 g pre-treated Amberlite XAD-2 beads. Stir at 200-250 rpm.

4. Take up the lipid mixtures with a 10-20 ml syringe and feed into porthole of a slide-a-lyzer cassette. Carefully withdraw all the air from the cassette. Dialysis for 8 h, change and dialyse overnight.

5. Retrieval of liposomes: Remove Amberlite beads sticking to the outside of the cassettes by rinsing with buffer. Fill sufficient air into cassette from an unused port with a 10 - 20 ml syringe, tilt the cassette and withdraw the liposomes.

6. Transfer to glass tube and store on ice in the dark. Keep in the cold-room until use and use within the next 3 days.

In accordance with this invention, a compound, in particular a tripartite compound of this invention, is considered as "raftophilic" when the ratio of the equilibrium constants as defined above is greater than 8, more preferably greater than 9, more preferably greater than 10, even more preferably greater than 11. As documented herein, even more preferred compounds (either the precursor of the moiety A and A' of the tripartite compound of this invention or the complete tripartite compound) are compounds where the ratio of the equilibrium constants is greater than 20, even more preferred greater than 30, most preferred greater than 40.

This test/assay as well as the following DRM assay is useful to deduce, verify and/or determine the raftophilicity of a given construct, e.g. a tripartite construct of the invention as well as the raftophilicity of a moiety A/A' of the compounds of this invention.

**B. The principle of Detergent Resistant Membrane Assay (DRM)**

The accumulation of test compounds in cellular membrane fractions derived from a non-raft and a raft membrane is determined. The test system involves treatment of cultured cells with test compound. Following incubation, cells are lysed in detergent solution and the DRM fraction (rafts) is isolated on a sucrose gradient. The DRM fraction is recovered and test compounds are measured by fluorimetry or quantitative mass spectrometry. Raftophilicity is determined as the proportion of test compound recovered in the DRM fraction compared to that contained in the total membrane.

A better comparison of results of different experiments is achieved by comparing the raftophilicity of a test compound to that of a known, raftophilic standard.

**Experimental protocol**

1. Cultured mammalian cells (e.g. MDCK, NIH3T3, E6.1 Jurkat T, RBL-2H3, NK3.3, Ramos, Caco-2, BHK) are grown to sub-confluence.

2. Cells are incubated with test compound (usually at 1-10 μM in ethanol or DMSO) for 1 h at 37°C; dishes are washed twice with 2 ml ice-cold TNE buffer (100 mM Tris (pH 7.5), 150 mM NaCl, and 0.2 mM EGTA (ethylene glycol-bis-(beta-amino-ethyl ether) N,N,N'N"-tetra-acetic acid)) and chilled 5 min. Cells are extracted for 30 min with 0.5 ml TNE buffer containing 1% (w/v) Triton X-100 at 4°C.

3. Cells are scraped with a cell lifter and homogenized by passing ten times through a 1 ml pipette tip. Lysates are transferred to Eppendorf tubes and ultrasonicated in an ice-water bath and subsequently centrifuged for 5 min at 3,000 rpm, 4°C.

4. Lysates are brought to about 47% sucrose by transferring 0.3 ml lysate to an Eppendorf tube containing 0.6 ml 65% (w/w) sucrose/TNE with vigorous vortexing. Of this lysate/sucrose sample, 0.7 ml are placed on the bottom of a SW-60 tube, overlayed with 2.7 ml 35% (w/w) sucrose/TNE and then 0.8 ml TNE. Gradients are centrifuged in an SW-60 rotor at 335,000 x g for 16 h at 4 °C.

5. DRM and non-DRM fractions of 1040 μl are collected starting from the top of the gradient. The DRM fraction equals pooled fractions 2 & 3 and the non-DRM fraction equals pooled fractions 6, 7 and 8. Compounds are detected in the fractions by fluorimetry or quantitative mass spectrometry.

**Computation of raftophilicity in accordance with DRM**

A dimensionless raftophilicity quotient rq can be derived:

$$r_q = \%\mathrm{DRM} / \%\mathrm{non\text{-}DRM}$$

The relative raftophilicity ($r_{rel}$) of an unknown compound in relation to a standard is computed as:

$$r_{rel} = r_q\ (\text{test compound}) / r_q\ (\text{standard})$$

Positive values indicate that the test compound is more raftophilic than the standard. A standard may be, but is not limited to, cholesteryl 4,4-difluoro-5,7-dimethyl-4-bora-3a,4a-diaza-s-indacene-3-dodecanoate (cholesteryl BODIPY® FL C12; Molecular Probes, Eugene, USA).

In accordance with the assay presented here and the invention, a compound, in particular a compound of the present invention, is considered as "raftophilic" when the corresponding relative value (in comparison to the standard) is greater than 0.1.

**Example 34: Exemplified LRA-Test**

This assay is used for all test compounds which are sufficiently water soluble to give a measurable aqueous concentration after incubation with liposomes. Other lipophilic test compounds (e.g. compound 27) are measured in the DRM assay (see Example 33).

The tripartite compound and cholesteryl glycolic acid were assessed for their ability to partition into liposomes composed of lipid mixtures representing rafts (cholesterol: sphingomyelin: phosphatidylcholine: phosphatidylethanolamine: gangliosides (bovine brain, Type III, Sigma-Aldrich Co.) (50:15:15:15:5)) compared to a mixture representing non-rafts (phoshphatidylcholine: phoshphatidylethanolamine (50:50)) at 37°C. Relative partitioning as defined above was defined as raftophilicity in the LRA assay. The compound was added at a final concentration of 0.2-2.0 μM from a

DMSO or ethanol stock solution to duplicate sets of liposomes using the compositions listed above. The maximum compound concentration was 2 mol% with respect to the lipid concentration. Liposomes were preincubated in PBS for 30 min at 37˚C in a Thermomixer before addition of compound and further incubation for 1 h at 37˚C. Liposomes were quantitatively transferred from one set of tubes and residual compound was eluted from the tube wall with 100 μl 40 mM octyl-β-D-glucopyranoside in PBS. A second set of tubes was centrifuged at 400,000 x g and the supernatant was collected. Compound concentrations were determined in the total liposome solution, the adherent fraction and the aqueous supernatant by fluorimetry or quantitative mass spectrometry.

A partition coefficient for the compound in each liposome type was determined as the ratio of the concentration of the compound in the liposome membrane versus the concentration in the aqueous supernatant. The volume of liposome membrane was calculated using a volume ratio of aqueous: membrane at 1 mg lipid/ml of 878.65. The raft affinity (raftophilicity) was calculated as the ratio of the partition-coefficients for raft and non-raft liposomes.
The LRA raftophilicity of the cholesterol-based raft anchor alone was approximately 50 (i.e. 50-fold more affinity for raft liposomes) and that of the tripartite compound was over 50.

As discussed herein above, in accordance with this invention, values of greater than 8, more preferably greater than 9 are considered as being a measure for raftophilicity in context of the LRA. Preferred raftophilic compounds are significantly raftophilic when their corresponding LRA value is greater than 10. Accordingly the tripartite compound tested above is considered as highly raftophilic compound.

**Example 35: Exemplified DRM-Assay/DRM-Test**

Sub-confluent MCDK (canine kidney epithelium) or RBL-2H3 (rat B-cell lymphoma) cells, grown in Minimum Essential Medium with Earle's Salts (MEM-E), 1x GlutaMax I (Invitrogen), 5% FCS, 250 μg/ml G418, are washed in MEM-E, 1x GlutaMax I, 10 mM HEPES, pH 7.3, and incubated in the same medium but containing compound 27, at a final concentration of 1.0-10 μM in combination with a raft marker substance e.g. cholesteryl BODIPY-FL C12 (Molecular Probes, Inc) at 1.0 μM, both from DMSO or ethanol stock solutions, for1hr at 37˚C. The cells are washed twice with 2 ml ice-cold Dulbecco's PBS with Ca2+, Mg2+, chilled for 5 min. at 4˚C and then extracted for 30 min with 0.5 ml 25 mM Tris-HCl, pH 7.5, 150 mM NaCl, 5 mM EDTA (ethylenediaminetetraacetic acid), 1% (w/v) Triton X-100 (TN-T) at 4˚C. The cells were scraped from the plate and homogenized by passing ten times through a 25G syringe needle. Lysates were ultrasonicated in an ice-water bath with a Bandelin Sonoplus HD200 sonifier (MS73 tip, power setting at MS72/D for 60 s. at cycle 10%) and subsequently centrifuged for 5 min at 3000 x g at 4˚C. Lysates are brought to 47% sucrose by transferring 0.3 ml lysate to an Eppendorf tube containing 0.6 ml 65% (w/w) sucrose/25 mM Tris-HCl, pH 7.5, 150 mM NaCl, 5 mM EDTA (TNE) and vigorous vortexing. A lysate/sucrose sample, 0.7 ml, was placed on bottom of a SW-60 tube and overlayed with 2.7 ml 35% (w/w) sucrose/TNE and 0.8 ml TNE. Gradients were centrifuged in a Beckman LE 80K centrifuge with a SW 60 rotor for 16 h at 335,000 x g, 4˚C. Fractions of each 520 μl were collected from the top to the bottom of the gradient. Pooled fractions 2 and 3 are collected. This represents the DRM fraction. Pooled fractions 6, 7 and 8 represent non-DRM fraction. Compound concentrations were determined in the DRM and non-DRM fractions by quantitative fluorimetry or mass spectrometry. A dimensionless raftophilicity quotient rq is derived rq = %DRM / %non-DRM, where % DRM and % non-DRM is the total fluorescence or mass of compound in the respective fractions. Test compound raftophilicity in the DRM assay, rq, is normalized to the raftophilicity of a standard e.g. cholesterol BODIPY-FL C12, obtainable from Molecular Probes, rrel = rq (test compound) / rq (standard).

In this assay tripartite compound 27 containing a ceramide as raftophile had an $r_{rel}$ of 0.48.

Accordingly, a compound, in particular a tripartite construct/compound as well as an individual moiety A and A' as defined herein may be considered as "raftophilic" when it has an rrel (in accordance with this assay system) of greater than 0.2, more preferably more than 0.3, even more preferably more than 0.4.

**Example 36: Inhibition of BACE-1 by a tripartite compound of the invention**

Tripartite compounds having formulae **24, 24b, 25** and **25b** were tested for their ability to inhibit β-secretase (BACE-1) in a whole cell assay and the potencies compared to that of the free inhibitor III. Murine neuroblastoma cells (N2a) grown in DMEM (Dulbecco's Modified Eagle Medium), 1x glutamine, 10% FCS (fetal calf serum) were infected with recombinant adenovirus containing the amyloid precursor protein (APP) gene. After infection for 75 min. cells were washed, trypsinized and subcultured, After about 20 hr (50% confluence) the medium is aspirated and replaced with fresh medium containing test compound at 10 nM to 10 μM in DMSO or methanol and cells incubated for a further 3-4 hr, 37˚C, 5% CO2. After incubation, a supernatant sample was collected and the production of β-cleaved ectodomain of APP (βAPPs) measured using an ELISA assay with a specific monoclonal antibody against βAPPs and an second antibody against the N-terminal portion of βAPPs. In this system inhibitor III alone is not inhibitory whereas the tripartite compounds **25** and, in particular, **25b** are potent inhibitors of βAPPs and therefore of β-secretase activity as also demonstrated in appended Figures. Furthermore, tripartite compounds containing a shorter linker [see compounds **26**

and **27**] are in this specific assay less effective demonstrating that the linker length is critical to the inhibition of beta-secretase by inhibitor III. Accordingly, compounds **26** and **27** do probably not place the specific pharmacophore inhibitor III at the correct locus on the BACE-1 enzyme. Yet, a linker as defined in compounds **26** and **27** may be useful in other test systems for inhibition of biological molecules where the corresponding binding/interaction site is located closer to the heads of the phospholipids of the raft.

**Example 37: Tripartite compounds of the invention in proteoliposome assays**

In the following, a further, non-limiting assay for verification and/or characterization is described employing the herein disclosed tripartite structures as models. The assay is a proteoliposome assay.

The principle of the BACE Proteoliposome Assay

Tripartite raftophilic test compounds are incorporated into liposomes representing raft membrane which are then reconstituted with recombinant BACE (BACE proteoliposomes) as described under A. BACE is membrane-anchored by a transmembrane domain. The lipid moiety of the test compound is anchored in the membrane while the spacer and pharmacophore project into the aqueous phase. At optimal topology the pharmacophore can block the BACE active site (Fig. 1: *Top*). For activity and inhibition assay BACE proteoliposomes are suspended in assay buffer and preincubated for 10 min at room temperature. The temperature is shifted to 37˚C, and an internally quenched fluorescent substrate analog FS-1 (Dabcyl-[Asn670,Leu671]-Amyloid β/A4 Protein Precursor770 Fragment (661-675)-Edans; Sigma A 4972) is added, the cleavage of which elicits a fluorescent signal. This signal is recorded at set intervals in a Thermoscan Ascent fluorimeter (see Fig. 1: *Bottom*).

Experimental protocol

Proteoliposomes are prepared in two steps:

1. liposome formation and test compound incorporation by serial dialysis; followed by
2. detergent-facilitated membrane reconstitution of BACE and purification by gel filtration and density gradient centrifugation.

Ad 1. Formation of liposomes incorporating test compounds by dialysis

1.1. Porcine brain lipids (Avanti 131101), 5 mg in chloroform solution, are spread in a round-bottomed flask in a rotary evaporator and evacuated over night in a desiccator. The lipid is taken up in 0.5 ml 400 mM 1-octyl-β-D-glucoside (OG) in water and rotated at 50 ˚C, then 1.166 ml phosphate buffered-saline (PBS), 0.02 % sodium azide ($NaN_3$) is added to a final OG concentration of 120 mM and lipid concentration of 3 mg/ml or 4.8 mM. The suspension is rotated again at 50˚C for about 5 min until homogenous.

1.1.a Proof of raft character of porcine brain lipid liposomes. Porcine brain lipids have a qualitatively and quantitatively similar but more complex composition compared to the raft lipid mix as used in the LRA. Raft (R) liposomes and non-raft (N) liposomes, prepared (described in, Example 33. A. Principle of LRA, Liposome preparation) and porcine brain lipid liposomes (P), are tested with a standard raftophilic tracer. Partition into P liposomes is shown to be identical or somewhat greater than into R liposomes; raftophilicity (Rf) ≥ 40.

1.2. The lipid suspension is aliquoted (0.35 ml for controls and 0.26 ml for incorporation of test compound) into glass tubes. To some aliquots add test compounds from 100x stock solutions in DMSO and vortex 10s. At the beginning of dialysis, total lipid:detergent ratio should be 0.04 and 1 % DMSO. Test compound starting concentration is between 0.0005 and 0.05 mol %.

1.3. Take up 0.25 ml (initial volume, $v_i$) lipid mixtures with a 1 ml syringe and feed into porthole of an overnight predialyzed 0.5 ml, slide-a-lyzer cassette (Pierce) with 10 kD exclusion. Carefully withdraw all the air from the cassette. Transfer each cassette to a Petri dish containing 375 μl PBS/0.02 % $NaN_3$ placed directly under and 375 μl PBS on top of the cassette. Dialyse for 3 h and exchange twice, using new Petri dishes for each change. The third dialysis is over-night. Continue on day 2 with 3 changes of 2 x 2.5 ml PBS.

1.4. In a 22˚C incubator prepare a 5 L glass beaker with 5 L PBS and 100 ml 20% pre-treated Amberlite XAD-2 beads (Supelco 20275). Transfer cassettes into beaker and dialyse for 16 h. Stir at 200-250 rpm.

1.5. Retrieval of liposomes: Remove Amberlite beads sticking to the outside of the cassettes by rinsing with buffer. Fill sufficient air into cassette from an unused port with a 1 ml syringe, tilt the cassette and withdraw the liposomes.

1.6. Measure the post-dialysis volume ($v_p$) with the syringe and transfer to brown glass tubes. Dilute each sample to 3x the initial volume. Determine the post-dialysis test compound concentration by fluorimetry, mass spectroscopy or other suitable method. Store on ice in the dark until use within 24 h.

Ad 2. Proteoliposome preparation

2.1. Pellet liposomes and take up in 70 μl 10 mM Hepes/150 mM NaCl pH 7.3 (buffer). Add 8 μl 10 % decanoyl-N-hydroxyethylglucamide (HEGA 10). Then add 2 μg/8 μl recombinant BACE (in 0.4 % Triton X-100).

2.2. Gel filtration over Sephadex G-50 in 10 mM Hepes/150 mM NaCl pH 7.3.

2.3. Float on 5 % Optiprep gradient to separate proteoliposomes from empty liposomes.

2.4. Harvest proteoliposome band, dilute and pellet. Resuspend pellet in 50-100 μl buffer and quantify protein.

**Exemplified BACE Proteoliposome Assay:**

1. Formation of liposomes incorporating test compounds by dialysis

1.1. Spreading and homogenization of lipids.

4 mg porcine brain lipids (Avanti 131101) in chloroform are dried in a round-bottomed flask in a rotary evaporator at 50 ˚C. 1.5 ml tert-butanol is added to redissolve the lipid. The flask is rotated at 50 ˚C until the lipid forms a homogeneous film. Traces of solvent are removed by drying the flask over night in a desiccator. The lipid is now taken up in 0.5 ml 400 mM l-octyl-β-D-glucoside (OG) in water and rotated at 50˚C, then 1.166 ml phosphate buffered-saline (PBS), 0.02 % sodium azide ($NaN_3$) is added to a final OG concentration of 120 mM and a lipid concentration of 3 mg/ml or 4.8 mM. The suspension is rotated again at 50˚C for about 5 min until homogenous.

1.2. Addition of test compound

The lipid suspension is aliquoted (0.35 ml for controls and 0.26 ml for incorporation of test compound) into glass tubes. Into aliquots compound **25b** is diluted 1:100 from 100x stock solutions in DMSO (cp. Table 1):

| | | |
|---|---|---|
| 0.05 mol % | 2.4 μM | 2.4 μl (250 μM stock) |
| 0.005 mol % | 0.24 μM | 2.4 μl (25 μM stock) |
| 0.0005 mol % | 0.024 μM | 2.4 μl (2.5 μM stock) |

The tubes are then vortexed for 10s.

1.3. Serial dialysis

0.25 ml (initial volume, $v_i$, Table 1) lipid mixtures are transferred with a 1 ml syringe and into a porthole of an overnight predialyzed 0.5 ml, slide-a-lyzer cassette (Pierce) with 10 kD exclusion. All the air is then withdrawn from the cassette. Each cassette is placed in a separate Petri dish containing 375 μl PBS/0.02 % $NaN_3$ pipetted directly under and 375 μl PBS on top of the cassette. After 3 h dialysis the cassettes are transferred to new Petri dishes and the procedure repeated. The third dialysis is over-night. On day 2 the procedure is repeated with 3 changes of 2 x 2.5 ml PBS (2.5 ml PBS below and 2.5 ml PBS on top of the cassette). During the whole procedure the Petri dishes are wrapped in aluminium foil to avoid bleaching.

1.4. Bulk dialysis

A 5 L glass beaker containing 5 L PBS with 100 ml 20% pre-treated Amberlite XAD-2 beads (Supelco 20275) and a magnetic stirrer is placed in a 22˚C incubator. All the cassettes are inserted into floats (Pierce), placed in the beaker and dialysed for 16 h at 200-250 rpm. The beaker is wrapped in aluminium foil.

1.5. Retrieval of liposomes

Amberlite beads sticking to the outside of the cassettes are rinsed off. Using a 1 ml syringe air is filled into the cassette from an unused port, the cassette is tilted and the liposomes withdrawn with the syringe. Using the syringe the post-dialysis volume ($v_p$) is measured and the liposomes transferred to brown glass tubes with screw tops. Each sample is diluted with PBS to 3x the initial volume $v_i$ (see Table 1).

1.6. Determination of final **25b** concentration

**25b** concentration standards 25, 250 and 2500 nM are prepared in PBS/40 mM OG and four 100 µl samples of each standard filled into wells of a 96-well plate (Nunc Maxisorb). 50 µl of each liposome preparation is diluted into 50 µl 80 mM OG in PBS in the 96-well plate. After addition of PBS and OG controls and brief shaking fluorescence is recorded in a Tecan Safire fluorimeter plate-reader at 553/592 nm (excitation/emission wavelength). The fluorescence readings of the standard are plotted and a regression line calculated (Excel) from which the final **25b** concentrations in the liposome preparations are calculated (see Table 1).

<u>2. Proteoliposome preparation</u>

2.1. The liposomes are pelleted 20 min at 48,000 rpm in a TLA-100 rotor and taken up in 70 µl 10 mM Hepes/150 mM NaCl pH 7.3 (buffer). 8 µl 10 % decanoyl-N-hydroxyethylglucamide (HEGA 10) are added. Finally 2 µg/8 µl recombinant BACE (in 0.4 % Triton X-100) are added and mixed by pipetting up and down.

2.2. Gel filtration over Sephadex G-50 in 10 mM Hepes/150 mM NaCl pH 7.3. The sample is pipetted onto the gel filtration column. The flow-through is collected, containing the proteoliposomes.

2.3. This material is pipetted onto a 5 % Optiprep gradient and centrifuged. The proteoliposome band is harvested, diluted and pelleted. The pellet is resuspended in 50-100 µl buffer. The protein is quantified.

2.4. BACE assay
Per well of 96-well plate are added 10 µl proteoliposomes (60 ng BACE, 4.5 µg lipid), 70 µl 80 mM NaOAc pH 5.1 and 20 µl 10 mM Hepes/150 mM NaCl pH 7.3 and mixed. The mixture is preincubated for 30 min at 37 ˚C. Finally, 2 µl substrate FS-1 in 1.5 M HAc (5 µM final conc.) is added. Fluorescence is recorded at 485 nm (excitation 340 nm) every 40 sec. with 8 sec. shaking before each measurement.

Table 1. Overview of lipid and test compound concentrations during proteoliposome preparation

| Initial vol. ($v_i$) [µl] | Final vol. ($v_p$) [µl] | Lipid | | Test compound | | | |
|---|---|---|---|---|---|---|---|
| | | Initial conc. (mM) | Final conc. (mM) | Name | Initial conc. (mol %[1]) | Final conc. (mol %[1]) | Final conc. (µM) |
| 340 | 400 | 4.8 | 1.6 | Control | 0 | 0 | 0 |
| 250 | 400 | 4.8 | 1.6 | 25b | 0.05 | 0.044 | 0.61 |
| 250 | 325 | 4.8 | 1.6 | 25b | 0.005 | 0.0039 | 0.051 |
| 250 | 340 | 4.8 | 1.6 | 25b | 0.0005 | 0.00038 | 0.005 |
| [1] mol % given with respect to lipid concentration | | | | | | | |

Enrichment of the inhibitor within the raft subcompartment by coupling to a raftophile should lead not simply

to a similar increase in potency proportional to inhibitor concentration but to a disproportional increase, due to the reduced ability of the inhibitor to diffuse away from the site of action. This "lock-in" effect exploits the same phenomenon used by the cell to increase protein-protein interactions. The results depicted in Fig. 1 show that **25b** is much more potent than inhibitor III. Measurements taken from the graph reveal that **25b** has an $ED_{50}$ (concentration at which BACE activity is reduced to 50%) of around 1 nM compared to inhibitor III with an $ED_{50}$ of 500-1000 nM. Thus the potency of the inhibitor is increased 500-1000 fold by incorporation into a tripartite structure of the type exemplified by **25b.**

The inhibitors were also tested in a functional assay incorporating neuronal cells expressing exogenous swAPP (a highly-cleavable form of APP) as described in Example 36 (see also Fig *2: Top).* Cells were treated with **25b** or inhibitor III and release of beta-cleavage products measured in the cell culture supernatant.

The results depicted in Fig. 2 show that **25b** is inhibitory in the whole cell assay whereas the commercial inhibitor III is completely inactive. **25b** could reduce activity by 65% at 1 μM. BACE-1 is active in acidic endosomes and free inhibitor III would thus need to pass through both cell and endosome membranes to reach the target. Without being bound by theory, it is likely that **25b** inserts into the raft membrane where BACE-1 is located and is taken up together with the protein. Hence, targeting and efficacy are assured by the presence of the tripartite construct (Fig 2: *Top*). Accordingly, inhibitor III does not cross the cell membrane and the raftophile coupled inhibitor **25b** has gained access to the cell interior as well as efficiently inhibited beta-secretase activity.

**Example 38: Illustrative inhibition of HIV infection by tripartite raftophilic structures**

The early stages of HIV infection - from absorption to entry of host cells - encompass the following consecutive steps (reviewed in Olson (2003) Infect. Disorders 3, 255): Via its spike protein gp120 HIV attaches to the primary receptor, CD4, a raft protein. Attachment elicits conformational change of gp120, enabling it to bind the co-receptor, one of several chemokine receptors, which is recruited to the raft (Fantini (2001) Glycoconj. J. *17*, 199-204). This in turn triggers a conformational change of gp41, the viral fusion protein closely associated with gp120. Gp41 adopts an extended pre-hairpin conformation where the N-terminal fusion peptide projects into the plasma membrane and the two heptad-repeat regions HR1 and HR2 are exposed. When three copies of HR2 fold back onto the HR1 trimer forming a hairpin, the viral and the plasma membrane - two apposed raft domains - are forged together and fused (Weissenhorn et (1997) Nature 387, 426-430.). The strong interaction between HR1 and HR2 can be blocked by soluble HR2 peptide analogues (Wild (1992) Proc Natl Acad Sci USA 91, 9770-9774), of which enfuvirtide (T20; DP178) is one. Also known are, inter alia, T1249 and pegylated forms of these peptide inhibitors. The hairpin does not form and fusion of the viral and host membranes is prevented. It is clear that the soluble inhibitor can only bind to the virus after it has engaged with its two receptors, i.e. it acts membrane-proximally. Indeed, T20 is also inhibitory when expressed on the cell membrane from an appropriate construct (Hildinger (2001) J. Virol. 75, 3038-3042.). In the tripartite structure of the invention the pharmacophore (enfuvirtide) is connected to a raft anchor (raftophile) via spacer elements (hinge and linker) so that the inhibitor projects out of the target membrane raft, towards the infecting virion (see Figure 3). The pharmacophore (HR2 analogue) of the tripartite drug can bind to HR1 elements exposed during the conformational change of gp41 and effectively lock the protein in its conformational transition state, as well as physically immobilizing it at the plasma membrane. The drug concentration to achieve this is predicted to be orders of magnitude lower than that of soluble inhibitors like enfuvirtide because (1) the tripartite drug is enriched in the raft domains about 10,000-fold with respect to the medium and about 50-fold with respect to non-raft membrane and (2) less tripartite drug molecules per virion are required to irreversibly block infection and mark the virion for destruction. In addition to inhibiting the entry of free virus the same inhibitory mechanism will block the fusion of infected to noninfected cells which depends on the same events.

HIV entry assay (after Salzwedel et al., 1999).

Human embryonic kidney 293T cells are transfected with a proviral clone of the HIV strain of interest. 60 - 72 h later the virus-containing cell culture supernatant is collected and filtered through a 0.45 μm pore-size filter. The virus is then used to infect HeLa-CD4/LTR-β-gal cells. Cells are stained with X-gal in situ, the monolayers are imaged with a CCD camera (Fuji LAS) and the number of blue foci is counted. As an alternative readout, the expression of HIV gp 24 can be monitored by ELISA (see, eg, Hildinger (2001), loc.cit.). Similarly, to measure cell-cell fusion, infected 293T cells are mixed with non-infected HeLa-CD4/LTR-β-gal cells and scored in the same way; see, for example, Salzwedel (1999) J. Virol. 73, 2469-2480.

**Synthesis of a compound of the invention 25c comprising T20 as the pharmacophore C**

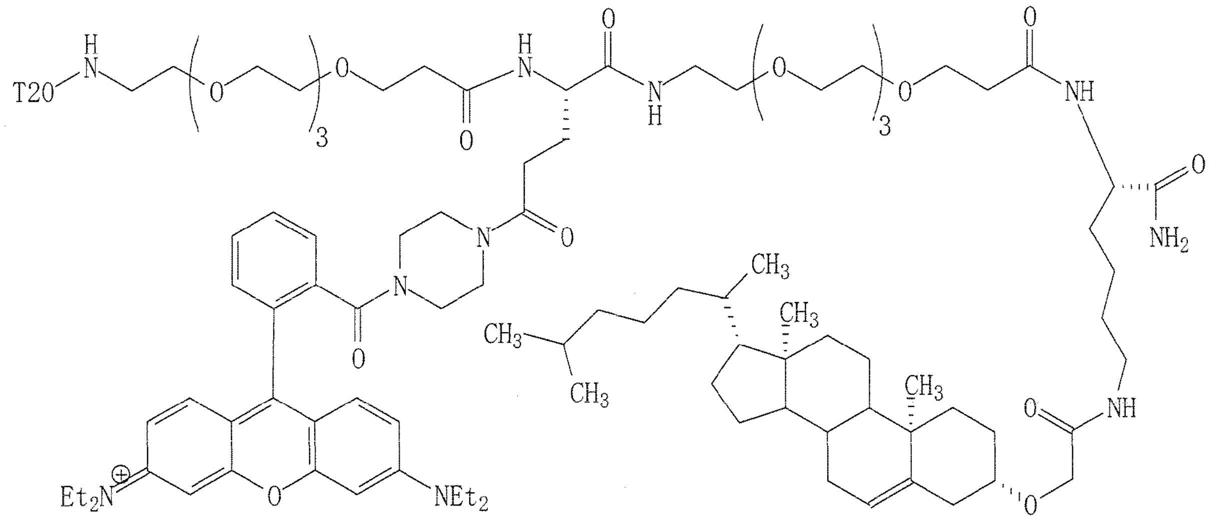

25c

Ac-Tyr-Thr-Ser-Leu-Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe-4Gl-Glu(Rho)-4Gl-Lys(CO-$CH_2$-dihydrocholesteryl)=$NH_2$

Couplings were performed using HATU, either by replacing the ABI-433's stock-solution of HBTU with HATU or by placing a solid mixture of HATU and Fmoc-amino acid (1 mmol each) into the amino acid cartridges of the synthesizer and modifying the synthesizer's software accordingly. PAL-PEG-PS resin (loading: 0,21 mmol/g) was used as the solid support. 0,1 mmol of resin were processed using the 0,25 mmol chemistry program and the 0,25 mmol reactor to allow for the considerable weight gain during synthesis. The raftophile was attached to the sidechain of lysine using Dde-Lys (Fmoc). [Novabiochem Catalog 2004/5, page 48; page 4-12.] Each coupling was followed by capping with $Ac_2O$.

Dde-Lys(Fmoc) was attached to the resin, deprotected and washed by automated synthesis. Dihydrocholesteryl-$CH_2$-COOH was coupled to the sidechain and the Dde-group was removed by treatment with 2% hydrazine hydrate in DMF (4 x 12 ml; 5 min each). The remaining sequence was coupled as described before. Only 0,5 mmol (5 eq.) of Glu(Rho) were used. UV-monitoring indicated decreasing coupling yield towards the end of the sequence.

Prior to cleavage from the resin, the trityl groups were removed by five washings with $CH_2Cl_2$/triisopropylsilane/trifluoroacetic acid (94:5:1). The resin was washed with $CH_2Cl_2$ (4x) and dried under vacuum. Cleavage and deprotection were carried out using trifluoroacetic acid/$H_2O$/dithiothreitol/triisopropylsilane (87:5:5:3) and 2h of reaction time. The solution was filtered off, concentrated to < 50% at the rotary evaporator (28 °C bath temperature) and triturated with petroleum ether / methyl tert-butyl ether (3:1). The oily crude product was separated by centrifugation and triturated with four portions of petroleum ether / methyl tert-butyl ether (4:1), which resulted in the formation of a red semisolid. This was dissolved in a mixture of acetonitrile (3,5 ml), $H_2O$ (2,5 ml) and acetic acid (65 μl), degassed by a stream of argon and left at room temperature overnight.

Analytical HPLC of the crude mixture was carried out using A: $H_2O$/MeCN (85:15) + 0,1% trifluoroacetic acid, B: MeCN + 0,1% TRIFLUOROACETIC ACID, a Vydac-C8 column type 208TP104 and a gradient of 10% to 100% B over 45 min at 1 ml/min flow rate. ESI-MS indicates RT = 32,2 min for the product.

Preparative purification was done in two steps. First, the material was chromatographed on a Vydac column type 208TP1030 using the same eluents as before and a gradient of 53% to 64% B at 20 ml/min. The fraction eluting at RT = 18,1 - 23,1 min was collected. Further purification of this material was achieved using a flowrate of 40 ml/min and a gradient of 30% to 40% B over 5 min, followed by a gradient of 40% to 50% B over 100 min (eluents as before.) The product (RT = 81,3 min) was separated, concentrated at the rotary evaporator and dried under vacuum. Yield: 4,5 mg of red solid.

The invention contains the following items.

1. A compound comprising a tripartite structure

C-B-A or C'-B'-A'

wherein moiety A and A' is a raftophile;
wherein moiety B and B' is a linker;

wherein moiety C and C' is a pharmacophore;
wherein the raftophilicity of moiety A and moiety A' comprises a partitioning into lipid membranes which are characterized by insolubility in non-ionic detergent at 4˚C.; and
wherein moiety B and B' is a linker which has a backbone of at least 8 carbon atoms and wherein one or more of said carbon atoms may be replaced by nitrogen, oxygen or sulfur.

2. The compound of item 1, wherein the raftophilicity of moiety A and moiety A' comprises an partitioning into lipid membranes which comprises a lipid composition comprising cholesterol and/or functional analogues of cholesterol, sphingolipids and/or functional analogues of sphingolipid, glycolipid and glycerophospholipids.

3. The compound of 2, wherein said lipid composition comprising glycolipids which are selected from the group consisting of gangliosides, cerebrosides, globosides and sulfatides.

4. The compound of item 3, wherein the ganglioside is GM1, GD1a, GD1b, GD3, GM2, GM3, GQ1a or GQ1b.

5. The compound of any one of items 2 to 4, wherein said sphingolipids and/or functional analogues of sphingolipids is a sphingomyelin or a ceramide.

6. The compound of any one of items 2 to 4, wherein said glycerophospholipids are selected from the group consisting of phosphatidylcholine, phosphatidylethanolamine, phosphatidylserine.

7. The compound of any one of items 2 to 6, wherein said lipid composition comprises cholesterol and/or functional analogues of cholesterol in a range of 5 to 60%, sphingolipids and/or functional analogues of sphingolipid in a range of 5 to 40% and phospholipids in a range of 20 to 80 %.

8. The compound of any one of items 2 to 7, wherein the lipid membrane comprises cholesterol, sphingomyelin, phosphatidylcholine, phosphatidylethanolamine and gangliosides.

9. The compound of item 8, wherein the lipid membrane comprises cholesterol in the range of 40 to 60%, sphingomyelin in the range of 10 to 20%, phosphatidylcholine in the range of 10 to 20%, phosphatidylethanolamine in the range of 10 to 20%, and gangliosides in the range of 1 to 10%.

10. The compound of item 9, wherein the lipid membrane consists of 50% of cholesterol, 15% of sphingomyelin, 15% of phosphatidylcholine, 15% of phosphatidylethanolamine, and 5% of gangliosides.

11. The compound of any one of items 2, 6 or 7, wherein said lipid membrane comprises said cholesterol and/or functional analogues thereof, said sphingolipid and/or functional analogues thereof and said phospholipid and/or functional analogues thereof in equal parts.

12. The compound of item 11, wherein said lipid membrane comprises 33% cholesterol , 33% sphingomyelin/ceramide and 33% phophatidylcholine.

13. The compound of any one of items 1 to 12 wherein said moiety B and said moiety B' has an overall length of 1 nm to 50 nm.

14. The compound of any of items 1 to 13, wherein the raftophile A or A' is represented by one of the following formulae 2 and 3:

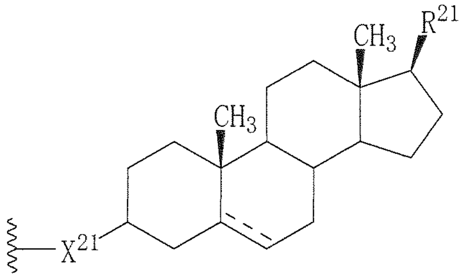

2

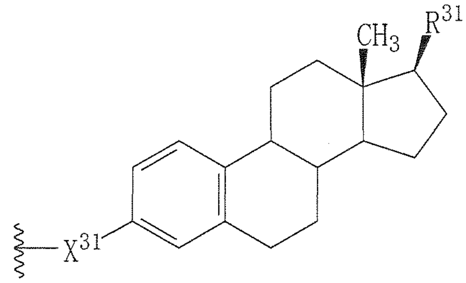

3

wherein

--- is a single bond or a double bond;

when the tripartite structure is C-B-A, $X^{21}$ and $X^{31}$ are directionally selected from NH, O, S, $NH(CH_2)_cOPO_3^-$, $NH(CH_2)_cSO_2CF_2$, $NH(CH_2)_cSO_2NH$, NHCONH, NHCOO, $NHCH(CONH_2)(CH_2)_dCOO$, $NHCH(COOH)(CH_2)_dCOO$, $NHCH(CONH_2)(CH_2)_dCONH$, $NHCH(COOH)(CH_2)_dCONH$, $NHCH(CONH_2)(CH_2)_4NH((CO)CH_2O)_f$ and $NHCH(COOH)(CH_2)_4NH((CO)CH_2O)_f$,

wherein c is an integer from 2 to 3, d is an integer from 1 to 2 and f is an integer from 0 to 1, and wherein the linker is bonded to $X^{21}$ or $X^{31}$;

when the tripartite structure is C'-F'-k', $X^{21}$ and $X^{31}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2CF_2$, $CO(CH_2)_bSO_2NH$, $CO(CH_2)_bNHCONH$, $CO(CH_2)_bOCONH$, $CO(CH_2)_eCH(CONH_2)NHCO(CH_2)_b(CO)_aNH$, $CO(CH_2)_eCH(COOH)NHCO(CH_2)_b(CO)_aNH$, $CO(CH_2)_eCH(CONH_2)NHCO(CH_2)_b(CO)_aO$, $CO(CH_2)_eCH(COOH)NHCO(CH_2)_b(CO)_aO$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{21}$ or $X^{31}$; and

$R^{21}$ and $R^{31}$ are a $C_{4-20}$ hydrocarbon group, wherein one or more hydrogens are optionally replaced by fluorine.

15. The compound of any of items 1 to 13, wherein the raftophile A or A' is represented by one of the following formulae 4a and 5a:

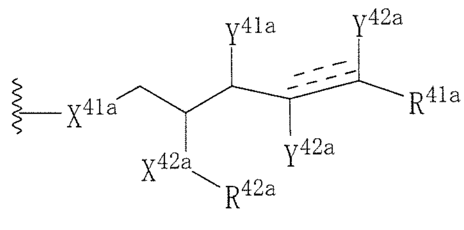

4a

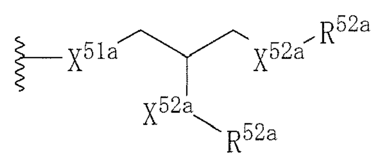

5a

wherein

the bond drawn as ≡ with dashes is a single bond, a double bond or a triple bond, provided that when it is a triple bond, each $Y^{42a}$ is not present;

when the tripartite structure is C-B-A, $X^{41a}$ and $X^{51a}$ are directionally selected from NH, O, $NH(CH_2)_cOPO_3^-$, $NH(CH_2)_cSO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, wherein c is an integer from 2 to 3 and d is an integer from 1 to 2, and wherein the linker is bonded to $X^{41a}$ or$X^{51a}$;

when the tripartite structure is C'-B'-A', $X^{41a}$ and $X^{51a}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, CO

$(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{41a}$ or$X^{51a}$;

$X^{42a}$ andeach $X^{52a}$ are independently NH, O, S, OCO, NHCO, NHCONH, $NHCO_2$ or $NHSO_2$;

$Y^{41a}$ is $NH_2$, $NHCH_3$, OH, H, halogen or O, provided that when $Y^{41a}$ is $NH_2$, $NHCH_3$, OH, H or halogen then --- is a single bond and when $Y^{41a}$ is O then --- is a double bond;

each $Y^{42a}$ is independently H or OH, provided that if $Y^{42a}$ is OH then --- is a single bond;

$R^{41a}$ is a $C_{10-30}$ hydrocarbon group, wherein one or more hydrogens are optionally replaced by fluorine; and

$R^{42a}$ and each $R^{52a}$ are independently a $C_{14-30}$ hydrocarbon group, wherein one or more hydrogens are optionally replaced by fluorine.

16. The compound of any of items 1 to 13, wherein the raftophile A or A' is represented by one of the following formulae **6** and **7:**

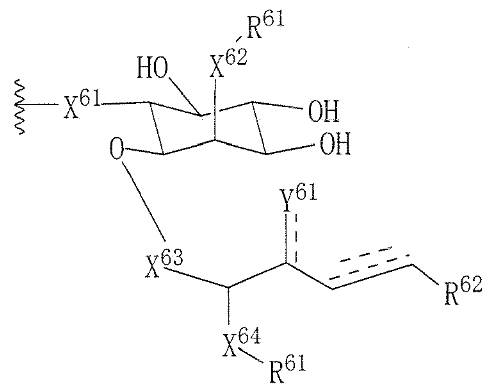

6

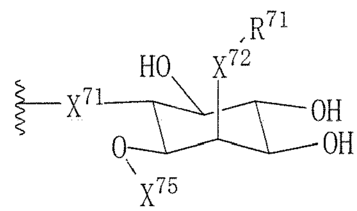

7

wherein

--- is a single bond, a double bond or a triple bond;

when the tripartite structure is C-B-A, $X^{61}$ and $X^{71}$ are O, wherein the linker is bonded to $X^{61}$ or $X^{71}$;

when the tripartite structure is C'-B'-A', $X^{61}$ and $X^{71}$ are $CO(CH_2)_b(CO)_aO$, wherein a is an integer from 0 to 1 and b is an integer from 1 to 3, and wherein the linker is bonded to the terminal carbonyl group of $X^{61}$ or $X^{71}$;

each $X^{75}$ is independently a CO-$C_{13-25}$ hydrocarbon group, wherein one or more hydrogens are optionally replaced by fluorine, a group of the following formula:

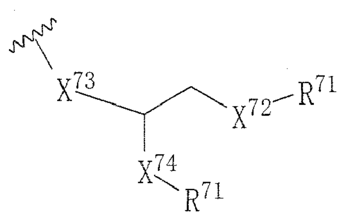

or a group of the following formula:

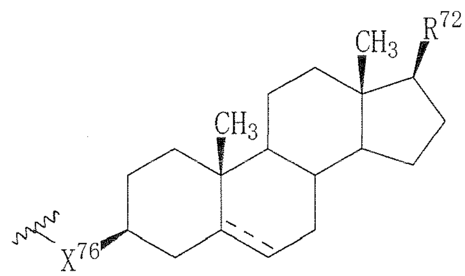

$X^{62}$ and each $X^{72}$ are independently O or OCO;
$X^{63}$ and $X^{73}$ are directionally selected from $PO_3^-CH_2$, $SO_3CH_2$, $CH_2$, $CO_2CH_2$ and a direct bond;
$X^{64}$ and $X^{74}$ are NH, O, S, OCO, NHCO, NHCONH, $NHCO_2$ or $NHSO_2$;
$X^{76}$ is directionally selected from $CO(CH_2)_b(CO)_aO$ and $CO(CH_2)_b(CO)_aNH$, wherein a is an integer from 0 to 1 and b is an integer from 1 to 3;
$Y^{61}$ is $NH_2$, $NHCH_3$, OH, H, halogen or O, provided that when $Y^{61}$ is $NH_2$, $NHCH_3$, OH, H or halogen then --- is a single bond and when $Y^{61}$ is O then --- is a double bond;
each $R^{61}$ and each $R^{71}$ are independently a $C_{16-30}$ hydrocarbon group, wherein one or more hydrogens are optionally replaced by fluorine;
$R^{62}$ is a $C_{13-25}$ hydrocarbon group, wherein one or more hydrogens are optionally replaced by fluorine; and
$R^{72}$ is a $C_{4-20}$ hydrocarbon group, wherein one or more hydrogens are optionally replaced by fluorine.

17. The compound of any of items 1 to 13, wherein the raftophile A or A' is represented by the following formulae **18a** and **18b:**

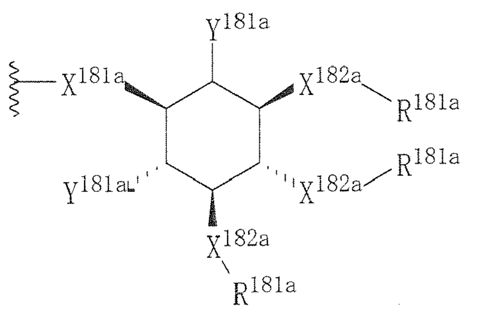

18a

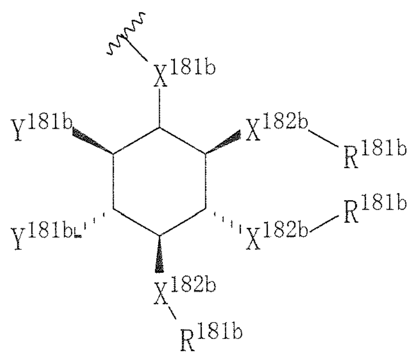

18b

wherein

when the tripartite structure is C-B-A, $X^{181a}$ and $X^{181b}$ are directionally selected from NH, O, $NH(CH_2)_cOPO_3^-$, $NH(CH_2)_cSO_2NH$, NHCONH and NHCOO, wherein c is an integer from 2 to 3, and wherein the linker is bonded to $X^{181a}$ or $X^{181b}$;
when the tripartite structure is C'-B'-A', $X^{181a}$ and $X^{181b}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$ or $CO(CH_2)_bNHCO_2$, wherein a is an integer from 0 to 1 and b is an integer from 1 to 3, and wherein the linker is bonded to the terminal carbonyl group of $X^{181a}$ or $X^{181b}$;
each $Y^{181a}$ and each $Y^{181b}$ is independently $NH_2$, $NHCH_3$, OH, H or halogen;
each $X^{182a}$ and each $X^{182b}$ is independently O, NH, OCO or NHCO; and
each $R^{181a}$ and each $R^{181b}$ is independently a $C_{15-30}$ hydrocarbon group, wherein one or more hydrogens are optionally replaced by fluorine.

18. The compound of any of items 1 to 13, wherein the raftophile A or A' is represented by the following formulae **1**9a and **19b:**

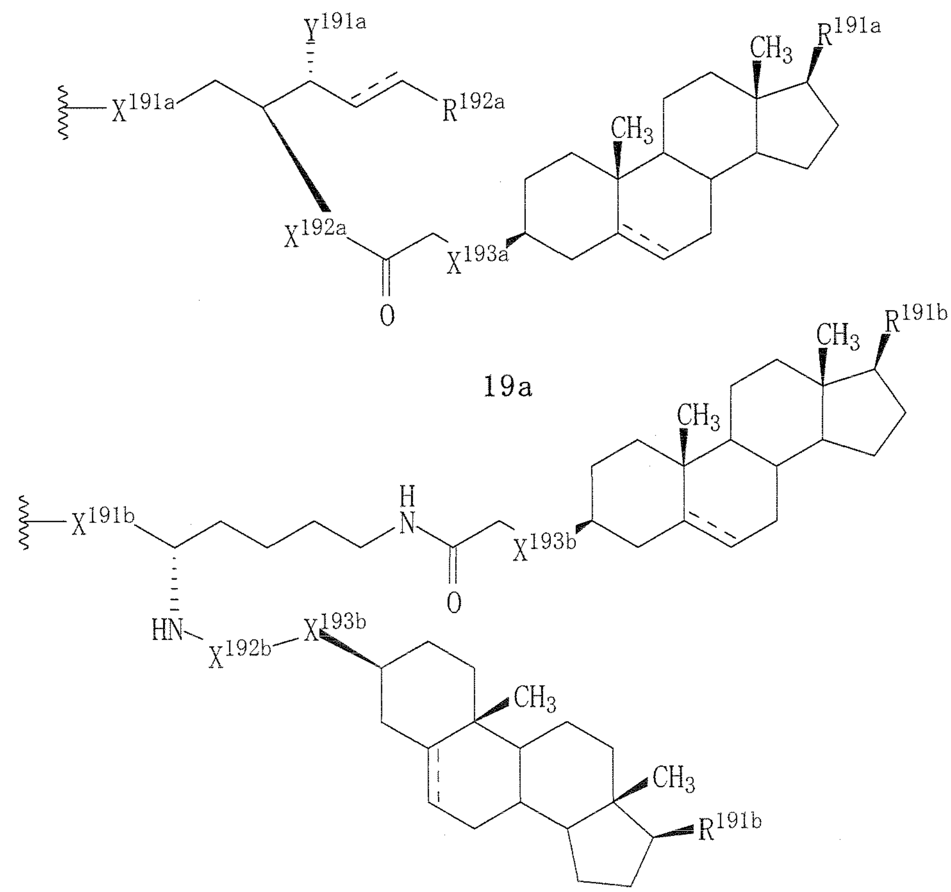

19a

19b

wherein

--- is a single bond or a double bond;

when the tripartite structure is C-B-A, $X^{191a}$ is directionally selected from NH, O, $NH(CH_2)_cOPO_3^-$, $NH(CH_2)_cSO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, wherein c is an integer from 2 to 3 and d is an integer from 1 to 2, and wherein the linker is bonded to $X^{191a}$;

when the tripartite structure is C'-B'-A', $X^{191a}$ is $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, $CO(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{191a}$;

when the tripartite structure is C-B-A, $X^{191b}$ is $NH(CH_2)_cNHCO$, wherein c is an integer from 2 to 3, and wherein the linker is bonded to the terminal amino group of $X^{191b}$;

when the tripartite structure is C'-B'-A', $X^{191b}$ is CO, wherein the linker is bonded to $X^{191b}$;

$X^{192a}$ is directionally selected from $NHCOCH_2NH$ or $NHCOCH_2OCH_2CH_2OCH_2CH_2NH$;

$X^{192b}$ is directionally selected from $COCH_2CH_2NHCOCH_2$ or $COCH_2$;

$X^{193a}$ and each $X^{193b}$ are independently directionally selected from O, NH, $C_{1-8}$ alkylene-O and $C_{1-8}$ alkylene-NH.;

$Y^{191a}$ is $NH_2$, OH or H;

$R^{191a}$ and each $R^{191b}$ are independently a $C_{4-18}$ hydrocarbon group, wherein one or more hydrogens are optionally replaced by fluorine; and

$R^{192a}$ is a $C_{13-25}$ hydrocarbon group, wherein one or more hydrogens are optionally replaced by fluorine.

19. The compound of any of items 1 to 18, wherein the linker B or B' is represented by the following formula **20:**

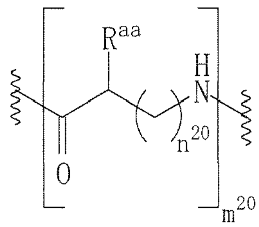

20

wherein

$m^{20}$ is an integer from 3 to 80;
each $n^{20}$ is independently an integer from 0 to 1;
each $R^{aa}$ is independently any of the side chains of naturally occurring amino acids, optionally substituted with a dye label;
wherein the C-terminus is bonded to the raftophile A and the N-terminus is bonded to the pharmacophore C in the tripartite structure C-B-A; and
wherein the N-terminus is bonded to the raftophile A' and the C-terminus is bonded to the pharmacophore C' in the tripartite structure C'-B'-A'.

20. The compound of any of items 1 to 18, wherein the linker B or B' is represented by the following formula **21:**

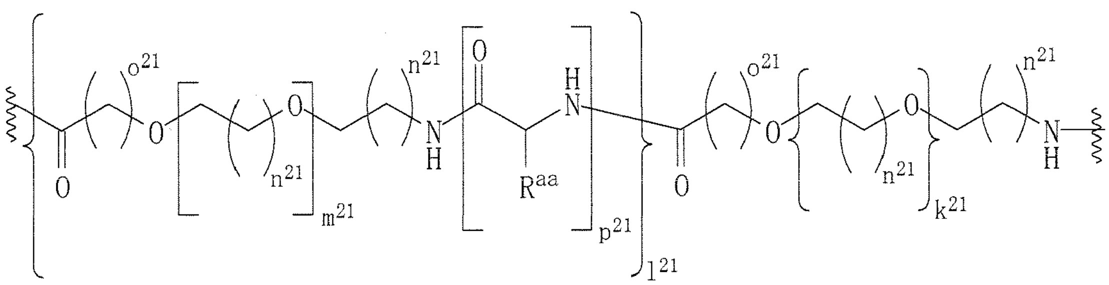

21

wherein

each $n^{21}$ is independently an integer from 1 to 2;
each $o^{21}$ is independently an integer from 1 to 3;
each $p^{21}$ is independently an integer from 0 to 1;
$k^{21}$ and each $m^{21}$ are independently integers from 0 to 5;
$l^{21}$ is an integer from 0 to 10, provided that the sum of $k^{21}$ and $l^{21}$ is at least 1; and
$R^{aa}$ is as defined in claim 19;
wherein the C-terminus is bonded to the raftophile A and the N-terminus is bonded to the pharmacophore C in the tripartite structure C-B-A; and
wherein the N-terminus is bonded to the raftophile A' and the C-terminus is bonded to the pharmacophore C' in the tripartite structure C'-B'-A'.

21. The compound of any of items 1 to 18, wherein the linker B or B' is represented by the following formula **22:**

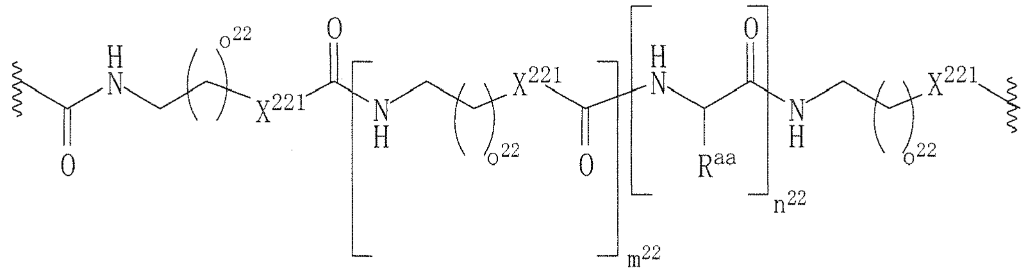

22

wherein

$m^{22}$ is an integer from 0 to 40;
$n^{22}$ is an integer from 0 to 1;
each $o^{22}$ is independently an integer from 1 to 5;
each $X^{221}$ is independently NH or O; and
$R^{aa}$ is as defined in claim 19;
wherein the C-terminus is bonded to the raftophile A and the $X^{221}$-terminus is bonded to the pharmacophore C in the tripartite structure C-B-A; and
wherein the $X^{221}$-terminus is bonded to the raftophile A' and the C-terminus is bonded to the pharmacophore C' in the tripartite structure C'-B'-A'.

22. The compound of any of items 1 to 18, wherein the linker B or B' is represented by the following formula **23:**

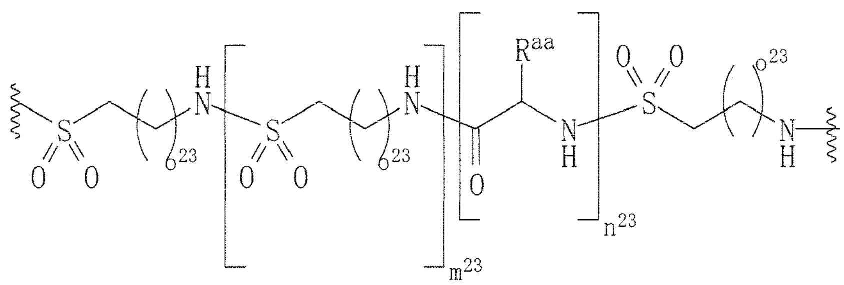

23

wherein

$m^{23}$ is an integer from 0 to 40;
$n^{23}$ is an integer from 0 to 1;
each $o^{23}$ is independently an integer from 1 to 5; and
$R^{aa}$ is as defined in claim 19;
wherein the $SO_2$-terminus is bonded to the raftophile A and the N-terminus is bonded to the pharmacophore C in the tripartite structure C-B-A; and
wherein the N-terminus is bonded to the raftophile A' and the $SO_2$-terminus is bonded to the pharmacophore C' in the tripartite structure C'-B'-A'.

23. The compound of any of items 1 to 22, wherein the pharmacophore C or C ' is selected from the group consisting of an enzyme, an antibody or a fragment or a derivative thereof, an aptamer, a peptide, a fusion protein, a small molecule inhibitor, a carbocyclic compound, a heterocyclic compound, a nucleoside derivative and an anilino-naphthalene compound.

24. The compound of any one of items 1 to 22, wherein the pharmacophore C or C' is an enzyme inhibitor.

25. The compound of item 24, wherein the enzyme inhibitor is beta-secretase inhibitor III.

26. The compound of any one of items 1 to 22, wherein the pharmacophore C or C' is a receptor inhibitor.

27. The compound of item 26, wherein the receptor inhibitor is EGF receptor inhibitor.

28. The compound of item 27, wherein said EGF receptor inhibitor is selected from the group consisting of aptamer A30, antibody IMC-C225 or a functional fragment thereof, antibody ABX-ECTF or a functional fragment thereof, antibody EMD7200 or a functional fragment thereof, antibody hR3 or a functional fragment thereof or the antibody trastuzumab or a functional fragment thereof.

29. The compound of any one of items 1 to 22, wherein the pharmacophore C or C' is an ETBR-antagonist.

30. The compound of item 29, wherein said ETBR-antagonist is A-192621.

31. The compound of any of items 1 to 22, wherein the pharmacophore C or C ' is Losartan, Valsartan, Candesartan cilexetil (TCV-116) or Irbesartan.

32. The compound of item 23, wherein said anilino-naphthalene compound is selected from the group consisting of bis-ANS (bis 8-anilino naphthalene sulfonate), ANS (8-anilino naphthalene sulfonate)and AmNS (1-amino-5-naphtalenesulfonate).

33. The compound of any of items 1 to 22, wherein the pharmacophore C or C ' is an antiviral agent.

34. The compound of items 33, wherein the antiviral agent is selected from the group consisting of Zanamivir (2,4-dideoxy-2,3-didehydro-4-guanidinosialic acid), Oseltamivir (ethyl(3R,4R,5S)-4-acetoamido-5-amino-3-(1-ethylpropoxy)-1-cyclohexene-1-carboxylate), RWJ-270201 (Peramivir), BCX-1812, BCX-1827, BCX-1898, BCX-1923, Norakin (1-tricyclo-(2,2,1,0)-heptyl-(2)-1-phenyl-3-piperidine-propanol; triperiden), Akineton (alpha-5-norbornen-2-yl-alpha-phenyl-1-piperidine propanol; biperiden), Antiparkin (ethylbenzhydramin) and Parkopan (trihexyphenidyl).

35. The compound of items 33, wherein the antiviral agent is selected from the group consisting of Fuzeon, T20, T1249, coselane, AMD3100, AMD070, SCH351125 and AD101.

36. The compound of item 25, which is represented by the following formula **24b** and pharmaceutically acceptable salts thereof:

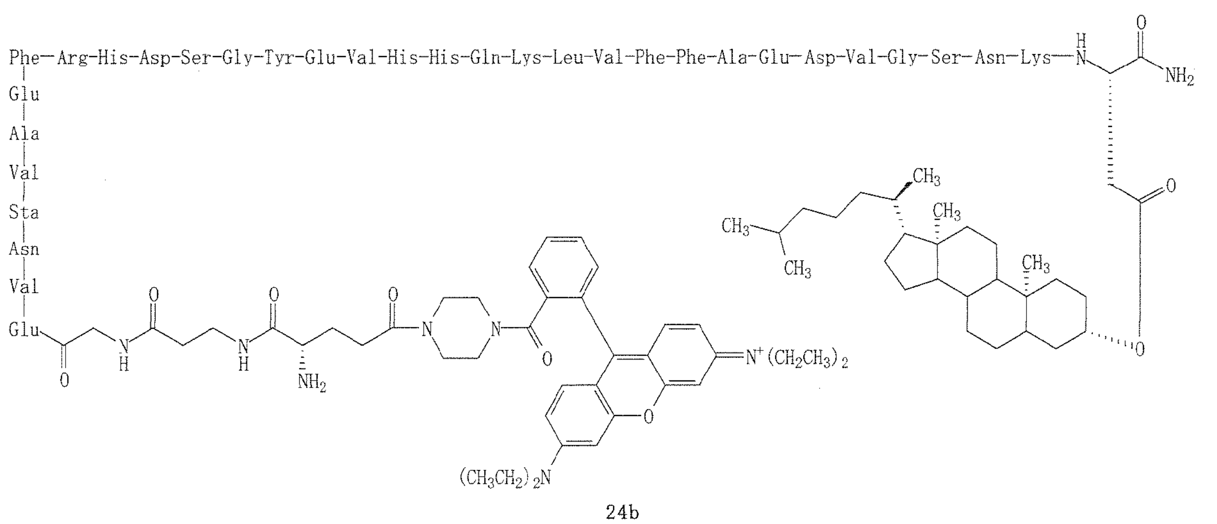

24b

37. The compound of item 25, which is represented by the following formula **25b** and pharmaceutically acceptable

salts thereof:

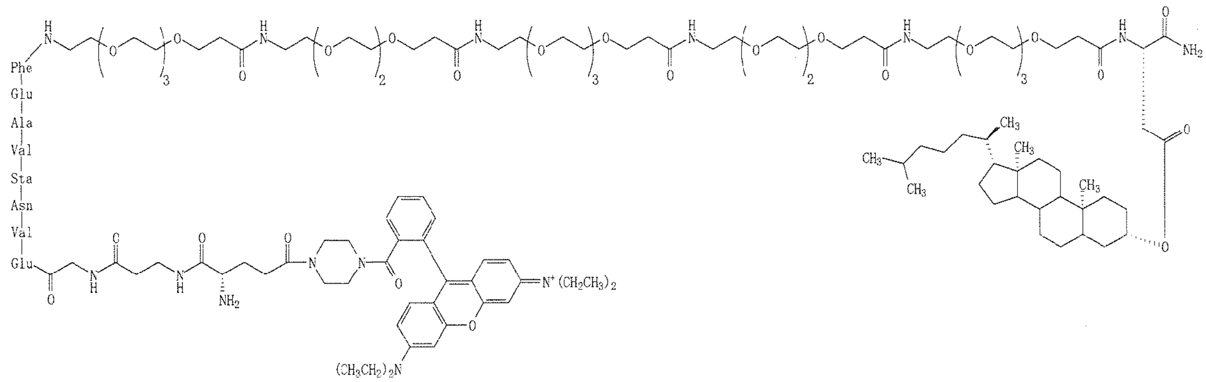

25b

38. A compound comprising the raftophile A' as described in any of items 14 to 18 and a linker represented by the following formula **28:**

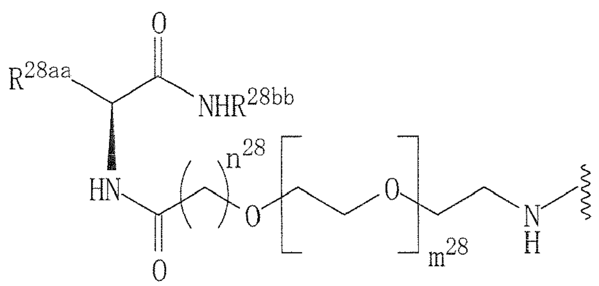

28

wherein

$R^{28aa}$ is the side chain of a naturally occurring amino acid substituted with a dye label;
$R^{28bb}$ is H or $CH_2CONH_2$; and
$m^{28}$ and $n^{28}$ are independently an integer from 1 to 3;
wherein the N-terminus of the linker is bonded to the raftophile A'.

39. The compound of item 38 wherein the linker is represented by the following formula **28a:**

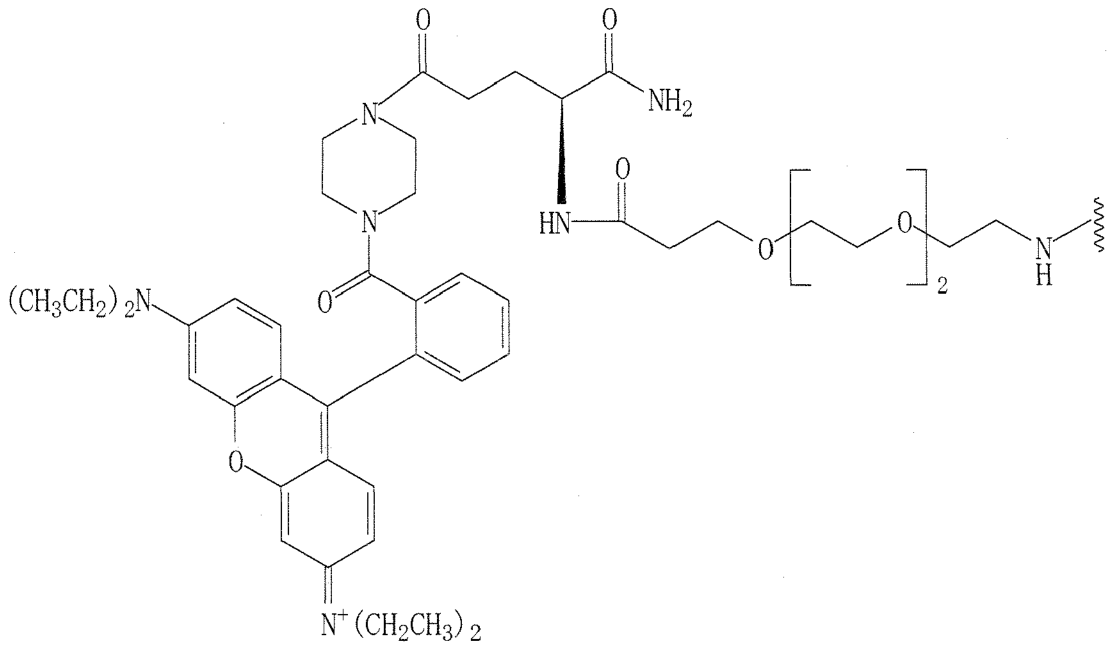

28a

40. A pharmaceutical composition comprising a compound of any one of items 1 to 39.

41. Use of a compound as defined in any one of items 1 to 25, 37 and 38 for the preparation of a pharmaceutical composition for the treatment, prevention and/or amelioration of a neurological disorder.

42. The use of item 41, wherein said neurological disorder is Alzheimer's disease or Down's syndrome.

43. Use of a compound as defined in any of items 1 to 23, 27 and 28 for the preparation of a pharmaceutical composition for the treatment, prevention and/or amelioration of a proliferative disorder or cancer.

44. Use of item 43, wherein said cancer is selected from the group consisting of breast cancer, colon cancer, stomach cancer, uro-genital cancer, liver cancer, head-neck cancer, lung cancer, or skin cancer (melanoma).

45. Use of a compound as defined in items 27 and 28 for the preparation of a pharmaceutical composition for the treatment, prevention and/or amelioration of breast cancer.

46. Use of a compound as defined in any of items 29 to 31 for the preparation of a pharmaceutical composition for the treatment, prevention and/or amelioration of hypertension and/or congestive heart failure.

47. Use of a compound as defined in item 43 for the preparation of a pharmaceutical composition for the treatment, prevention and/or amelioration of a prion (PrP)-related disease.

48. Use of a compound as defined in items 1 to 23 and 33 to 35 for the preparation of a pharmaceutical composition for the treatment, prevention and/or amelioration of an infectious disease.

49. The use of item 48, wherein said infectious disease is a viral infection.

50. The use of item 49, wherein said viral infection is an HIV-infection.

51. The use of item 49, wherein said viral infection is an influenza infection.

52. The use of item 48, wherein said infectious disease is a bacterial infection.

53. The use of item 52, wherein said bacterial infection is a mycobacterial infection or an infection with Escherichia coli, Campylobacter jejuni, Vibrio cholerae, Clostridium difficile, Clostridium tetani, Salmonella, Shigella.

54. The use of item 53, wherein said mycobacterial infection is an infection with M. tuberculosis or an infection with M. kansasii or M. bovis.

55. Use of a compound as defined in any one of items 1 to 23 for the preparation of a pharmaceutical composition for the treatment of a parasite infection.

56. The use of item 55, wherein said parasite infection is a Plasmodium falsiparum infection or infection by Trypanasoma or Leishmania or Toxoplasma gondii.

57. A method for the preparation of a compound as described in anyone of items 1 to 35 comprising the steps of

a) determining the distance between a phosphoryl head group or an equivalent head group of a raft lipid and a binding and/or interaction site of a pharmacophore C/C' on a raft-associated target molecule;
b) selecting a linker B/B' which is capable of spanning the distance as determined in a); and
c) bonding a raftophile A/A' and the pharmacophore C/C' by the linker as selected in b).

58. A method for the preparation of a pharmaceutical composition comprising admixing the compound of any one of items 1 to 35 with one or more pharmaceutically acceptable excipients.

## Claims

1. A compound comprising a tripartite structure

C-B-A or C'-B'-A'

or a pharmaceutically acceptable salt thereof;

wherein moiety A and A' is a raftophile;
wherein moiety B and B' is a linker;
wherein moiety C and C' is a pharmacophore;
wherein the raftophilicity of moiety A and moiety A' comprises a partitioning into lipid membranes which are **characterized by** insolubility in non-ionic detergent at 4°C.; and
wherein moiety B and B' is a linker which has a backbone of at least 8 carbon atoms and wherein one or more of said carbon atoms may be replaced by nitrogen, oxygen or sulfur.

2. The compound of claim 1, wherein the raftophilicity of moiety A and moiety A' comprises an partitioning into lipid membranes which comprises a lipid composition comprising cholesterol and/or functional analogues of cholesterol, sphingolipids and/or functional analogues of sphingolipid, glycolipid and glycerophospholipids.

3. The compound of claim 2, wherein the lipid membrane comprises cholesterol, sphingomyelin, phosphatidylcholine, phosphatidylethanolamine and gangliosides.

4. The compound of any one of claims 1 to 3 wherein said moiety B and said moiety B' has an overall length of 1 nm to 50 nm.

**5.** The compound of any of claims I to 4, wherein the raftophile A or A' is represented by one of the following formulae **2** and **3**:

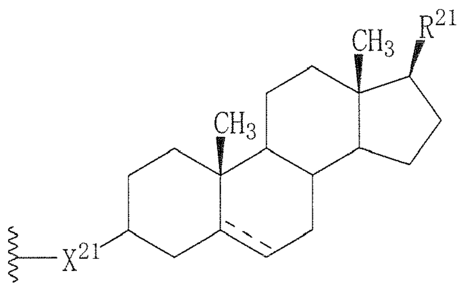

2

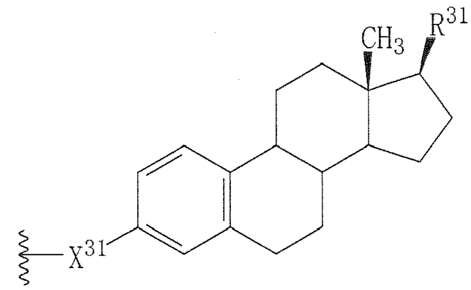

3

wherein

--- is a single bond or a double bond;
when the tripartite structure is C-B-A, $X^{21}$ and $X^{31}$ are directionally selected from NH, O, S, $NH(CH_2)_cOPO_3^-$, $NH(CH_2)_cSO_2CF_2$, $NH(CH_2)_cSO_2NH$, NHCONH, NHCOO, $NHCH(CONH_2)(CH_2)_dCOO$, $NHCH(COOH)(CH_2)_dCOO$, $NHCH(CONH_2)(CH_2)_dCONH$, $NHCH(COOH)(CH_2)_dCONH$, $NHCH(CONH_2)(CH_2)_4NH((CO)CH_2O)_f$ and $NHCH(COOH)(CH_2)_4NH((CO)CH_2O)_f$,
wherein c is an integer from 2 to 3, d is an integer from 1 to 2 and f is an integer from 0 to 1, and wherein the linker is bonded to $X^{21}$ or $X^{31}$;
when the tripartite structure is C'-B'-A', $X^{21}$ and $X^{31}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2CF_2$, $CO(CH_2)_bSO_2NH$, $CO(CH_2)_bNHCONH$, $CO(CH_2)_bOCONH$, $CO(CH_2)_eCH(CONH_2)NHCO(CH_2)_b(CO)_aNH$, $CO(CH_2)_eCH(COOH)NHCO(CH_2)_b(CO)_aNH$, $CO(CH_2)_eCH(CONH_2)NHCO(CH_2)_b(CO)_aO$, $CO(CH_2)_eCH(COOH)NHCO(CH_2)_b(CO)_aO$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{21}$ or $X^{31}$; and
$R^{21}$ and $R^{31}$ are a $C_{4-20}$ hydrocarbon group, wherein one or more hydrogens are optionally replaced by fluorine;
or wherein the raftophile A or A' is represented by one of the following formulae 4a and **5a:**

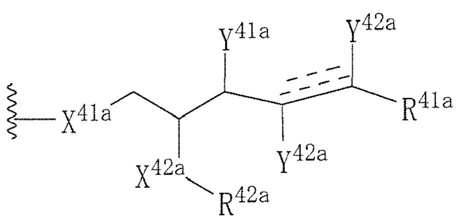

4a

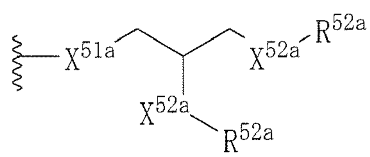

5a

wherein

--- is a single bond, a double bond or a triple bond, provided that when --- is a triple bond, each $Y^{42a}$ is not present;
when the tripartite structure is C-B-A, $X^{41a}$ and $X^{51a}$ are directionally selected from NH, O, $NH(CH_2)_cOPO_3^-$, $NH(CH_2)_cSO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, wherein c is an integer from 2 to 3 and d is an integer from 1 to 2, and wherein the linker is bonded to $X^{41a}$ or$X^{51a}$;
when the tripartite structure is C'-B'-A', $X^{41a}$ and $X^{51a}$ are $CO(CH_2)_b(CO)_aNH$, $CO(CH_2)_b(CO)_aO$, $CO(CH_2)_bS$,

$CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, $CO(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{41a}$ or$X^{51a}$;

$X^{42a}$ andeach $X^{52a}$ are independently NH, O, S, OCO, NHCO, NHCONH, $NHCO_2$ or $NHSO_2$;

$Y^{41a}$ is $NH_2$, $NHCH_3$, OH, H, halogen or O, provided that when $Y^{41a}$ is $NH_2$, $NHCH_3$, OH, H or halogen then --- is a single bond and when $Y^{41a}$ is O then --- is a double bond;

each $Y^{42a}$ is independently H or OH, provided that if $Y^{42a}$ is OH then --- is a single bond;

$R^{41a}$ is a $C_{10-30}$ hydrocarbon group, wherein one or more hydrogens are optionally replaced by fluorine; and

$R^{42a}$ and each $R^{52a}$ are independently a $C_{14-30}$ hydrocarbon group, wherein one or more hydrogens are optionally replaced by fluorine;

or wherein the raftophile A or A' is represented by one of the following formulae **6** and **7:**

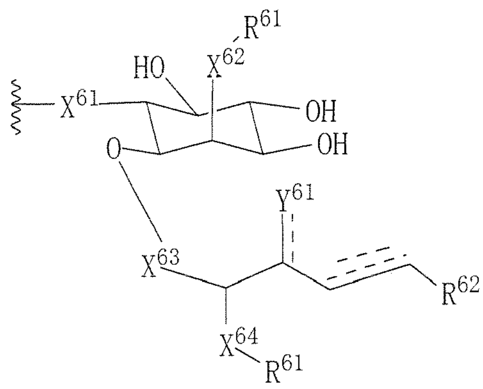

6

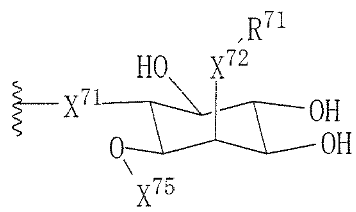

7

wherein

--- is a single bond, a double bond or a triple bond;

when the tripartite structure is C-B-A, $X^{61}$ and $X^{71}$ are O, wherein the linker is bonded to $X^{61}$ or $X^{71}$;

when the tripartite structure is C'-B'-A', $X^{61}$ and $X^{71}$ are $CO(CH_2)_b(CO)_aO$, wherein a is an integer from 0 to 1 and b is an integer from 1 to 3, and wherein the linker is bonded to the terminal carbonyl group of $X^{61}$ or $X^{71}$;

each $X^{75}$ is independently a $CO\text{-}C_{13-25}$ hydrocarbon group, wherein one or more hydrogens are optionally replaced by fluorine, a group of the following formula:

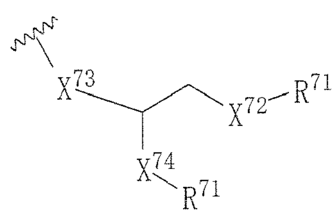

or a group of the following formula:

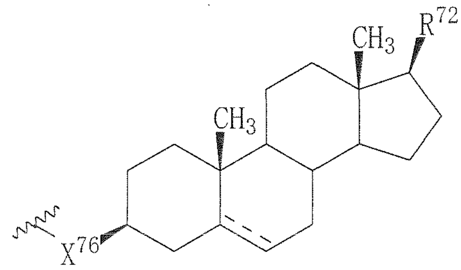

$X^{62}$ and each $X^{72}$ are independently O or OCO;
$X^{63}$ and $X^{73}$ are directionally selected from $PO_3^-CH_2$, $SO_3CH_2$, $CH_2$, $CO_2CH_2$ and a direct bond;
$X^{64}$ and $X^{74}$ are NH, O, S, OCO, NHCO, NHCONH, $NHCO_2$ or $NHSO_2$;
$X^{76}$ is directionally selected from $CO(CH_2)_b(CO)_aO$ and $CO(CH_2)_b(CO)_aNH$, wherein a is an integer from 0 to 1 and b is an integer from 1 to 3;
$Y^{61}$ is $NH_2$, $NHCH_3$, OH, H, halogen or O, provided that when $Y^{61}$ is $NH_2$, $NHCH_3$, OH, H or halogen then --- is a single bond and when $Y^{61}$ is O then --- is a double bond;
each $R^{61}$ and each $R^{71}$ are independently a $C_{16-30}$ hydrocarbon group, wherein one or more hydrogens are optionally replaced by fluorine;
$R^{62}$ is a $C_{13-25}$ hydrocarbon group, wherein one or more hydrogens are optionally replaced by fluorine; and
$R^{72}$ is a $C_{4-20}$ hydrocarbon group, wherein one or more hydrogens are optionally replaced by fluorine;
or wherein the raftophile A or A' is represented by the following formulae **18a** and **18b:**

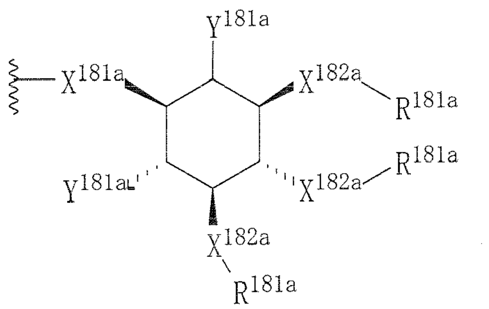

18a

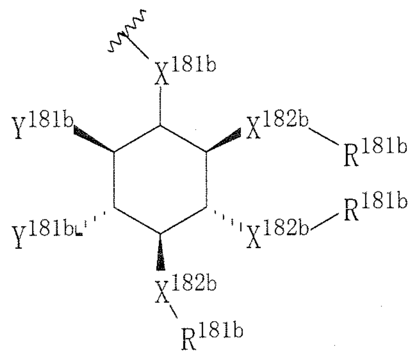

18b

wherein
when the tripartite structure is C-B-A, $X^{181a}$ and $X^{181b}$ are directionally selected from NH, O, $NH(CH_2)_cOPO_3^-$, $NH(CH_2)_cSO_2NH$, NHCONH and NHCOO, wherein c is an integer from 2 to 3, and wherein the linker is bonded to $X^{181a}$ or $X^{181b}$;
when the tripartite structure is C'-B'-A', $X^{181a}$ and $X^{181b}$ are $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$ or $CO(CH_2)_bNHCO_2$,
wherein a is an integer from 0 to 1 and b is an integer from 1 to 3, and wherein the linker is bonded to the terminal carbonyl group of $X^{181a}$ or $X^{181b}$;
each $Y^{181a}$ and each $y^{181b}$ is independently $NH_2$, $NHCH_3$, OH, H or halogen;
each $X^{182a}$ and each $X^{182b}$ is independently O, NH, OCO or NHCO; and each $R^{181a}$ and each $R^{181b}$ is independently a $C_{15-30}$ hydrocarbon group, wherein one or more hydrogens are optionally replaced by fluorine;
or wherein the raftophile A or A' is represented by the following formulae **19a** and **19b:**

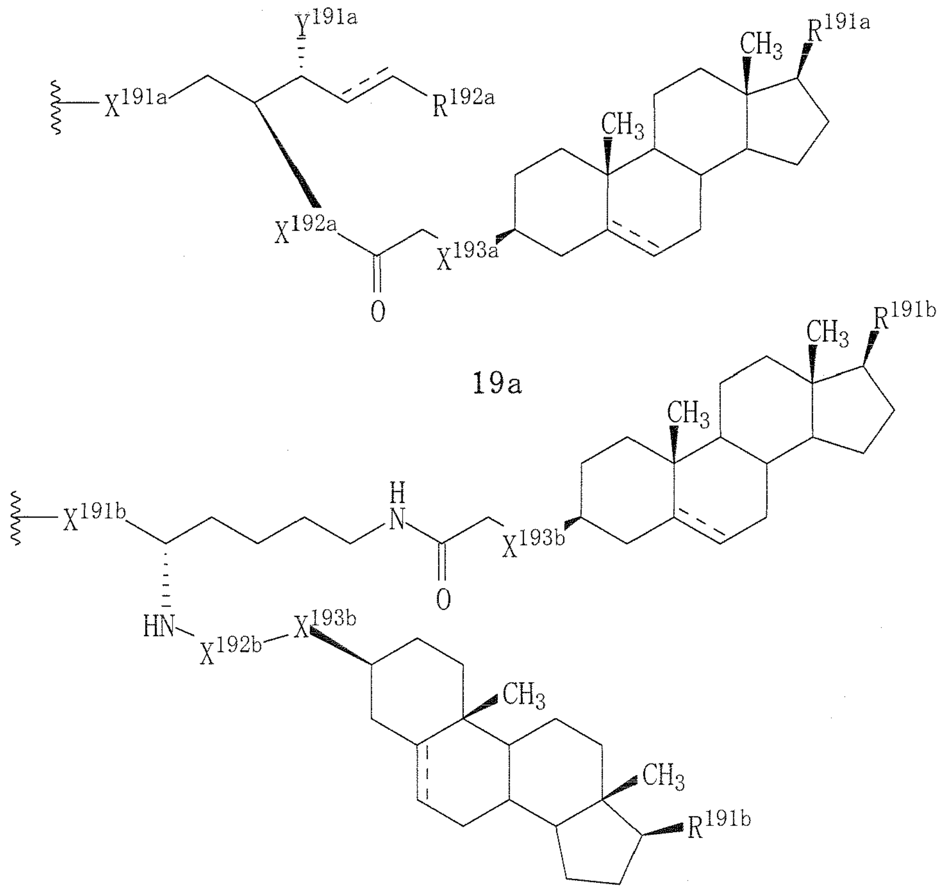

19a

19b

wherein

--- is a single bond or a double bond;

when the tripartite structure is C-B-A, $X^{191a}$ is directionally selected from NH, O, $NH(CH_2)_cOPO_3^-$, $NH(CH_2)_eSO_2NH$, NHCONH, NHCOO, $NHCH(CONH_2)(CH_2)_dCOO$, $NHCH(COOH)(CH_2)_dCOO$, $NH(CH_2)_4CH(CONH_2)NH$, $NH(CH_2)_4CH(COOH)NH$, $NHCH(CONH_2)(CH_2)_4NH$ and $NHCH(COOH)(CH_2)_4NH$, wherein c is an integer from 2 to 3 and d is an integer from 1 to 2, and wherein the linker is bonded to $X^{191a}$;

when the tripartite structure is C'-B'-A', $X^{191a}$ is $CO(CH_2)_b(CO)_aNH$, $CO(CH_2)_b(CO)_aO$, $CO(CH_2)_bS$, $CO(CH_2)_bOPO_3^-$, $CO(CH_2)_bSO_2NH$, $CO(CH_2)_bNHCONH$, $CO(CH_2)_bOCONH$, $CO(CH_2)_bOSO_3$, $CO(CH_2)_bNHCO_2$, $CO(CH_2)_eCH(CONH_2)NH$, $CO(CH_2)_eCH(COOH)NH$, $COCH(NH_2)(CH_2)_eCOO$ or $COCH(NHCOCH_3)(CH_2)_eCOO$, wherein a is an integer from 0 to 1, b is an integer from 1 to 3 and e is an integer from 1 to 2, and wherein the linker is bonded to the terminal carbonyl group of $X^{191a}$;

when the tripartite structure is C-B-A, $X^{191b}$ is $NH(CH_2)_cNHCO$, wherein c is an integer from 2 to 3, and wherein the linker is bonded to the terminal amino group of $X^{191b}$;

when the tripartite structure is C'-B'-A', $X^{191b}$ is CO, wherein the linker is bonded to $X^{191b}$;

$X^{192a}$ is directionally selected from $NHCOCH_2NH$ or $NHCOCH_2OCH_2CH_2OCH_2CH_2NH$;

$X^{192b}$ is directionally selected from $COCH_{2C}H_2NHCOCH_2$ or $COCH_2$;

$X^{193a}$ and each $X^{193b}$ are independently directionally selected from O, NH, $C_{1-8}$ alkylene-O and $C_{1-8}$ alkylene-NH.;

$Y^{191a}$ is $NH_2$, OH or H;

$R^{191a}$ and each $R^{191b}$ are independently a $C_{4-18}$ hydrocarbon group, wherein one or more hydrogens are optionally replaced by fluorine; and

$R^{192a}$ is a $C_{13-25}$ hydrocarbon group, wherein one or more hydrogens are optionally replaced by fluorine.

**6.** The compound of any of claims 1 to 5, wherein the linker B or B' is represented by the following formula **20:**

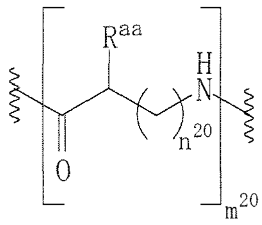

20

wherein

$m^{20}$ is an integer from 3 to 80;
each $n^{20}$ is independently an integer from 0 to 1;
each $R^{aa}$ is independently any of the side chains of naturally occurring amino acids, optionally substituted with a dye label;
wherein the C-terminus is bonded to the raftophile A and the N-terminus is bonded to the pharmacophore C in the tripartite structure C-B-A; and
wherein the N-terminus is bonded to the raftophile A' and the C-terminus is bonded to the pharmacophore C' in the tripartite structure C'-B'-A';
or wherein the linker B or B' is represented by the following formula **21:**

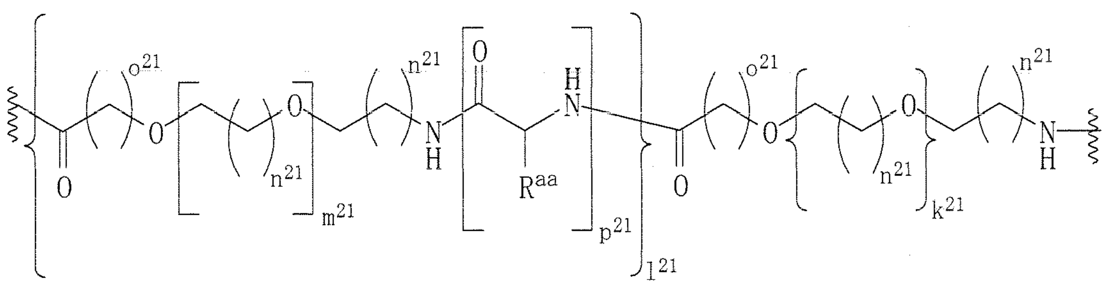

21

wherein
each $n^{21}$ is independently an integer from 1 to 2;
each $o^{21}$ is independently an integer from 1 to 3;
each $p^{21}$ is independently an integer from 0 to 1;
$k^{21}$ and each $m^{21}$ are independently integers from 0 to 5;
$l^{21}$ is an integer from 0 to 10, provided that the sum of $k^{21}$ and $l^{21}$ is at least 1; and $R^{aa}$ is as defined in claim 19;
wherein the C-terminus is bonded to the raftophile A and the N-terminus is bonded to the pharmacophore C in the tripartite structure C-B-A; and
wherein the N-terminus is bonded to the raftophile A' and the C-terminus is bonded to the pharmacophore C' in the tripartite structure C'-B'-A';
or wherein the linker B or B' is represented by the following formula **22:**

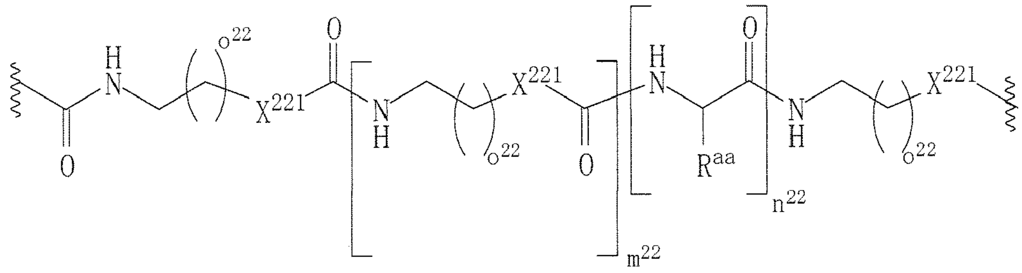

22

wherein
$m^{22}$ is an integer from 0 to 40;
$n^{22}$ is an integer from 0 to 1;
each $o^{22}$ is independently an integer from 1 to 5;
each $X^{221}$ is independently NH or O; and
$R^{aa}$ is as defined in claim 19;
wherein the C-terminus is bonded to the raftophile A and the $X^{221}$-terminus is bonded to the pharmacophore C in the tripartite structure C-B-A; and wherein the $X^{221}$-terminus is bonded to the raftophile A' and the C-terminus is bonded to the pharmacophore C' in the tripartite structure C'-B'-A';
or wherein the linker B or B' is represented by the following formula **23:**

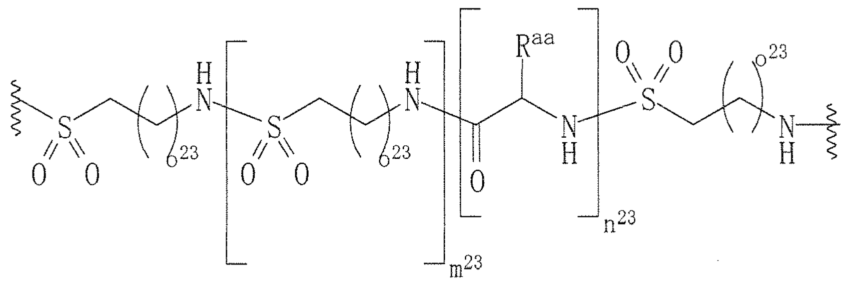

23

wherein
$m^{23}$ is an integer from 0 to 40;
$n^{23}$ is an integer from 0 to 1;
each $o^{23}$ is independently an integer from 1 to 5; and
$R^{aa}$ is as defined in claim 19;
wherein the $SO_2$-terminus is bonded to the raftophile A and the N-terminus is bonded to the pharmacophore C in the tripartite structure C-B-A; and wherein the N-terminus is bonded to the raftophile A' and the $SO_2$-terminus is bonded to the pharmacophore C' in the tripartite structure C'-B'-A'.

**7.** The compound of any of claims 1 to 6, wherein the pharmacophore C or C ' is selected from the group consisting of an enzyme, an antibody or a fragment or a derivative thereof, an aptamer, a peptide, a fusion protein, a small molecule inhibitor, a carbocyclic compound, a heterocyclic compound, a nucleoside derivative and an anilino-naphthalene compound.

**8.** The compound of any one of claims 1 to 6, wherein the pharmacophore C or C' is an enzyme inhibitor.

**9.** The compound of any one of claims 1 to 6, wherein the pharmacophore C or C' is a receptor inhibitor.

**10.** The compound of claim 9, wherein the receptor inhibitor is EGF receptor inhibitor.

**11.** The compound of any one of claims 1 to 6, wherein the pharmacophore C or C' is an ETBR-antagonist.

**12.** The compound of any of claims 1 to 6, wherein the pharmacophore C or C ' is an antiviral agent.

**13.** The compound of claim 12, wherein the antiviral agent is selected from the group consisting of Zanamivir (2,4-dideoxy-2,3-didehydro-4-guanidinosialic acid), Oseltamivir (ethyl(3R,4R,5S)-4-acetoamido-5-amino-3-(l-ethylpropoxy)-1-cyclohexene-1-carboxylate), RWJ-270201 (Peramivir), BCX-1812, BCX-1827, BCX-1898, BCX-1923, Norakin (1-tricyclo-(2,2,1,0)-heptyl-(2)-1-phenyl-3-piperidine-propanol; triperiden), Akineton (alpha-5-norbornen-2-yl-alpha-phenyl-1-piperidine propanol; biperiden), Antiparkin (ethylbenzhydramin) and Parkopan (trihexyphenidyl).

**14.** The compound of claim 12, wherein the antiviral agent is selected from the group consisting of Fuzeon, T20, T1249, coselane, AMD3100, AMD070, SCH351125 and AD101.

**15.** A compound comprising the raftophile A' as described in claim 5 and a linker represented by the following formula **28:**

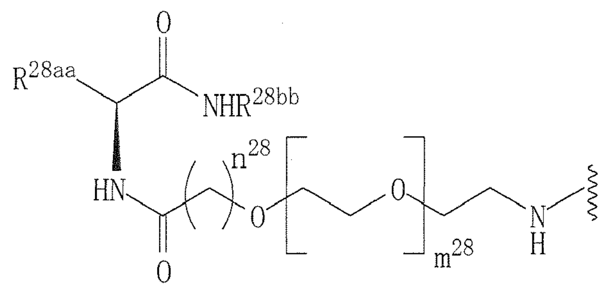

28

wherein

$R^{28aa}$ is the side chain of a naturally occurring amino acid substituted with a dye label;
$R^{28bb}$ is H or $CH_2CONH_2$; and
$m^{28}$ and $n^{28}$ are independently an integer from 1 to 3;
wherein the N-terminus of the linker is bonded to the raftophile A'.

**16.** The compound of claim 15 wherein the linker is represented by the following formula **28a:**

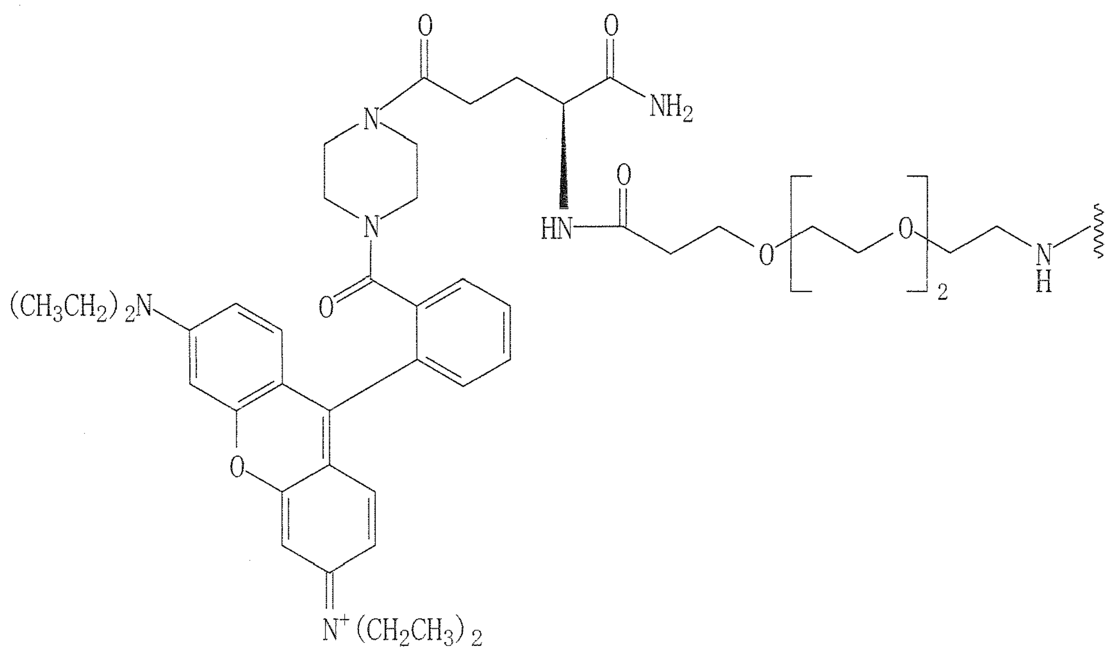

28a

**17.** A pharmaceutical composition comprising a compound of any one of claims 1 to 16.

**18.** A compound as defined in any of claims 7 and 10 for use in the treatment, prevention and/or amelioration of a proliferative disorder or cancer.

**19.** A compound as defined in claim 11 for use in the treatment, prevention and/or amelioration of hypertension and/or congestive heart failure.

**20.** A compound as defined in claim 18 for us in the treatment, prevention and/or amelioration of a prion (PrP)-related disease.

**21.** A compound as defined in claims 1 to 7 and 12 to 14 for use in the treatment, prevention and/or amelioration of an infectious disease.

**22.** The compound of claim 21, wherein said infectious disease is a viral infection.

**23.** The compound of claim 22, wherein said viral infection is an HIV-infection.

**24.** The compound of claim 22, wherein said viral infection is an influenza infection.

**25.** The compound of claim 21, wherein said infectious disease is a bacterial infection.

**26.** A compound as defined in any one of claims 1 to 7 for use in the treatment of a parasite infection.

**27.** A method for the preparation of a compound as described in anyone of claims 1 to 14 comprising the steps of

(a) determining the distance between a phosphoryl head group or an equivalent head group of a raft lipid and a binding and/or interaction site of a pharmacophore C/C' on a raft-associated target molecule;
(b) selecting a linker B/B' which is capable of spanning the distance as determined in a); and
(c) bonding a raftophile A/A' and the pharmacophore C/C' by the linker as selected in b).

**28.** A method for the preparation of a pharmaceutical composition comprising admixing the compound of any one of claims 1 to 14 with one or more pharmaceutically acceptable excipients.

# Figure 1

## Proposed Mechanism of Action of compound 25b
## BACE inhibitor III

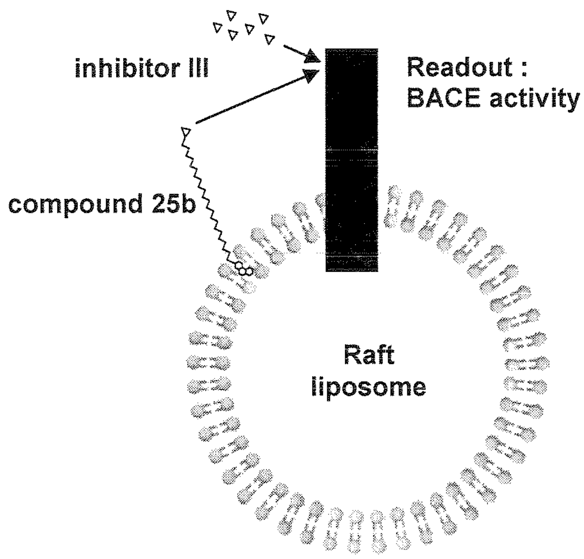


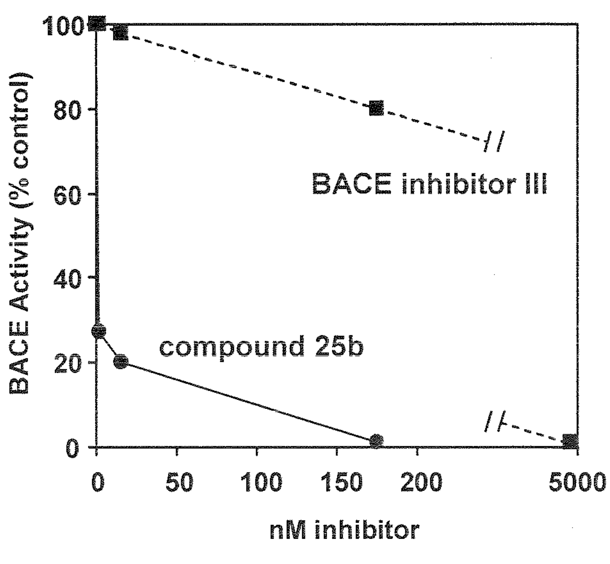

## Figure 2

## Proposed Mechanism of Action of Inhibition of ß-Secretase by compound 25b

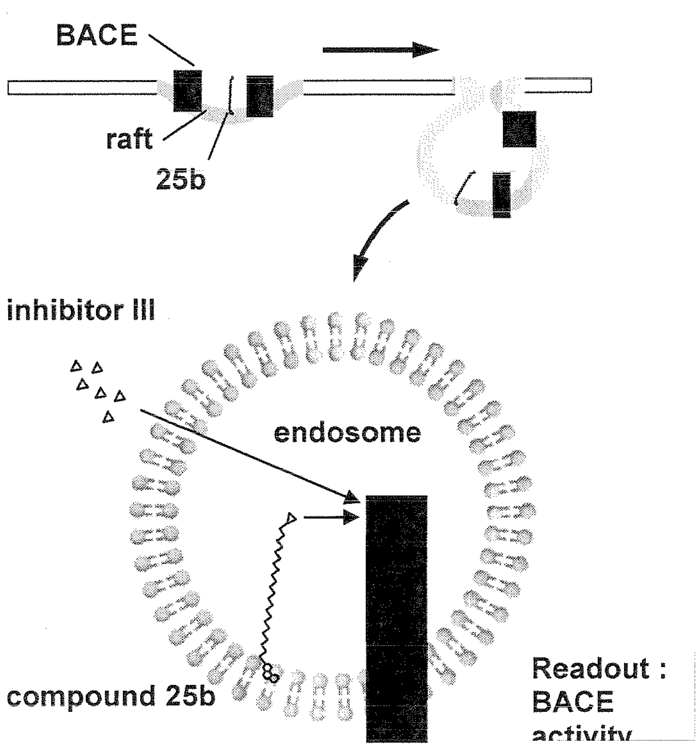


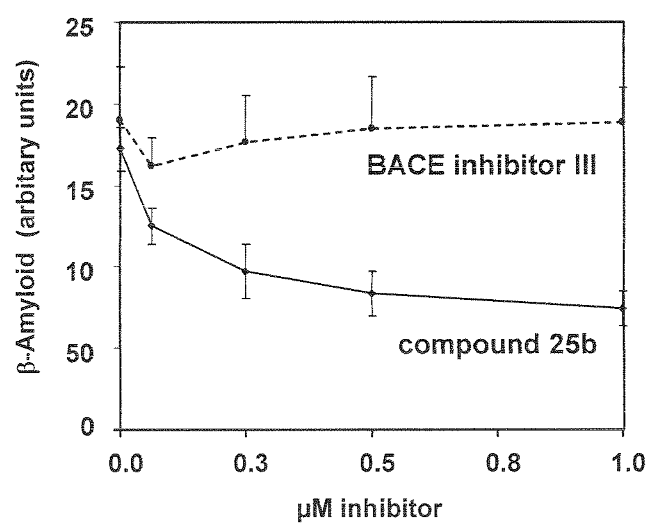

Figure 3:

Mechanism of Action of a Tripartite Raftophile comprising an HIV Fusion Inhibitor (as pharmacophore C/C')

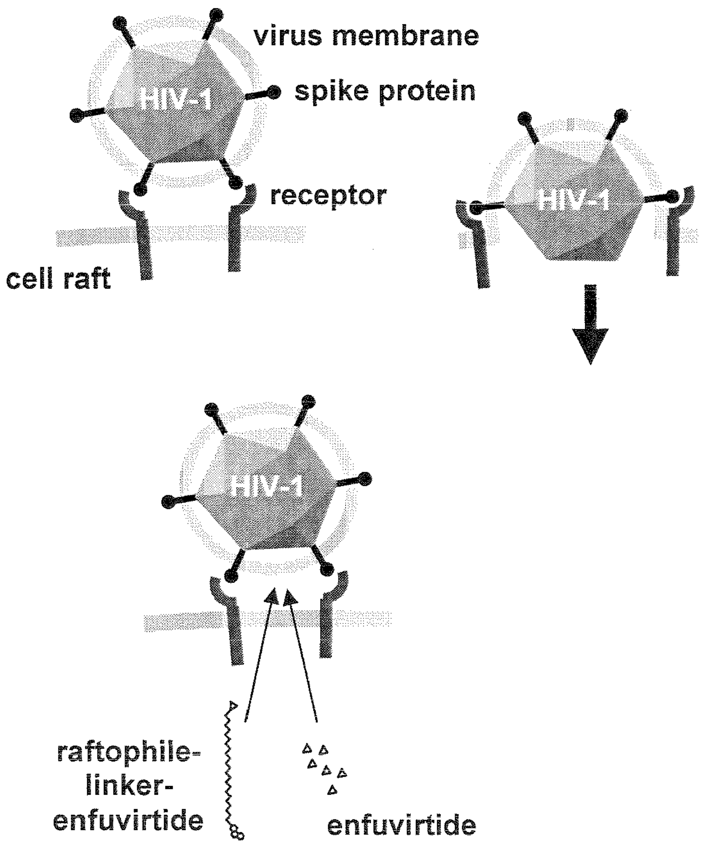

Europäisches Patentamt
European Patent Office
Office européen des brevets

## PARTIAL EUROPEAN SEARCH REPORT

which under Rule 63 of the European Patent Convention shall be considered, for the purposes of subsequent proceedings, as the European search report

Application Number
EP 09 16 9971

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/09163 A (UNIVERSITY OF GLASGOW) 8 February 2001 (2001-02-08) * the whole document * ----- | 1-28 | INV. A61K47/48 |
| A | US 5 446 026 A (M RUFF ET AL.) 29 August 1995 (1995-08-29) * the whole document * ----- | 1-28 | |
| A | EP 0 417 735 A (HOECHST AG) 20 March 1991 (1991-03-20) * the whole document * ----- | 1-28 | |
| A | BIOCONJUGATE CHEMISTRY., vol. 8, 1997, pages 520-525, XP002158726 ACS, WASHINGTON, DC. * the whole document * ----- | 1-28 | |
| A | BIOCHEMICAL PHARMACOLOGY, vol. 43, no. 9, 1992, pages 1969-1974, XP000982833 PERGAMON, OXFORD * the whole document * ----- | 1-28 | TECHNICAL FIELDS SEARCHED (IPC) A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 9 October 2009 | Masturzo, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04E07)

Europäisches Patentamt
European Patent Office
Office européen des brevets

**INCOMPLETE SEARCH SHEET C**

Application Number
EP 09 16 9971

Claim(s) searched incompletely:
1-28

Reason for the limitation of the search:

The starting products of the method claimed in claims 1-28 are tripartite compounds or conjugates comprising a raftophile and a pharmacophore joined by a linker. The starting compounds are not clearly defined because , even though it is clear what a raftophile and what a pharmacophore is, their nature is not defined except (for the raftophile) through a series of cumbersome, cascading definitons, where no fixed element is provided. This would require an equally unquantifiable and thus unreasonable amount of experimentation, imposing a severe and undue burden on all those wishing to ascertain the scope of the claim, which is not in compliance with the clarity requirement of Article 84 EPC. The non-compliance with the substantive provisions is to such an extent, that a meaningful search of the whole claimed subject-matter of the claim could not be carried out (Rule 63 EPC and Guidelines B-VIII, 3).

The extent of the search was consequently limited to the clearly defined examples in the description.

## ANNEX TO THE EUROPEAN SEARCH REPORT ON EUROPEAN PATENT APPLICATION NO. EP 09 16 9971

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2009

| Patent document cited in search report | | Publication date | Patent family member(s) | | | Publication date |
|---|---|---|---|---|---|---|
| WO 0109163 | A | 08-02-2001 | AU | 6173900 | A | 19-02-2001 |
| | | | DE | 60032926 | T2 | 25-10-2007 |
| | | | DK | 1228093 | T3 | 14-05-2007 |
| | | | EP | 1228093 | A2 | 07-08-2002 |
| | | | ES | 2280232 | T3 | 16-09-2007 |
| | | | GB | 2355009 | A | 11-04-2001 |
| | | | US | 7153930 | B1 | 26-12-2006 |
| US 5446026 | A | 29-08-1995 | NONE | | | |
| EP 0417735 | A | 20-03-1991 | DE | 69016897 | D1 | 23-03-1995 |
| | | | DE | 69016897 | T2 | 14-09-1995 |
| | | | ES | 2070964 | T3 | 16-06-1995 |
| | | | HK | 1007646 | A1 | 16-04-1999 |
| | | | IT | 1234909 | B | 02-06-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5439899 A
- US 6562805 B
- US 20030212045 A
- US 6143740 A
- US 4946778 A
- WO 94282920 A
- US 5464933 A
- WO 2004013165 A
- US 5955422 A
- US 6348568 B


### Non-patent literature cited in the description


- **Glaser.** *Curr. Opin. Struct. Biol.,* 1993, vol. 3, 475-481
- **Jacobson.** *Comments Mol. Cell Biophys.,* 1992, vol. 8, 1-144
- **Jain.** *Adv. Lipid Res.,* 1977, vol. 15, 1-60
- **Vaz.** *Curr. Opin. Struct. Biol.,* 1993, 3
- **Simons.** *Biochemistry,* 1988, vol. 27, 6197-6202
- **Simons.** *Nature,* 1997, vol. 387, 569-572
- **Brown.** *Cell,* 1992, vol. 68, 533-544
- **Ilangumaran.** *Biochem. J.,* 1998, vol. 335, 433-440
- **Scheiffele.** *Embo J.,* 1997, vol. 16, 5501-5508
- **Ipsen.** *Biochem. Biophys. Acta,* 1987, vol. 905, 162-172
- **Ipsen.** *Biophys. J.,* 1989, vol. 56, 661-667
- **Sankaram.** *Biochemistry,* 1990, vol. 29, 10676-10684
- **Nielsen.** *Phys. Rev. E. Stat. Phys. Plasmas Fluids Relat. Interdiscip. Topics,* 1999, vol. 59, 5790-5803
- **de Almeida.** *Biophys. J.,* 2003, vol. 85, 2406-2416
- **Cushman.** *J. Chem.,* 1994, vol. 37, 3040
- **Golebiewski.** *Bioorg. & Med. Chemistry,* 1996, vol. 4, 1637
- **Ruell.** *J. Org. Chem.,* 1999, vol. 64, 5858
- **Garcia.** *J. Bioorg. Med. Biochem.,* 2000, vol. 8, 191
- *Hussey Organic Letters,* 2001, vol. 4, 415-418
- **Hussey.** *J. Am. Chem. Soc.,* 2001, vol. 123, 1271-1273
- **Hussey.** *J. Am. Chem. Soc.,* 2002, vol. 124, 6265-6273
- **Martin.** *Bioconjugate Chem.,* 2003, vol. 14, 67-74
- **Danielson.** *Biochem. Biophys.,* 2003, vol. 1617, 1-9
- X-ray chrystallography methods and interpretation. **McRee.** Practical Protein Crystallography. Academic Press, 1999
- **De Strooper, B. ; Annaert, W.** Proteolytic processing and cell biological functions of the amyloid precursor protein. *J. Cell Sci.,* 2000, vol. 113, 1857-70
- **Capell.** *J. Biol. Chem.,* 2002, vol. 277, 5637
- **Tung.** *J. Med. Chem.,* 2002, vol. 45, 259
- **Chen.** *Proc. Natl. Acad. Sci. (USA),* 2003, vol. 100, 9226-31
- **Park.** *Nat. Biotech.,* 2000, vol. 18, 1948
- **Xu.** *J. Biol. Chem.,* 2001, vol. 276, 33540-33546
- **Wang.** *Biochemistry,* 2004, vol. 43, 1010-8
- **Svennerholm ; Asbury ; Reisfeld.** Biological Function of Gangliosides. Elsevier Science Ltd, 1994
- **S. L. Hussey ; E. He ; B. R. Peterson.** *J. Am. Chem. Soc,* 2001, vol. 123, 12712-12713
- **S. L. Hussey ; E. He ; B. R. Peterson.** *Org. Lett.,* 2002, vol. 4, 415-418
- **S. E. Martin ; B. R. Peterson.** *Bioconjugate Chem.,* 2003, vol. 14, 67-74
- **R. L. Hinman ; L. Locatell.** *J. Am. Chem. Soc,* 1959, vol. 81, 5655-5658
- **H.-J. Knölker ; T. Braxmeier ; G. Schlechtingen.** *Angew. Chem. Int. Ed.,* 1995, vol. 34, 2497
- **H.-J. Knölker ; T. Braxmeier ; G. Schlechtingen.** *Synlett,* 1996, 502
- **H.-J. Knölker ; T. Braxmeier.** *Tetrahedron Lett,* 1996, vol. 37, 5861
- **Golebriewski ; Keyes ; Cushman.** *Bioorg. Med. Chem.,* 1996, vol. 4, 1637-1648
- **Cusinato ; Habeler et al.** *J. Lipid Res.,* 1998, vol. 39, 1844-1851
- **Himber ; Missano et al.** *J. Lipid Res.,* 1995, vol. 36, 1567-1585

- **J. G. Parkes ; H. R. Watson ; A. Joyce ; R. Phadke ; I.C. P. Smith.** *Biochim. Biophys. Acta,* 1982, vol. 691, 24-29
- **J. Lapierre ; V. Ahmed ; M.-J. Chen ; M. Ispahany ; J. G. Guillemette ; S. D. Taylor.** *Bioorg. Med. Chem. Lett.,* 2004, vol. 14, 151-155
- **E. D. Bergmann ; M. Rabinovitz ; Z. H. Levinson.** *J. Am. Chem. Soc.,* 1959, vol. 81, 1239-1243
- **X. Zhang ; Z. Sui.** *Tetrahedron Lett,* 2003, vol. 44, 3071-3073
- **L. W. L. Woo ; M. Lightowler ; A. Purohit ; M. J. Reed ; B. V. L. Potter.** *J. Steroid Biochem. Molec. Biol.,* 1996, vol. 57, 79-88
- **R. H. Peters ; D. F. Crowe ; M. A. Avery ; W. K. M. Chong ; M. Tanabe.** *J. Med. Chem.,* 1989, vol. 32, 1642-1652
- **A. M. Krubiner ; E. P. Oliveto.** *J. Org. Chem.,* 1966, vol. 31, 24-26
- Methods in Enzymology. Academic Press, 1999, vol. 311
- **P. M. Koskinen ; A. M. P. Koskinen.** *Synthesis,* 1998, 1075
- **T. Yamanoi et al.** *Chem. Lett.,* 1989, 335
- **A. R. Howell ; A. J. Ndakala.** *Curr. Org. Chem.,* 2002, vol. 6, 365-391
- **P. Garner et al.** *J. Org. Chem,* 1988, vol. 53, 4395
- **P. Herold.** *Helv. Chim. Acta,* 1988, vol. 74, 354
- **H.-E. Radunz et al.** *Liebigs Ann. Chem.,* 1988, 1103
- **H. J. Harwood.** *Chem. Rev.,* 1962, vol. 62, 99-154
- **W. J. Gensler.** *Chem. Rev.,* 1957, vol. 57, 191-280
- **S. Müller et al.** *J. Prakt. Chem.,* 2000, vol. 342, 779
- **U. Schlueter ; J. Lu ; B. Fraser-Reid.** *Org. Lett.,* 2003, vol. 5, 255-257
- **K. Henrick ; M. McPartlin ; S. Munjoma ; P. G. Owston ; R. Peters ; S. A. Sangokoya ; P. A. Tasker.** *J. Chem. Soc. Dalton Trans.,* 1982, 225-227
- **J. Xue ; Z. Guo.** *Bioorg. Med. Chem. Lett.,* 2002, vol. 12, 2015-2018
- **J. Xue ; Z. Guo.** *J. Am. Chem. Soc,* 2003, 16334-16339
- **J. Xue ; N. Shao ; Z. Guo.** *J. Org. Chem.,* 2003, vol. 68, 4020-4029
- **N. Shao ; J. Xue ; Z. Guo.** *Angew. Chem. Int. Ed.,* 2004, vol. 43, 1569-1573
- **H.-J. Knölker.** *Chem. Soc. Rev.,* 1999, vol. 28, 151-157
- **H.-J. Knölker ; K. R. Reddy.** *Chem. Rev.,* 2002, vol. 102, 4303-4427
- **H.-J. Knölker ; J. Knöll.** *Chem. Commun.,* 2003, 1170-1171
- **H.-J. Knölker.** *Curr. Org. Synthesis,* 2004, 1
- **N. K. Djedovic ; R. Ferdani ; P. H. Schlesinger ; G. W. Gokel.** *Tetrahedron,* 2002, vol. 58, 10263-10268
- **G. S. Hird ; T. J. McIntosh ; M. W. Grinstaff.** *J. Am. Chem. Soc,* 2000, vol. 122, 8097-8098
- **J. G. Witteveen ; A. J. A. Van der Weerdt.** *Rec. Trav. Chim. Pays-Bas,* 1987, vol. 106, 29-34
- **M. S. Newman ; R. L. Childers.** *J. Org. Chem,* 1967, vol. 32, 62-66
- **W. Sucrow ; H. Minas ; H. Stegemeyer ; P. Geschwinder ; H. R. Murawski ; C. Krueger.** *Chem. Ber.,* 1985, vol. 118, 3332-3349
- **H. Minas ; H. R. Murawski ; H. Stegemeyer ; W. Sucrow.** *J. Chem. Soc. Chem. Commun,* 1982, 308-309
- **D.-S. Wang ; C.-S. Chen.** *J. Org. Chem.,* 1996, vol. 61, 5905-5910
- **Harlow ; Lane.** Antibodies, A Laboratory Manual. CSH Press, 1988
- **Gold.** *Ann. Rev. Biochem.,* 1995, vol. 64, 763-797
- **Azios.** *Oncogene,* 2001, vol. 20, 5199-5209
- **Chen.** *Proc. Natl. Acad. Sci. USA,* 2003, vol. 100, 9226-9231
- **von Itzstein M.** *Nature,* 1993, vol. 363, 418-23
- **Woods JM.** *Antimicrob Agents Chemother.,* 1993, vol. 37, 1473-9
- **Eisenberg EJ.** *Antimicrob Agents Chemother.,* 1997, vol. 41, 1949-52
- **Kati WM.** *Biochem Biophys Res Commun.,* 1998, vol. 244, 408-13
- **Babu YS.** *J Med Chem,* 2000, vol. 43, 3482-6
- **Smee DF.** *Antimicrob Agents Chemother.,* 2001, vol. 45, 743-8
- **Hartt JK.** *Biochem Biophys Res Commun.,* 2000, vol. 272, 699-704
- **Tremblay CL.** *J Acquir Immune Defic Syndr.,* 2000, vol. 25, 99-102
- **De Clercq. E.** *Antimicrob Agents Chemother.,* 1994, vol. 38, 668-74
- **Palani A.** *J Med Chem,* 2001, vol. 44, 3339-42
- **Smee.** *Antimicrob. Agents Chemother.,* 2001, vol. 45, 743-748
- Remington's Pharmaceutical Sciences
- **Simons.** *Annu. Rev. Biophys. Biomol. Struct.,* 2004, vol. 33, 269-295
- **Harder.** *J. Cell Biol.,* 1998, vol. 141, 929-942
- **Harder.** *Curr. Opin. Cell Biol.,* 1997, vol. 9, 534-542
- **London.** *Curr. Opin. Struct. Biol.,* 2002, vol. 12, 480-486
- **London.** *Biochim. Biophys. Acta,* 2000, vol. 1508, 182-195
- **Ehehalt.** *J. Cell Biol.,* 2003, vol. 160, 113-123
- **Simons.** *Proc. Natl. Acad. Sci. USA,* 1998, vol. 95, 6460-6464
- **Riddell.** *Curr. Biol.,* 2001, vol. 11, 1288-1293

- **Israelachvili.** *Biochim. Biophys. Acta,* 1977, vol. 469, 221-225
- **Jorgensen.** *J. Phys. Chem.,* 2000, vol. 104, 11763-11773
- **Keller.** *Phys. Rev. Lett.,* 1998, vol. 81, 5019-5022
- **Kenworthy.** *Mol. Biol. Cell,* vol. 11, 1645-1644
- **Kholodenko.** *Trends Cell Biol.,* 2000, vol. 10, 173-178
- **Kurzchalia.** *Curr. Opin. Cell Biol.,* 1999, vol. 11, 424-431
- **Lipowsky.** *J. Biol. Phys.,* 2002, vol. 28, 195-210
- **Nguyen.** *J. Virol.,* 2000, vol. 74, 3264-72
- **Jiang.** *Curr. Pharm. Des.,* 2002, vol. 8, 563-80
- **Wadia.** *Nat, Med.,* 2004, vol. 10, 310-315
- **Wild.** *PNAS,* 1992, vol. 91, 9770
- **Peyron.** *J. Immunol.,* 2000, vol. 165, 5186-5191
- **Samuel.** *J. Biol. Chem.,* 2001, vol. 276, 29319-29329
- **Alessio.** *Blood,* 1993, vol. 82, 3637-3647
- **McConnell.** *Annu, Rev. Biophys. Biomol. Struct.,* 2003, vol. 32, 469-492
- **McLaughlin.** *Annu. Rev. Biophys. Biomol. Struct.,* 2002, vol. 31, 151-175
- **Milhiet.** *Single Mol.,* 2001, vol. 2, 119-121
- **Murata.** *Proc. Nat. Acad. Sci. USA,* 1995, vol. 92, 10339-10343
- **Cordeiro.** *J. Biol. Chem.,* 2004, vol. 279 (7), 5346-5352
- **Ringerike, J.** *Cell Sci.,* 2002, vol. 115, 1331-1340
- **Parton.** *Trends Cell Biol.,* 2004, vol. 14, 141-147
- **Miaczynska.** *Curr. Opin. Cell Biol.,* 2004, vol. 16
- **Yamaguchi.** *Eur. J. Biochem.,* 2003, vol. 270, 1816-1827
- **Bagnato.** *Cancer Res.,* 2004, vol. 64, 1436-1443
- **Khan.** *Diabetologia,* 2002, vol. 45, 1475-1483
- *J. Org. Chem.,* 2003, vol. 68, 4020
- *Biochemistry,* 1994, vol. 33, 11586
- Synthetic Peptides: A User's Cuide. W. H. Freeman & Co, 1992
- **D. Boumrah ; M. M. Campbell ; S. Fenner ; R. G. Kinsman.** *Tetrahedron,* 1997, vol. 53, 6977-6992
- **Weissenhorn.** *Nature,* 1997, vol. 387, 426-430
- **Wild.** *Proc Natl Acad Sci USA,* 1992, vol. 91, 9770-9774
- **Hildinger.** *J. Virol.,* 2001, vol. 75, 3038-3042
- **Salzwedel.** *J. Virol.,* 1999, vol. 73, 2469-2480